(19) Europäisches Patentamt
European Patent Office
Office européen des brevets

(11) EP 3 842 575 A1

(12) EUROPEAN PATENT APPLICATION
published in accordance with Art. 153(4) EPC

(43) Date of publication:
**30.06.2021 Bulletin 2021/26**

(21) Application number: **19853195.6**

(22) Date of filing: **21.08.2019**

(51) Int Cl.:
*C30B 29/54* (2006.01) *C07D 333/20* (2006.01)
*C07D 401/12* (2006.01) *C07D 409/04* (2006.01)
*C07D 495/04* (2006.01) *C07H 1/06* (2006.01)
*C07H 17/00* (2006.01) *C07H 17/08* (2006.01)
*C30B 7/08* (2006.01) *C30B 29/60* (2006.01)
*A61K 31/198* (2006.01) *A61K 31/381* (2006.01)
*A61K 31/4365* (2006.01) *A61K 31/4439* (2006.01)
*A61K 31/4535* (2006.01) *A61K 31/7048* (2006.01)
*A61K 31/7052* (2006.01) *A61K 33/24* (2019.01)

(86) International application number:
**PCT/JP2019/032722**

(87) International publication number:
**WO 2020/040233 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **21.08.2018 JP 2018154760**

(71) Applicants:
- **TOWA PHARMACEUTICAL CO., LTD.
Kadoma-shi,
Osaka 571-8580 (JP)**
- **The University of Tokyo
Bunkyo-ku,
Tokyo 113-8654 (JP)**

(72) Inventors:
- **SUKEGAWA, Junpei
Kadoma-shi,
Osaka 571-8580 (JP)**
- **ARAKI, Daigo
Kadoma-shi,
Osaka 571-8580 (JP)**
- **SUZUKI, Shunpei
Kadoma-shi,
Osaka 571-8580 (JP)**
- **MINAMI, Masaki
Kadoma-shi,
Osaka 571-8580 (JP)**
- **ONAI, Takuma
Kadoma-shi,
Osaka 571-8580 (JP)**
- **WAKITA, Fumihiro
Kadoma-shi,
Osaka 571-8580 (JP)**
- **LIU, Chao
Kadoma-shi,
Osaka 571-8580 (JP)**
- **NAKAMURA, Eiichi
Tokyo 113-8654 (JP)**
- **HARANO, Koji
Tokyo 113-8654 (JP)**

(74) Representative: **advotec.
Patent- und Rechtsanwälte
Widenmayerstrasse 4
80538 München (DE)**

# (54) SPECIFICALLY-SHAPED CRYSTAL OF COMPOUND AND METHOD FOR PRODUCING SAME

(57) The present invention provides a method for obtaining a specifically-shaped crystal (specifically, spherocrystal) of a compound with good reproducibility. This method for producing a specifically-shaped crystal (specifically spherocrystal) of a compound comprises: (1) a step for preparing a supersaturated solution of a compound having a degree of supersaturation equal to or higher than a critical degree of supersaturation; and (2) a step for precipitating a specifically-shaped crystal (specifically spherocrystal) of a compound from the supersaturated solution.

EP 3 842 575 A1

FIG. 90

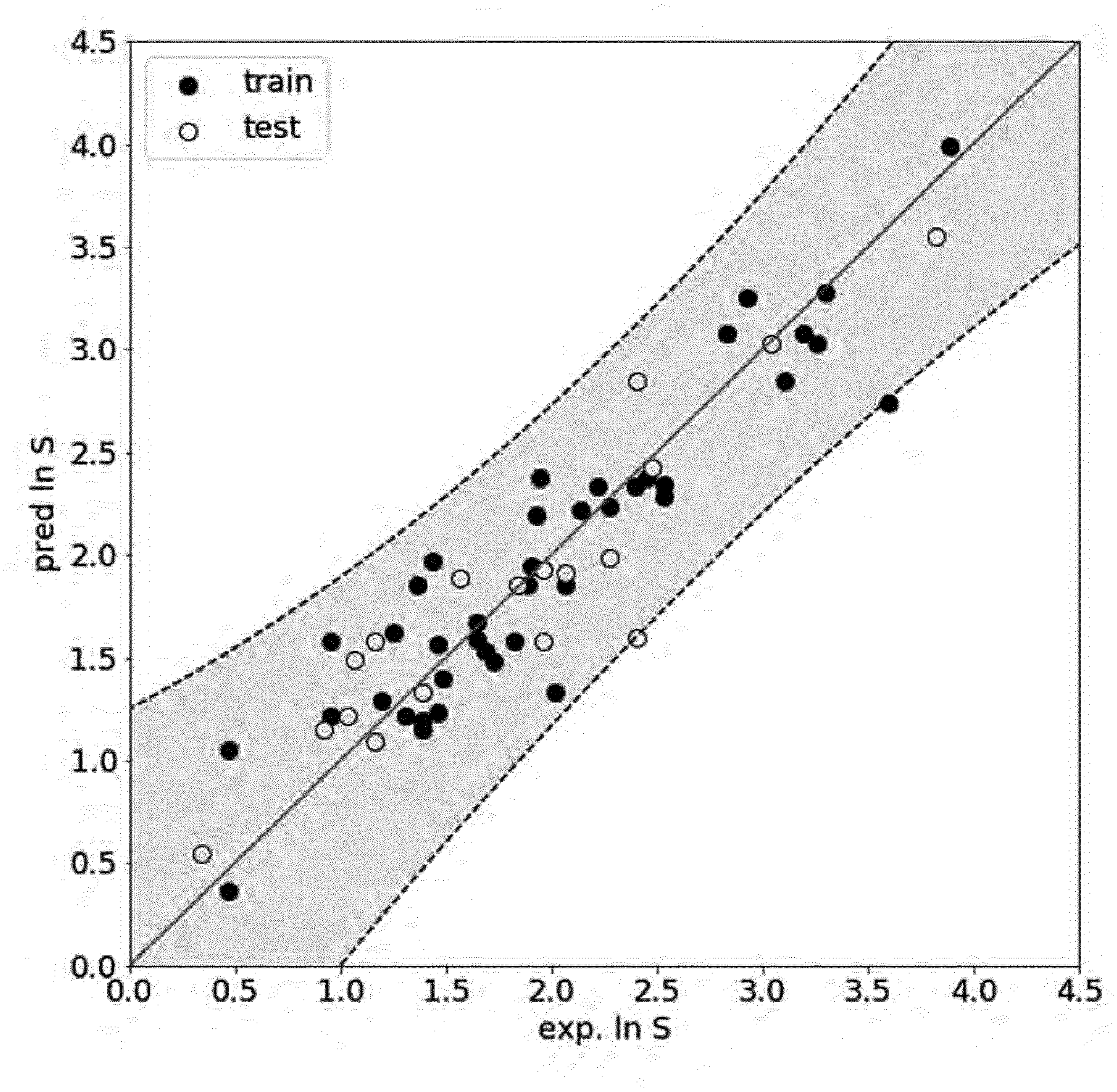

## Description

TECHNICAL FIELD

The present invention relates to a specifically-shaped crystal of a compound and a method for producing the same. Particularly, the present invention relates to a spherulite of a compound and a method for producing the same.

BACKGROUND ART

Powder properties of compounds are greatly influenced by a crystal shape thereof. Therefore, the method for controlling the crystal shape has wide applicability in the fields of pharmaceutical manufacturing, pesticide manufacturing, food manufacturing, printing technology, and organic electronic devices.

The crystal shape of compounds is closely involved in crystal growth mechanism. With an increase in driving force or impurities in crystal growth, compounds as single crystals change from polyhedron crystals to hopper crystals (crystals with recesses on crystal faces) and symmetric dendritic crystals due to spiral growth and two-dimensional nucleation and growth. When the driving force or impurities further increase(s), adhesive growth leads to formation of asymmetric dendritic crystals as polycrystals, which finally become spherulites (NPL 1).

Because of having the smallest specific surface area, spherulites have various advantages in the manufacturing process of many chemical products such as pharmaceuticals, pesticides, foods, and electronic materials. When solid-liquid separation is performed in the crystallization step, short operation time and high washing operation are achieved because of high filterability, thus making it possible to obtain high purity crystals. Further, due to high fillability to manufacturing equipment, the amount of processing at one time can be increased in these operations. Further, in the step of processing spherulites, productivity is expected to be improved by an improvement in fluidity and fillability. For example, in the step of manufacturing pharmaceutical preparations, fine drug substance has poor adhesion/cohesiveness, fluidity, fillability, and wettability, and it may be difficult to formulate the drug substance as it is. In this case, conventionally, excipients are added to granulate the drug substance into granules, followed by formulation. However, if the drug substance is a spherulite, the spherulite has high fluidity and fillability, so that such a granulation operation is unnecessary, and there is an advantage that direct tableting and coating can be performed (PTL 1).

In addition to the spherical crystallization method in which spherical crystals are obtained by radially crystallizing from a homogeneous solution of a single compound, there is also known the spherical granulation method in which a quasi-emulsion is formed by adding an organic solvent (dichloromethane, etc.) containing the compound dissolved therein to water and a water-miscible organic solvent (ethanol, etc.), the organic solvent being immiscible in both, thus obtaining spherical agglomerate in which microcrystals obtained by solvent diffusion are directly accumulated in a spherical shape in the system (PTL 1 and PTL 2). In this manufacturing method, not only applicable compound is limited to those which are dissolved in halogen-based solvents such as dichloromethane, but also strict control of residual solvent is required due to high toxicity of halogen-based solvents in the field of pharmaceutical manufacturing. Meanwhile, there is also known the method in which an emulsion is formed using a surfactant instead of using a halogen-based solvent (NPL 2). In this method, since all the compounds dissolved in a good solvent are crystallized, it is impossible to essentially expect the purification effect by crystallization. Therefore, it is necessary to use a compound highly purified in advance as a raw material, thus failing to be used in applications where the productivity of the manufacturing process of chemical products is improved. From the above, the spherical crystallization method for obtaining spherulites from a homogeneous solution of a single compound has a wider range of coverage and range of application and is therefore highly industrially useful.

[CITATION LIST]

[PATENT LITERATURE]

[PTL 1] JP S58-143832 A
[PTL 2] JP H1-279869 A

[NON-PATENT LITERATURE]

[NPL 1] Ichiro Sunagawa "Crystals: Growth, Morphology and Perfection", KYORITSU SHUPPAN CO., LTD. (Tokyo,

2003), Chapter 3, pp. 47 - 48
[NPL 2] F. Espitalier, B. Biscans, J.-R. Authelin, C. Laguerie, Institution of Chemical Engineers, 1997, 75(2), pp. 257-267
[NPL 3] L. Granasy, et al., Nature Materials 2004, vol. 3, pp. 645-650

SUMMARY

[TECHNICAL PROBLEM]

There is currently no universal method capable of preparing a crystal having a desired shape as necessary. Particularly, as mentioned above, since spherulites of compounds are highly useful in industry, a method capable of producing spherulites of all compounds with satisfactory reproducibility is desired.

Spherulite is a crystal shape which is universally observed regardless of the type of compounds, however, in many cases, the spherulite is obtained from compounds such as polymers, minerals, and inorganic materials, and there are relatively few examples obtained from molecular compounds such as active pharmaceutical ingredients, pharmaceutical intermediates, and pesticides. In many cases, the spherulite is obtained from a supercooled state of the compound, and theoretical studies suggest that, as the degree of supercooling increases, rotational motion becomes relatively slower than translational motion, and nucleation in a non-crystallographic orientation occurs on crystal faces, whereby, branching of crystals leads to formation of polycrystals, which finally become spherulites (NPL 3). Meanwhile, crystallization of the compound from a supersaturated solution is a common crystallization process in the manufacture of fine chemicals and is of high industrial importance, but spherulites are rarely obtained and theoretical research is not sufficiently conducted. Therefore, it is difficult to solve the problem of obtaining spherulites from a supersaturated solution of a compound with good reproducibility in a unified manner, which is largely due to trial and error. This is a major obstacle to shortening the product development period. Therefore, it is industrially useful to systematically research and develop a method for obtaining spherulites from a supersaturated solution of the compound.

An object of the present invention is to provide a method for obtaining a crystal of a compound having a desired shape with satisfactory reproducibility. Particularly, an object of the present invention is to provide a method for obtaining a spherulite of a compound with satisfactory reproducibility.

[SOLUTION TO PROBLEM]

As a result of intensive study in light of the above problems, the present inventors have found that there exists a minimum degree of supersaturation (referred to as "critical degree of supersaturation" in the present description) required to obtain a specifically-shaped crystal of a compound. The present inventors have also found that a specifically-shaped crystal can be obtained with satisfactory reproducibility from a supersaturated solution of a compound having a degree of supersaturation equal to or higher than the critical degree of supersaturation. The present inventors have also developed a statistical model of the critical degree of supersaturation by machine-learning the data of the critical degree of supersaturation and crystallization conditions of a large number of newly acquired compounds, and clarified the physical quantity which determines the critical degree of supersaturation. Thus, it has been found that the critical degree of supersaturation of any compound can be predicted in crystallization from a solution without trial and error.

One or more embodiments of the present invention include the following.

<1> A method for producing a spherulite of a compound, which includes:

(1) a step of preparing a supersaturated solution of the compound having a degree of supersaturation equal to or higher than a critical degree of supersaturation required to obtain the spherulite of the compound; and
(2) a step of precipitating the spherulite of the compound from the supersaturated solution.

<2> The method according to <1>, wherein the step (1) is performed under a condition where nucleation does not occur until a degree of supersaturation equal to or higher than a critical degree of supersaturation is reached.
<3> The method according to <1>, which further includes a step of removing crystal nuclei produced by the time a degree of supersaturation equal to or higher than a critical degree of supersaturation is reached in the step (1).
<4> The method according to any one of <1> to <3>, which further includes a step of inputting data including:

information on a compound obtained as a spherulite, and
at least one of information on a solvent used for crystallization and a solution temperature during crystallization into a predictive model of a critical degree of supersaturation required to obtain the spherulite of the compound, and outputting a predictive value of the critical degree of supersaturation from the predictive model, wherein

the critical degree of supersaturation in the step (1) is the predictive value.

<5> The method according to <4>, wherein, when the predictive value is outputted as a numerical value range, the critical degree of supersaturation in the step (1) is a lower limit of the numerical value range.
<6> The method according to any one of <1> to <3>, wherein the critical degree of supersaturation is an actual measured value.
<7> The method according to any one of <1> to <6>, wherein a sphericity of the spherulite is 0.60 or more.
<8> The method according to any one of <1> to <7>, wherein the compound obtained as a spherulite is a compound selected from the following (1) and (2):

(1) a compound represented by the following formula I, or a tautomer thereof, or an optical isomer thereof, or a salt thereof, or a solvate thereof:

[Chemical Formula 1]

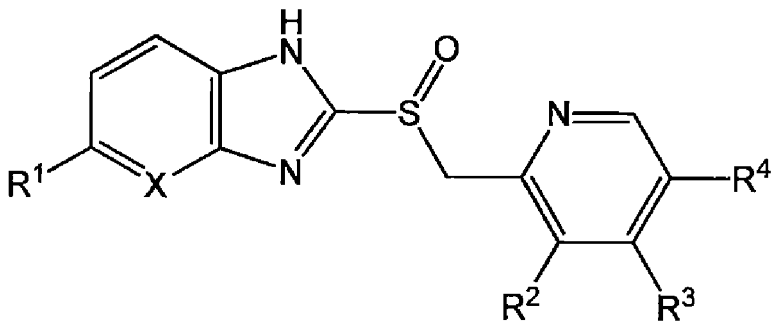

I

wherein

X is CH or N,
$R^1$ is a hydrogen atom or an optionally substituted $C_{1-6}$ alkoxy group, and
$R^2$, $R^3$, and $R^4$ are the same or different and each represent a hydrogen atom, an optionally substituted $C_{1-6}$ alkyl group, an optionally substituted $C_{1-6}$ alkoxy group, or an optionally substituted amino group;

(2) azithromycin, duloxetine, clarithromycin, lanthanum carbonate, glutamic acid, clopidogrel, ketotifen, escitalopram, dabigatran etexilate, theophylline, teneligliptin, pilsicainide, tramadol, vildagliptin, linagliptin, glutathione, mirabegron, tolvaptan, valacyclovir, bepotastine, olopatadine, or an optical isomer thereof, or a salt thereof, or a solvate thereof.

<9> The method according to any one of <1> to <8>, wherein the compound obtained as a spherulite is esomeprazole or lansoprazole, or a salt thereof, or a solvate thereof.
<10> The method according to any one of <1> to <9>, wherein the compound obtained as a spherulite is esomeprazole magnesium trihydrate.
<11> A spherulite of a compound which is produced by the method according to any one of <1> to <10>.
<12> A spherulite of a compound selected from the following (1) and (2):

(1) a compound represented by the following formula I, or a tautomer thereof, or an optical isomer thereof, or a salt thereof, or a solvate thereof:

[Chemical Formula 2]

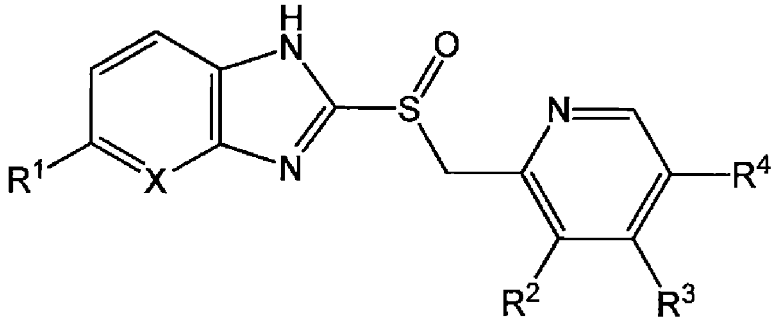

I

wherein

X is CH or N,
$R^1$ is a hydrogen atom or an optionally substituted $C_{1-6}$ alkoxy group, and
$R^2$, $R^3$, and $R^4$ are the same or different and each represent a hydrogen atom, an optionally substituted $C_{1-6}$ alkyl group, an optionally substituted $C_{1-6}$ alkoxy group, or an optionally substituted amino group;

(2) azithromycin, duloxetine, clarithromycin, lanthanum carbonate, glutamic acid, clopidogrel, ketotifen, escitalopram, dabigatran etexilate, theophylline, teneligliptin, pilsicainide, tramadol, vildagliptin, linagliptin, glutathione, mirabegron, tolvaptan, valacyclovir, bepotastine, olopatadine, or an optical isomer thereof, or a salt thereof, or a solvate thereof.

<13> The spherulite according to <12>, wherein the compound selected from (1) and (2) is esomeprazole or lansoprazole, or a salt thereof, or a solvate thereof.
<14> The spherulite according to <12> or <13>, wherein the compound selected from (1) and (2) is esomeprazole magnesium trihydrate.
<15> The spherulite according to any one of <12> to <14>, wherein the sphericity is 0.60 or more.
<16> A device for predicting a critical degree of supersaturation required to obtain a spherulite of a target compound, the device including:

a storage unit for recording a predictive model of a critical degree of supersaturation which is previously learned so as to input data including information on a compound obtained as a spherulite and at least one of information on a solvent used for crystallization and a solution temperature during crystallization, and to output a predictive value of a critical degree of supersaturation required to obtain the spherulite of the compound; and
a calculation unit for inputting data including information on a target compound obtained as a spherulite and at least one of information on a solvent used for crystallization of the target compound and a solution temperature during crystallization into the predictive model, and calculating a predictive value of a critical degree of supersaturation required to obtain the spherulite of the target compound.

<17> A method for predicting a critical degree of supersaturation required to obtain a spherulite of a compound, which includes a step of inputting data including information on a compound obtained as a spherulite and at least one of information on a solvent used for crystallization and a solution temperature during crystallization into a predictive model of a critical degree of supersaturation required to obtain the spherulite of the compound, and outputting a predictive value of a critical degree of supersaturation from the predictive model.
<18> A computer program for predicting a critical degree of supersaturation required to obtain a spherulite of a compound, which makes a computer run a process including a step of inputting data including information on a compound obtained as a spherulite and at least one of information on a solvent used for crystallization and a solution temperature during crystallization into a predictive model of a critical degree of supersaturation required to obtain the spherulite of the compound, and outputting a predictive value of a critical degree of supersaturation from the predictive model.
<19> A method for producing a specifically-shaped crystal of a compound, which includes:

(1) a step of preparing a supersaturated solution of the compound having a degree of supersaturation equal to or higher than a critical degree of supersaturation required to obtain the specifically-shaped crystal of the compound; and
(2) a step of precipitating the specifically-shaped crystal of the compound from the supersaturated solution.

<20> A device for predicting a critical degree of supersaturation required to obtain a specifically-shaped crystal of a target compound, the device including:

a storage unit for recording a predictive model of a critical degree of supersaturation which is previously learned so as to input data including information on a compound obtained as a specifically-shaped crystal and at least one of information on a solvent used for crystallization and a solution temperature during crystallization, and to output a predictive value of a critical degree of supersaturation required to obtain the specifically-shaped crystal of the compound; and
a calculation unit for inputting data including information on a target compound obtained as a specifically-shaped crystal and at least one of information on a solvent used for crystallization of the target compound and a solution temperature during crystallization into the predictive model, and calculating a predictive value of a critical degree of supersaturation required to obtain the specifically-shaped crystal of the target compound.

<21> A method for predicting a critical degree of supersaturation required to obtain a specifically-shaped crystal of a compound, which includes a step of inputting data including information on a compound obtained as a specifically-shaped crystal and at least one of information on a solvent used for crystallization and a solution temperature during crystallization into a predictive model of a critical degree of supersaturation required to obtain the specifically-shaped crystal of the compound, and outputting a predictive value of a critical degree of supersaturation from the predictive model.
<22> A computer program for predicting a critical degree of supersaturation required to obtain a specifically-shaped crystal of a compound, which makes a computer run a process including a step of inputting data including information on a compound obtained as a specifically-shaped crystal and at least one of information on a solvent used for crystallization and a solution temperature during crystallization into a predictive model of a critical degree of supersaturation required to obtain the specifically-shaped crystal of the compound, and outputting a predictive value of a critical degree of supersaturation from the predictive model.

[ADVANTAGEOUS EFFECTS OF INVENTION]

Use of the method of the present invention makes it possible to obtain a specifically-shaped crystal of a compound (particularly, spherulite) with satisfactory reproducibility. Since the spherulite has high fluidity and fillability, the time required for solid-liquid separation in the manufacturing step is short and high crystal cleaning effect is exerted. Furthermore, when pharmaceuticals are produced using this spherulite, the spherulite can be directly tableted without adding an additive to form granules. The spherulite can be uniformly coated with a small amount of base material. Since the spherulite has a small specific surface area, adhesion to the surface of the pestle during tableting can be suppressed. Therefore, tablet failure can be reduced. Accordingly, the spherulites produced by the method of the present invention enables an improvement in both the quality and the productivity of pharmaceuticals.

BRIEF DESCRIPTION OF DRAWINGS

FIG. 1 shows scanning electron microscope (SEM) images of crystals of ketotifen fumarate obtained in Example 1.
FIG. 2 shows the results of powder X-ray diffraction of ketotifen fumarate obtained in Example 1.
FIG. 3 shows a dissolution profile and a regression model of spherulites and non-spherulites of esomeprazole magnesium trihydrate of Example 8.
FIG. 4 shows a SEM image taken by cutting spherulites of azithromycin monohydrate obtained in Example 12.
FIG. 5 shows a SEM image of spherulites of esomeprazole magnesium trihydrate obtained in Example 2.
FIG. 6 shows the results of powder X-ray diffraction of spherulites of esomeprazole magnesium trihydrate obtained in Example 2.
FIG. 7 shows particle size distribution of spherulites of esomeprazole magnesium trihydrate obtained in Example 2.
FIG. 8 shows a SEM image of spherulites of esomeprazole magnesium trihydrate obtained in Example 3.
FIG. 9 shows a SEM image of spherulites of esomeprazole magnesium trihydrate obtained in Example 4.
FIG. 10 shows a SEM image of spherulites of esomeprazole magnesium trihydrate obtained in Example 5.

FIG. 11 shows the results of powder X-ray diffraction of spherulites of esomeprazole magnesium trihydrate obtained in Example 5.
FIG. 12 shows the results of particle size distribution of spherulites of esomeprazole magnesium trihydrate obtained in Example 5.
FIG. 13 shows a SEM image of spherulites of esomeprazole magnesium trihydrate obtained in Example 6.
FIG. 14 shows the results of powder X-ray diffraction of spherulites of esomeprazole magnesium trihydrate obtained in Example 6.
FIG. 15 shows the results of particle size distribution of spherulites of esomeprazole magnesium trihydrate obtained in Example 6.
FIG. 16 shows a SEM image of spherulites of esomeprazole magnesium trihydrate obtained in Example 7.
FIG. 17 shows the results of powder X-ray diffraction of spherulites of esomeprazole magnesium trihydrate obtained in Example 7.
FIG. 18 shows the results of particle size distribution of spherulites of esomeprazole magnesium trihydrate obtained in Example 7.
FIG. 19 shows a SEM image of spherulites of lansoprazole obtained in Example 11.
FIG. 20 shows the results of powder X-ray diffraction of spherulites of lansoprazole obtained in Example 11.
FIG. 21 shows a SEM image of spherulites of azithromycin monohydrate obtained in Example 12.
FIG. 22 shows the results of powder X-ray diffraction of spherulites of azithromycin monohydrate obtained in Example 12.
FIG. 23 shows the results of the particle size distribution of spherulites of azithromycin monohydrate obtained in Example 12.
FIG. 24 shows a SEM image of spherulites of clarithromycin obtained in Example 13.
FIG. 25 shows the results of powder X-ray diffraction of spherulites of clarithromycin obtained in Example 13.
FIG. 26 shows the results of particle size distribution of spherulites of clarithromycin obtained in Example 13.
FIG. 27 shows a SEM image of spherulites of DL-glutamic acid obtained in Example 14.
FIG. 28 shows the results of powder X-ray diffraction of spherulites of DL-glutamic acid obtained in Example 14.
FIG. 29 shows the results of particle size distribution of spherulites of DL-glutamic acid obtained in Example 14.
FIG. 30 shows a SEM image of spherulites of duloxetine hydrochloride obtained in Example 15.
FIG. 31 shows the results of powder X-ray diffraction of spherulites of duloxetine hydrochloride obtained in Example 15.
FIG. 32 shows the results of particle size distribution of spherulites of duloxetine hydrochloride obtained in Example 15.
FIG. 33 shows a SEM image of spherulites of clopidogrel sulfate obtained in Example 16.
FIG. 34 shows the results of powder X-ray diffraction of spherulites of clopidogrel sulfate obtained in Example 16.
FIG. 35 shows the results of particle size distribution of spherulites of clopidogrel sulfate obtained in Example 16.
FIG. 36 shows a SEM image of spherulites of lanthanum carbonate octahydrate obtained in Example 17.
FIG. 37 shows the results of powder X-ray diffraction of spherulites of lanthanum carbonate octahydrate obtained in Example 17.
FIG. 38 shows the results of particle size distribution of spherulites of lanthanum carbonate octahydrate obtained in Example 17.
FIG. 39 is a diagram showing an example of a schematic block diagram of an information processor 100 used in the step of predicting a critical degree of supersaturation.
FIG. 40 shows a flowchart showing an example of the operation of the entire processing.
FIG. 41 is a graph in which a predictive value with respect to an experimental value of a critical degree of supersaturation is plotted. The dashed lines show the 95% confidence interval.
FIG. 42 is a diagram showing a method for calculating a sphericity.
FIG. 43 is a graph showing a relationship between the RMSE value and the sphericity.
FIG. 44 shows a SEM image of spherulites of esomeprazole magnesium trihydrate obtained in Example 8(1).
FIG. 45 shows the results of powder X-ray diffraction of spherulites of esomeprazole magnesium trihydrate obtained in Example 8(1).
FIG. 46 shows the results of particle size distribution of spherulites of esomeprazole magnesium trihydrate obtained in Example 8 (1).
FIG. 47 shows a SEM image of non-spherulites of esomeprazole magnesium trihydrate used in Example 8(2).
FIG. 48 shows the results of powder X-ray diffraction of the non-spherulites of esomeprazole magnesium trihydrate used in Example 8(2).
FIG. 49 shows the results of particle size distribution of non-spherulites of esomeprazole magnesium trihydrate used in Example 8(2).
FIG. 50 shows a SEM image of spherulites of esomeprazole magnesium trihydrate obtained in Example 10(1).

FIG. 51 shows the results of particle size distribution of spherulites of esomeprazole magnesium trihydrate obtained in Example 10(1).

FIG. 52 shows a SEM image of crystals of esomeprazole magnesium trihydrate obtained in Example 10(2).

FIG. 53 shows the results of particle size distribution of crystals of esomeprazole magnesium trihydrate obtained in Example 10(2).

FIG. 54 shows a SEM image of crystals of escitalopram oxalate obtained in Example 19.

FIG. 55 shows the results of powder X-ray diffraction of crystals of escitalopram oxalate obtained in Example 19.

FIG. 56 shows the results of particle size distribution of crystals of escitalopram oxalate obtained in Example 19.

FIG. 57 shows a SEM image of crystals of vildagliptin obtained in Example 20.

FIG. 58 shows the results of powder X-ray diffraction of crystals of vildagliptin obtained in Example 20.

FIG. 59 shows the results of particle size distribution of crystals of vildagliptin obtained in Example 20.

FIG. 60 shows a SEM image of crystals of linagliptin obtained in Example 21.

FIG. 61 shows the results of powder X-ray diffraction of crystals of linagliptin obtained in Example 21.

FIG. 62 shows the results of particle size distribution of crystals of linagliptin obtained in Example 21.

FIG. 63 shows a SEM image of crystals of teneligliptin hydrobromide hydrate obtained in Example 22.

FIG. 64 shows the results of powder X-ray diffraction of crystals of teneligliptin hydrobromide hydrate obtained in Example 22.

FIG. 65 shows the results of particle size distribution of crystals of teneligliptin hydrobromide hydrate obtained in Example 22.

FIG. 66 shows a SEM image of crystals of glutathione obtained in Example 23.

FIG. 67 shows the results of powder X-ray diffraction of crystals of glutathione obtained in Example 23.

FIG. 68 shows the results of particle size distribution of crystals of glutathione obtained in Example 23.

FIG. 69 shows a SEM image of crystals of dabigatran etexilate methanesulfonate obtained in Example 24.

FIG. 70 shows the results of powder X-ray diffraction of crystals of dabigatran etexilate methanesulfonate obtained in Example 24.

FIG. 71 shows the results of particle size distribution of crystals of dabigatran etexilate methanesulfonate obtained in Example 24.

FIG. 72 shows a SEM image of crystals of pilsicainide hydrochloride obtained in Example 25.

FIG. 73 shows the results of powder X-ray diffraction of crystals of pilsicainide hydrochloride obtained in Example 25.

FIG. 74 shows the results of particle size distribution of crystals of pilsicainide hydrochloride obtained in Example 25.

FIG. 75 shows a SEM image of crystals of theophylline magnesium salt tetrahydrate obtained in Example 26.

FIG. 76 shows the results of powder X-ray diffraction of crystals of theophylline magnesium salt tetrahydrate obtained in Example 26.

FIG. 77 shows the results of particle size distribution of crystals of theophylline magnesium salt tetrahydrate obtained in Example 26.

FIG. 78 shows a SEM image of crystals of mirabegron obtained in Example 27.

FIG. 79 shows the results of powder X-ray diffraction of crystals of mirabegron obtained in Example 27.

FIG. 80 shows the results of particle size distribution of crystals of mirabegron obtained in Example 27.

FIG. 81 shows a SEM image of crystals of tolvaptan obtained in Example 28.

FIG. 82 shows the results of powder X-ray diffraction of crystals of tolvaptan obtained in Example 28.

FIG. 83 shows a SEM image of crystals of tramadol hydrochloride obtained in Example 29.

FIG. 84 shows the results of powder X-ray diffraction of crystals of tramadol hydrochloride obtained in Example 29.

FIG. 85 shows a SEM image of crystals of bepotastine besilate obtained in Example 30.

FIG. 86 shows the results of powder X-ray diffraction of crystals of bepotastine besilate obtained in Example 30.

FIG. 87 shows the results of particle size distribution of crystals of bepotastine besilate obtained in Example 30.

FIG. 88 shows a SEM image of crystals of olopatadine obtained in Example 31.

FIG. 89 shows the results of powder X-ray diffraction of crystals of olopatadine obtained in Example 31.

FIG. 90 is a graph in which a predictive value with respect to an experimental value of a critical degree of supersaturation is plotted. The dashed lines show the 95% confidence interval.

FIG. 91 shows a flowchart showing an example of the operation of the entire processing.

## DESCRIPTION OF EMBODIMENTS

The method of the present invention is a method for producing a specifically-shaped crystal of a compound, which includes the following steps:

(1) a step of preparing a supersaturated solution of the compound having a degree of supersaturation equal to or higher than a critical degree of supersaturation required to obtain the specifically-shaped crystal of the compound; and

(2) a step of precipitating the specifically-shaped crystal of the compound from the supersaturated solution.

In the present invention, "crystal of a compound" means a single crystal or a polycrystal of the compound. In the present invention, "specifically-shaped crystal of a compound" is not particularly limited as long as it is known as a single crystal or polycrystal shape of the compound. Specific examples thereof include plate crystals, needle crystals, columnar crystals, hopper crystals, symmetric dendritic crystals, asymmetric dendritic crystals, spherulites, and the like, of which spherulites are preferable.

The compound obtained as the specifically-shaped crystal by the method of the present invention is not particularly limited, and may be either an organic compound or an inorganic compound.

When the compound obtained as the specifically-shaped crystal by the method of the present invention is an organic compound, it may be in any form of a free substance (i.e., a form which does not form a complex with other substances), a salt thereof or a solvate thereof, or a mixture thereof. When the compound obtained as the specifically-shaped crystal by the method of the present invention is an inorganic compound, it may be a free substance, a solvate thereof, or a mixture thereof.

In the present description, "salt" of the organic compound is not particularly limited, and examples thereof include salts with inorganic acids such as sulfuric acid, hydrochloric acid, hydrobromic acid, phosphoric acid, and nitric acid; salts with organic acids such as acetic acid, oxalic acid, lactic acid, tartaric acid, fumaric acid, maleic acid, citric acid, benzoic acid, benzenesulfonic acid (besylic acid), methanesulfonic acid, p-toluenesulfonic acid, 10-camphorsulfonic acid, ethanesulfonic acid, glucoheptonic acid, gluconic acid, glutamic acid, glycolic acid, malic acid, malonic acid, mandelic acid, galactal acid, and naphthalene-2-sulfonic acid; salts with single or multiple metal ions such as lithium ion, sodium ion, potassium ion, calcium ion, magnesium ion, zinc ion, and aluminum ion; and salts with amines such as ammonia, arginine, lysine, piperazine, choline, diethylamine, 4-phenylcyclohexylamine, 2-aminoethanol, and benzathine.

In the present description, "solvate" of the organic or inorganic compound is not particularly limited, and examples thereof include hydrates, alcohol solvates (e.g., methanol solvate, ethanol solvate, 1-propanol solvate, 2-propanol solvate, etc.), ketone solvates (e.g., acetone solvate, methyl ethyl ketone solvate, methyl isopropyl solvate, methyl isobutyl ketone solvate, etc.), nitrile solvates (e.g., acetonitrile solvate, propionitrile solvate, etc.), ester solvates (e.g., ethyl acetate solvate, isopropyl acetate solvate), ether solvates (e.g., ethyl ether solvate, tert-butyl methyl ether solvate, etc.), aliphatic hydrocarbon solvates (e.g., normal pentane solvate, normal hexane solvate, cyclohexane solvate, normal heptane solvate, isooctane solvate, etc.), toluene solvates, N,N-dimethylformamide solvate, dimethyl sulfoxide solvates, and the like.

When the compound obtained as the specifically-shaped crystal by the method of the present invention is an organic compound, the molecular weight thereof is not particularly limited, and is preferably 100 to 2,000, and more preferably 200 to 1,500, from the viewpoint of the operation of dissolving the compound once, followed by crystallization and further drying under reduced pressure. When the compound obtained as the specifically-shaped crystal by the method of the present invention is an inorganic compound, the formula weight thereof is not particularly limited, and is preferably 50 to 2,000, and more preferably 200 to 1,500, from the same viewpoint as above.

The compound obtained as the specifically-shaped crystal by the method of the present invention is preferably a compound selected from the following (1) and (2):

(1) a compound represented by the following formula I, or a tautomer thereof, or an optical isomer thereof, or a salt thereof, or a solvate thereof:

[Chemical Formula 3]

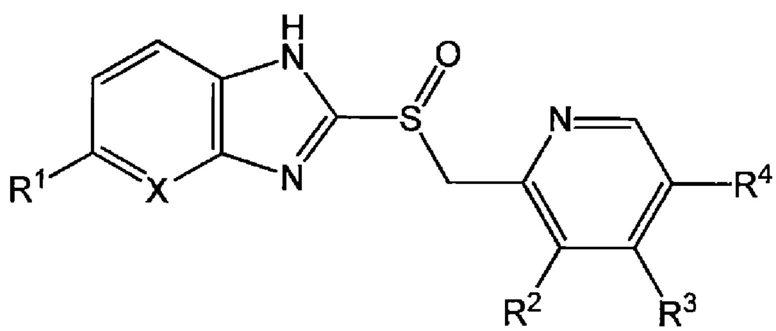

I

wherein

X is CH or N,
$R^1$ is a hydrogen atom or an optionally substituted $C_{1-6}$ alkoxy group, and
$R^2$, $R^3$, and $R^4$ are the same or different and each represent a hydrogen atom, an optionally substituted $C_{1-6}$ alkyl group, an optionally substituted $C_{1-6}$ alkoxy group, or an optionally substituted amino group;

(2) azithromycin, duloxetine, clarithromycin, lanthanum carbonate ($La_2(CO_3)_3$), glutamic acid, clopidogrel, ketotifen, escitalopram, dabigatran etexilate, theophylline, teneligliptin, pilsicainide, tramadol, vildagliptin, linagliptin, glutathione, mirabegron, tolvaptan, valacyclovir, bepotastine, olopatadine, or an optical isomer thereof (if any), or a salt thereof (if any), or a solvate thereof.

In the present description, "$C_{1-6}$ alkyl group" is a linear or branched alkyl group having 1 to 6 carbon atoms. Examples of the $C_{1-6}$ alkyl group include a methyl group, an ethyl group, a propyl group, a butyl group, a pentyl group, a hexyl group, and an isomer thereof. When the $C_{1-6}$ alkyl group is substituted, it may be mono-substituted or poly-substituted. In the case of poly-substitution, the substituent may be the same or different. The substituent to the $C_{1-6}$ alkyl group is not particularly limited, and examples thereof include a halogen, a hydroxy group, an amino group, and a $C_{1-6}$ alkoxy group.

In the present description, "$C_{1-6}$ alkoxy group" is a group in which the above $C_{1-6}$ alkyl group is substituted with an oxygen atom. Examples of the $C_{1-6}$ alkoxy group include a methoxy group, an ethoxy group, a propoxy group, a butoxy group, a pentyloxy group, a hexyloxy group, and an isomer thereof. When the $C_{1-6}$ alkoxy group is substituted, it may be mono-substituted or poly-substituted. In the case of poly-substitution, the substituent may be the same or different. The substituent to the $C_{1-6}$ alkoxy group is not particularly limited, and examples thereof include a halogen, a hydroxy group, an amino group, and a $C_{1-6}$ alkoxy group.

In the present description, "halogen" is a monovalent group of a halogen atom, and specific examples thereof include a fluoro group, a chloro group, a bromo group, and an iodo group.

In the present description, when the amino group is substituted, it may be mono-substituted or di-substituted. In the case of di-substitution, the substituent may be the same or different. The substituent to the amino group is not particularly limited, and examples thereof include a $C_{1-6}$ alkyl group.

Examples of the compound obtained as the specifically-shaped crystal include omeprazole (X = CH, $R^1$ = methoxy, $R^2$ = methyl, $R^3$ = methoxy, $R^4$ = methyl), lansoprazole (X = CH, $R^1$ = hydrogen atom, $R^2$ = methyl, $R^3$ = 2,2,2-trifluoroethoxy, $R^4$ = hydrogen atom), tenatoprazole (X = N, $R^1$ = methoxy, $R^2$ = methyl, $R^3$ = methoxy, $R^4$ = methyl), pantoprazole (X = CH, $R^1$ = difluoromethoxy, $R^2$ = methoxy, $R^3$ = methoxy, $R^4$ = hydrogen atom), esomeprazole (X = CH, $R^1$ = methoxy, $R^2$ = methyl, $R^3$ = methoxy, $R^4$ = methyl), dexlansoprazole (X = CH, $R^1$ = hydrogen atom, $R^2$ = methyl, $R^3$ = 2,2,2-trifluoroethoxy, $R^4$ = hydrogen atom), labeprazole (X = CH, $R^1$ = hydrogen atom, $R^2$ = methyl, $R^3$ = 3-methoxypropoxy, $R^4$ = hydrogen atom), and leminoprazole (X = CH, $R^1$ = hydrogen atom, $R^2$ = N-methyl-N-(2-methylpropyl)amino, $R^3$ = hydrogen atom, $R^4$ = hydrogen atom), of which a salt thereof and a solvate thereof are preferable, and esomeprazole and lansoprazole, and a salt thereof and a solvate thereof are more preferable.

In the method of the present invention, the compound used as a starting material (also referred to as "compound to be crystallized" or "substrate" in the present description) is sometimes the same as or different from "compound obtained as the specifically-shaped crystal" by the method. For example, the compound used as the starting material may be a crystal of a potassium salt, and the compound obtained as the specifically-shaped crystal may be a magnesium salt (see Example 6). The compound used as the starting material may be a dihydrate crystal, and the compound obtained as the specifically-shaped crystal may be a monohydrate (see Example 12). The compound used as the starting material may be amorphous. The compound obtained as the specifically-shaped crystal may be formed in a supersaturated solution during preparation (see Examples 15 to 17).

In the present description, "spherulite" means a crystal aggregate (polycrystal) which has a radial or concentric thin layered structure and has a spherical outer shape. Whether the crystal obtained by the method of the present invention is a spherulite or not can be determined, for example, by observing the outer shape of the crystal using SEM. For example, it can be discriminated by cutting the crystal aggregate and observing the internal structure thereof using SEM. The sphericity of the spherulite obtained by the method of the present invention is usually 0.60 or more, preferably 0.70 or more, more preferably 0.80 or more, particularly preferably 0.90 or more, and most preferably 0.95 or more. The sphericity can be calculated by the following method.

When an image of particles taken by SEM is analyzed using image processing software ImageJ, a coordinate group representing the position of pixels forming the outline of a particle is obtained. The length of the coordinate group is defined as N, and one pixel coordinate of each outline is defined as p[i] ($1 \leq i \leq N$). The angle between a straight line l(k) connecting two points p[k] and p[k + N/D] and a straight line m(k) connecting two points p[k + N/D] and p[k + 2N/D] is defined as $\theta$(k) ($-180° < \theta < 180°$) ($1 \leq k \leq N$, $1 \leq D \leq N$). D corresponds to the number of partitions of the outline.

When N/D is not a natural number, the value is defined as the quotient obtained by dividing N by D. By calculating this angle with k = 1 to N, N pieces of θ value can be obtained. FIG. 42 shows the position of θ(k).

There are some methods for calculating θ(k), and, for example, when the slope of the straight line l(k) is defined as a(k) and the slope of the straight line m(k) is defined as b(k), θ(k) can be calculated by the following formula:

[Equation 1]

$$\theta(k) = \arctan\frac{a(k) - b(k)}{1 + a(k)b(k)}$$

When the particle is a true sphere, θ(k) is 360/D, and the closer the particle is to a true sphere, the closer θ(k) is to this value. The error between θ(k) and the true value 360/D is evaluated by the root-mean-square error (RMSE) of the following formula:

[Equation 2]

$$\mathrm{RMSE} = \sqrt{\frac{1}{\mathrm{N}}\sum_{k=1}^{N}\left(\frac{360}{D} - \theta(k)\right)^2}$$

Using the RMSE value of when D is 10 (θ(k) = 36°), the sphericity is defined by the following formula:

[Equation 3]

$$\mathrm{Sphericity} = 1 - \frac{1}{1 + \exp(-6(\log(\mathrm{RMSE}) - 1.3))}$$

The sphericity is 1 in a true sphere, and the closer the particle is to a true sphere, the closer the sphericity is to 1. Meanwhile, the sphericity of an ellipse whose long axis is infinitely long is zero. The sphericity is measured for five or more particles to calculate the mean. This mean is defined as the sphericity of each sample (see FIGs. 42 and 43).

$d_{10}$ in the present description is a value obtained from a particle size distribution analyzer, and represents a particle size of a particle corresponding to 10% of accumulation from the smaller particle side in the particle size distribution based on the volume.

$d_{50}$ in the present description is a value obtained from a particle size distribution analyzer, and represents a particle size of a particle corresponding to 50% of accumulation from the smaller particle side in the particle size distribution based on the volume.

$d_{90}$ in the present description is a value obtained from a particle size distribution analyzer, and represents a particle size of a particle corresponding to 90% of accumulation from the smaller particle side in the particle size distribution based on the volume.

The lower limit of $d_{50}$ of a specifically-shaped crystal obtained by the method of the present invention is not particularly limited, and is preferably 1 μm, 10 μm, 15 μm, 20 μm, 25 μm, 30 μm, 35 μm, 40 μm, 45 μm, or 50 μm. The upper limit of $d_{50}$ of a specifically-shaped crystal obtained by the method of the present invention is not particularly limited, and is preferably 100 μm, 120 μm, 150 μm, 200 μm, 250 μm, 300 μm, 500 μm, or 1,000 μm. More preferably, $d_{50}$ is 1 to 500 μm or 10 to 300 μm.

"Equivalent circle diameter" in the present description is a diameter of a perfect circle corresponding to the area of individual particles in an image of particles taken by SEM, and is calculated from randomly selected 5 to 800 particles using image processing software ImageJ. The equivalent circle diameter of a spherulite obtained by the method of the present invention is preferably 25 μm or more, 30 μm or more, 35 μm or more, 40 μm or more, 45 μm or more, or 50 μm or more and 100 μm or less, 120 μm or less, 150 μm or less, 200 μm or less, 250 μm or less, 300 μm or less, 500 μm or less, or 1,000 μm or less. More preferably, the equivalent circle diameter is 25 to 500 μm or 30 to 300 μm.

"Sharpness index" in the present description is an index representing the uniformity of a particle size of a particle

in a powder, and a sharpness index of 1.0 represents a powder having the most uniform particle size. The closer the sharpness index is to 1.0, the more the particle size of the powder is uniform. Specifically, the sharpness index represents a value calculated using the following formula from the values of $d_{10}$, $d_{50}$, and $d_{90}$ measured by a particle size distribution analyzer:

[Equation 4]

$$\text{Sharpness index} = \left(\frac{d_{90}}{d_{50}} + \frac{d_{50}}{d_{10}}\right)/2$$

The sharpness index of a specifically-shaped crystal obtained by the method of the present invention is preferably 1.0 to 5.0, more preferably 1.0 to 4.0, still more preferably 1.0 to 3.0, yet more preferably 1.0 to 2.5, particularly preferably 1.0 to 2.0, and most preferably 1.0 to 1.5.

Whether a specifically-shaped crystal obtained by the method of the present invention is a crystal or not can be determined by, for example, showing a diffraction peak in powder X-ray diffraction or performing SEM observation.

"Supersaturated solution" in the present description means a solution in a state of containing a solute exceeding the solubility. As the degree of supersaturation, "degree of supersaturation" can be used. Regarding the degree of supersaturation, when the concentration (mass concentration (g/g)) of a compound dissolved in a supersaturated solution is defined as C and the solubility of the compound is defined as Cs, the degree of supersaturation S can be represented by the following formula:

[Equation 5]

$$S = C / Cs$$

In the method of the present invention, when a compound obtained as a specifically-shaped crystal is a solvate, the degree of supersaturation of the compound is calculated by dividing the concentration of a solvate of the compound dissolved in a supersaturated solution by the solubility of the solvate of the compound.

"Critical degree of supersaturation" in the present description means the lowest degree of supersaturation required to obtain a specifically-shaped crystal. The present inventors have found that, from a supersaturated solution of a compound having a degree of supersaturation equal to or higher than the critical degree of supersaturation, a specifically-shaped spherulite of the compound is reproducibly obtained. In other words, when crystallization is performed by preparing supersaturated solutions each having a different degree of supersaturation using the same compound and the same solvent or the same combination of solvents, all supersaturated solutions having a degree of supersaturation equal to or higher than the critical degree of supersaturation produce a crystal having a desired specific shape. However, supersaturated solutions having a degree of supersaturation below the critical degree of supersaturation do not produce a crystal having the specific shape. The value of a critical degree of supersaturation varies depending on the shape of a crystal. For example, all of values of a critical degree of supersaturation required to obtain a plate crystal, a critical degree of supersaturation required to obtain a needle crystal, and a critical degree of supersaturation required to obtain a spherulite are different. The critical degree of supersaturation required to obtain a spherulite is higher than the critical degree of supersaturation required to obtain a plate crystal or a needle crystal.

"Critical degree of supersaturation" in the method of the present invention may be an actual measured value obtained by the measurement method described below or a predictive value outputted from the predictive model of a critical degree of supersaturation described below. When the predictive value is outputted within a numerical value range having an upper limit and a lower limit, the "critical degree of supersaturation" may be the lower limit, the upper limit, or any value between the upper limit and the lower limit of the predictive value. In this case, the "critical degree of supersaturation" is preferably the lower limit of the predictive value.

The actual measured value of the critical degree of supersaturation (S*) can be measured as follows:

(1) 100 particles are observed by SEM.
(2) 5 to 10 particles whose shape is close to a desired specific shape are selected, and the shape is evaluated. In particular, when the desired specific shape is a sphere, the sphericity is evaluated.
(3) Based on the evaluation index of the shape, whether the selected particles have the desired specific shape or not is determined. In particular, when the desired specific shape is a sphere, the case where the sphericity is 0.60 or more is determined as a spherulite.
(4) The above-mentioned operation is performed for each sample having a different degree of supersaturation during

crystallization, and the minimum value of a degree of supersaturation having the specifically-shaped particles is determined as the critical degree of supersaturation. The difference between the maximum value of a degree of supersaturation not having the specifically-shaped particles and the critical degree of supersaturation should be 20% or less.

A step of preparing a supersaturated solution having a degree of supersaturation equal to or higher than a critical degree of supersaturation can be performed under a condition where nucleation does not occur before the critical degree of supersaturation is reached (namely, a condition where crystal nuclei of a crystal not having a desired shape are not formed before a critical degree of supersaturation required to obtain a crystal having a desired shape is reached). For example, the step can be performed by preparing a supersaturated solution earlier than the formation of crystal nuclei of a crystal not having a desired shape.

Even if crystal nuclei of a crystal not having a desired shape are formed before the critical degree of supersaturation is reached during preparation of a supersaturated solution, it is possible to prepare the supersaturated solution by separating the crystal nuclei from the solution by a method such as filtration before the supersaturated state are completely resolved.

Regarding the step of preparing a supersaturated solution having a degree of supersaturation equal to or higher than a critical degree of supersaturation, the conditions (e.g., rate of addition of a poor solvent to a solution of a compound, rate of addition of a solution of a compound to a poor solvent, rate of addition of a reaction reagent to a solution of a precursor of a compound, rate of addition of a precursor of a compound to a solution of a reaction reagent, crystallization temperature, stirring rate, stirring time, etc.) are not particularly limited as long as it is possible to prepare a solution having a degree of supersaturation equal to or higher than a critical degree of supersaturation. This step of preparing a supersaturated solution can be performed by, for example, the following methods (1) to (6).

(1) Preparation of Supersaturated Solution by Reverse Addition

By reversely adding dropwise a solution in which a compound to be crystallized is dissolved in a good solvent to a poor solvent, it is possible to prepare a supersaturated solution of a compound obtained as a specifically-shaped crystal having a degree of supersaturation equal to or higher than a critical degree of supersaturation. The combination of a good solvent and a poor solvent used can be appropriately changed according to the compound to be crystallized.

(2) Preparation of Supersaturated Solution by Normal Addition

By adding dropwise a poor solvent to a solution in which a compound to be crystallized is dissolved in a good solvent, it is possible to prepare a supersaturated solution of a compound obtained as a specifically-shaped crystal having a degree of supersaturation equal to or higher than a critical degree of supersaturation. In this case, when an amorphous solid of the compound is precipitated in the supersaturated solution, filtration may be performed. The combination of a good solvent and a poor solvent used can be appropriately changed according to the compound to be crystallized.

(3) Preparation of Supersaturated Solution by Neutralization Reaction

When a compound to be crystallized is an acidic compound, by (i) preparing a solution in which a compound to be crystallized is suspended in a good solvent, (ii) adding a base to the suspension prepared in (i) to prepare a solution of a base addition salt of the compound, and (iii) adding a poor solvent containing an acid to the solution of the base addition salt, it is possible to prepare a supersaturated solution of a compound obtained as a specifically-shaped crystal having a degree of supersaturation equal to or higher than a critical degree of supersaturation. The combination of a good solvent and a poor solvent used can be appropriately changed according to the compound to be crystallized. The base used can be appropriately selected according to the compound to be crystallized.

When a compound to be crystallized is a basic compound, by (i) preparing a solution in which a compound to be crystallized is suspended in a good solvent, (ii) adding an acid to the suspension prepared in (i) to prepare a solution of an acid addition salt of the compound, and (iii) adding a poor solvent containing a base to the solution of the acid addition salt, it is possible to prepare a supersaturated solution of a compound obtained as a specifically-shaped crystal having a degree of supersaturation equal to or higher than a critical degree of supersaturation. The combination of a good solvent and a poor solvent used can be appropriately changed according to the compound to be crystallized. The acid used can be appropriately selected according to the compound to be crystallized.

(4) Preparation of Supersaturated Solution by Salt Formation

When a compound obtained as a specifically-shaped crystal is a salt of an organic compound and a free form of the organic compound is a basic compound, by (i) preparing a solution in which the free form of the organic compound is dissolved in a solvent, and (ii) adding an acid to the solution prepared in (i), it is possible to prepare a supersaturated solution of the salt of the organic compound having a degree of supersaturation equal to or higher than a critical degree of supersaturation. The acid used can be appropriately selected according to the free form of the organic compound.
When a compound obtained as a specifically-shaped crystal is a salt of an organic compound and a free form of the organic compound is an acidic compound, by (i) preparing a solution in which the free form of the organic compound is dissolved in a solvent, and (ii) adding a base to the solution prepared in (i), it is possible to prepare a supersaturated solution of the salt of the organic compound having a degree of supersaturation equal to or higher than a critical degree of supersaturation. The base used can be appropriately selected according to the free form of the organic compound.

(5) Preparation of Supersaturated Solution by Chemical Conversion

When a compound obtained as a specifically-shaped crystal is a compound obtained by chemical conversion of a precursor compound, by (i) preparing a solution in which the precursor compound is dissolved in a solvent, and (ii) adding a conversion reagent to the solution prepared in (i), it is possible to prepare a supersaturated solution of the compound obtained by the chemical conversion having a degree of supersaturation equal to or higher than a critical degree of supersaturation. The conversion reagent used can be appropriately selected according to the precursor compound.

(6) Preparation of Supersaturated Solution by Ion Exchange

When a compound obtained as a specifically-shaped crystal is a salt with a cation, by (i) preparing a solution of a compound which formed a salt using a cation different from the cation, and (ii) adding a solution containing a cation constituting a compound obtained as a specifically-shaped crystal to the solution prepared in (i), it is possible to prepare a supersaturated solution of the compound obtained as a specifically-shaped crystal having a degree of supersaturation equal to or higher than a critical degree of supersaturation. The solvent used can be appropriately changed according to the compound to be crystallized. The cation used can be appropriately selected according to the compound.
When a compound obtained as a specifically-shaped crystal is a salt with an anion, by (i) preparing a solution of a compound which formed a salt using an anion different from the anion, and (ii) adding a solution containing an anion constituting a compound obtained as a specifically-shaped crystal to the solution prepared in (i), it is possible to prepare a supersaturated solution of the compound obtained as a specifically-shaped crystal having a degree of supersaturation equal to or higher than a critical degree of supersaturation. The solvent used can be appropriately changed according to the compound to be crystallized. The anion used can be appropriately selected according to the compound.
The solvent used in the method of the present invention is not particularly limited, and is determined based on the solubility of a compound to be crystallized and a compound obtained as a specifically-shaped crystal and the like. Examples thereof include water, alcohols (e.g., linear or branched monohydric, dihydric, or trihydric alcohol having 1 to 6 carbon atoms, specifically, methanol, ethanol, 1-propanol, 2-propanol, 1-butanol, 2-butanol, tert-butanol, ethylene glycol, propylene glycol, glycerin, diethylene glycol, diethylene glycol monoethyl ether, and the like), tetrahydrofuran, ketones (e.g., acetone, methyl ethyl ketone, methyl isopropyl ketone, methyl isobutyl ketone), acetonitrile, ethyl acetate, isopropyl acetate, tert-butyl methyl ether, toluene, aliphatic hydrocarbons (e.g., n-pentane, n-hexane, cyclohexane, n-heptane, isooctane), aromatic hydrocarbons (e.g., toluene), N,N-dimethylformamide, dimethyl sulfoxide, and mixed solvents thereof.
A supersaturated solution can be prepared at room temperature, or can also be prepared under cooling or in a warmed state. For example, preparation can be performed at -30 °C to a solvent's boiling point, and preferably at 0 °C to 100 °C.
In the method of the present invention, a step of precipitating a specifically-shaped crystal from a supersaturated solution (crystallization step) may be performed by allowing the supersaturated solution to stand or may be performed while stirring. The step is performed for preferably 0.1 to 400 hours, more preferably 0.1 to 200 hours, and still more preferably 0.1 to 100 hours. The step may be performed by inoculating a seed crystal of a compound into the supersaturated solution. The form of the seed crystal is not particularly limited, and may be or may not be a crystal having the same shape as that of a crystal to be precipitated. The amount of the seed crystal inoculated is preferably 0.00001 to 10% (w/w), more preferably 0.00005 to 1% (w/w), still more preferably 0.0001 to 0.5% (w/w), and particularly preferably 0.0001 to 0.1% (w/w) based on the amount of the compound in the supersaturated solution. It is possible to adjust the particle size of the crystal obtained according to the amount of the seed crystal added.
In the method of the present invention, the step of precipitating a specifically-shaped crystal from a supersaturated solution can be prepared at room temperature, or can also be prepared under cooling or in a warmed state. This step can be performed at a crystallization temperature of, for example, -70 °C to a solvent's boiling point, preferably -20 °C to 60 °C, and more preferably -10 °C to 40 °C.

The method of the present invention may further include, after the step of precipitating a specifically-shaped crystal from a supersaturated solution, a step of isolating the specifically-shaped crystal thus precipitated by, for example, filtration, centrifugation, or decantation. A step of washing the specifically-shaped crystal thus isolated with an appropriate solvent may also be included.

The method of the present invention may further include, after the step of isolating and washing the specifically-shaped crystal, a step of drying a wet body of the crystal by, for example, air drying, through-flow drying, drying under reduced pressure, and/or freeze-drying.

Sizes of individual crystals constituting a specifically-shaped crystal obtained by the method of the present invention can be controlled by the degree of supersaturation and the crystallization temperature during crystallization. For example, in the case of a specifically-shaped crystal as a spherulite, when a method in which the degree of supersaturation decreases as the crystal grows is adopted, a spherulite is formed with the crystal in the center of the spherulite being finest as a result of radial polycrystalline growth while individual crystals become gradually large (see Example 12). This characteristic can be observed by SEM by cutting the spherulite (see FIG. 4). As another example, when a method for keeping a high degree of supersaturation during crystal growth is adopted, a spherulite is formed with individual crystal sizes being small. By controlling crystal sizes inside the spherulite, it is possible to control the dissolution profile.

The dissolution profile is described by the zero-order model, the first-order model, the Weibull model, the Higuchi model, the Hixson-Crowell model, the Korsmeyer-Peppas model, the Baker-Lonsdale model, the Hopfenberg model, and the like. The Weibull model is represented by the following formula:

[Equation 6]

$$C = C_0\left(1 - \exp\left[-\left(\frac{t}{a}\right)^b\right]\right)$$

where C represents a solution concentration, $C_0$ represents solubility, t represents time, a represents a scale factor, and b represents a shape factor.

"Particle density" in the present description is mass per unit volume when the volume of the substance itself as well as the volume of the open void and the closed void are included as the volume of polycrystalline particles. For example, when the polycrystalline particles are spherical particles, the particle density is calculated by the following formula using the mean mass of the polycrystalline particles and the mean volume of the polycrystalline particles:

[Equation 7]

$$\text{Particle density} = \frac{\bar{M}}{\bar{V}}$$

[where *M* represents the mean mass of polycrystalline particles and *V* represents the mean volume of polycrystalline particles.]

Here, the mean mass of polycrystalline particles is calculated by measuring the number of polycrystalline particles contained in 1.0 mg or more, preferably 5.0 mg or more, and more preferably 10.0 mg or more of a sample by a scanner, etc. The mean volume of crystal particles is obtained by averaging the volume calculated by photographing 500 or more, preferably 1,000 or more, and more preferably 5,000 or more crystal particles by a light microscope, etc., using an equivalent circle diameter obtained from the projected area of polycrystalline particles, and assuming that the polycrystalline particles are true spheres. At this time, it is desirable that the distribution of the equivalent circle diameter is unimodal and the sharpness value is less than 1.5.

When a specifically-shaped crystal obtained by the method of the present invention is a polycrystal (e.g., spherulite), the particle density and the particle strength of the polycrystal can be controlled by the degree of supersaturation, the stirring rate, and the crystallization temperature during crystallization. This is also associated with the control of crystal size mentioned above. For example, in the case of a specifically-shaped crystal as a spherulite, when a method for keeping a high degree of supersaturation during crystal growth is adopted, individual crystal sizes become small,

and a densely packed spherulite is formed. As another example, when a method in which the degree of supersaturation decreases as the crystal grows is adopted, individual crystals become gradually large, and thus the particle density does not become high.

When a specifically-shaped crystal obtained by the method of the present invention is a polycrystal (e.g., spherulite), the particle density is preferably 0.6 $g/cm^3$ or more, more preferably 0.7 $g/cm^3$ or more, still more preferably 0.9 $g/cm^3$ or more, and particularly preferably 1.0 $g/cm^3$ or more.

"Particle packing rate" of a polycrystal in the present description is a value calculated by the following formula:

[Equation 8]

$$\text{Particle packing rate (\%)} = \text{(particle density)} / \text{(true density of compound)} \times 100$$

Here, "true density of compound" is a density of a substance itself not including a void. If no void exists at all inside or on the surface of a particle, the particle packing rate is 100%. The true density of a compound can be obtained by dry density measurement by the constant volume expansion method. Examples of the measuring device include a dry automatic densimeter AccuPyc II 1340-10CC manufactured by Shimadzu Corporation.

When a specifically-shaped crystal obtained by the method of the present invention is a polycrystal (e.g., spherulite), the particle packing rate is preferably 30% or more, more preferably 50% or more, still more preferably 60% or more, and particularly preferably 80% or more.

Furthermore, a polycrystal with a high particle density also has a high particle strength. When a polycrystal, particularly a spherulite, is used as a raw material for production of drugs, a problem occurs in which wear in a formulation step leads to deterioration of yield or quality or both, and a polycrystal with a high particle strength has an advantage in this regard. When a specifically-shaped crystal obtained by the present invention is a polycrystal, the particle strength is preferably 1.0 MPa or more, more preferably 1.5 MPa or more, still more preferably 2.0 MPa, yet more preferably 2.5 MPa or more, and particularly preferably 3.0 MPa or more. The particle strength is calculated by measuring the force at which a particle breaks when load is applied to one particle and the particle size. Specifically, the particle strength is calculated by the following formula in accordance with JIS R 1639-5. Examples of a measuring device include a particle hardness measuring device NEW GRANO manufactured by OKADA SEIKO CO., LTD. and a micro compression testing machine MCT manufactured by Shimadzu Corporation.

[Equation 9]

$$\text{Particle strength} = 2.48 \times P / (\pi \times d \times d)$$

where P represents a test force (N), and d represents a particle size (mm).

The more the shape of particles to be filtered is true spherical and the larger the particle size is, the faster the filtration rate of the particles is. The more the shape of the particles is true spherical, the thinner the cake thickness is and the smaller the difference between before and after compression is.

In one embodiment, the method of the present invention further includes a step of inputting data including:

information on a compound obtained as a specifically-shaped crystal, and
at least one of information on a solvent used for crystallization and a solution temperature during crystallization into a predictive model of a critical degree of supersaturation required to obtain the specifically-shaped crystal of the compound, and outputting a predictive value of the critical degree of supersaturation from the predictive model (hereinafter also referred to as "step of predicting a critical degree of supersaturation").

In the step of predicting a critical degree of supersaturation, as information on a compound and a solvent, a variable for a compound and/or a solvent used by paid or free descriptor calculation software, for example, alvaDesc, RDKit, Dragon, ChemoPy, MOE, Cinfony, PaDEL-descriptor, Mordred, and the like, and information based on the chemical structure of a compound and/or a solvent can be optionally used from a MOL file and an SDF file of a compound structure. In another aspect, a variable for a compound and/or a solvent may be used in combination with information based on the chemical structure of a compound and/or a solvent.

The variable for a compound and/or a solvent may be one or a plurality of variables belonging to at least one parameter selected from 1) molecule-related parameters, for example, molecular weight, species/number of atoms,

species/number of bonds, and the like, 2) topological parameters, for example, molecule binding indices, Hosoya indices, and the like, 3) physical property-related parameters, for example, molecular refractivity, parachor, LogP, and the like, and 4) other parameters, for example, substructure-related parameters (appearance information, appearance frequency, etc.), partial charge parameters, and the like. More specifically, as a variable describing a compound and a solvent, it is possible to use one or a plurality of variables belonging to at least one classification selected from constitutional indices, Ring descriptors, topological indices, walk and path counts, connectivity indices, information indices, 2D matrix-based descriptors, 2D autocorrelations, Burden eigenvalues, PVSA-like descriptors, ETA indices, edge adjacency indices, geometrical descriptors, 3D matrix-based descriptors, 3D autocorrelations, RDF descriptors, 3D-MoRSE descriptors, WHIM descriptors, GETAWAY descriptors, Randic molecular profiles, functional group counts, atom-centered fragments, atom-type E-state indices, pharmacophore descriptors, 2D Atom Pairs, 3D Atom Pairs, charge descriptors, molecular properties, drug-like indices, CATS 3D descriptors, 2D Monte Carlo descriptors, 3D Monte Carlo descriptors, and quantum-chemical descriptors as the above-mentioned descriptors.

In the step of predicting a critical degree of supersaturation, when a variable for a compound obtained as a specifically-shaped crystal and/or a variable for a solvent is/are used, the number of variables may be one or plural. The variable for a compound obtained as a specifically-shaped crystal and a solvent may be an actual measured value or a value obtained by calculation based on the molecule structure. When the solvent is a mixed solvent, not only a variable for respective solvents contained in the mixed solvent but also the mixing ratio of these solvents may be used as a variable.

As the variable for a compound, it is possible to use, as an example, at least one variable for descriptors selected from the group consisting of the following descriptors (1,905 types) obtained by calculation based on the molecule structure:

MW, AMW, Sv, Se, Sp, Si, Mv, Me, Mp, Mi, GD, nAT, nSK, nTA, nBT, nBO, nBM, SCBO, RBN, RBF, nDB, nTB, nAB, nH, nC, nN, nO, nS, nF, nCL, nHM, nHet, nX, H%, C%, N%, O%, X%, nCsp3, nCsp2, nCsp, max_conj_path, nCIC, nCIR, TRS, Rperim, Rbrid, MCD, RFD, RCI, NRS, NNRS, nR05, nR06, nR07, nR08, nR09, nR10, nR11, nBnz, ARR, D/Dtr05, D/Dtr06, D/Dtr07, D/Dtr08, D/Dtr09, D/Dtr10, D/Dtr11, ZM1, ZM1V, ZM1Kup, ZM1Mad, ZM1Per, ZM1MulPer, ZM2, ZM2V, ZM2Kup, ZM2Mad, ZM2Per, ZM2MulPer, ON0, ON0V, ON1, ON1V, Qindex, BBI, DBI, SNar, HNar, GNar, Xt, Dz, Ram, BLI, Pol, LPRS, MSD, SPI, PJI2, ECC, AECC, DECC, MDDD, UNIP, CENT, VAR, ICR, MaxTD, MeanTD, MaxDD, MeanDD, SMTI, SMTIV, GMTI, GMTIV, Xu, CSI, Wap, S1K, S2K, S3K, PHI, PW2, PW3, PW4, PW5, MAXDN, MAXDP, DELS, TIE, Psi_i_s, Psi_i_0, Psi_i_1, Psi_i_t, Psi_i_0d, Psi_i_1d, Psi_i_1s, Psi_e_A, Psi_e_0, Psi_e_1, Psi_e_0d, BAC, LOC, MWC01, MWC02, MWC03, MWC04, MWC05, MWC06, MWC07, MWC08, MWC09, MWC10, SRW02, SRW04, SRW05, SRW06, SRW07, SRW08, SRW09, SRW10, MPC02, MPC03, MPC04, MPC05, MPC06, MPC07, MPC08, MPC09, MPC10, piPC01, piPC02, piPC03, piPC04, piPC05, piPC06, piPC07, piPC08, piPC09, piPC10, TWC, TPC, piID, PCR, PCD, CID, BID, X0, X1, X2, X3, X4, X5, X0A, X1A, X2A, X3A, X4A, X5A, X0v, X1v, X2v, X3v, X4v, X5v, X0Av, X1Av, X2Av, X3Av, X4Av, X5Av, X0sol, X1sol, X2sol, X3sol, X4sol, X5sol, XMOD, RDCHI, RDSQ, X1Kup, X1Mad, X1Per, X1MulPer, ISIZ, AAC, IDE, IDM, IDDE, IDDM, IDET, IDMT, IVDE, IVDM, Ges, rGes, S0K, HVcpx, HDcpx, Uindex, Vindex, Xindex, Yindex, IC0, IC1, IC2, IC3, IC4, IC5, TIC0, TIC1, TIC2, TIC3, TIC4, TIC5, SIC0, SIC1, SIC2, SIC3, SIC4, SIC5, CIC0, CIC1, CIC2, CIC3, CIC4, CIC5, BIC0, BIC1, BIC2, BIC3, BIC4, BIC5, J_A, SpPos_A, SpPosLog_A, SpMax_A, SpMaxA_A, SpDiam_A, SpMAD_A, Ho_A, EE_A, VE1_A, VE2_A, VE3_A, VE1sign_A, VE2sign_A, VR1_A, VR2_A, VR3_A, Wi_D, AVS_D, H_D, Chi_D, ChiA_D, J_D, HyWi_D, SpPos_D, SpPosA_D, SpPosLog_D, SpMaxA_D, SpDiam_D, Ho_D, SM2_D, SM3_D, SM4_D, SM5_D, SM6_D, QW_L, TI1_L, TI2_L, STN_L, SpPosA_L, SpPosLog_L, SpMax_L, SpMaxA_L, SpDiam_L, SpAD_L, SpMAD_L, Ho_L, EE_L, SM2_L, SM3_L, SM4_L, SM5_L, SM6_L, VE1_L, VE2_L, VE3_L, VE1sign_L, VE2sign_L, VE3sign_L, VR1_L, VR2_L, VR3_L, AVS_X, H_X, Chi_X, ChiA_X, J_X, HyWi_X, SpPosX, SpPosA_X, SpPosLog_X, SpMaxA_X, SpDiam_X, SpMAD_X, Ho_X, EE_X, SM2_X, SM3_X, SM4_X, SM5_X, SM6_X, VE1_X, VE2_X, VE3_X, VE1sign_X, VE2sign_X, VR1_X, VR2_X, VR3_X, Wi_H2, WiA_H2, AVS_H2, Chi_H2, ChiA_H2, J_H2, HyWi_H2, SpPos_H2, SpPosA_H2, SpPosLog_H2, SpMax_H2, SpMaxA_H2, SpDiam_H2, Ho_H2, EE_H2, SM2_H2, SM3_H2, SM4_H2, SM5_H2, SM6_H2, VE1_H2, VE2_H2, VE3_H2, VE1sign_H2, VE2sign_H2, VR1_H2, VR2_H2, VR3_H2, Wi_Dt, AVS_Dt, H_Dt, Chi_Dt, ChiA_Dt, J_Dt, HyWi_Dt, SpPos_Dt, SpPosA_Dt, SpPosLog_Dt, SpMax_Dt, SpMaxA_Dt, SpDiam_Dt, Ho_Dt, SM2_Dt, SM3_Dt, SM4_Dt, SM5_Dt, SM6_Dt, Wi_D/Dt, WiA_D/Dt, AVS_D/Dt, H_D/Dt, Chi_D/Dt, ChiA_D/Dt, J_D/Dt, HyWi_D/Dt, SpPos_D/Dt, SpPosA_D/Dt, SpPosLog_D/Dt, SpMax_D/Dt, SpMaxA_D/Dt, SpDiam_D/Dt, Ho_D/Dt, EE_D/Dt, SM2_D/Dt, SM3_D/Dt, SM4_D/Dt, SM5_D/Dt, SM6_D/Dt, Wi_Dz(Z), WiA_Dz(Z), AVS_Dz(Z), H_Dz(Z), Chi_Dz(Z), ChiA_Dz(Z), J_Dz(Z), HyWi_Dz(Z), SpAbs_Dz(Z), SpPos_Dz(Z), SpPosA_Dz(Z), SpPosLog_Dz(Z), SpMax_Dz(Z), SpMaxA_Dz(Z), SpDiam_Dz(Z), SpAD_Dz(Z), SpMAD_Dz(Z), Ho_Dz(Z), SM1_Dz(Z), SM2_Dz(Z), SM3_Dz(Z), SM4_Dz(Z), SM5_Dz(Z), SM6_Dz(Z), VE1_Dz(Z), VE2_Dz(Z), VE3_Dz(Z), VE1sign_Dz(Z), VE2sign_Dz(Z), VR1_Dz(Z), VR2_Dz(Z), VR3_Dz(Z), Wi_Dz(m), WiA_Dz(m), AVS_Dz(m), H_Dz(m), Chi_Dz(m), ChiA_Dz(m), J_Dz(m), HyWi_Dz(m), SpAbs_Dz(m), SpPos_Dz(m), SpPosA_Dz(m), SpPosLog_Dz(m), SpMax_Dz(m), SpMaxA_Dz(m), SpDiam_Dz(m), SpAD_Dz(m), SpMAD_Dz(m), Ho_Dz(m), SM1_Dz(m), SM2_Dz(m), SM3_Dz(m), SM4_Dz(m), SM5_Dz(m), SM6_Dz(m), VE1_Dz(m), VE2_Dz(m), VE3_Dz(m), VE1sign_Dz(m), VE2sign_Dz(m), VR1_Dz(m), VR2_Dz(m), VR3_Dz(m), Wi_Dz(v), WiA_Dz(v), AVS_Dz(v), H_Dz(v), Chi_Dz(v), ChiA_Dz(v), J_Dz(v), HyWi_Dz(v),

SpAbs_Dz(v), SpPos_Dz(v), SpPosA_Dz(v), SpPosLog_Dz(v), SpMaxA_Dz(v), SpDiam_Dz(v), SpAD_Dz(v), SpMAD_Dz(v), Ho_Dz(v), EE_Dz(v), SM1_Dz(v), SM2_Dz(v), SM3_Dz(v), SM4_Dz(v), SM5_Dz(v), SM6_Dz(v), VE1_Dz(v), VE2_Dz(v), VE3_Dz(v), VE1sign_Dz(v), VE2sign_Dz(v), VE3sign_Dz(v), VR1_Dz(v), VR2_Dz(v), VR3_Dz(v), Wi_Dz(e), WiA_Dz(e), AVS_Dz(e), H_Dz(e), Chi_Dz(e), ChiA_Dz(e), J_Dz(e), HyWi_Dz(e), SpAbs_Dz(e), SpPos_Dz(e), SpPosA_Dz(e), SpPosLog_Dz(e), SpMax_Dz(e), SpMaxA_Dz(e), SpDiam_Dz(e), SpAD_Dz(e), SpMAD_Dz(e), Ho_Dz(e), EE_Dz(e), SM1_Dz(e), SM2_Dz(e), SM3_Dz(e), SM4_Dz(e), SM5_Dz(e), SM6_Dz(e), VE1_Dz(e), VE2_Dz(e), VE3_Dz(e), VE1sign_Dz(e), VE2sign_Dz(e), VR1_Dz(e), VR2_Dz(e), VR3_Dz(e), Wi_Dz(p), WiA_Dz(p), AVS_Dz(p), H_Dz(p), Chi_Dz(p), ChiA_Dz(p), J_Dz(p), HyWi_Dz(p), SpAbs_Dz(p), SpPos_Dz(p), SpPosA_Dz(p), SpPosLog_Dz(p), SpMax_Dz(p), SpMaxA_Dz(p), SpDiam_Dz(p), SpAD_Dz(p), SpMAD_Dz(p), Ho_Dz(p), EE_Dz(p), SM1_Dz(p), SM2_Dz(p), SM3_Dz(p), SM4_Dz(p), SM5_Dz(p), SM6_Dz(p), VE1_Dz(p), VE2_Dz(p), VE3_Dz(p), VE1sign_Dz(p), VE2sign_Dz(p), VE3sign_Dz(p), VR1_Dz(p), VR2_Dz(p), VR3_Dz(p), Wi_Dz(i), WiA_Dz(i), AVS_Dz(i), H_Dz(i), Chi_Dz(i), ChiA_Dz(i), J_Dz(i), HyWi_Dz(i), SpAbs_Dz(i), SpPos_Dz(i), SpPosA_Dz(i), SpPosLog_Dz(i), SpMaxA_Dz(i), SpDiam_Dz(i), SpAD_Dz(i), SpMAD_Dz(i), Ho_Dz(i), EE_Dz(i), SM1_Dz(i), SM2_Dz(i), SM3_Dz(i), SM4_Dz(i), SM5_Dz(i), SM6_Dz(i), VE1_Dz(i), VE2_Dz(i), VE3_Dz(i), VE1sign_Dz(i), VE2sign_Dz(i), VR1_Dz(i), VR2_Dz(i), VR3_Dz(i), Wi_B(m), WiA_B(m), AVS_B(m), Chi_B(m), ChiA_B(m), J_B(m), HyWi_B(m), SpAbs_B(m), SpPos_B(m), SpPosA_B(m), SpPosLog_B(m), SpMax_B(m), SpMaxA_B(m), SpDiam_B(m), SpAD_B(m), SpMAD_B(m), Ho_B(m), EE_B(m), SM1_B(m), SM2_B(m), SM3_B(m), SM4_B(m), SM5_B(m), SM6_B(m), VE1_B(m), VE2_B(m), VE3_B(m), VE1sign_B(m), VE2sign_B(m), VE3sign_B(m), VR1_B(m), VR2_B(m), VR3_B(m), Wi_B(v), WiA_B(v), AVS_B(v), Chi_B(v), ChiA_B(v), J_B(v), HyWi_B(v), SpAbs_B(v), SpPos_B(v), SpPosA_B(v), SpPosLog_B(v), SpMax_B(v), SpMaxA_B(v), SpDiam_B(v), SpAD_B(v), SpMAD_B(v), Ho_B(v), EE_B(v), SM1_B(v), SM2_B(v), SM3_B(v), SM4_B(v), SM5_B(v), SM6_B(v), VE1_B(v), VE2_B(v), VE3_B(v), VE1sign_B(v), VE2sign_B(v), VE3sign_B(v), VR1_B(v), VR2_B(v), VR3_B(v), Wi_B(e), WiA_B(e), AVS_B(e), Chi_B(e), ChiA_B(e), J_B(e), HyWi_B(e), SpAbs_B(e), SpPos_B(e), SpPosA_B(e), SpPosLog_B(e), SpMax_B(e), SpMaxA_B(e), SpDiam_B(e), SpAD_B(e), SpMAD_B(e), Ho_B(e), EE_B(e), SM1_B(e), SM2_B(e), SM3_B(e), SM4_B(e), SM5_B(e), SM6_B(e), VE1_B(e), VE2_B(e), VE3_B(e), VE1sign_B(e), VE2sign_B(e), VE3sign_B(e), VR1_B(e), VR2_B(e), VR3_B(e), Wi_B(p), WiA_B(p), AVS_B(p), Chi_B(p), ChiA_B(p), J_B(p), HyWi_B(p), SpAbs_B(p), SpPos_B(p), SpPosA_B(p), SpPosLog_B(p), SpMax_B(p), SpMaxA_B(p), SpDiam_B(p), SpAD_B(p), SpMAD_B(p), Ho_B(p), EE_B(p), SM1_B(p), SM2_B(p), SM3_B(p), SM4_B(p), SM5_B(p), SM6_B(p), VE1_B(p), VE2_B(p), VE3_B(p), VE1sign_B(p), VE2sign_B(p), VE3sign_B(p), VR1_B(p), VR2B(p), VR3_B(p), Wi_B(i), WiA_B(i), AVS_B(i), Chi_B(i), ChiA_B(i), J_B(i), HyWi_B(i), SpAbs_B(i), SpPos_B(i), SpPosA_B(i), SpPosLog_B(i), SpMax_B(i), SpMaxA_B(i), SpDiam_B(i), SpAD_B(i), SpMAD_B(i), Ho_B(i), EE_B(i), SM1_B(i), SM2_B(i), SM3_B(i), SM4_B(i), SM5_B(i), SM6_B(i), VE1_B(i), VE2_B(i), VE3_B(i), VE1sign_B(i), VE2sign_B(i), VE3sign_B(i), VR1_B(i), VR2 _B(i), VR3_B(i), Wi_B(s), WiA_B(s), AVS_B(s), Chi_B(s), ChiA_B(s), J_B(s), HyWi_B(s), SpAbs_B(s), SpPos_B(s), SpPosA_B(s), SpPosLog_B(s), SpMax_B(s), SpMaxA_B(s), SpDiam_B(s), SpAD_B(s), SpMAD_B(s), Ho_B(s), EE_B(s), SM1_B(s), SM2_B(s), SM3_B(s), SM4_B(s), SM5_B(s), SM6_B(s), VE1_B(s), VE2_B(s), VE3_B(s), VE1sign_B(s), VE2sign_B(s), VE3sign_B(s), VR1_B(s), VR2_B(s), VR3_B(s), ATS1m, ATS2m, ATS3m, ATS4m, ATS5m, ATS6m, ATS7m, ATS8m, ATS1v, ATS2v, ATS3v, ATS4v, ATS5v, ATS6v, ATS7v, ATS8v, ATS1e, ATS2e, ATS3e, ATS4e, ATS5e, ATS6e, ATS7e, ATS8e, ATS1p, ATS2p, ATS3p, ATS4p, ATS5p, ATS6p, ATS7p, ATS8p, ATS1i, ATS2i, ATS3i, ATS4i, ATS5i, ATS6i, ATS7i, ATS8i, ATS1s, ATS2s, ATS3s, ATS4s, ATS5s, ATS6s, ATS7s, ATS8s, ATSC1m, ATSC2m, ATSC3m, ATSC4m, ATSC5m, ATSC6m, ATSC7m, ATSC8m, ATSC1v, ATSC2v, ATSC3v, ATSC4v, ATSC5v, ATSC6v, ATSC7v, ATSC8v, ATSC1e, ATSC2e, ATSC3e, ATSC4e, ATSC5e, ATSC6e, ATSC7e, ATSC8e, ATSC1p, ATSC2p, ATSC3p, ATSC4p, ATSC5p, ATSC6p, ATSC7p, ATSC8p, ATSC1i, ATSC2i, ATSC3i, ATSC4i, ATSC5i, ATSC6i, ATSC7i, ATSC8i, ATSC1s, ATSC2s, ATSC3s, ATSC4s, ATSC5s, ATSC6s, ATSC7s, ATSC8s, MATS1m, MATS2m, MATS3m, MATS4m, MATS5m, MATS6m, MATS7m, MATS8m, MATS1v, MATS2v, MATS3v, MATS4v, MATS5v, MATS6v, MATS7v, MATS8v, MATS1e, MATS2e, MATS3e, MATS4e, MATS5e, MATS6e, MATS7e, MATS8e, MATS1p, MATS2p, MATS3p, MATS4p, MATS5p, MATS6p, MATS7p, MATS8p, MATS1i, MATS2i, MATS3i, MATS4i, MATS5i, MATS6i, MATS7i, MATS8i, MATS1s, MATS2s, MATS3s, MATS4s, MATS5s, MATS6s, MATS7s, MATS8s, GATS1m, GATS2m, GATS3m, GATS4m, GATS5m, GATS6m, GATS7m, GATS8m, GATS1v, GATS2v, GATS3v, GATS4v, GATS5v, GATS6v, GATS7v, GATS8v, GATS1e, GATS2e, GATS3e, GATS4e, GATS5e, GATS6e, GATS7e, GATS8e, GATS1p, GATS2p, GATS3p, GATS4p, GATS5p, GATS6p, GATS7p, GATS8p, GATS1i, GATS2i, GATS3i, GATS4i, GATS5i, GATS6i, GATS7i, GATS8i, GATS1s, GATS2s, GATS3s, GATS4s, GATS5s, GATS6s, GATS7s, GATS8s, GGI1, GGI2, GGI3, GGI4, GGI5, GGI6, GGI7, GGI8, GGI9, GGI10, JGI1, JGI2, JGI3, JGI4, JGI5, JGI6, JGI7, JGI8, JGI9, JGI10, JGT, SpMax1_Bh(m), SpMax2_Bh(m), SpMax3 _Bh(m), SpMax4 _Bh(m), SpMax5_Bh(m), SpMax6_Bh(m), SpMax7_Bh(m), SpMax8_Bh(m), SpMax1_Bh(v), SpMax2_Bh(v), SpMax3_Bh(v), SpMax4_Bh(v), SpMax5_Bh(v), SpMax6_Bh(v), SpMax7_Bh(v), SpMax8_Bh(v), SpMax1_Bh(e), SpMax2_Bh(e), SpMax3_Bh(e), SpMax4_Bh(e), SpMax5_Bh(e), SpMax6 _Bh(e), SpMax7_Bh(e), SpMax8 _Bh(e), SpMax1_Bh(p), SpMax2_Bh(p), SpMax3 _Bh(p), SpMax4 _Bh(p), SpMax5 _Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i),

SpMax8_Bh(i), SpMax1_Bh(s), SpMax2_Bh(s), SpMax3_Bh(s), SpMax4_Bh(s), SpMax5_Bh(s), SpMax6_Bh(s), SpMax7_Bh(s), SpMax8_Bh(s), SpMin1_Bh(m), SpMin2_Bh(m), SpMin3_Bh(m), SpMin4_Bh(m), SpMin5_Bh(m), SpMin6_Bh(m), SpMin7_Bh(m), SpMin8_Bh(m), SpMin1_Bh(v), SpMin2_Bh(v), SpMin3_Bh(v), SpMin4_Bh(v), SpMin5_Bh(v), SpMin6_Bh(v), SpMin7_Bh(v), SpMin8_Bh(v), SpMin1_Bh(e), SpMin2_Bh(e), SpMin3_Bh(e), SpMin4_Bh(e), SpMin5_Bh(e), SpMin6_Bh(e), SpMin7_Bh(e), SpMin8_Bh(e), SpMin1_Bh(p), SpMin2_Bh(p), SpMin3_Bh(p), SpMin4_Bh(p), SpMin5_Bh(p), SpMin6_Bh(p), SpMin7_Bh(p), SpMin8_Bh(p), SpMin1_Bh(i), SpMin2_Bh(i), SpMin3_Bh(i), SpMin4_Bh(i), SpMin5_Bh(i), SpMin6_Bh(i), SpMin7_Bh(i), SpMin8_Bh(i), SpMin1_Bh(s), SpMin2_Bh(s), SpMin3_Bh(s), SpMin4_Bh(s), SpMin5_Bh(s), SpMin6_Bh(s), SpMin7_Bh(s), SpMin8_Bh(s), P_VSA_LogP_1, P_VSA_LogP_2, P_VSA_LogP_3, P_VSA_LogP_4, P_VSA_LogP_5, P_VSA_LogP_6, P_VSA_LogP_7, P_VSA_LogP_8, P_VSA_MR_1, P_VSA_MR_2, P_VSA_MR_3, P_VSA_MR_4, P_VSA_MR_5, P_VSA_MR_6, P_VSA_MR_7, P_VSA_MR_8, P_VSA_m_1, P_VSA_m_2, P_VSA_m_3, P_VSA_m_4, P_VSA_v_2, P_VSA_v_3, P_VSA_e_2, P_VSA_e_3, P_VSA_e_4, P_VSA_e_5, P_VSA_p_1, PVSA_p2, P_VSA_i_1, P_VSA_i_2, P_VSA_i_3, P_VSA_i_4, P_VSA_s_2, P_VSA_s_3, P_VSA_s_4, P_VSA_s_5, P_VSA_s_6, P_VSA_ppp_L, P_VSA_ppp_P, P_VSA_ppp_N, P_VSA_ppp_D, P_VSA_ppp_A, P_VSA_ppp_ar, P_VSA_ppp_con, P_VSA_ppp_hal, P_VSA_ppp_cyc, P_VSA_app_ter, Eta_alpha, Eta_alpha A, Eta_epsi, Eta_epsi_A, Eta_betaS, Eta_betaS_A, Eta_betaP, Eta_betaP_A, Eta_beta, Eta_beta_A, Eta_C, Eta_C_A, Eta_L, Eta_L_A, Eta_F, Eta_F_A, Eta_FL, Eta_FL_A, Eta_B, Eta_B_A, Eta_sh_p, Eta_sh_y, Eta_sh_x, Eta_D_AlphaA, Eta_D_AlphaB, Eta_epsi_2, Eta_epsi_3, Eta_epsi_4, Eta_epsi_5, Eta_D_epsiA, Eta_D_epsiB, Eta_D_epsiC, Eta_D_epsiD, Eta_psi1, Eta_D_psiA, Eta_D_beta, Eta_D_beta_A, SpMax_EA, SpMaxA_EA, SpDiam_EA, SpAD_EA, SpMAD_EA, SpMax_EA(ed), SpMaxA_EA(ed), SpDiam_EA(ed), SpAD_EA(ed), SpMAD_EA(ed), SpMax_EA(bo), SpMaxA_EA(bo), SpDiam_EA(bo), SpAD_EA(bo), SpMAD_EA(bo), SpMaxEA(dm), SpMaxA_EA(dm), SpDiam_EA(dm), SpAD_EA(dm), SpMAD_EA(dm), SpMax_EA(ri), SpMaxA_EA(ri), SpDiam_EA(ri), SpAD_EA(ri), SpMAD_EA(ri), SpMax_AEA(ed), SpMaxA_AEA(ed), SpDiam_AEA(ed), SpAD_AEA(ed), SpMAD_AEA(ed), SpMax_AEA(bo), SpMaxA_AEA(bo), SpDiam_AEA(bo), SpADAEA(bo), SpMAD_AEA(bo), SpMax_AEA(dm), SpMaxA_AEA(dm), SpDiam_AEA(dm), SpAD_AEA(dm), SpMAD_AEA(dm), SpMax_AEA(ri), SpMaxA_AEA(ri), SpDiam_AEA(ri), SpAD_AEA(ri), SpMAD AEA(ri), Chi0_EA, Chi1_EA, Chi0_EA(ed), Chi1_EA(ed), Chi0_EA(bo), Chi1_EA(bo), Chi0_EA(dm), Chi1_EA(dm), Chi0_EA(ri), Chi1_EA(ri), SM02_EA, SM03_EA, SM04_EA, SM05_EA, SM06_EA, SM07_EA, SM08_EA, SM09_EA, SM10_EA, SM11_EA, SM12_EA, SM13_EA, SM14_EA, SM15_EA, SM02_EA(ed), SM03_EA(ed), SM04_EA(ed), SM05_EA(ed), SM06_EA(ed), SM07_EA(ed), SM08_EA(ed), SM09_EA(ed), SM10_EA(ed), SM11_EA(ed), SM12_EA(ed), SM13_EA(ed), SM14_EA(ed), SM15_EA(ed), SM02_EA(bo), SM03_EA(bo), SM04_EA(bo), SM05_EA(bo), SM06_EA(bo), SM07_EA(bo), SM08_EA(bo), SM09_EA(bo), SM10_EA(bo), SM11_EA(bo), SM12_EA(bo), SM13_EA(bo), SM14_EA(bo), SM15_EA(bo), SM02_EA(dm), SM03_EA(dm), SM04_EA(dm), SM05_EA(dm), SM06_EA(dm), SM07_EA(dm), SM08_EA(dm), SM09_EA(dm), SM10_EA(dm), SM11_EA(dm), SM12_EA(dm), SM13_EA(dm), SM14_EA(dm), SM15_EA(dm), SM02_EA(ri), SM03_EA(ri), SM04_EA(ri), SM05_EA(ri), SM06_EA(ri), SM07_EA(ri), SM08_EA(ri), SM09_EA(ri), SM10_EA(ri), SM11_EA(ri), SM12_EA(ri), SM13_EA(ri), SM14_EA(ri), SM15_EA(ri), SM02_AEA(ed), SM03_AEA(ed), SM04_AEA(ed), SM05_AEA(ed), SM06_AEA(ed), SM07_AEA(ed), SM08_AEA(ed), SM09_AEA(ed), SM10_AEA(ed), SM11_AEA(ed), SM12_AEA(ed), SM13_AEA(ed), SM14_AEA(ed), SM15_AEA(ed), SM02_AEA(bo), SM03_AEA(bo), SM04_AEA(bo), SM05_AEA(bo), SM06_AEA(bo), SM07_AEA(bo), SM08_AEA(bo), SM10_AEA(bo), SM11_AEA(bo), SM12_AEA(bo), SM13_AEA(bo), SM14_AEA(bo), SM15_AEA(bo), SM02_AEA(dm), SM03_AEA(dm), SM04_AEA(dm), SM05_AEA(dm), SM06_AEA(dm), SM07_AEA(dm), SM08_AEA(dm), SM09_AEA(dm), SM11_AEA(dm), SM12_AEA(dm), SM13_AEA(dm), SM14_AEA(dm), SM15_AEA(dm), SM02_AEA(ri), SM03_AEA(ri), SM04_AEA(ri), SM05_AEA(ri), SM06_AEA(ri), SM07_AEA(ri), SM08_AEA(ri), SM09_AEA(ri), SM10_AEA(ri), SM12_AEA(ri), SM13_AEA(ri), SM14_AEA(ri), SM15_AEA(ri), Eig06_EA, Eig11_EA, Eig14_EA, Eig05_EA(ed), Eig10_EA(ed), Eig13_EA(ed), Eig14_EA(ed), Eig02_EA(bo), Eig05_EA(bo), Eig06_EA(bo), Eig07_EA(bo), Eig08_EA(bo), Eig09_EA(bo), Eig10_EA(bo), Eig11_EA(bo), Eig12_EA(bo), Eig13_EA(bo), Eig14_EA(bo), Eig15_EA(bo), Eig01_EA(dm), Eig02_EA(dm), Eig03_EA(dm), Eig04_EA(dm), Eig05_EA(dm), Eig06_EA(dm), Eig07_EA(dm), Eig08_EA(dm), Eig09_EA(dm), Eig10_EA(dm), Eig11_EA(dm), Eig12_EA(dm), Eig13_EA(dm), Eig14_EA(dm), Eig02_EA(ri), Eig03_EA(ri), Eig04_EA(ri), Eig05_EA(ri), Eig06_EA(ri), Eig07_EA(ri), Eig08_EA(ri), Eig09_EA(ri), Eig10_EA(ri), Eig11_EA(ri), Eig12_EA(ri), Eig13_EA(ri), Eig14_EA(ri), Eig15_EA(ri), Eig01_AEA(ed), Eig02_AEA(ed), Eig03_AEA(ed), Eig04_AEA(ed), Eig05_AEA(ed), Eig06_AEA(ed), Eig07_AEA(ed), Eig08_AEA(ed), Eig09_AEA(ed), Eig10_AEA(ed), Eig11_AEA(ed), Eig12_AEA(ed), Eig13_AEA(ed), Eig14_AEA(ed), Eig15_AEA(ed), Eig02AEA(bo), Eig03_AEA(bo), Eig04_AEA(bo), Eig05_AEA(bo), Eig06_AEA(bo), Eig07_AEA(bo), Eig08_AEA(bo), Eig09AEA(bo), Eig10_AEA(bo), Eig11_AEA(bo), Eig12_AEA(bo), Eig13_AEA(bo), Eig14_AEA(bo), Eig15_AEA(bo), Eig01_AEA(dm), Eig02_AEA(dm), Eig03_AEA(dm), Eig04_AEA(dm), Eig05_AEA(dm), Eig06_AEA(dm), Eig07_AEA(dm), Eig08_AEA(dm), Eig09_AEA(dm), Eig10_AEA(dm), Eig11_AEA(dm), Eig12_AEA(dm), Eig13_AEA(dm), Eig14_AEA(dm), Eig15_AEA(dm), Eig02_AEA(ri), Eig03_AEA(ri), Eig04_AEA(ri), Eig05_AEA(ri), Eig06_AEA(ri), Eig07_AEA(ri), Eig08_AEA(ri), Eig09_AEA(ri), Eig10_AEA(ri), Eig11_AEA(ri), Eig12_AEA(ri), Eig13_AEA(ri), Eig14_AEA(ri), Eig15_AEA(ri), nCp, nCs,

nCt, nCq, nCrs, nCrt, nCrq, nCar, nCbH, nCb-, nCconj, nR=Ct, nRCOOH, nRCOOR, nRCONHR, nArCONHR, nRCONR2, nArCONR2, nCONN, nN=C-N<, nRNH2, nRNHR, nRNR2, nArNR2, nN(CO)2, nROH, nOHs, nOHt, nROR, nArOR, nSO, nArX, nPyrrolidines, nImidazoles, nThiophenes, nPyridines, nHDon, nHAcc, C-001, C-002, C-003, C-005, C-006, C-007, C-008, C-009, C-011, C-024, C-025, C-026, C-027, C-028, C-029, C-033, C-034, C-035, C-040, C-041, C-042, C-044, H-046, H-047, H-048, H-049, H-050, H-051, H-052, H-053, H-054, O-056, O-058, O-059, O-060, N-067, N-068, N-072, N-073, N-074, N-075, S-107, S-109, SsCH3, SssCH2, SaaCH, SsssCH, StsC, SdssC, SaasC, SaaaC, SssssC, SsNH2, SssNH, SsssN, SdsN, SaaN, StN, SaasN, SaaNH, SsOH, SdO, SssO, SaaS, SdssS, SsF, SsCl, NsCH3, NssCH2, NaaCH, NsssCH, NdssC, NaasC, NaaaC, NssssC, NssNH, NsssN, NdsN, NaaN, NtN, NaasN, NaaNH, NdO, NssO, NdssS, CATS2D_00_DD, CATS2D_03_DD, CATS2D_05_DD, CATS2D_06_DD, CATS2D_08_DD, CATS2D_09_DD, CATS2D_02_DA, CATS2D_03_DA, CATS2D_04_DA, CATS2D_05_DA, CATS2D_06_DA, CATS2D_07_DA, CATS2D_08_DA, CATS2D_09_DA, CATS2D_03_DP, CATS2D_06_DP, CATS2D_02_DN, CATS2D_04_DN, CATS2D_05_DN, CATS2D_02_DL, CATS2D_03_DL, CATS2D_04_DL, CATS2D_05_DL, CATS2D_06_DL, CATS2D_07_DL, CATS2D_08_DL, CATS2D_09_DL, CATS2D_00_AA, CATS2D_02_AA, CATS2D_03_AA, CATS2D_04_AA, CATS2D_05_AA, CATS2D_06_AA, CATS2D_07_AA, CATS2D_08_AA, CATS2D_09_AA, CATS2D_02_AP, CATS2D_03_AP, CATS2D_04_AP, CATS2D_05_AP, CATS2D_06_AP, CATS2D_08_AP, CATS2D_09_AP, CATS2D_04_AN, CATS2D_05_AN, CATS2D_07_AN, CATS2D_08_AN, CATS2D_02_AL, CATS2D_03_AL, CATS2D_04_AL, CATS2D_05_AL, CATS2D_06_AL, CATS2D_07_AL, CATS2D_08_AL, CATS2D_09_AL, CATS2D_02_PN, CATS2D_04_PN, CATS2D_02_PL, CATS2D_03_PL, CATS2D_04_PL, CATS2D_05_PL, CATS2D_07_PL, CATS2D_08_PL, CATS2D_09_PL, CATS2D_00_NN, CATS2D_01_NL, CATS2D_02_NL, CATS2D_03_NL, CATS2D_04_NL, CATS2D_05_NL, CATS2D_06_NL, CATS2D_07_NL, CATS2D_08_NL, CATS2D_00_LL, CATS2D_01_LL, CATS2D_02_LL, CATS2D_03_LL, CATS2D_04_LL, CATS2D_05_LL, CATS2D_06_LL, CATS2D_07_LL, CATS2D_08_LL, CATS2D_09_LL, SHED_DD, SHED_DA, SHED_DP, SHED_DN, SHED_DL, SHED_AA, SHED_AP, SHED_AN, SHED_AL, SHED_PN, SHED_PL, SHED_NN, SHED_NL, SHED_LL, T(N..N), T(N..O), T(N..S), T(N..F), T(N..Cl), T(O..O), T(O..S), T(O..Cl), B01[C-O], B01[C-F], B01[O-S], B02[C-F], B02[N-N], B02[N-O], B02[N-S], B02[O-O], B03[N-N], B03[N-O], B03[N-S], B03[O-O], B04[C-S], B04[C-F], B04[N-N], B04[N-O], B04[N-S], B04[O-O], B04[O-S], B05[C-C], B05[C-O], B05[C-S], B05[C-F], B05[N-N], B05[N-O], B05[N-S], B05[O-O], B05[O-S], B05[O-Cl], B06[C-C], B06[C-N], B06[C-O], B06[C-F], B06[N-N], B06[N-O], B06[O-O], B07[C-C], B07[C-N], B07[C-O], B07[C-S], B07[C-F], B07[N-N], B07[N-O], B07[N-S], B07[O-O], B07[O-S], B08[C-C], B08[C-N], B08[C-O], B08[C-S], B08[N-N], B08[N-O], B08[O-O], B09[C-C], B09[C-N], B09[C-O], B09[C-S], B09[C-F], B09[C-Cl], B09[N-N], B09[N-O], B09[O-O], B10[C-C], B10[C-N], B10[C-O], B10[N-N], B10[N-O], B10[O-O], F01[C-C], F01[C-N], F01[C-O], F01[C-S], F01[O-S], F02[C-C], F02[C-N], F02[C-O], F02[C-S], F02[C-F], F02[N-N], F02[N-O], F02[N-S], F02[O-O], F03[C-C], F03[C-N], F03[C-O], F03[C-S], F03[C-Cl], F03[N-N], F03[N-O], F03[O-O], F04[C-C], F04[C-N], F04[C-O], F04[C-S], F04[C-Cl], F04[N-N], F04[N-O], F04[N-S], F04[O-O], F04[O-S], F05[C-C], F05[C-N], F05[C-O], F05[C-S], F05[C-F], F05[C-Cl], F05[N-N], F05[NO], F05[N-S], F05[O-O], F05[O-Cl], F06[C-C], F06[C-N], F06[C-O], F06[C-S], F06[C-F], F06[C-Cl], F06[N-N], F06[N-O], F06[O-O], F07[C-C], F07[C-N], F07[C-O], F07[C-S], F07[C-F], F07[C-Cl], F07[N-N], F07[N-O], F07[O-O], F07[O-S], F08[C-C], F08[C-N], F08[C-O], F08[C-S], F08[C-Cl], F08[N-N], F08[N-O], F08[O-O], F09[C-C], F09[C-N], F09[C-O], F09[C-S], F09[C-Cl], F09[N-N], F09[N-O], F09[O-O], F10[C-C], F10[C-N], F10[C-O], F10[N-N], F10[N-O], F10[O-O], Uc, Ui, Hy, TPSA(NO), TPSA(Tot), MLOGP, MLOGP2, SAtot, SAacc, VvdwMG, VvdwZAZ, PDI, BLTD48, BLTA96, Ro5, DLS_01, DLS_02, DLS_03, DLS_04, DLS_05, DLS_06, DLS_07, DLS_cons, LLS_01, LLS_02.

The above-mentioned descriptors can be classified into constitutional indices, Ring descriptors, topological indices, walk and path counts, connectivity indices, information indices, 2D matrix-based descriptors, 2D autocorrelations, Burden eigenvalues, P_VSA-like descriptors, ETA indices, edge adjacency indices, geometrical descriptors, 3D matrix-based descriptors, 3D autocorrelations, RDF descriptors, 3D-MoRSE descriptors, WHIM descriptors, GETAWAY descriptors, Randic molecular profiles, functional group counts, atom-centered fragments, atom-type E-state indices, pharmacophore descriptors, 2D Atom Pairs, 3D Atom Pairs, charge descriptors, molecular properties, drug-like indices, and CATS 3D descriptors. All variables for the above-mentioned descriptors may be used, or a combination of variables for any optionally selected descriptor may be used. When a variable for a plurality of descriptors is used, in terms of selecting descriptors with high explanatory power for a critical degree of supersaturation, a component (principal component) obtained by statistical processing such as principal component analysis, and descriptors selected by least absolute shrinkage and selection operator (LASSO) regression, genetic algorithm, variable importance in random forest, Boruta, forward selection, backward elimination, stepwise, and a value of variable importance in projection (VIP) in partial least squares regression (PLSR) can be used as a variable. As the descriptor on a compound, it is possible to use, as an example, a variable for at least one descriptor belonging to descriptors belonging to 2D autocorrelations, P_VSA-like descriptors, drug-like indices, edge adjacency indices, topological indices, and Burden eigenvalues. Particularly, when descriptors belonging to 2D autocorrelations are used and/or both of descriptors belonging to drug-like indices and descriptors belonging to edge adjacency indices are used, a model with high prediction accuracy can be built. Using descriptors with a value of 0.50 or more in Table 10, for example, 2D autocorrelations or edge adjacency indices, or a

combination of descriptors with a value of 0.50 or more, for example, 2D autocorrelations and P_VSA-like descriptors, 2D autocorrelations and drug-like indices, 2D autocorrelations and edge adjacency indices, 2D autocorrelations and Burden eigenvalues, 2D autocorrelations and topological indices, 2D autocorrelations and others, PVSA-like descriptors and edge adjacency indices, drug-like indices and edge adjacency indices, drug-like indices and Burden eigenvalues, drug-like indices and topological indices, edge adjacency indices and Burden eigenvalues, or edge adjacency indices and topological indices, a model can be produced. In terms of building a model with higher prediction accuracy, preferably using descriptors with a value of 0.65 or more in Table 10, for example, 2D autocorrelations, or a combination of descriptors with a value of 0.65 or more, for example, 2D autocorrelations and P_VSA-like descriptors, 2D autocorrelations and drug-like indices, 2D autocorrelations and edge adjacency indices, 2D autocorrelations and Burden eigenvalues, 2D autocorrelations and topological indices, 2D autocorrelations and others, or drug-like indices and edge adjacency indices, a model can be produced.

More specifically, it is possible to use a variable for at least one descriptor selected from the group consisting of the following descriptors:

MATS5i, SM03_EA(dm), P_VSA_MR_6, MAXDP, MATS6m, DLS_04, P_VSA_s_3, GATS8s, ATSC1e, P_YSA_MR_8, GATS5i, SM13_AEA(ri), MATS2s, P_VSA_LogP_2, SpMax1_Bh(m) and SpMax1_Bh(p)

or for descriptors including descriptors having the substantially same content.

These descriptors specifically have the following meanings:

[Table 1]

Table 1

| Descriptor | Explanation |
|---|---|
| MATS5i | Moran autocorrelation of lag 5 weighted by ionization potential |
| SM03_EA(dm) | Spectral moment of order 3 from edge adjacency mat. weighted by dipole moment |
| P_VSA_MR_6 | P VSA-like on Molar Refractivity, bin 6: $3.0 \leq MR < 4.0$ |
| MAXDP | Maximal electrotopological positive variation |
| MATS6m | Moran autocorrelation of lag 6 weighted by mass |
| DLS 04 | Modified drug-like score from Chen et al. (7 rules) |
| P_VSA_s_3 | P VSA-like on I-state (s), bin 3: $1.5 \leq s < 2.0$ |
| GATS8s | Geary autocorrelation of lag 8 weighted by I-state |
| ATSCle | Centered Broto-Moreau autocorrelation of lag 1 weighted by Sanderson electronegativity |
| P_VSA_MR_8 | P VSA-like on Molar Refractivity, bin 8: $6.0 \leq MR < +\infty$ |
| GATS5i | Geary autocorrelation of lag 5 weighted by ionization potential |
| SM13_AEA(ri) | Spectral moment of order 13 from augmented edge adjacency mat. weighted by resonance integral |
| MATS2s | Moran (Geary) autocorrelation of lag 2 weighted by I-state |
| P_VSA_LogP_2 | P_VSA-like on octanol/water partition coefficient (LogP), bin 2: $-1.5 \leq logP < -0.5$ |
| SpMax1_ Bh(m) | Largest eigenvalue n. 1 of Burden matrix weighted by mass |
| SpMax1_ Bh(p) | Largest eigenvalue n. 1 of Burden matrix weighted by polarizability |

As the variable for a solvent, it is possible to use, as an example, one or a plurality of variables for descriptors selected from the group consisting of the following descriptors (373 types) obtained by calculation based on the molecule structure:

MW, AMW, Sv, Se, Sp, Si, Mv, Me, Mp, Mi, GD, RBF, H%, C%, O%, MCD, ZM1Kup, ZM1Mad, ZM1Per, ZM1MulPer, ZM2Kup, ZM2Mad, ZM2Per, ZM2MulPer, ON0, ON0V, ON1, ON1V, DBI, SNar, HNar, GNar, Xt, Dz, LPRS, MSD, SPI, AECC, DECC, MDDD, ICR, MeanTD, MeanDD, S1K, S2K, S3K, PHI, PW2, PW3, PW4, PW5, MAXDN, MAXDP, DELS, LOC, MWC01, MWC02, MWC03, MWC04, MWC05, MWC06, MWC07, MWC08, MWC09, MWC10, SRW02, SRW04, SRW06, SRW08, SRW10, MPC01, MPC02, MPC03, MPC04, MPC05, piPC01, piPC02, piPC03, piPC04, piPC05, TWC, TPC, piID, PCD, CID, BID, ISIZ, IAC, AAC, IDE, IDM, IDDE, IDDM, IDET, IDMT, IVDE, IVDM, HVcpx, HDcpx, Uindex, Vindex, Xindex, Yindex, IC0, IC1, IC2, IC3, IC4, IC5, TIC0, TIC1, TIC2, TIC3, TIC4, TIC5, SIC0, SIC1, SIC2, SIC3, SIC4, SIC5, CIC0, CIC1, CIC2, CIC3, CIC4, CIC5, BIC0, BIC1, BIC2, BIC3, BIC4, BIC5, ATS1m, ATS2m, ATS3m, ATS4m, ATS5m, ATS6m, ATS1v, ATS2v, ATS3v, ATS4v, ATS5v, ATS6v, ATS1e, ATS2e, ATS3e, ATS4e, ATS5e,

ATS6e, ATS1p, ATS2p, ATS3p, ATS4p, ATS5p, ATS6p, ATS1i, ATS2i, ATS3i, ATS4i, ATS5i, ATS6i, ATSC1m, ATSC2m, ATSC3m, ATSC4m, ATSC5m, ATSC6m, ATSC1v, ATSC2v, ATSC3v, ATSC4v, ATSC5v, ATSC6v, ATSC1e, ATSC2e, ATSC3e, ATSC4e, ATSC5e, ATSC6e, ATSC1p, ATSC2p, ATSC3p, ATSC4p, ATSC5p, ATSC6p, ATSC1i, ATSC2i, ATSC3i, ATSC4i, ATSC5i, ATSC6i, MATS1m, MATS2m, MATS3m, MATS4m, MATS5m, MATS6m, MATS1v, MATS2v, MATS3v, MATS4v, MATS5v, MATS6v, MATS1e, MATS2e, MATS3e, MATS4e, MATS5e, MATS6e, MATS1p, MATS2p, MATS3p, MATS4p, MATS5p, MATS6p, MATS1i, MATS2i, MATS3i, MATS4i, MATS5i, MATS6i, GATS1m, GATS2m, GATS3m, GATS4m, GATS5m, GATS1v, GATS2v, GATS3v, GATS4v, GATS5v, GATS1e, GATS2e, GATS3e, GATS4e, GATS5e, GATS1p, GATS2p, GATS3p, GATS4p, GATS5p, GATS1i, GATS2i, GATS3i, GATS4i, GATS5i, GGI1, GGI2, GGI3, JGI1, JGI2, JGI3, JGT, SpMax1_Bh(m), SpMax2_Bh(m), SpMax3_Bh(m), SpMax4_Bh(m), SpMax5_Bh(m), SpMax6_Bh(m), SpMax7_Bh(m), SpMax8_Bh(m), SpMax1_Bh(v), SpMax2_Bh(v), SpMax3_Bh(v), SpMax4_Bh(v), SpMax5_Bh(v), SpMax6_Bh(v), SpMax7_Bh(v), SpMax1_Bh(e), SpMax2_Bh(e), SpMax3_Bh(e), SpMax4_Bh(e), SpMax5_Bh(e), SpMax6_Bh(e), SpMax7_Bh(e), SpMax8_Bh(e), SpMax1_Bh(p), SpMax2_Bh(p), SpMax3_Bh(p), SpMax4_Bh(p), SpMax5_Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i), SpMax8_Bh(i), SpMin1_Bh(m), SpMin2_Bh(m), SpMin3_Bh(m), SpMin4_Bh(m), SpMin5_Bh(m), SpMin1_Bh(v), SpMin2_Bh(v), SpMin3_Bh(v), SpMin4_Bh(v), SpMin5_Bh(v), SpMin1_Bh(e), SpMin2_Bh(e), SpMin3_Bh(e), SpMin4_Bh(e), SpMin1_Bh(p), SpMin2_Bh(p), SpMin3_Bh(p), SpMin4_Bh(p), SpMin5_Bh(p), SpMin1_Bh(i), SpMin2_Bh(i), SpMin3_Bh(i), SpMin4 Bh(i), P VSA LogP_1, P_VSA_LogP_2, P_VSA_LogP_3, P_VSA_LogP_4, P_VSA_LogP_5, P_VSA_LogP_7, P_VSA_MR_1, P_VSA_MR_2, P_VSA_MR_3, P_VSA_MR_5, P_VSA_MR_6, P_VSA_m1, P_VSA_m_2, P_VSA_m_3, P_VSA_v_2, P_VSA_v_3, P_VSA_e_2, P_VSA_e_5, P_VSA_i_2, P_VSA_i_3, P_VSA_s_2, P_VSA_s_3, P_VSA_s_4, P_VSA_s_6, P_VSA_ppp_L, P_VSA_ppp_D, P_VSA_ppp_cyc, P_VSA_ppp_ter, SsCH3, SssCH2, SsssCH, SdssC, SsOH, SdO, SssO, SHED AL, SHED LL, Uc, Ui, Hy, AMR, TPSA(NO), TPSA(Tot), MLOGP2, ALOGP, ALOGP2, SAtot, SAdon, VvdwMG, VvdwZAZ, PDI, BLTF96, DLS_02, DLS_04, DLS_05, DLS_cons.

The above-mentioned descriptors can be classified into constitutional indices, Ring descriptors, topological indices, walk and path counts, connectivity indices, information indices, 2D matrix-based descriptors, 2D autocorrelations, Burden eigenvalues, P_VSA-like descriptors, ETA indices, edge adjacency indices, geometrical descriptors, 3D matrix-based descriptors, 3D autocorrelations, RDF descriptors, 3D-MoRSE descriptors, WHIM descriptors, GETAWAY descriptors, Randic molecular profiles, functional group counts, atom-centered fragments, atom-type E-state indices, pharmacophore descriptors, 2D Atom Pairs, 3D Atom Pairs, charge descriptors, molecular properties, drug-like indices, and CATS 3D descriptors. All variables for the above-mentioned descriptors may be used, or a combination of variables for any optionally selected descriptor may be used. When a variable for a plurality of descriptors is used, in terms of selecting descriptors with high explanatory power for a critical degree of supersaturation, a component (principal component) obtained by statistical processing such as principal component analysis, and descriptors selected by LASSO regression, genetic algorithm, variable importance in random forest, Boruta, forward selection, backward elimination, stepwise, and a value of VIP in PLSR can be used as a variable. As the descriptor on a solvent, it is possible to use, as an example, a variable for descriptors belonging to Burden eigenvalues and 2D autocorrelations.

More specifically, it is possible to use a variable for at least one descriptor selected from the group consisting of the following descriptors:

SpMax5_Bh(m), SpMax5_Bh(v) and MATS3v

or for descriptors including descriptors having the substantially same content. These descriptors specifically have the following meanings:

[Table 2]

Table 2

| Descriptor | Explanation |
|---|---|
| SpMax5_ Bh(m) | Largest eigenvalue n. 5 of Burden matrix weighted by mass |
| SpMax5 _Bh(v) | Largest eigenvalue n. 5 of Burden matrix weighted by van der Waals volume |
| MATS3v | Moran autocorrelation of lag 3 weighted by van der Waals volume |

The descriptor having the substantially same content refers to a descriptor represented by a different descriptor due to difference in software, etc. although it means a content which is the same as or similar to that of the above-mentioned descriptors. As an example, a descriptor which is ATSCle in alvaDesc is represented by ATSC1se in Mordred, but only the name of the descriptor is different, and the content is the same. Some software sometimes adopts a descriptor in which only a numerical value used for the definition of the descriptor is different even if it is a descriptor having the same definition. Therefore, a descriptor meaning a similar content to that of one descriptor is intended to include a

descriptor in which only a numerical value used for the definition is different. As an example, a descriptor which is P_VSA_logP_5 in alvaDesc is represented by SlogP_VSA4+MR_VSA5+MR_VSA6+MR_VSA7 in RDKit, but the domain of logP is the same. Since the atomic physical property has a different numerical value according to the definition but means the same physical quantity, the content is substantially the same. For example, in Mordred, ATSC1se and ATSC1pe have different numerical values based on Sanderson electronegativity and Pauling electronegativity, respectively, but the contents are the same.

In the step of predicting a critical degree of supersaturation, "solution temperature during crystallization" is a temperature when a crystal is precipitated in the step (2) (step of precipitating a spherulite of a compound from a supersaturated solution) of the method of the present invention.

The information based on the chemical structure is information based on the three-dimensional structure of a compound in the case of structure optimization as an example. As the information based on the three-dimensional structure, a captured image of a compound can be used. In other words, regarding a compound with a three-dimensional structure being built, a captured image from a plurality of directions is produced, and the produced image can be inputted as information on a compound and/or a solvent.

The three-dimensional structure of a compound may be produced using known software on a computer, or a structure determined by crystal structure analysis may be used. Regarding the chemical formula of the compound inputted, a three-dimensional structure may be built considering conditions such as a solvent, temperature, and pH, or a three-dimensional structure built not considering a part or all of these conditions may be used. One three-dimensional structure may be created for one compound, or a plurality of three-dimensional structures may be created considering a degree of freedom. Regarding the three-dimensional structure, the ball-and-stick representation in which an atom is represented by a sphere and a bond is represented by a bar may be used, or representation methods such as wireframe in which the structure is represented only by a bond, the spacefill representation in which space is packed with atoms, and the surface representation which represents a molecule surface which comes in contact with a solvent are used. In such representation method, it is preferable to represent the type of an atom by distinguishing by color, which can improve the prediction accuracy of the physical property of a compound, etc.

An image of a three-dimensional structure built on a computer can be taken by using a virtual camera on a computer. An image can be taken from a plurality of directions, and as an example, an image may be taken from each direction of the X-axis, the Y-axis, and the Z-axis, or an image may be taken by rotating each predetermined angle for each axis. A plurality of images taken by such a way can be inputted into a predictive model as information on a compound and/or a solvent.

In the step of predicting a critical degree of supersaturation, the input data may be data including or consisting of information on a compound obtained as a specifically-shaped crystal and information on a solvent used for crystallization, data including or consisting of information on a compound obtained as a specifically-shaped crystal and a solution temperature during crystallization, or data including or consisting of information on a compound obtained as a specifically-shaped crystal and information on a solvent used for crystallization and a solution temperature during crystallization. The input data are preferably data including information on a compound obtained as a specifically-shaped crystal and information on a solvent used for crystallization and a solution temperature during crystallization.

The step of predicting a critical degree of supersaturation is performed using an information processor. FIG. 39 is a diagram showing an example of a schematic block diagram of an information processor 100 used for prediction of a critical degree of supersaturation in the method of the present invention.

The information processor 100 is an information processor such as a personal computer, which is used by a user. The information processor 100 has a communication device 101, an input device 102, a display device 103, a storage device 110, and a central processing unit (CPU) 120. Each unit of the information processor 100 will be described in detail below.

The communication device 101 has a communication interface circuit to communicate with a network such as LAN. The communication device 101 transmits and receives data to/from an external server device (not shown) via a network. The communication device 101 supplies data received from the server device via the network to the CPU 120, and transmits data supplied from the CPU 120 to the server device via the network. The communication device 101 may be any type as long as it can communicate with an external device. The communication device 101 may receive input data used in the step of predicting a critical degree of supersaturation from an external server device, and supply them to the CPU 120. The communication device 101 may transmit a predictive value of a critical degree of supersaturation outputted from the CPU 120 to an external device.

The input device 102 is an example of an operation unit, and has input devices such as a touch panel input device, a keyboard, and a mouse, and an interface circuit which obtains a signal from the input devices. The input device 102 accepts an input by a user, and outputs a signal according to the input by the user to the CPU 120. The input data used in the step of predicting a critical degree of supersaturation may be inputted from the input device 102.

The display device 103 is an example of a display unit, and has a display composed of a liquid crystal, organic electro-luminescence (EL), and the like and an interface circuit which outputs image data or various types of information

to the display. The display device 103 is connected to the CPU 120, and displays a predictive value of a critical degree of supersaturation outputted from the CPU 120 on the display.

The storage device 110 is an example of a storage unit. The storage device 110 has memory devices such as random-access memory (RAM) and read-only memory (ROM), fixed disk devices such as a hard disk, or portable storage devices such as a flexible disk and an optical disk. The storage device 110 stores a computer program, a database, a table, and the like used for various types of processing of the information processor 100. The computer program may be installed from, for example, portable storage media which can be read by a computer such as compact disk read-only memory (CD-ROM) and digital versatile disk read-only memory (DVD-ROM). The computer program is installed on the storage device 110 using a known setup program and the like. The storage device stores a predictive model used in the step of predicting a critical degree of supersaturation and a parameter set to describe the predictive model.

The CPU 120 operates based on the program prestored in the storage device 110. The CPU 120 may be a general-purpose processor. In place of the CPU 120, digital signal processor (DSP), large scale integration (LSI), application specific integrated circuit (ASIC), field-programmable gate array (FPGA) or the like may be used.

The CPU 120 is connected to the communication device 101, the input device 102, the display device 103, and the storage device 110, and controls these respective units.

FIG. 40 and FIG. 91 are flow charts showing an example of the operation of the entire processing by the information processor 100.

With reference to the flow chart shown in FIG. 40 or FIG. 91, an example of the operation of the entire processing by the information processor 100 will be described below. The operation flow described below is run in collaboration with each element of the information processor 100 mainly by the CPU 120 based on the program prestored in the storage device 110.

First, preprocessing S100 is performed to produce information on a compound obtained as a specifically-shaped crystal and information of a solvent used for crystallization inputted into the input device 102. In one aspect, in the preprocessing S100, information on a compound obtained as a specifically-shaped crystal and information on a solvent used for crystallization are determined based on descriptors on and a mixing ratio of each of the compound and the solvent. In another aspect, in the preprocessing S100, for the three-dimensional structure of the compound and/or the solvent built on a computer, an image of the compound can be taken from a predetermined direction by using a virtual camera on a computer to obtain an image. The preprocessing step S100 may be performed in the information processor 100, or may be performed in advance in an information processor different from the information processor 100. When the preprocessing step S100 is performed in the information processor 100, information produced by the preprocessing S100 is stored in the storage device 110. When the preprocessing step S100 is performed in advance in an information processor different from the information processor 100, information produced by the preprocessing S100 is inputted into the information processor 100 via the input device 102 or the communication device 101.

Variables for descriptors on the compound and variables for descriptors on the solvent determined in the preprocessing S100 may be inputted as they are into a predictive model, or principal component analysis (PCA) may be performed for various variables which are explanatory variables when the predictive model is built. One or a plurality of principal components produced by the principal component analysis can be used as information.

Next, the CPU 120 accepts data including information on a compound obtained as a specifically-shaped crystal which was inputted by a user using the input device 102, or received by the communication device 101 from an external server device, or stored in the storage device 110, and at least one of information on a solvent used for crystallization and a solution temperature during crystallization (step S101).

Next, the CPU 120 calculates a predictive value of a critical degree of supersaturation (step S102). The CPU 120 calculates a predictive value of a critical degree of supersaturation by inputting the accepted data into a predictive model which is previously learned so as to output a critical degree of supersaturation for each of data when data including information on a compound obtained as a specifically-shaped crystal and at least one of information on a solvent used for crystallization and a solution temperature during crystallization are inputted.

Next, the CPU 120 displays the calculated predictive value of a critical degree of supersaturation on the display device 103 (step S103).

The predictive model is prestored in the storage device 110. For example, by performing partial least squares regression shown in Examples 18, 32, and/or 33 below, it is possible to obtain a set of weight coefficients for various types of information as a parameter set. It is also possible to obtain a predictive model showing the relationship between various variables which are explanatory variables and a critical degree of supersaturation. A method for building a predictive model is not limited to the method mentioned in Example 18, and, for example, the relationship between the inputted information and a critical degree of supersaturation may be learned using a known machine learning technique such as deep learning. Deep learning is machine learning using neural network with a multilayer structure composed of an input layer, an intermediate layer, and an output layer. Into each node of the input layer, data including information on a compound obtained as a specifically-shaped crystal and at least one of information on a solvent used for crystallization and a solution temperature during crystallization are inputted. Each node of the intermediate layer outputs the sum of

values obtained by multiplying each feature vector outputted from each node of the input layer by weight, and furthermore, the output layer outputs the sum of values obtained by multiplying each feature vector outputted from each node of the intermediate layer by weight. By prior learning, learning is performed so that the difference between output values from the output layer and a critical degree of supersaturation becomes small while adjusting each weight.

By preparing a supersaturated solution having a higher degree of supersaturation than the calculated predictive value of a critical degree of supersaturation, it is possible to reproducibly obtain a specifically-shaped crystal of a compound.

One embodiment of the method of the present invention is a method for producing a spherulite of a compound, which includes the following steps:

(1) a step of preparing a supersaturated solution of the compound having a degree of supersaturation equal to or higher than a critical degree of supersaturation required to obtain a crystal of the spherulite of the compound; and
(2) a step of precipitating a crystal including the spherulite of the compound from the supersaturated solution.

Here, the proportion of the spherulite in the crystal precipitated is, for example, more than 0% by weight, 0.1% by weight or more, 1% by weight or more, 5% by weight or more, 10% by weight or more, 20% by weight or more, 30% by weight or more, 40% by weight or more, 50% by weight or more, 60% by weight or more, 70% by weight or more, 80% by weight or more, 90% by weight or more, or 95% by weight or more based on weight. The proportion is preferably 50% by weight or more, more preferably 70% by weight or more, and particularly preferably 90% by weight or more. The proportion (based on weight) of the spherulite in the crystal precipitated can be calculated by, for example, classifying the crystals precipitated, and measuring each of the weight of the spherulite and the weight of the crystals other than the spherulite. The proportion of the spherulite in the crystal precipitated is, for example, more than 0%, 0.1% or more, 1% or more, 5% or more, 10% or more, 20% or more, 30% or more, 40% or more, 50% or more, 60% or more, 70% or more, 80% or more, 90% or more, or 95% or more based on number. The proportion is preferably 50% or more, more preferably 70% or more, and particularly preferably 90% or more. The proportion (based on number) of the spherulite in the crystal precipitated can be calculated using, for example, a dry distributed image analyzer, etc.

One embodiment of the method of the present invention is a method for producing a spherulite of azithromycin monohydrate, which includes the following steps:

(1) a step of dissolving azithromycin or a hydrate thereof in a water-miscible organic solvent (e.g., lower alcohols such as methanol and ethanol, tetrahydrofuran, acetone, and a mixed solvent thereof) to prepare a solution of azithromycin;
(2) a step of adding dropwise the solution prepared in the step (1) to water (for example, over 1 second to 3 hours) at 0 °C to 55 °C to prepare a supersaturated solution of azithromycin monohydrate; and
(3) a step of precipitating a spherulite of azithromycin monohydrate at 0 °C to 55 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 11, 14, 15, 16, 17, or 18 in Table 3 below are used, the solution has a degree of supersaturation equal to or higher than the actual measured value of the critical degree of supersaturation mentioned in No. 11, 14, 15, 16, 17, or 18, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of azithromycin monohydrate may be inoculated in a step between the steps (2) and (3). The volume ratio of the water-miscible organic solvent to water (water-miscible organic solvent:water) used in this production method is not particularly limited, and is preferably 1:0.1 to 1:100, and more preferably 1:1 to 1:20.

Another embodiment of the method of the present invention is a method for producing a spherulite of lansoprazole, which includes the following steps:

(1) a step of dissolving lansoprazole or a hydrate thereof in a water-miscible organic solvent (e.g., lower alcohols such as methanol and ethanol, tetrahydrofuran, acetone, and a mixed solvent thereof) to prepare a solution of lansoprazole;
(2) a step of adding dropwise the solution prepared in the step (1) to water (for example, over 1 second to 3 hours) at 0 °C to 55 °C to prepare a supersaturated solution of lansoprazole; and
(3) a step of precipitating a spherulite of lansoprazole at 0 °C to 55 °C.

The solution obtained in the step (2) of this production method has a degree of supersaturation equal to or

higher than a critical degree of supersaturation required to obtain the spherulite. For example, when the substrate, the solvent, the solvent ratio, and the crystallization temperature mentioned in No. 2 or 20 in Table 3 below are used, the solution has a degree of supersaturation equal to or higher than the actual measured value of the critical degree of supersaturation mentioned in No. 2 or 20, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of lansoprazole may be inoculated in a step between the steps (2) and (3). The volume ratio of the water-miscible organic solvent to water (water-miscible organic solvent:water) used in this production method is not particularly limited, and is preferably 1:0.1 to 1:100, and more preferably 1:1 to 1:20.

Another embodiment of the method of the present invention is a method for producing a spherulite of esomeprazole magnesium trihydrate, which includes the following steps:

(1) a step of dissolving esomeprazole magnesium or a hydrate thereof in a water-miscible organic solvent (e.g., lower alcohols such as methanol, ethanol, and 2-propanol; tetrahydrofuran, acetone, and a mixed solvent thereof) to prepare a solution of esomeprazole magnesium;
(2) a step of adding dropwise the solution prepared in the step (1) to water (for example, over 1 second to 3 hours) at 0 °C to 55 °C to prepare a supersaturated solution of esomeprazole magnesium trihydrate; and
(3) a step of precipitating a spherulite of esomeprazole magnesium trihydrate at 0 °C to 55 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 1 or 19 in Table 3 below are used, the solution has a degree of supersaturation equal to or higher than the actual measured value of the critical degree of supersaturation mentioned in No. 1 or 19, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of esomeprazole magnesium trihydrate may be inoculated in a step between the steps (2) and (3). The volume ratio of the water-miscible organic solvent to water (water-miscible organic solvent: water) used in this production method is not particularly limited, and is preferably 1:0.1 to 1:100, and more preferably 1:1 to 1:20.

Another embodiment of the method of the present invention is a method for producing a spherulite of esomeprazole magnesium trihydrate, which includes the following steps:

(1) a step of dissolving esomeprazole potassium in a water-miscible organic solvent (e.g., lower alcohols such as methanol, ethanol, and 2-propanol; tetrahydrofuran, acetone, and a mixed solvent thereof) to prepare a solution of esomeprazole potassium;
(2) a step of adding dropwise an aqueous magnesium chloride solution to the solution prepared in the step (1) at 0 °C to 55 °C to prepare a supersaturated solution of esomeprazole magnesium trihydrate;
(3) a step of filtering the supersaturated solution prepared in the step (2); and
(4) a step of precipitating a spherulite of esomeprazole magnesium trihydrate at 0 °C to 55 °C.

The solution obtained in the step (2) of this production method has a degree of supersaturation equal to or higher than a critical degree of supersaturation required to obtain the spherulite. In this production method, a seed crystal of esomeprazole magnesium trihydrate may be inoculated in a step between the steps (3) and (4). The volume ratio of the water-miscible organic solvent to water (water-miscible organic solvent:water) used in this production method is not particularly limited, and is preferably 1:0.1 to 1:100, and more preferably 1:1 to 1:20.

This production method preferably further includes (5) a step of adding dropwise an aqueous magnesium chloride solution at 0 °C to 55 °C to prepare a supersaturated solution of esomeprazole magnesium and to simultaneously grow a spherulite of esomeprazole magnesium trihydrate. The amount of magnesium chloride used in the step (2) and the amount of magnesium chloride used in the step (5) can be appropriately adjusted.

Another embodiment of the method of the present invention is a method for producing a spherulite of duloxetine hydrochloride, which includes the following steps:

(1) a step of mixing duloxetine with a water-miscible organic solvent (e.g., lower alcohols such as methanol, ethanol, and 2-propanol; tetrahydrofuran, acetone, and a mixed solvent thereof) and a surfactant (e.g., polyol ester) to prepare a solution of duloxetine;
(2) a step of adding dropwise a solvent (e.g., ethyl acetate, 1,4-dioxane, ethanol, and water) containing hydrogen

chloride to the solution prepared in the step (1) (for example, over 1 second to 1 hour) at 0 °C to 55 °C to prepare a supersaturated solution of duloxetine hydrochloride; and
(3) a step of precipitating a spherulite of duloxetine hydrochloride at 0 °C to 55 °C.

The solution obtained in the step (2) of this production method has a degree of supersaturation equal to or higher than a critical degree of supersaturation required to obtain the spherulite.
Another embodiment of the method of the present invention is a method for producing a spherulite of ketotifen fumarate, which includes the following steps:

(1) a step of adding an organic solvent (e.g., lower alcohols such as methanol and ethanol, tetrahydrofuran, acetonitrile, and a mixed solvent thereof) to ketotifen fumarate to dissolve, and to prepare a solution of ketotifen fumarate;
(2) a step of adding dropwise the solution prepared in the step (1) to another type of an organic solvent (lower alcohols such as 1-propanol, 2-propanol, 1-butanol, 2-butanol, isobutanol, and tert-butanol; ethyl acetate, tert-butyl methyl ether, toluene, acetone, and a mixed solvent thereof) (for example, over 1 second to 1 hour) at -20 °C to 30 °C to prepare a supersaturated solution of ketotifen fumarate; and
(3) a step of precipitating a spherulite of ketotifen fumarate at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 4, 5, 6, and 7 in Table 3 below are used, the solution has a degree of supersaturation equal to or higher than the actual measured value of the critical degree of supersaturation mentioned in No. 4, 5, 6, and 7, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation.
Another embodiment of the method of the present invention is a method for producing a spherulite of lanthanum carbonate octahydrate, which includes the following steps:

(1) a step of dissolving lanthanum oxide in hydrochloric acid to prepare an aqueous lanthanum chloride solution; and
(2) a step of adding dropwise an aqueous ammonium carbonate solution (for example, over 1 hour to 72 hours) at 0 °C to 55 °C to prepare a supersaturated solution of lanthanum carbonate octahydrate and to simultaneously precipitate a spherulite of lanthanum carbonate octahydrate.

The solution obtained in the step (2) of this production method has a degree of supersaturation equal to or higher than a critical degree of supersaturation required to obtain the spherulite.
Another embodiment of the method of the present invention is a method for producing a spherulite of clarithromycin, which includes the following steps:

(1) a step of adding a water-miscible organic solvent (e.g., lower alcohols such as methanol, ethanol, and 2-propanol; tetrahydrofuran, acetone, and a mixed solvent thereof) to clarithromycin to dissolve, and to prepare a solution of clarithromycin;
(2) a step of adding dropwise the solution prepared in the step (1) to water (for example, over 1 second to 1 hour) at -20 °C to 50 °C to prepare a supersaturated solution of clarithromycin; and
(3) a step of precipitating a spherulite of clarithromycin at -20°C to 50°C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 8, 9, or 10 in Table 3 below are used, the solution has a degree of supersaturation equal to or higher than the actual measured value of the critical degree of supersaturation mentioned in No. 8, 9, or 10, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation.
Another embodiment of the method of the present invention is a method for producing a spherulite of DL-glutamic acid, which includes the following steps:

(1) a step of adding a water-miscible organic solvent (e.g., lower alcohols such as methanol, ethanol, and 2-propanol; tetrahydrofuran, acetone, and a mixed solvent thereof) to DL-glutamic acid to dissolve, and to prepare a solution of

DL-glutamic acid;
(2) a step of adding dropwise the solution prepared in the step (1) to water (for example, over 1 second to 1 hour) at -20 °C to 50 °C to prepare a supersaturated solution of DL-glutamic acid; and
(3) a step of precipitating a spherulite of DL-glutamic acid at -20 °C to 50 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 12 or 13 in Table 3 below are used, the solution has a degree of supersaturation equal to or higher than the actual measured value of the critical degree of supersaturation mentioned in No. 12 or 13, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation.

Another embodiment of the method of the present invention is a method for producing a spherulite of escitalopram oxalate, which includes the following steps:

(1) a step of adding water and an organic solvent (e.g., lower alcohols such as methanol and ethanol, acetonitrile, and acetone) and a mixed solvent thereof to escitalopram oxalate to dissolve, and to prepare a solution of escitalopram oxalate;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; ethyl acetate, tert-butyl methyl ether, toluene, and a mixed solvent thereof) at - 20 °C to 30 °C to prepare a supersaturated solution of escitalopram oxalate; and
(3) a step of precipitating a spherulite of escitalopram oxalate at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 21, 22, or 23 in Table 3 below are used, the solution has a degree of supersaturation equal to or higher than the actual measured value of the critical degree of supersaturation mentioned in No. 21, 22, or 23, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of escitalopram oxalate may be inoculated in a step between the steps (2) and (3). The volume ratio of the good solvent to the poor solvent used in this production method is not particularly limited, and is preferably 1:0.1 to 1:100, and more preferably 1:5 to 1:30.

Another embodiment of the method of the present invention is a method for producing a spherulite of dabigatran etexilate methanesulfonate, which includes the following steps:

(1) a step of adding an organic solvent (e.g., alcohols such as methanol, ethanol, 1-propanol, 2-propanol, 1-butanol, 2-butanol, and 1-hexanol; acetonitrile, and a mixed solvent thereof) to dabigatran etexilate methanesulfonate to dissolve, and to prepare a solution of dabigatran etexilate methanesulfonate;
(2) a step of adding dropwise the solution prepared in the step (1) to another type of an organic solvent (e.g., acetates such as ethyl acetate, propyl acetate, and isopropyl acetate; tert-butyl methyl ether, toluene, tetrahydrofuran, acetone, and a mixed solvent thereof) at -20 °C to 40 °C to prepare a supersaturated solution of dabigatran etexilate methanesulfonate; and
(3) a step of precipitating a spherulite of dabigatran etexilate methanesulfonate at -20 °C to 40 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 24, 25, 26, or 27 in Table 3 below are used, the solution has a degree of supersaturation equal to or higher than the actual measured value of the critical degree of supersaturation mentioned in No. 24, 25, 26, or 27, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of dabigatran etexilate methanesulfonate may be inoculated in a step between the steps (2) and (3). The volume ratio of the good solvent to the poor solvent used in this production method

is preferably 1:3 to 1:1,000, and more preferably 1:5 to 1:20.

Another embodiment of the method of the present invention is a method for producing a spherulite of theophylline magnesium salt, which includes the following steps:

(1) a step of dissolving magnesium chloride hexahydrate in water, an organic solvent (e.g., lower alcohols such as methanol and ethanol, acetone, acetonitrile, and dimethyl sulfoxide) and a mixed solvent thereof to prepare a solution of magnesium chloride;
(2) a step of adding dropwise an aqueous theophylline potassium salt solution to the solution prepared in the step (1) at 0 °C to 55 °C to prepare a supersaturated solution of theophylline magnesium salt; and
(3) a step of precipitating a spherulite of theophylline magnesium salt at -20 °C to 50 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 28, 29, 30, or 31 in Table 3 below are used, the solution has a degree of supersaturation equal to or higher than the actual measured value of the critical degree of supersaturation mentioned in No. 28, 29, 30, or 31, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of theophylline magnesium salt may be inoculated in a step between the steps (2) and (3). The volume ratio of the good solvent to the poor solvent used in this production method is not particularly limited, and is preferably 1:0.1 to 1:100, and more preferably 1:1 to 1:20.

Another embodiment of the method of the present invention is a method for producing a spherulite of teneligliptin hydrobromide hydrate, which includes the following steps:

(1) a step of adding water and an organic solvent (e.g., lower alcohols such as methanol and ethanol) and a mixed solvent thereof to teneligliptin hydrobromide hydrate to dissolve, and to prepare a solution of teneligliptin hydrobromide hydrate;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; ethyl acetate, tert-butyl methyl ether, toluene, acetone, and a mixed solvent thereof) at -20 °C to 30 °C to prepare a supersaturated solution of teneligliptin hydrobromide hydrate; and
(3) a step of precipitating a spherulite of teneligliptin hydrobromide hydrate at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 32, 33, or 34 in Table 3 below are used, the solution has a degree of supersaturation equal to or higher than the actual measured value of the critical degree of supersaturation mentioned in No. 32, 33, or 34, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of teneligliptin hydrobromide hydrate may be inoculated in a step between the steps (2) and (3). The volume ratio of the good solvent to the poor solvent used in this production method is not particularly limited, and is preferably 1:0.1 to 1:100, and more preferably 1:5 to 1:30.

Another embodiment of the method of the present invention is a method for producing a spherulite of pilsicainide hydrochloride, which includes the following steps:

(1) a step of adding an organic solvent (e.g., alcohols such as methanol, ethanol, 1-propanol, 2-propanol, 1-butanol, 2-butanol, and 1-hexanol; acetonitrile, ethyl acetate, and a mixed solvent thereof) to pilsicainide hydrochloride to dissolve, and to prepare a solution of pilsicainide hydrochloride;
(2) a step of adding dropwise the solution prepared in the step (1) to another type of an organic solvent (e.g., toluene, tert-butyl methyl ether, tetrahydrofuran, acetone, and a mixed solvent thereof) at -20 °C to 50 °C to prepare a supersaturated solution of pilsicainide hydrochloride; and
(3) a step of precipitating a spherulite of pilsicainide hydrochloride at -20 °C to 50 °C.

The solution obtained in the step (2) of this production method has a degree of supersaturation equal to or higher than a critical degree of supersaturation required to obtain the spherulite. For example, when the substrate, the

solvent, the solvent ratio, and the crystallization temperature mentioned in No. 35 in Table 3 below are used, the solution has a degree of supersaturation equal to or higher than the actual measured value of the critical degree of supersaturation mentioned in No. 35, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of pilsicainide hydrochloride may be inoculated in a step between the steps (2) and (3). The volume ratio of the good solvent to the poor solvent used in this production method is not particularly limited, and is preferably 1:0.1 to 1:100, and more preferably 1:1 to 1:20.

Another embodiment of the method of the present invention is a method for producing a spherulite of tramadol hydrochloride, which includes the following steps:

(1) a step of adding water, an organic solvent (e.g., lower alcohols such as methanol and ethanol, acetone, acetonitrile, and dimethyl sulfoxide), and a mixed solvent thereof to tramadol hydrochloride to dissolve, and to prepare a solution of tramadol hydrochloride;
(2) a step of adding dropwise the solution prepared in the step (1) to another type of an organic solvent (e.g., acetone, tetrahydrofuran, ethyl acetate, isopropyl acetate, butyl acetate, tert-butyl methyl ether, and a mixed solvent thereof) at -10 °C to 40 °C to prepare a supersaturated solution of tramadol hydrochloride; and
(3) a step of precipitating a spherulite of tramadol hydrochloride at -10 °C to 40 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 36, 37, or 38 in Table 3 below are used, the solution has a degree of supersaturation equal to or higher than the actual measured value of the critical degree of supersaturation mentioned in No. 36, 37, or 38, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of tramadol hydrochloride may be inoculated in a step between the steps (2) and (3). The volume ratio of the good solvent to the poor solvent used in this production method is not particularly limited, and is preferably 1:0.1 to 1:100, and more preferably 1:1 to 1:40.

Another embodiment of the method of the present invention is a method for producing a spherulite of vildagliptin, which includes the following steps:

(1) a step of adding an organic solvent (e.g., lower alcohols such as methanol, ethanol, 1-propanol, 2-propanol, 1-butanol, 2-butanol, isobutanol, and tert-butanol; acetone, 2-butanone, acetonitrile, and a mixed solvent thereof) to vildagliptin to dissolve, and to prepare a solution of vildagliptin;
(2) a step of adding dropwise the solution prepared in the step (1) to another type of an organic solvent (e.g., tert-butyl methyl ether, diisopropyl ether, toluene, ethyl acetate, isopropyl acetate, butyl acetate, cyclopentyl methyl ether, cyclohexane, heptane, and a mixed solvent thereof) at - 20 °C to 30 °C to prepare a supersaturated solution of vildagliptin; and
(3) a step of precipitating a spherulite of vildagliptin at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 39, 40, 41, 42, 43, or 44 in Table 3 below are used, the solution has a degree of supersaturation equal to or higher than the actual measured value of the critical degree of supersaturation mentioned in No. 39, 40, 41, 42, 43, or 44, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of vildagliptin may be inoculated in a step between the steps (2) and (3). The volume ratio of the good solvent to the poor solvent used in this production method is not particularly limited, and is preferably 1:0.1 to 1:100, and more preferably 1:0.5 to 1:50.

Another embodiment of the method of the present invention is a method for producing a spherulite of linagliptin, which includes the following steps:

(1) a step of adding an organic solvent (e.g., lower alcohols such as methanol and ethanol, tetrahydrofuran, and

acetone) and a mixed solvent thereof to linagliptin to dissolve, and to prepare a solution of linagliptin;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; ethyl acetate, tert-butyl methyl ether, toluene, and a mixed solvent thereof) at - 20 °C to 30 °C to prepare a supersaturated solution of linagliptin; and
(3) a step of precipitating a spherulite of linagliptin at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 45 or 46 in Table 3 below are used, the solution has a degree of supersaturation equal to or higher than the actual measured value of the critical degree of supersaturation mentioned in No. 45 or 46, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of linagliptin may be inoculated in a step between the steps (2) and (3). The volume ratio of the good solvent to the poor solvent used in this production method is not particularly limited, and is preferably 1:0.1 to 1:100, and more preferably 1:1 to 1:20.

Another embodiment of the method of the present invention is a method for producing a spherulite of glutathione, which includes the following steps:

(1) a step of adding water and an organic solvent (e.g., lower alcohols such as methanol) and a mixed solvent thereof to glutathione to dissolve, and to prepare a solution of glutathione;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; ethyl acetate, isopropyl acetate, tert-butyl methyl ether, toluene, acetone, and a mixed solvent thereof) at -20 °C to 30 °C to prepare a supersaturated solution of glutathione; and
(3) a step of precipitating a spherulite of glutathione at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 47 in Table 3 below are used, the solution has a degree of supersaturation equal to or higher than the actual measured value of the critical degree of supersaturation mentioned in No. 47, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of glutathione may be inoculated in a step between the steps (2) and (3). The volume ratio of the good solvent to the poor solvent used in this production method is not particularly limited, and is preferably 1:0.1 to 1:100, and more preferably 1:2 to 1:30.

Another embodiment of the method of the present invention is a method for producing a spherulite of mirabegron, which includes the following steps:

(1) a step of adding an organic solvent (lower alcohols such as methanol, ethanol, 1-propanol, 2-propanol, 1-butanol, 2-butanol, isobutanol, and tert-butanol; acetone, 2-butanone, acetonitrile, and a mixed solvent thereof) to mirabegron to dissolve, and to prepare a solution of mirabegron;
(2) a step of adding dropwise the solution prepared in the step (1) to water, another type of an organic solvent (e.g., tert-butyl methyl ether, diisopropyl ether, toluene, ethyl acetate, isopropyl acetate, butyl acetate, cyclopentyl methyl ether, and cyclohexane), and a mixed solvent thereof at -20 °C to 40 °C to prepare a supersaturated solution of mirabegron; and
(3) a step of precipitating a spherulite of mirabegron at -20 °C to 40 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 48, 49, 50, 51, 52, or 53 in Table 3 below are used, the solution has a degree of supersaturation equal to or higher than the actual measured value of the critical degree of supersaturation mentioned in No. 48, 49, 50, 51, 52, or 53, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of

supersaturation equal to or higher than the upper limit of the 95% prediction interval of the critical degree of supersaturation. In this production method, a seed crystal of mirabegron may be inoculated in a step between the steps (2) and (3). The volume ratio of the good solvent to the poor solvent used in this production method is not particularly limited, and is preferably 1:0.1 to 1:100, and more preferably 1:0.2 to 1:50.

Another embodiment of the method of the present invention is a method for producing a spherulite of tolvaptan, which includes the following steps:

(1) a step of adding a water-miscible organic solvent (e.g., lower alcohols such as methanol, ethanol, 1-propanol, 2-propanol, 1-butanol, and 2-butanol, and a mixed solvent thereof) to tolvaptan to dissolve, and to prepare a solution of tolvaptan;
(2) a step of adding dropwise the solution prepared in the step (1) to water at -10 °C to 50 °C to prepare a supersaturated solution of tolvaptan; and
(3) a step of precipitating a spherulite of tolvaptan at -10 °C to 50 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 54 or 55 in Table 3 below are used, the solution has a degree of supersaturation equal to or higher than the actual measured value of the critical degree of supersaturation mentioned in No. 54 or 55, respectively, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of tolvaptan may be inoculated in a step between the steps (2) and (3). The volume ratio of the good solvent to the poor solvent used in this production method is not particularly limited, and is preferably 1:0.1 to 1:20, and more preferably 1:0.1 to 1:10.

Another embodiment of the method of the present invention is a method for producing a spherulite of valacyclovir hydrochloride, which includes the following steps:

(1) a step of adding water, an organic solvent (e.g., lower alcohols such as methanol; tert-butyl methyl ether, and dimethyl sulfoxide), and a mixed solvent thereof to valacyclovir hydrochloride to dissolve, and to prepare a solution of valacyclovir hydrochloride;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; tetrahydrofuran, toluene, and a mixed solvent thereof) at -10 °C to 40 °C to prepare a supersaturated solution of valacyclovir hydrochloride; and
(3) a step of precipitating a spherulite of valacyclovir hydrochloride at -10 °C to 40 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 56 in Table 3 below are used, the solution has a degree of supersaturation equal to or higher than the actual measured value of the critical degree of supersaturation mentioned in No. 56, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of valacyclovir hydrochloride may be inoculated in a step between the steps (2) and (3). The volume ratio of the good solvent to the poor solvent used in this production method is not particularly limited, and is preferably 1:0.1 to 1:100, and more preferably 1:1 to 1:50.

Another embodiment of the method of the present invention is a method for producing a spherulite of bepotastine besilate, which includes the following steps:

(1) a step of adding water, an organic solvent (e.g., lower alcohols such as methanol and ethanol), and a mixed solvent thereof to bepotastine besilate to dissolve, and to prepare a solution of bepotastine besilate;
(2) a step of adding dropwise the solution prepared in the step (1) to another type of an organic solvent (e.g., lower alcohols such as 1-propanol, 2-propanol, 1-butanol, 2-butanol, isobutanol, and tert-butanol; ethyl acetate, isopropyl acetate, tert-butyl methyl ether, toluene, acetone, and a mixed solvent thereof) at -20 °C to 30 °C to prepare a supersaturated solution of bepotastine besilate; and
(3) a step of precipitating a spherulite of bepotastine besilate at -20 °C to 30 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 57 in Table 3 below are used, the solution has a degree of supersaturation equal to or higher than the actual measured value of the critical degree of supersaturation mentioned in No. 57, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of bepotastine besilate may be inoculated in a step between the steps (2) and (3). The volume ratio of the good solvent to the poor solvent used in this production method is not particularly limited, and is preferably 1:0.1 to 1:100, and more preferably 1:5 to 1:30.

Another embodiment of the method of the present invention is a method for producing a spherulite of olopatadine, which includes the following steps:

(1) a step of adding an organic solvent (e.g., lower alcohols such as methanol and ethanol, tetrahydrofuran, and a mixed solvent thereof) to olopatadine to dissolve, and to prepare a solution of olopatadine;
(2) a step of adding dropwise the solution prepared in the step (1) to another type of an organic solvent (e.g., acetates such as ethyl acetate, propyl acetate, and isopropyl acetate; lower alcohols such as 1-propanol, 2-propanol, 1-butanol, and 2-butanol; tert-butyl methyl ether, toluene, acetone, and a mixed solvent thereof) at -20 °C to 40 °C to prepare a supersaturated solution of olopatadine; and
(3) a step of precipitating a spherulite of olopatadine at -20 °C to 40 °C.

The solution obtained in the step (2) of this production method has a degree of supersaturation equal to or higher than a critical degree of supersaturation required to obtain the spherulite. For example, when the substrate, the solvent, the solvent ratio, and the crystallization temperature mentioned in No. 58 in Table 3 below are used, the solution has a degree of supersaturation equal to or higher than the actual measured value of the critical degree of supersaturation mentioned in No. 58, has a degree of supersaturation equal to or higher than the predictive value of the critical degree of supersaturation, has a degree of supersaturation equal to or higher than the lower limit of the 95% prediction interval of the critical degree of supersaturation, or has a degree of supersaturation equal to or higher than the upper limit of the 95% prediction interval of the critical degree of supersaturation. In this production method, a seed crystal of olopatadine may be inoculated in a step between the steps (2) and (3). The volume ratio of the good solvent to the poor solvent used in this production method is not particularly limited, and is preferably 1:3 to 1:100, and more preferably 1:5 to 1:20.

## EXAMPLES

The present invention will be described in more detail with reference to the Examples shown below, but it is needless to say that the scope of the present invention is not limited by the Examples.

Various reagents used in the Examples are commercially available products unless otherwise specified, or those produced by a known method were used.

The particle size distribution was measured in a dry manner using a spray particle size distribution analyzer AEROTRAC LDSA-SPR3500A manufactured by Microtrac Inc. The particle size distribution in Examples 2 and 7 was measured in a dry manner using a laser diffraction particle size distribution measurement MASTERSIZER 3000 manufactured by Malvern Panalytical Ltd. SEM images were measured using a scanning electron microscope JSM-IT100 manufactured by JEOL Ltd. Light microscope images were measured using an upright microscope BX53M manufactured by Olympus Corporation and a microscope digital camera DP74 manufactured by Olympus Corporation. Images for measuring the number of particles were obtained by a scanner GT-X830 manufactured by SEIKO EPSON CORPORATION. The powder X-ray diffraction (XRD) was measured using a desktop X-ray diffraction device MiniFlex300 manufactured by Rigaku Corporation. The specific surface area was measured by the BET fluid process (gas used: pure nitrogen) using a fully automatic specific surface area measuring device Macsorb HM-1208 manufactured by Mountech Co., Ltd. The moisture content was measured using a trace moisture measuring device AQ-2200 manufactured by HIRANUMA SANGYO Co., Ltd. The dissolution profile was measured using μDISS Profiler manufactured by Pion Inc. Centrifugal classification was performed using a high-efficiency precision forced air classifier Lab Calssiel N-01 manufactured by SEISHIN ENTERPRISE Co., Ltd. Image analysis was performed using image processing software ImageJ developed by the National Institute of Health (NIH). The particle strength was measured using a particle hardness measuring device NEW GRANO manufactured by OKADA SEIKO CO., LTD. The true density was measured using a dry automatic densimeter AccuPyc II 1340-10CC manufactured by Shimadzu Corporation. The purity of a compound was measured by a high performance liquid chromatograph (LC-20A) manufactured by Shimadzu Corporation.

Example 1

Measurement of Critical Degree of Supersaturation of Spherulite

The critical degree of supersaturation of a spherulite of ketotifen fumarate was determined as follows. To 2.0 g of ketotifen fumarate, 10 mL (5 v/w) of methanol was added, followed by reflux to completely dissolve. This solution was added dropwise to 50 mL of isopropanol at 20 °C to prepare a supersaturated solution of ketotifen fumarate. The degree of supersaturation of the supersaturated solution at the end of dropwise addition was 9.0. The supersaturated solution was crystallized. The crystal was collected by filtration, followed by drying under reduced pressure at 40 °C, thus obtaining a crystal of ketotifen fumarate. The sphericity of the spherulite was 0.78 ± 0.02.

In order to gradually decrease the degree of supersaturation, the amount of methanol was set at 11.5 v/w and 13.5 v/w and the amount of isopropanol was set at 5 times the amount of methanol, and after dropwise addition, a seed crystal was added to crystallize. At this time, the degree of supersaturation was 4.0 and 3.4, respectively. The results of observation of each sample by SEM are shown in FIG. 1. At a degree of supersaturation of 9.0 to 4.0, a spherulite having a sphericity of 0.60 or more was observed (FIGs. 1(a) and (b)), but not observed at a degree of supersaturation of 3.4 (FIG. 1(c)). Since the difference between a degree of supersaturation of 3.4, which is the maximum value of a degree of supersaturation at which a spherulite having a sphericity of 0.60 or more does not exist at all, and a critical degree of supersaturation of 4.0 is within 20% of the critical degree of supersaturation, a degree of supersaturation of 4.0 was determined as a critical degree of supersaturation of a spherulite of ketotifen fumarate at a ratio of methanol to isopropanol of 1:5 and at a temperature during crystallization of 20 °C (see FIGs. 1 and 2).

Similarly, when a substrate and a solvent different from those mentioned above are used, a plurality of samples having a different degree of supersaturation are prepared and crystallized, and then the critical degree of supersaturation of the spherulite was measured. The results are shown in Table 3.

[Table 3-1]

Table 3

| No | Substrate | Solvent 1 | Solvent 2 | Solvent 2/solvent 1 | Temperature during crystallization | Critical degree of supersaturation S | Method for Preparing supersaturate d solution |
|---|---|---|---|---|---|---|---|
| | | | | Ratio | (°C) | Experimental value | |
| 1 | Esomeprazole magnesium trihydrate | MeOH | H2O | 1 | 35 | 1.4 | B |
| 2 | Lansoprazole | MeOH | H2O | 3 | 0 | 6.9 | B |
| 3 | Clopidogrel sulfate | H2O | 2-BuOH | 222 | 20 | 9.7 | C |
| 4 | Ketotifen fumarate | MeOH | IPA | 5 | 0 | 2.5 | C |
| 5 | Ketotifen fumarate | MeOH | IPA | 5 | 20 | 4.0 | C |
| 6 | Ketotifen fumarate | MeOH | 2-BuOH | 5 | 0 | 2.6 | C |
| 7 | Ketotifen fumarate | MeOH | 2-BuOH | 5 | 20 | 4.4 | C |
| 8 | Clarithromycin | THF | H2O | 25 | 0 | 21 | A |
| 9 | Clarithromycin | THF | H2O | 20 | 0 | 26 | A |
| 10 | Clarithromycin | THF | H2O | 25 | 10 | 17 | A |
| 11 | Azithromycin monohydrate | IPA | H2O | 10 | 0 | 3.2 | A |

(continued)

| No | Substrate | Solvent 1 | Solvent 2 | Solvent 2/solvent 1 | Temperature during crystallization | Critical degree of supersaturation S | Method for Preparing supersaturate d solution |
|---|---|---|---|---|---|---|---|
| | | | | Ratio | (°C) | Experimental value | |
| 12 | DL-glutamic acid | H2O | Acetone | 5 | 0 | 45.8 | A |
| 13 | DL-glutamic acid | H2O | THF | 5 | 0 | 24.3 | A |
| 14 | Azithromycin | IPA | H2O | 20 | 0 | 2.6 | A |
| 15 | Azithromycin | Acetone | H2O | 20 | 0 | 6.2 | A |
| 16 | Azithromycin | EtOH | H2O | 20 | 0 | 7.1 | A |
| 17 | Azithromycin | MeOH | H2O | 20 | 0 | 7.5 | A |
| 18 | Azithromvcin | IPA | H2O | 10 | 20 | 11.1 | A |
| 19 | Esomeprazole magnesium trihydrate | EtOH | H2O | 1 | 35 | 1.6 | A |
| 20 | Lansoprazole | MeOH | H2O | 3 | 15 | 12.6 | A |
| 21 | Escitalopram oxalate | H2O | 2-BuOH | 30 | 0 | 2.8 | A |
| 22 | Escitalopram oxalate | H2O | 2-BuOH | 15 | 0 | 3.7 | A |
| 23 | Escitalopram oxalate | H2O | IPA | 15 | 0 | 4.0 | A |
| 24 | Dabigatran etexilate methane sulfonate | MeOH | TBME | 10 | 20 | 3.3 | A |
| 25 | Dabigatran etexilate methane sulfonate | EtOH | EtOAc | 10 | 5 | 2.9 | A |
| 26 | Dabigatran etexilate methane sulfonate | EtOH | EtOAc | 10 | 20 | 4.3 | A |
| 27 | Dabigatran etexilate methane sulfonate | EtOH | EtOAc | 10 | 35 | 5.2 | A |
| 28 | Theophylline magnesium salt | MeOH | H2O | 3 | 0 | 3.5 | C |
| 29 | Theophylline magnesium salt | EtOH | H2O | 3 | 0 | 3.9 | C |

(continued)

| No | Substrate | Solvent 1 | Solvent 2 | Solvent 2/solvent 1 | Temperature during crystallization | Critical degree of supersaturation S | Method for Preparing supersaturate d solution |
|---|---|---|---|---|---|---|---|
| | | | | Ratio | (°C) | Experimental value | |
| 30 | Theophylline magnesium salt | Acetone | H2O | 3 | 0 | 6.3 | C |

[Table 3-2]

| No | Substrate | Solvent 1 | Solvent 2 | Solvent 2/solvent 1 | Temperature during crystallization | Critical degree of supersaturation S | Method for preparing supersaturated solution |
|---|---|---|---|---|---|---|---|
| | | | | Ratio | (°C) | Experimental value | |
| 31 | Theophylline magnesium salt | IPA | H2O | 3 | 0 | 6.6 | C |
| 32 | Teneligliptin hydrobromide hydrate | MeOH | 1-BuOH | 10 | 0 | 5.2 | A |
| 33 | Teneligliptin hydrobromide hydrate | MeOH | 1-BuOH | 10 | 20 | 7.9 | A |
| 34 | Teneligliptin hydrobromide hydrate | MeOH | 1-BuOH | 10 | 40 | 12.6 | A |
| 35 | Pilsicainide hydrochloride anhydride | IPA | Toluene | 10 | 5 | 27 | A |
| 36 | Tramadol hydrochloride | MeOH | i-PrOAc | 20 | 10 | 4.2 | A |
| 37 | Tramadol hydrochloride | IPA | TBME | 30 | 10 | 11.1 | A |
| 38 | Tramadol hydrochloride | EtOH | TBME | 30 | 10 | 22.4 | A |
| 39 | Vildagliptin | EtOH | TBME | 10 | 0 | 9.2 | A |
| 40 | Vildagliptin | EtOH | TBME | 10 | 10 | 7.0 | A |
| 41 | Vildagliptin | MEK | Toluene | 10 | 0 | 8.5 | A |
| 42 | Vildagliptin | IPA | TBME | 10 | 0 | 11.0 | A |
| 43 | Vildagliptin | IPA | TBME | 10 | 10 | 11.6 | A |
| 44 | Vildagliptin | IPA | TBME | 10 | 20 | 11.9 | A |
| 45 | Linagliptin | EtOH | TBME | 15 | 0 | 7.9 | A |

(continued)

| No | Substrate | Solvent 1 | Solvent 2 | Solvent 2/solvent 1 | Temperature during crystallization | Critical degree of supersaturation S | Method for preparing supersaturated solution |
|---|---|---|---|---|---|---|---|
| | | | | Ratio | (°C) | Experimental value | |
| 46 | Linagliptin | EtOH | TBME | 15 | 20 | 6.7 | A |
| 47 | Glutathione | H2O | EtOH | 5 | 20 | 18.7 | A |
| 48 | Mirabegron | H2O | MeOH | 1.25 | 0 | 1.6 | A |
| 49 | Mirabegron | H2O | MeOH | 1.25 | 10 | 3.2 | A |
| 50 | Mirabegron | MeOH | TBME | 10 | 0 | 4.0 | A |
| 51 | Mirabegron | MeOH | TBME | 10 | 10 | 4.3 | A |
| 52 | Mirabegron | EtOH | TBME | 10 | 0 | 5.6 | A |
| 53 | Mirabegron | EtOH | TBME | 10 | 10 | 5.4 | A |
| 54 | Tolvaptan | MeOH | H2O | 0.4 | 0 | 4.8 | B |
| 55 | Tolvaptan | MeOH | H2O | 0.4 | 10 | 7.1 | B |
| 56 | Valacyclovir hydrochloride | H2O | 2-BuOH | 35 | 10 | 48.6 | A |
| 57 | Bepotastine besilate | EtOH | AcOiPr | 10 | 0 | 36.4 | A |
| 58 | Olopatadine | MeOH | EtOAc | 20 | 15 | 9.7 | A |
| A: Crystallization by reverse addition<br>B: Crystallization by normal addition<br>C: Crystallization by salt formation | | | | | | | |

Example 2

Production of Spherulite of Esomeprazole Magnesium Trihydrate (Crystallization by Normal Addition)

To 1.7 kg of esomeprazole magnesium trihydrate, 13.43 kg (17.0 L) of methanol was added, followed by dissolution while stirring at room temperature. To this solution, 51.0 kg (51.0 L) of water was added dropwise at 25 °C for 11 minutes to prepare a supersaturated solution of esomeprazole magnesium trihydrate. The degree of supersaturation of the supersaturated solution at the end of dropwise addition was 6.7. In the supersaturated solution, a precipitate of an amorphous solid of esomeprazole magnesium was observed (the fact that the precipitate is an amorphous solid was confirmed by powder X-ray diffraction). As a seed crystal, 25.5 mg of esomeprazole magnesium trihydrate suspended in 2.6 mL of water was added to the above-mentioned supersaturated solution, and allowed to stand at 35 °C for 45 hours, followed by stirring for 3 hours. After addition of the seed crystal, the amorphous solid in the supersaturated solution was dissolved, and crystallization of esomeprazole magnesium trihydrate proceeded. Thereafter, the precipitate was isolated by centrifugation, and after washing with a mixed solution of 1.3 kg (1.7 L) of methanol and 5.1 kg (5.1 L) of water, drying under reduced pressure was performed at 40 °C for 38 hours to obtain 1.4 kg of a spherulite of esomeprazole magnesium trihydrate (yield of 80%). The $d_{50}$ of the crystal thus obtained was 56.1 μm, and the sharpness index was 2.5. The crystal thus obtained was isolated by centrifugal classification into fine powders and coarse powders. The fine powders thus obtained were classified with a 235 mesh (M) (63 μm) and 390 M (38 μm). Furthermore, the crystal on the 390 M (38 μm) was treated by a suction machine to obtain a spherulite of esomeprazole magnesium trihydrate. The $d_{50}$ of the fine powders was 54.6 μm, the sharpness index was 1.3, and the sphericity of the spherulite was 0.95 ± 0.03 (see FIGs. 5 to 7).

Example 3

Production of Spherulite of Esomeprazole Magnesium Trihydrate (Crystallization by Normal Addition)

To 10 g of esomeprazole magnesium trihydrate, 50 mL of methanol was added, followed by dissolution while stirring at room temperature. To this solution, 150 mL of water was added dropwise at 25 °C, and then 0.1 mg of esomeprazole magnesium trihydrate was added and stirring was stopped. The temperature was raised to 45 °C, and after allowing to stand for 3 hours, 100 mL of methanol was added while stirring. Thereafter, the solid was isolated by filtration under reduced pressure, and drying under reduced pressure was performed at 40 °C for 20 hours to obtain 1.84 g of a spherulite of esomeprazole magnesium trihydrate (yield of 18%). The crystal thus obtained was classified with a 149 mesh (M) (100 μm) and 390 M (38 μm), and the crystal on the 390 M (38 μm) was treated by a suction machine to obtain a spherulite of esomeprazole magnesium trihydrate. The sphericity of the spherulite was 0.97 ± 0.01, and the equivalent circle diameter was 58 ± 7 μm (see FIG. 8).

Example 4

Method for Producing Spherulite of Esomeprazole Magnesium Trihydrate (Crystallization by Normal Addition)

To 5 g of esomeprazole magnesium trihydrate, 50 mL of methanol was added, followed by dissolution while stirring at room temperature. To this solution, 50 mL of water was added dropwise at 35 °C, and then the solid was removed by filtration under pressure. To the solution thus obtained, 50 mg of a spherulite (equivalent circle diameter of 51 ± 3 μm) of esomeprazole magnesium trihydrate was added, followed by stirring at 35 °C for 114 hours. Thereafter, the solid was isolated by filtration under reduced pressure, and drying under reduced pressure was performed at 40 °C for 20 hours to obtain a spherulite of esomeprazole magnesium trihydrate (quantitative yield of 66%). The sphericity of the spherulite was 0.93 ± 0.02, and the equivalent circle diameter was 183 ± 8 μm (see FIG. 9).

Example 5

Production of Spherulite of Esomeprazole Magnesium Trihydrate (Crystallization by Normal Addition)

To 20.0 g of esomeprazole magnesium trihydrate, 360 mL of methanol was added, followed by dissolution while stirring at room temperature. To this solution, 360 mL of water was added dropwise at 35 °C, and then the insoluble matter was removed by filtration under pressure, and 20.0 mg of esomeprazole magnesium trihydrate was added, followed by stirring at 35 °C for 186 hours. Thereafter, the solid was isolated by filtration under reduced pressure, and drying under reduced pressure was performed at 40 °C for 23 hours to obtain 3.66 g of a spherulite of esomeprazole magnesium trihydrate (yield of 18%). The crystal thus obtained was classified with 149 M (100 μm). The sharpness index of the crystal thus obtained was 1.4, the $d_{50}$ was 149.9 μm, and the sphericity of the spherulite was 0.89 ± 0.05 (see FIGs. 10 to 12).

Example 6

Production of Spherulite of Esomeprazole Magnesium Trihydrate (Crystallization by Salt Exchange)

To 23.7 g of esomeprazole potassium salt dimethanol solvate (purity of 98.59%), 30.3 mL of water and 66.4 mL of acetone were added, followed by dissolution at room temperature. This solution was stirred, and 28.08 g of an aqueous 5.8% magnesium chloride solution was added dropwise over 30 minutes. The solution after dropwise addition was filtered by filtration under pressure, and then 1.1 mg of esomeprazole magnesium trihydrate was added to this filtrate. After stirring was continued for 22 hours, 65.43 g of an aqueous 5.5% magnesium chloride solution was added dropwise over 16 hours. After stirring was continued for 23 hours, the crystal was collected by filtration, and drying under reduced pressure was performed at 50 °C for 7 hours to obtain 14.8 g of a spherulite of esomeprazole magnesium trihydrate (yield of 73.0%, purity of 99.90%). The sharpness index of the crystal thus obtained was 1.45, the $d_{50}$ was 91.5 μm, and the sphericity of the spherulite was 0.96 ± 0.01 (see FIGs. 13 to 15).

Example 7

Production of Spherulite of Esomeprazole Magnesium Trihydrate (Crystallization by Salt Exchange)

To 0.88 g of esomeprazole magnesium trihydrate, 33 mL of acetone and 62 mL of water were added, followed

by stirring at 30 °C for 2 hours to dissolve. After dust removal and filtration, 8.9 mg of ground esomeprazole magnesium trihydrate ($d_{50}$: 1.8 μm) was added, followed by stirring to prepare a suspension. To this, a solution obtained by dissolving 5.49 g of magnesium chloride hexahydrate in 17 mL of acetone and 31 mL of water and a solution obtained by dissolving 23.7 g of esomeprazole potassium dimethanol solvate (purity of 98.59%) in 17 mL of acetone and 31 mL of water were simultaneously added dropwise over 33 hours. After stirring for 11 hours from completion of dropwise addition, the crystal was collected by filtration, and washed with a mixed solution of 21 mL of acetone and 38 mL of water. Drying under reduced pressure was performed at 40 °C for 20 hours to obtain 16.4 g of a spherulite of esomeprazole magnesium trihydrate (yield of 77%, purity of 99.87%). The sharpness index of the crystal thus obtained was 1.40, the $d_{50}$ was 189 μm, and the sphericity of the spherulite was 0.97 ± 0.01 (see FIGs. 16 to 18).

Example 8

Measurement of Dissolution Profile

(1) Production of Spherulite of Esomeprazole Magnesium Trihydrate

To 100 g of esomeprazole magnesium trihydrate, 1.0 L of methanol was added, followed by dissolution while stirring at 35 °C. To this solution, 3.0 L of water was added dropwise at 35 °C, and then 0.1 g of esomeprazole magnesium trihydrate was added to allow to stand for 46 hours. Thereafter, the solid was isolated by filtration under reduced pressure, and drying under reduced pressure was performed at 40 °C for 63 hours to obtain 69.9 g of a spherulite of esomeprazole magnesium trihydrate (yield of 70%). The sharpness index of the crystal thus obtained was 2.2, the $d_{50}$ was 33.0 μm, and the sphericity of the spherulite was 0.95 ± 0.03 (see FIGs. 44 to 46).

(2) Measurement of Dissolution Profile of Spherulite and Non-spherulite of Esomeprazole Magnesium Trihydrate

At 37 °C, 30 mg of a spherulite of esomeprazole magnesium trihydrate produced in (1) mentioned above was added to 10 mL of a phosphate buffer with pH 6.8, followed by stirring at 300 rpm, and the solution concentration was measured over time by μDISS Profiler. As a comparison, measurement was similarly performed for a ground product (sphericity of 0.45 ± 0.23) of a non-spherulite of esomeprazole magnesium trihydrate. Data on the ground product are shown in FIGs. 47 to 49. Compared with the non-spherulite, more rapid dissolution of the spherulite was observed (see FIG. 3). The particle size and the specific surface area of the samples used are shown in Table 4 below.

[Table 4]

Table 4

| Sample | $d_{50}$ (μm) | Specific surface area ($m^2$/g) |
|---|---|---|
| Example 8(1) | 33.0 | 22.6 |
| Ground product of non-spherulite | 5.2 | 44.0 |

Example 9

Measurement of Particle Density and Particle Strength

The particle density and the particle strength of a spherulite of esomeprazole magnesium trihydrate obtained in the same manner as in Example 2 (sample 1), a spherulite of esomeprazole magnesium trihydrate obtained in the same manner as in Example 4 (sample 2), and a spherulite of esomeprazole magnesium trihydrate obtained in the same manner as in Example 6 (sample 3) were measured.

The true density of esomeprazole magnesium trihydrate was calculated as 1.37 g/$cm^3$. The measurement results are shown in Table 5 below.

[Table 5]

Table 5

| Sample | Particle density (g/$cm^3$) | Particle packing rate (%) |
|---|---|---|
| Sample 1 | 0.74 | 54 |

(continued)

| Sample | Particle density (g/cm$^3$) | Particle packing rate (%) |
|---|---|---|
| Sample 2 | 0.87 | 64 |
| Sample 3 | 1.21 | 88 |

The particle strength of the spherulite of esomeprazole magnesium trihydrate obtained in Example 2, the spherulite of esomeprazole magnesium trihydrate obtained in Example 5, and the spherulite of esomeprazole magnesium trihydrate obtained in Example 6 was measured. The results are shown in Table 6.

[Table 6]

Table 6

| Sample | Particle strength (MPa) |
|---|---|
| Example 2 | Measurement was impossible due to malperformance of breakability of particles |
| Example 5 | 2.3 ± 0.4 |
| Example 6 | 2.9 ± 0.5 |

Example 10

Measurement of Filtration Time and Cake Thickness

(1) Production of Spherulite of Esomeprazole Magnesium Trihydrate (Crystallization by Salt Exchange)

To 23.7 g of esomeprazole potassium salt dimethanol solvate, 30.3 mL of water and 66.4 mL of acetone were added, followed by dissolution at room temperature. This solution was stirred, and 28.08 g of an aqueous 5.8% magnesium chloride solution was added dropwise over 30 minutes. The solution after dropwise addition was filtered by filtration under pressure, and then 71.1 mg of esomeprazole magnesium trihydrate was added to this filtrate. After stirring was continued for 2.5 hours, 65.43 g of an aqueous 5.5% magnesium chloride solution was added dropwise over 12 hours. After stirring was continued for 8 hours, the crystal was filtered under reduced pressure with Nutsche (outer diameter of 70 mm, retained particle size of filter paper of 1 μm) and washed with 20 mL of acetone/water (35:65). Drying under reduced pressure was performed at 50 °C for 4.5 hours to obtain 17.4 g of a spherulite of esomeprazole magnesium trihydrate (yield of 80%). The sharpness index of the crystal thus obtained was 1.51, the $d_{50}$ was 39.9 μm, and the sphericity of the spherulite was 0.97 ± 0.01 (see FIGs. 50 to 51). All crystals thus obtained were spherulites insofar as they were observed by SEM.

(2) Production of Crystal of Esomeprazole Magnesium Trihydrate (Crystallization by Salt Exchange)

To 23.7 g of esomeprazole potassium salt dimethanol solvate, 30.3 mL of water and 66.4 mL of acetone were added, followed by dissolution at room temperature. This solution was stirred, and 28.08 g of an aqueous 5.8% magnesium chloride solution was added dropwise over 30 minutes. The solution after dropwise addition was filtered by filtration under pressure, and then 71.1 mg of esomeprazole magnesium trihydrate was added to this filtrate. After stirring was continued for 2.5 hours, 65.43 g of an aqueous 5.5% magnesium chloride solution was added dropwise over 30 minutes. After stirring was continued for 14 hours, the crystal was filtered under reduced pressure with Nutsche (outer diameter of 70 mm, retained particle size of filter paper of 1 μm) and washed with 20 mL of acetone/water (35:65). Drying under reduced pressure was performed at 50 °C for 4.5 hours to obtain 17.9 g of a crystal of esomeprazole magnesium trihydrate (yield of 82%). The sharpness index of the crystal thus obtained was 1.48, and the $d_{50}$ was 9.6 μm. In the crystal thus obtained, a spherulite and a non-spherulite coexisted. From the SEM image and the particle size distribution, it was confirmed that the proportion of the non-spherulite was higher than that of the spherulite. The sphericity of the spherulite contained in the crystal thus obtained was 0.80 ± 0.05. The sphericity of the non-spherulite contained in the crystal thus obtained was 0.50 ± 0.06 (see FIGs. 52 and 53).

The filtration time and the cake thickness when esomeprazole magnesium trihydrate was isolated in (1) and (2) mentioned above are shown in Table 7 below.

[Table 7]

Table 7

| Sample | $d_{50}$ (μM) | Filtration time (min) | | Cake thickness (mm) | |
|---|---|---|---|---|---|
| | | Solid-liquid separation | Washing | Before compression | After compression |
| Example 10(1) | 39.9 | 1 | 0.2 | 10 | 10 |
| Example 10(2) | 9.6 | 2 | 14 | 20 | 11 |

Example 11

Production of Spherulite of Lansoprazole (Crystallization by Normal Addition)

To 1 g of lansoprazole, 20 mL of methanol was added, followed by heating at 35 °C to dissolve. This solution was added dropwise to 60 mL of water at 0 to 5 °C over 10 minutes to prepare a supersaturated solution of lansoprazole. The degree of supersaturation of the supersaturated solution at the end of dropwise addition was 35. This value was higher than the actual measured value (10.9) of the critical degree of supersaturation shown in No. 2 in Table 3 mentioned above. Thereafter, 0.1 mg of lansoprazole was added as a seed crystal, followed by allowing to stand at 0 to 5 °C for 4 hours. Thereafter, the precipitate was isolated by filtration under reduced pressure and dried to obtain 0.17 g of a spherulite of lansoprazole (yield of 17%). The SEM image of the spherulite thus obtained and the results of powder X-ray diffraction are shown in FIGs. 19 and 20. The sphericity of the spherulite was 0.94 ± 0.04.

Example 12

Production of Spherulite of Azithromycin Monohydrate (Crystallization by Reverse Addition)

To 1.0 g of azithromycin dihydrate, 10 mL of ethanol was added, followed by dissolution while stirring at room temperature. This solution was added dropwise to 100 mL of water at 0 °C over 100 minutes to prepare a supersaturated solution of azithromycin. When the temperature was raised to 8 °C in 1 minute and 18 seconds, a crystal was precipitated. At this time, the degree of supersaturation was 13.5. Thereafter, the temperature was raised to 20 °C in 3 minutes and 9 seconds, followed by stirring for 3 hours. Thereafter, the precipitate was isolated by filtration under reduced pressure, and drying under reduced pressure was performed at 40 °C for 17 hours to obtain 0.81 g of a spherulite of azithromycin monohydrate (yield of 81%). The $d_{50}$ of the crystal thus obtained was 75 μm, the sharpness index was 1.5, and the sphericity of the spherulite was 0.89 ± 0.02 (see FIGs. 21 to 23).

The spherulite of azithromycin monohydrate produced in Example 12 was cut, and the section was observed by SEM (FIG. 4). The crystal size at the outer edge was 5 to 10 μm, while the crystal size at the center was 0.2 to 1 μm.

Example 13

Production of Spherulite of Clarithromycin (Crystallization by Reverse Addition)

To 1 g of clarithromycin, 2 mL of tetrahydrofuran was added, followed by dissolution at 50 °C. This solution was added dropwise to 50 mL of water while stirring at 10 °C over about 5 seconds to prepare a supersaturated solution of clarithromycin. The degree of supersaturation of the supersaturated solution at the end of dropwise addition was 86. This value was higher than the actual measured value (17) of the critical degree of supersaturation shown in No. 10 in Table 3 mentioned above. Immediately after the end of dropwise addition, a crystal of clarithromycin started to be precipitated. About 6 minutes after the end of dropwise addition, the precipitated crystal was collected by filtration, followed by drying under reduced pressure at 40 °C for 17 hours to obtain 0.73 g of a spherulite of clarithromycin (yield of 73%). The $d_{50}$ of the crystal thus obtained was 16 μm, the sharpness index was 4.6, and the sphericity of the spherulite thus obtained was 0.87 ± 0.08 (see FIGs. 24 to 26).

Example 14

Production of Spherulite of DL-Glutamic Acid (Crystallization by Reverse Addition)

To 0.21 g of DL-glutamic acid, 4 mL (20 v/w) of water was added, followed by complete dissolution at 60 °C. This solution was added dropwise to 20 mL of acetone cooled to 0 °C over 3 seconds, followed by crystallization. At this

time, the degree of supersaturation was 91. The crystal was collected by filtration, followed by drying under reduced pressure at 40 °C, thus obtaining a spherulite of DL-glutamic acid. The sphericity of the spherulite was 0.95 ± 0.03 (see FIGs. 27 and 28).

Example 15

Production of Spherulite of Duloxetine Hydrochloride (Crystallization by Salt Formation)

To 5 g of duloxetine, 50 mL of 2-propanol and 5 mL of Span80 were added, followed by dissolution at room temperature. While stirring this solution, 16 mL of a 1 mol/L hydrogen chloride-ethyl acetate solution was added dropwise at 20 °C over about 1 second to prepare a supersaturated solution of duloxetine hydrochloride. The degree of supersaturation of the supersaturated solution at the end of dropwise addition was 73. About 3 minutes after the end of dropwise addition, a crystal of duloxetine hydrochloride started to be precipitated. About 1 hour after the end of dropwise addition, the precipitated crystal was collected by filtration, followed by drying under reduced pressure at 40 °C for 16 hours to obtain 4.7 g of a spherulite of duloxetine hydrochloride (yield of 84%). The $d_{50}$ of the crystal thus obtained was 138 μm, the sharpness index was 4.7, and the sphericity of the spherulite was 0.92 ± 0.07 (see FIGs. 30 to 32).

Example 16

Method for Producing Spherulite of Clopidogrel Sulfate (Crystallization by Salt Formation)

To 2 g of clopidogrel, 40 mL of 2-butanol and 180 μL of water were added, followed by dissolution at room temperature. While stirring this solution, 0.63 g of an aqueous 98% by weight sulfuric acid solution was added dropwise over about 1 second to prepare a supersaturated solution of clopidogrel sulfate. The degree of supersaturation of the supersaturated solution at the end of dropwise addition was 23. After the end of dropwise addition, 2 mg of the seed crystal was inoculated, and stirring was continued for 102 hours.

The crystal was collected by filtration, followed by drying under reduced pressure at 70 °C for 16 hours to obtain 1.87 g of a spherulite of clopidogrel sulfate (yield of 94%). The $d_{50}$ of the crystal thus obtained was 133 μm, the sharpness index was 1.7, and the sphericity of the spherulite was 0.96 ± 0.02 (see FIGs. 33 to 35).

Example 17

Production of Spherulite of Lanthanum Carbonate Octahydrate (Crystallization by Chemical Conversion)

To 35.1 g of lanthanum oxide, 140 mL of water and 119.4 g of 20% hydrochloric acid were added, followed by dissolution. This solution was subjected to dust removal and filtration. While stirring the aqueous lanthanum chloride solution after filtration, an aqueous ammonium carbonate solution (prepared using 35.2 g of ammonium carbonate and 175.5 mL of water) was added dropwise to the solution at 40 °C over 46 hours. In 3 hours after the start of dropwise addition, a crystal of lanthanum carbonate was precipitated. Two hours after the end of dropwise addition, the precipitated crystal was collected by filtration, and cake washing was performed five times using 526 mL of water. Drying under reduced pressure was performed at 40 °C to obtain 61.9 g of a spherulite of lanthanum carbonate octahydrate (yield of 96%). The crystal thus obtained was classified with a 149 mesh (M) (100 μm). The $d_{50}$ of the crystal thus obtained was 105 μm, the sharpness index was 1.4, and the sphericity of the spherulite was 0.69 ± 0.08 (see FIGs. 36 to 38).

Example 18

Building of Predictive Model of Critical Degree of Supersaturation

From the database chembl_23 of ChEMBL (https://www.ebi.ac.uk/chembl/) and the database of PubChem (https://pubchem.ncbi.nlm.nih.gov/), 50,000 structures of respective compounds were randomly extracted. Of these, structures with corrupted structure data were excluded, and desalting treatment was performed to make a total of 89,203 structures. A total of 89,224 types of compound data combining these compounds with 21 types of desalted forms of pharmaceutical drug substances (azithromycin, clarithromycin, DL-glutamic acid, esomeprazole, lansoprazole, clopidogrel, ketotifen, theophylline, vildagliptin, valacyclovir, tramadol, escitalopram, dabigatran etexilate, pilsicainide, linagliptin, glutathione, mirabegron, teneligliptin, tolvaptan, bepotastine, and olopatadine) were used. As solvent data, data on 14 types of solvents (1-butanol, 2-butanol, 2-propanol, acetone, ethanol, ethyl acetate, heptane, isopropyl acetate, methanol, methyl ethyl ketone, tert-butyl methyl ether, tetrahydrofuran, toluene, and water) were used. Descriptors were calculated using alvaDesc 1.0 (https://www.alvascience.com/alvadesc/). After all two-dimensional descriptors were calculated, descriptors which could not be calculated for even one structure, descriptors having the same value for all structures, and one descriptor of a set of two descriptors having a correlation coefficient of 1.0 were deleted. Descriptors in which all values are integers, including those showing the proportion of the number of specific substructures and atoms, were excluded. As the descriptors on a compound, 436 types remained, and as the descriptors on a solvent, 373 types remained.

Descriptors on a compound (436 types)

MW, AMW, Se, Sp, Si, Me, Mp, Mi, GD, SCBO, RBF, H%, C%, N%, O%, X%, MCD, RFD, RCI, NNRS, ARR, D/Dtr03, D/Dtr04, D/Dtr05, D/Dtr06, D/Dtr07, D/Dtr08, D/Dtr09, D/Dtr10, D/Dtr11, D/Dtr12, LPRS, MSD, SPI, AECC, DECC, MDDD, ICR, MeanTD, MeanDD, S1K, S2K, S3K, PHI, PW2, PW3, PW4, PW5, MAXDN, MAXDP, DELS, LOC, MWC01, MWC02, MWC03, MWC04, MWC05, MWC06, MWC07, MWC08, MWC09, MWC10, SRW02, SRW03, SRW04, SRW05, SRW06, SRW07, SRW08, SRW09, SRW10, MPC02, MPC03, MPC04, MPC05, MPC06, MPC07, MPC08, MPC09, MPC10, piPC01, piPC02, piPC03, piPC04, piPC05, piPC06, piPC07, piPC08, piPC09, piPC10, TWC, TPC, piID, PCD, CID, BID, ATS1m, ATS2m, ATS3m, ATS4m, ATS5m, ATS6m, ATS7m, ATS8m, ATS1e, ATS2e, ATS3e, ATS4e, ATS5e, ATS6e, ATS7e, ATS8e, ATS1p, ATS2p, ATS3p, ATS4p, ATS5p, ATS6p, ATS7p, ATS8p, ATS1i, ATS2i, ATS3i, ATS4i, ATS5i, ATS6i, ATS7i, ATS8i, ATSC1m, ATSC2m, ATSC3m, ATSC4m, ATSC5m, ATSC6m, ATSC7m, ATSC8m, ATSC1e, ATSC2e, ATSC3e, ATSC4e, ATSC5e, ATSC6e, ATSC7e, ATSC8e, ATSC1p, ATSC2p, ATSC3p, ATSC4p, ATSC5p, ATSC6p, ATSC7p, ATSC8p, ATSC1i, ATSC2i, ATSC3i, ATSC4i, ATSC5i, ATSC6i, ATSC7i, ATSC8i, MATS1m, MATS2m, MATS3m, MATS4m, MATS5m, MATS6m, MATS7m, MATS8m, MATS1e, MATS2e, MATS3e, MATS4e, MATS5e, MATS6e, MATS7e, MATS8e, MATS1p, MATS2p, MATS3p, MATS4p, MATS5p, MATS6p, MATS7p, MATS8p, MATS1i, MATS2i, MATS3i, MATS4i, MATS5i, MATS6i, MATS7i, MATS8i, GATS1m, GATS2m, GATS3m, GATS4m, GATS5m, GATS6m, GATS7m, GATS8m, GATS1e, GATS2e, GATS3e, GATS4e, GATS5e, GATS6e, GATS7e, GATS8e, GATS1p, GATS2p, GATS3p, GATS4p, GATS5p, GATS6p, GATS7p, GATS8p, GATS1i, GATS2i, GATS3i, GATS4i, GATS5i, GATS6i, GATS7i, GATS8i, GGI1, GGI2, GGI3, GGI4, GGI5, GGI6, GGI7, GGI8, GGI9, GGI10, JGI1, JGI2, JGI3, JGI4, JGI5, JGI6, JGI7, JGI8, JGI9, JGI10, JGT, SpMax1_Bh(m), SpMax2_Bh(m), SpMax3_Bh(m), SpMax4_Bh(m), SpMax5_Bh(m), SpMax6_Bh(m), SpMax7_Bh(m), SpMax8_Bh(m), SpMax1_Bh(e), SpMax2_Bh(e), SpMax3_Bh(e), SpMax4_Bh(e), SpMax5_Bh(e), SpMax6_Bh(e), SpMax7_Bh(e), SpMax8_Bh(e), SpMax1_Bh(p), SpMax2_Bh(p), SpMax3_Bh(p), SpMax4_Bh(p), SpMax5_Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i), SpMax8_Bh(i), SpMin1_Bh(m), SpMin2_Bh(m), SpMin3_Bh(m), SpMin4_Bh(m), SpMin5_Bh(m), SpMin6_Bh(m), SpMin7_Bh(m), SpMin8_Bh(m), SpMin1_Bh(e), SpMin2_Bh(e), SpMin3_Bh(e), SpMin4_Bh(e), SpMin5_Bh(e), SpMin6_Bh(e), SpMin7_Bh(e), SpMin8_Bh(e), SpMin1_Bh(p), SpMin2_Bh(p), SpMin3_Bh(p), SpMin4_Bh(p), SpMin5 _Bh(p), SpMin6_Bh(p), SpMin7_Bh(p), SpMin8_Bh(p), SpMin1_Bh(i), SpMin2_Bh(i), SpMin3_Bh(i), SpMin4_Bh(i), SpMin5_Bh(i), SpMin6_Bh(i), SpMin7_Bh(i), SpMin8_Bh(i), P_VSA_LogP_1, P_VSA_LogP_2, PVSA LogP_3, P_VSA_LogP_4, PVSA_LogP_5, P_VSA_LogP_6, P_VSA_LogP_7, P_VSA_LogP_8, P_VSA_MR_1, P_VSA_MR_2, P_VSA_MR_3, P_VSA_MR_4, P_VSA_MR_5, P_VSA_MR_6, P_VSA_MR_7, P_VSA_MR_8, P_VSA_m_1, P_VSA_m_2, P_VSA_m_3, P_VSA_m_4, P_VSA_m_5, P_VSA_v_2, P_VSA_v_3, P_VSA_v_4, P_VSA_e_1, P_VSA_e_2, P_VSA_e_3, P_VSA_e_4, P_VSA_e_5, P_VSA_e_6, P_VSA_p_1, P_VSA_p_2, P_VSA_p_3, P_VSA_p_4, P_VSA_i_1, P_VSA_i_2, P_VSA_i_3, P_VSA_i_4, P_VSA_s_1, P_VSA_s_2, P_VSA_s_3, P_VSA_s_4, P_VSA_s_5, P_VSA_s_6, P_VSA_ppp_L, P_VSA_ppp_P, P_VSA_ppp_N, P_VSA_ppp_D, P_VSA_ppp_A, P_VSA_ppp_ar, P_VSA_ppp_con, P_VSA_ppp_hal, P_VSA_ppp_cyc, P_VSA_ppp_ter, SsCH3, SdCH2, SssCH2, StCH, SdsCH, SaaCH, SsssCH, SddC, StsC, SdssC, SaasC, SaaaC, SssssC, SsNH2, SssNH, SdNH, SsssN, SdsN, SaaN, StN, SsNH3+, SssNH2+, SdNH2+, SsssNH+, SssssN+, SddsN, SaasN, SaaNH, SsOH, SdO, SssO, SaaO, SsssP, SdsssP, SssssssP, SsSH, SdS, SssS, SaaS, SdssS, SddssS, SsF, SsCl, SsBr, SsI, SsssB, SHED_DD, SHED_DA, SHED_DP, SHED_DN, SHED DL, SHED AA, SHED AP, SHED AN, SHED AL, SHED_PP, SHED PN, SHED PL, SHED_NN, SHED NL, SHED LL, Ro5, cRo5, DLS_01, DLS_02, DLS_03, DLS_04, DLS_05, DLS_06, DLS_07, DLS_cons, LLS_01, LLS_02.

Descriptors on a solvent (373 types)

MW, AMW, Sv, Se, Sp, Si, Mv, Me, Mp, Mi, GD, RBF, H%, C%, O%, MCD, ZM1Kup, ZM1Mad, ZM1Per, ZM1MulPer, ZM2Kup, ZM2Mad, ZM2Per, ZM2MulPer, ON0, ON0V, ON1, ON1V, DBI, SNar, HNar, GNar, Xt, Dz, LPRS, MSD, SPI, AECC, DECC, MDDD, ICR, MeanTD, MeanDD, S1K, S2K, S3K, PHI, PW2, PW3, PW4, PW5, MAXDN, MAXDP, DELS, LOC, MWC01, MWC02, MWC03, MWC04, MWC05, MWC06, MWC07, MWC08, MWC09, MWC10, SRW02, SRW04, SRW06, SRW08, SRW10, MPC01, MPC02, MPC03, MPC04, MPC05, piPC01, piPC02, piPC03, piPC04, piPC05, TWC, TPC, piID, PCD, CID, BID, ISIZ, IAC, AAC, IDE, IDM, IDDE, IDDM, IDET, IDMT, IVDE, IVDM, HVcpx, HDcpx, Uindex, Vindex, Xindex, Yindex, IC0, IC1, IC2, IC3, IC4, IC5, TIC0, TIC1, TIC2, TIC3, TIC4, TIC5, SIC0,

SIC1, SIC2, SIC3, SIC4, SIC5, CIC0, CIC1, CIC2, CIC3, CIC4, CIC5, BIC0, BIC1, BIC2, BIC3, BIC4, BIC5, ATS1m, ATS2m, ATS3m, ATS4m, ATS5m, ATS6m, ATS1v, ATS2v, ATS3v, ATS4v, ATS5v, ATS6v, ATS1e, ATS2e, ATS3e, ATS4e, ATS5e, ATS6e, ATS1p, ATS2p, ATS3p, ATS4p, ATS5p, ATS6p, ATS1i, ATS2i, ATS3i, ATS4i, ATS5i, ATS6i, ATSC1m, ATSC2m, ATSC3m, ATSC4m, ATSC5m, ATSC6m, ATSC1v, ATSC2v, ATSC3v, ATSC4v, ATSC5v, ATSC6v, ATSC1e, ATSC2e, ATSC3e, ATSC4e, ATSC5e, ATSC6e, ATSC1p, ATSC2p, ATSC3p, ATSC4p, ATSC5p, ATSC6p, ATSC1i, ATSC2i, ATSC3i, ATSC4i, ATSC5i, ATSC6i, MATS1m, MATS2m, MATS3m, MATS4m, MATS5m, MATS6m, MATS1v, MATS2v, MATS3v, MATS4v, MATS5v, MATS6v, MATS1e, MATS2e, MATS3e, MATS4e, MATS5e, MATS6e, MATS1p, MATS2p, MATS3p, MATS4p, MATS5p, MATS6p, MATS1i, MATS2i, MATS3i, MATS4i, MATS5i, MATS6i, GATS1m, GATS2m, GATS3m, GATS4m, GATS5m, GATS1v, GATS2v, GATS3v, GATS4v, GATS5v, GATS1e, GATS2e, GATS3e, GATS4e, GATS5e, GATS1p, GATS2p, GATS3p, GATS4p, GATS5p, GATS1i, GATS2i, GATS3i, GATS4i, GATS5i, GGI1, GGI2, GGI3, JGI1, JGI2, JGI3, JGT, SpMax1_Bh(m), SpMax2_Bh(m), SpMax3_Bh(m), SpMax4_Bh(m), SpMax5_Bh(m), SpMax6_Bh(m), SpMax7_Bh(m), SpMax8_Bh(m), SpMax1_Bh(v), SpMax2_Bh(v), SpMax3_Bh(v), SpMax4_Bh(v), SpMax5_Bh(v), SpMax6_Bh(v), SpMax7_Bh(v), SpMax1_Bh(e), SpMax2_Bh(e), SpMax3_Bh(e), SpMax4_Bh(e), SpMax5_Bh(e), SpMax6_Bh(e), SpMax7_Bh(e), SpMax8_Bh(e), SpMax1_Bh(p), SpMax2_Bh(p), SpMax3_Bh(p), SpMax4_Bh(p), SpMax5_Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i), SpMax8_Bh(i), SpMin1_Bh(m), SpMin2_Bh(m), SpMin3_Bh(m), SpMin4_Bh(m), SpMin5_Bh(m), SpMin1_Bh(v), SpMin2_Bh(v), SpMin3_Bh(v), SpMin4_Bh(v), SpMin5_Bh(v), SpMin1_Bh(e), SpMin2_Bh(e), SpMin3_Bh(e), SpMin4_Bh(e), SpMin1_Bh(p), SpMin2_Bh(p), SpMin3_Bh(p), SpMin4_Bh(p), SpMin5 _Bh(p), SpMin1_Bh(i), SpMin2_Bh(i), SpMin3_Bh(i), SpMin4 Bh(i), P_VSA_LogP_1, P_VSA_LogP_2, P_VSA_LogP_3, P_VSA_LogP_4, P_VSA_LogP_5, P_VSA_LogP_7, P_VSA_MR_1, P_VSA_MR_2, P_VSA_MR_3, P_VSA_MR_5, P_VSA_MR_6, P_VSA_m_1, P_VSA_m_2, P_VSA_m_3, P_VSA_v_2, P_VSA_v_3, P_VSA_e_2, P_VSA_e_5, P_VSA_i_2, P_VSA_i_3, P_VSA_s_2, P_VSA_s_3, P_VSA_s_4, P_VSA_s_6, P_VSA_pppL, P_VSA_ppp_D, P_VSA_ppp_cyc, P_VSA_ppp_ter, SsCH3, SssCH2, SsssCH, SdssC, SsOH, SdO, SssO, SHED AL, SHED LL, Uc, Ui, Hy, AMR, TPSA(NO), TPSA(Tot), MLOGP2, ALOGP, ALOGP2, SAtot, SAdon, VvdwMG, VvdwZAZ, PDI, BLTF96, DLS_02, DLS_04, DLS_05, DLS_cons.

Principal component analysis (PCA) was performed for each of the descriptors on a compound and the descriptors on a solvent to reduce dimension. Principal component analysis is a method for synthesizing variables which are a few uncorrelated variables most representing the entire dispersion (principal components) from many correlated variables. When the original variable is defined as X, the principal component is defined as T, and the residual is defined as E, principal component analysis is represented by the following formula:

[Equation 10]

$$X = AT + E$$

where A represents the weight of the variable. A is maximized so that the variance of T becomes maximum. By repeating principal component analysis for the residual E again, it is possible to obtain principal components for up to the Nth component.

When the principal components of a compound were 20 components and the descriptors on a solvent were 9 components, each cumulative contribution rate was 75.8% for compound and 91.8% for solvent.

Using 40 variables combining 20 components of the compound and 9 components each (a total of 18 components) of 2 types of solvents obtained by PCA with a solvent ratio and a crystallization temperature and a total of 860 variables further combining their interaction terms as explanatory variables, partial least squares regression (PLSR) was performed with the logarithm of a critical degree of supersaturation as an objective variable.

Partial least squares regression is one of linear regression methods, and a method for synthesizing variables which are a few uncorrelated variables and contribute to regression of objective variables from many correlated variables, which is a method that can be applied even when the number of data is fewer than the number of explanatory variables. When the explanatory variable is defined as X, the objective variable is defined as y, the latent variable is defined as T, one projected to each coordinate axis X is defined as P, and one projected to y is defined as q, the basic formula is represented by the following two formulas:

[Equation 11]

$$\mathbf{X} = \mathbf{T}\,\mathbf{P}^{\mathrm{T}} + \mathbf{E}$$
$$\mathbf{y} = \mathbf{T}\,\mathbf{q} + \mathbf{f}$$

where E and f are the residual of X and y, respectively. T is defined by the following formula:

[Equation 12]

$$T = XW$$

The norm of W is 1 (constraint condition). At this time, by determining W so that the covariance of T and y becomes maximum, and performing simple regression using T, X, and y thus obtained, P and q are determined. By repeating partial least squares regression using the residuals E and f again, it is possible to determine the regression equation for up to the Nth component.

As an evaluation index of the generalization performance of the model created, a coefficient of determination (R2) was used. The coefficient of determination is an index showing a good fit of a model represented by the following formula, and the value is 1 in the case of the best fit, and the value is smaller in the case of a bad fit.

[Equation 13]

$$R^2 = 1 - \frac{\Sigma_i \left(y_i - f(x_i)\right)^2}{\Sigma_i (y_i - \overline{y_i})^2}$$

where $y_i$ is an actual measured value, f(x) is a predictive value, and $\overline{y_i}$ is a mean of actual measured values.

Of 58 types of experimental data (data shown in Table 3), 70% (40 types) were used as learning data, and 30% (18 types) were used as validation data. When the number of components in PLS was determined using the R2 value in 3-fold cross validation as an evaluation function, a model with R2 for learning data of 0.888 and R2 for validation data of 0.817 was obtained in the case of the number of components of 4. FIG. 41 is a diagram in which predictive values for experimental values of a critical degree of supersaturation were plotted.

Using the model, a predictive value of a critical degree of supersaturation and a prediction interval for the substrates in Table 3 were calculated. The values obtained by converting the results outputted in a logarithmic scale to values in a linear scale are shown in Table 8. As the type of data in the table, "validation" represents data used as validation data, and "learning" represents data used as learning data.

[Table 8-1]

Table 8

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 1 | Esomeprazole magnesium hydrate | MeOH | H2O | 1 | 35 | 1.4 | Validation | 1.4 | 0.9 | 2.2 |
| 2 | Lansoprazole | MeOH | H2O | 3 | 0 | 6.9 | Learning | 8.0 | 5.1 | 12.6 |
| 3 | Clopidogrel sulfate | H2O | 2-BuOH | 222 | 20 | 9.7 | Learning | 9.8 | 6.2 | 15.5 |
| 4 | Ketotifen fumarate | MeOH | IPA | 5 | 0 | 2.5 | Validation | 4.4 | 2.8 | 7.0 |
| 5 | Ketotifen fumarate | MeOH | IPA | 5 | 20 | 4 | Learning | 4.2 | 2.7 | 6.7 |
| 6 | Ketotifen fumarate | MeOH | 2-BuOH | 5 | 0 | 2.6 | Validation | 4.5 | 2.9 | 7.2 |
| 7 | Ketotifen fumarate | MeOH | 2-BuOH | 5 | 20 | 4.4 | Learning | 4.4 | 2.8 | 7.0 |
| 8 | Clarithromycin | THF | H2O | 25 | 0 | 21 | Validation | 17.1 | 10.8 | 27.0 |
| 9 | Clarithromycin | THF | H2O | 20 | 0 | 26 | Learning | 17.2 | 10.9 | 27.1 |
| 10 | Clarithromycin | THF | H2O | 25 | 10 | 17 | Learning | 18.8 | 11.9 | 29.7 |
| 11 | Azithromycin | IPA | H2O | 10 | 0 | 3.2 | Learning | 5.1 | 3.2 | 8.0 |
| 12 | DL-glutamic acid | H2O | Acetone | 5 | 0 | 45.8 | Learning | 57.4 | 36.4 | 90.7 |
| 13 | DL-glutamic acid | H2O | THF | 5 | 0 | 24.3 | Validation | 23.0 | 14.6 | 36.4 |
| 14 | Azithromycin | IPA | H2O | 20 | 0 | 2.6 | Learning | 5.0 | 3.2 | 7.9 |
| 15 | Azithromycin | Acetone | H2O | 20 | 0 | 6.2 | Learning | 6.7 | 4.2 | 10.5 |
| 16 | Azithromycin | EtOH | H2O | 20 | 0 | 7.1 | Learning | 5.3 | 3.3 | 8.3 |
| 17 | Azithromycin | MeOH | H2O | 20 | 0 | 7.5 | Validation | 5.4 | 3.4 | 8.5 |
| 18 | Azithromycin | IPA | H2O | 10 | 20 | 11.1 | Learning | 6.1 | 3.8 | 9.6 |
| 19 | Esomeprazole magnesium hydrate | EtOH | H2O | 1 | 35 | 1.6 | Learning | 2.0 | 1.3 | 3.1 |
| 20 | Lansoprazole | MeOH | H2O | 3 | 15 | 12.6 | Learning | 7.5 | 4.7 | 11.8 |
| 21 | Escitalopram oxalate | H2O | 2-BuOH | 30 | 0 | 2.8 | Validation | 4.0 | 2.5 | 6.2 |

(continued)

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 22 | Escitalopram oxalate | H2O | 2-BuOH | 15 | 0 | 3.7 | Learning | 3.9 | 2.5 | 6.1 |
| 23 | Escitalopram oxalate | H2O | IPA | 15 | 0 | 4 | Learning | 3.8 | 2.4 | 5.9 |
| 24 | Dabigatran etexilate methanesulfonate | MeOH | TBME | 10 | 20 | 3.3 | Learning | 3.5 | 2.2 | 5.6 |
| 25 | Dabigatran etexilate methanesulfonate | EtOH | EtOAc | 10 | 5 | 2.9 | Learning | 3.9 | 2.4 | 6.1 |
| 26 | Dabigatran etexilate methanesulfonate | EtOH | EtOAc | 10 | 20 | 4.3 | Learning | 3.4 | 2.2 | 5.4 |
| 27 | Dabigatran etexilate methanesulfonate | EtOH | EtOAc | 10 | 35 | 5.2 | Validation | 3.0 | 1.9 | 4.7 |

[Table 8-2]

| | Substrate | Solvent 1 | Solvent 2 | Solvent 2/solvent 1 | Temperature during crystallization | Critical degree of supersaturation S | Type of data | Critical degree of supersaturation S | Lower 95% prediction | upper 95% prediction |
|---|---|---|---|---|---|---|---|---|---|---|
| | | | | Ratio | (°C) | Experimental value | | Predictive value | | |
| 28 | Theophylline magnesium salt | MeOH | H2O | 3 | 0 | 3.5 | Learning | 3.6 | 2.3 | 5.6 |
| 29 | Theophylline magnesium salt | EtOH | H2O | 3 | 0 | 3.9 | Validation | 3.6 | 2.3 | 5.7 |
| 30 | Theophylline magnesium salt | Acetone | H2O | 3 | 0 | 6.3 | Learning | 6.4 | 4.0 | 10.1 |
| 31 | Theophylline magnesium salt | IPA | H2O | 3 | 0 | 6.6 | Learning | 4.5 | 2.9 | 7.1 |
| 32 | Teneligliptin hydrobromide hydrate | MeOH | 1-BuOH | 10 | 0 | 5.2 | Learning | 6.7 | 4.2 | 10.5 |
| 33 | Teneligliptin hydrobromide hydrate | MeOH | 1-BuOH | 10 | 20 | 7.9 | Learning | 6.8 | 4.3 | 10.7 |
| 34 | Teneligliptin hydrobromide hydrate | MeOH | 1-BuOH | 10 | 40 | 12.6 | Validation | 6.6 | 4.2 | 10.5 |
| 35 | Pilsicainide hydrochloride anhydride | IPA | Toluene | 10 | 5 | 27 | Learning | 24.5 | 15.5 | 38.7 |
| 36 | Tramadol hydrochloride | MeOH | i-PrOAc | 20 | 10 | 4.2 | Learning | 6.9 | 4.3 | 10.8 |
| 37 | Tramadol hydrochloride | IPA | TBME | 30 | 10 | 11.1 | Learning | 17.3 | 10.9 | 27.3 |
| 38 | Tramadol hydrochloride | EtOH | TBME | 30 | 10 | 22.4 | Learning | 17.0 | 10.8 | 26.9 |
| 39 | Vildagliptin | EtOH | TBME | 10 | 0 | 9.2 | Validation | 8.7 | 5.5 | 13.8 |
| 40 | Vildagliptin | EtOH | TBME | 10 | 10 | 7 | Learning | 8.6 | 5.5 | 13.6 |
| 41 | Vildagliptin | MEK | Toluene | 10 | 0 | 8.5 | Learning | 8.3 | 5.3 | 13.1 |

(continued)

| | Substrate | Solvent 1 | Solvent 2 | Solvent 2/solvent 1 | Temperature during crystallization | Critical degree of supersaturation S | Type of data | Critical degree of supersaturation S | Lower95% prediction | upper95% prediction |
|---|---|---|---|---|---|---|---|---|---|---|
| | | | | Ratio | (°C) | Experimental value | | Predictive value | | |
| 42 | Vildagliptin | IPA | TBME | 10 | 0 | 11 | Learning | 9.3 | 5.9 | 14.7 |
| 43 | Vildagliptin | IPA | TBME | 10 | 10 | 11.6 | Learning | 9.3 | 5.9 | 14.7 |
| 44 | Vildagliptin | IPA | TBME | 10 | 20 | 11.9 | Validation | 9.2 | 5.8 | 14.5 |
| 45 | Linagliptin | EtOH | TBME | 15 | 0 | 7.9 | Validation | 8.8 | 5.6 | 13.9 |
| 46 | Linagliptin | EtOH | TBME | 15 | 20 | 6.7 | Learning | 8.3 | 5.3 | 13.1 |
| 47 | Glutathione | H2O | EtOH | 5 | 20 | 18.7 | Validation | 13.3 | 8.4 | 21.0 |
| 48 | Mirabegron | H2O | MeOH | 1.25 | 0 | 1.6 | Learning | 2.1 | 1.3 | 3.3 |
| 49 | Mirabegron | H2O | MeOH | 1.25 | 10 | 3.2 | Learning | 2.1 | 1.3 | 3.3 |
| 50 | Mirabegron | MeOH | TBME | 10 | 0 | 4 | Learning | 4.3 | 2.7 | 6.7 |
| 51 | Mirabegron | MeOH | TBME | 10 | 10 | 4.3 | Learning | 4.2 | 2.6 | 6.6 |
| 52 | Mirabegron | EtOH | TBME | 10 | 0 | 5.6 | Validation | 6.0 | 3.8 | 9.4 |
| 53 | Mirabegron | EtOH | TBME | 10 | 10 | 5.4 | Validation | 5.7 | 3.6 | 9.0 |
| 54 | Tolvaptan | MeOH | H2O | 0.4 | 0 | 4.8 | Validation | 5.8 | 3.7 | 9.2 |
| 55 | Tolvaptan | MeOH | H2O | 0.4 | 10 | 7.1 | Validation | 5.6 | 3.6 | 8.9 |
| 56 | Valacvclovir hydrochloride | H2O | 2-BuOH | 35 | 10 | 48.6 | Learning | 53.4 | 33.8 | 84.4 |
| 57 | Bepotastine besilate | EtOH | AcOiPr | 10 | 0 | 36.4 | Learning | 26.5 | 16.8 | 41.8 |
| 58 | Olopatadine | MeOH | EtOAc | 20 | 15 | 9.7 | Learning | 7.9 | 5.0 | 12.5 |

Even when a crystallization temperature is not used as an explanatory variable, it was possible to create a predictive model of a critical degree of supersaturation by partial least squares regression using components of a compound, components of two types of solvents, and a solvent ratio. In this model, it was also confirmed that a critical degree of supersaturation predicted based on learning data is fitted well to validation data. Even when variables for a solvent used for crystallization are not used, it was possible to create a predictive model of a critical degree of supersaturation by partial least squares regression using components of a compound and a solution temperature during crystallization. In this model, it was also confirmed that a critical degree of supersaturation predicted based on learning data is fitted well to validation data.

Example 19

Escitalopram Oxalate (Crystallization by Reverse Addition)

To 0.5 g of escitalopram oxalate, 1 mL of water was added, followed by dissolution at 60 °C. This solution was added dropwise to 15 mL of 2-butanol at 0 °C over about 1 second to prepare a supersaturated solution of escitalopram oxalate. The degree of supersaturation of the supersaturated solution at the end of dropwise addition was 7.4. This value was higher than the actual measured value (3.7) of the critical degree of supersaturation shown in No. 22 in Table 3 mentioned above. 27 minutes after the end of dropwise addition, a crystal began to be precipitated, and after stirring was continued for 2 hours, the crystal was collected by filtration, followed by drying under reduced pressure at room temperature for 15 hours to obtain a spherulite of escitalopram oxalate (quantitative yield of 86%: calculated by measuring the supernatant concentration of the mother liquor by HPLC). The $d_{50}$ of the crystal thus obtained was 50.7 μm, the sharpness index was 4.87, and the sphericity was 0.98 ± 0.01 (see FIGs. 54 to 56).

Example 20

Vildagliptin (Crystallization by Reverse Addition)

To 0.8 g of vildagliptin, 1.0 mL of ethanol was added, followed by dissolution at 80 °C. This solution was added dropwise to 10 mL of t-butyl methyl ether at 0 °C over about 1 second to prepare a supersaturated solution of vildagliptin. The supersaturation ratio of the supersaturated solution at the end of dropwise addition was 21.5. This value was higher than the actual measured value (9.2) of the critical degree of supersaturation shown in No. 39 in Table 3 mentioned above. 20 minutes after the end of dropwise addition, a crystal began to be precipitated, and after stirring was continued for 1 hour, the crystal was collected by filtration, followed by drying under reduced pressure at room temperature for 18 hours to obtain a spherulite of vildagliptin (0.187 g, 23%). The sharpness index of the crystal thus obtained was 1.66, the $d_{50}$ was 246 μm, and the sphericity was 0.99 ± 0.01 (see FIGs. 57 to 59).

Example 21

Linagliptin (Crystallization by Reverse Addition)

To 0.5 g of linagliptin, 2.5 mL of ethanol was added, followed by dissolution at 70 °C. This solution was added dropwise to 37.5 mL of t-butyl methyl ether at 0 °C over about 1 second to prepare a supersaturated solution of linagliptin. The degree of supersaturation of the supersaturated solution at the end of dropwise addition was 23.3. This value was higher than the actual measured value (7.9) of the critical degree of supersaturation shown in No. 45 in Table 3 mentioned above. 32 minutes after the end of dropwise addition, a crystal began to be precipitated, and after stirring was continued for 5 hours, the crystal was collected by filtration, followed by drying under reduced pressure at room temperature for 17 hours to obtain a spherulite of linagliptin (quantitative yield of 93%: calculated by measuring the supernatant concentration of the mother liquor by HPLC). The $d_{50}$ of the crystal thus obtained was 69.1 μm, the sharpness index was 2.42, and the sphericity was 0.98 ± 0.01 (see FIGs. 60 to 62).

Example 22

Teneligliptin Hydrobromide Hydrate (Crystallization by Reverse Addition)

To 1.0 g of teneligliptin hydrobromide hydrate, 4 mL of methanol was added, followed by dissolution at 60 °C. This solution was added dropwise to 40 mL of 1-butanol at 20 °C over about 1 second to prepare a supersaturated solution of teneligliptin hydrobromide hydrate. The degree of supersaturation of the supersaturated solution at the end of dropwise addition was 13.7. This value was higher than the actual measured value (7.9) of the critical degree of

supersaturation shown in No. 33 in Table 3 mentioned above. After stirring was continued for 18 hours, the crystal was collected by filtration, followed by drying under reduced pressure at room temperature for 24 hours to obtain a spherulite of teneligliptin hydrobromide hydrate (quantitative yield of 93%: calculated by measuring the supernatant concentration of the mother liquor by HPLC). The $d_{50}$ of the crystal thus obtained was 33.5 μm, the sharpness index was 3.77, and the sphericity was 0.93 ± 0.04 (see FIGs. 63 to 65).

Example 23

Glutathione (Crystallization by Reverse Addition)

To 1.0 g of glutathione, 10 mL of water was added, followed by dissolution at 30 °C. This solution was added dropwise to 50 mL of ethanol at 20 °C over about 3 seconds to prepare a supersaturated solution of glutathione. The degree of supersaturation of the supersaturated solution at the end of dropwise addition was 46.1. This value was higher than the actual measured value (18.7) of the critical degree of supersaturation shown in No. 47 in Table 3 mentioned above. After stirring was continued for 20 hours, the crystal was collected by filtration, followed by drying under reduced pressure at room temperature for 24 hours to obtain a spherulite of glutathione (0.97 g, 97%: calculated by measuring the supernatant concentration of the mother liquor by HPLC). The $d_{50}$ of the crystal thus obtained was 48.4 μm, the sharpness index was 2.27, and the sphericity was 0.85 ± 0.07 (see FIGs. 66 to 68).

Example 24

Production of Spherulite of Dabigatran Etexilate Methanesulfonate (Crystallization by Reverse Addition)

To 0.8 g of dabigatran etexilate methanesulfonate, 4 mL of ethanol was added, followed by dissolution at 60 °C. This solution was added dropwise to 40 mL of ethyl acetate at 20 °C over about 3 seconds to prepare a supersaturated solution. The degree of supersaturation of the supersaturated solution at the end of dropwise addition was 82. This value was higher than the actual measured value (4.3) of the critical degree of supersaturation shown in No. 26 in Table 3 mentioned above. About 4 minutes after the end of dropwise addition, a crystal began to be precipitated, and after 42 minutes, the crystal was collected by filtration. Drying under reduced pressure was performed at 40 °C to obtain a spherulite of dabigatran etexilate methanesulfonate (quantitative yield: 98.7%). The sharpness index of the crystal thus obtained was 2.61, the $d_{50}$ was 61.3 μm, and the sphericity was 0.95 ± 0.03 (see FIGs. 69 to 71).

Example 25

Production of Spherulite of Pilsicainide Hydrochloride (Crystallization by Reverse Addition)

To 1.5 g of pilsicainide hydrochloride mono/dihydrate, 4.5 mL of isopropanol was added, followed by dissolution at 80 °C. This solution was added dropwise to 45 mL of toluene at 5 °C over about 3 seconds to prepare a supersaturated solution. The degree of supersaturation of the supersaturated solution at the end of dropwise addition was 27. This value was the same as the actual measured value (27) of the critical degree of supersaturation shown in No. 35 in Table 3 mentioned above. About 3 minutes after the end of dropwise addition, a crystal began to be precipitated, and after 1 hour, the crystal was collected by filtration. Drying under reduced pressure was performed at 40 °C to obtain a spherulite of pilsicainide hydrochloride anhydride (quantitative yield: 93.6%). The sharpness index of the crystal thus obtained was 2.29, the $d_{50}$ was 116 μm, and the sphericity was 0.69 ± 0.18 (see FIGs. 72 to 74).

Example 26

Theophylline Magnesium Salt Tetrahydrate (Crystallization by Salt Formation)

To 0.333 g of magnesium chloride hexahydrate, 2.50 mL of water and 2.50 mL of methanol were added, followed by dissolution under room temperature, and then the solution was cooled to 0 °C. To 0.236 g of theophylline, 5.00 mL of water and 0.0734 g of potassium hydroxide were added, followed by dissolution under room temperature, and the solution thus obtained was added dropwise to the magnesium chloride solution over 10 minutes. The supersaturation ratio of the supersaturated solution immediately after dropwise addition was 8.4. This value was higher than the actual measured value (3.5) of the critical degree of supersaturation shown in No. 28 in Table 3 mentioned above. About 1 minute after the end of dropwise addition, a crystal began to be precipitated, and after allowing to stand for 30 minutes, the crystal was collected by filtration, followed by drying under reduced pressure at room temperature for 18 hours to obtain a spherulite of theophylline magnesium salt tetrahydrate (0.140 g, 47%). The sharpness index of the crystal thus

obtained was 3.06, the $d_{50}$ was 43.0 μm, and the sphericity was 0.95 ± 0.02 (see FIGs. 75 to 77).

Example 27

Mirabegron (Crystallization by Reverse Addition)

To 0.051 g of mirabegron, 1.0 mL of ethanol was added, followed by dissolution at 80 °C. This solution was added dropwise to 10 mL of t-butyl methyl ether at 0 °C over about 1 second to prepare a supersaturated solution of mirabegron, and then 2,000 ppm of a seed crystal was added. The supersaturation ratio of the supersaturated solution at the end of dropwise addition was 6.9. This value was higher than the actual measured value (5.6) of the critical degree of supersaturation shown in No. 52 in Table 3 mentioned above. 35 minutes after inoculation, a crystal began to be precipitated, and after stirring was continued for 19 hours, the crystal was collected by filtration, followed by drying under reduced pressure at room temperature for 18 hours to obtain a spherulite of mirabegron (0.027 g, 53%). The sharpness index of the crystal thus obtained was 1.66, the $d_{50}$ was 148 μm, and the sphericity was 0.98 ± 0.01 (see FIGs. 78 to 80).

Example 28

Tolvaptan (Crystallization by Normal Addition)

To 0.1 g of tolvaptan, 5.0 mL of methanol was added, followed by dissolution at room temperature. This solution was cooled to 0 °C, and 2.0 mL of water at room temperature was added dropwise over about 1 second to prepare a supersaturated solution of tolvaptan. The supersaturation ratio of the supersaturated solution at the end of dropwise addition was 6.6. This value was higher than the actual measured value (4.8) of the critical degree of supersaturation shown in No. 54 in Table 3 mentioned above. About 1 hour after the end of dropwise addition, a crystal began to be precipitated, and after stirring was continued for 1.5 hours, the crystal was collected by filtration, followed by drying under reduced pressure at room temperature for 18 hours to obtain a spherulite of tolvaptan (0.031 g, 31%). The mean particle size of the crystal thus obtained was 145 μm, and the sphericity of the crystal thus obtained was 0.94 ± 0.02 (see FIGs. 81 and 82).

Example 29

Production of Spherulite of Tramadol Hydrochloride (Crystallization by Reverse Addition)

To 0.5 g of tramadol hydrochloride, 0.5 mL of methanol was added, followed by dissolution at 30 °C. This solution was added dropwise to 10 mL of isopropyl acetate at 10 °C over about 1 second to prepare a supersaturated solution. The degree of supersaturation of the supersaturated solution at the end of dropwise addition was 13. This value was higher than the actual measured value (4.2) of the critical degree of supersaturation shown in No. 36 in Table 3 mentioned above. About 25 minutes after the end of dropwise addition, a crystal had been already precipitated, and after 70 minutes, the crystal was collected by filtration. Drying under reduced pressure was performed at room temperature to obtain a spherulite of tramadol hydrochloride. The mean particle size of the spherulite thus obtained was 319 μm, and the sphericity of the spherulite was 0.90 ± 0.08 (see FIGs. 83 and 84).

Example 30

Bepotastine Besilate (Crystallization by Reverse Addition)

To 1.0 g of bepotastine besilate, 4 mL of ethanol was added, followed by dissolution under reflux conditions. This solution was added dropwise to 40 mL of isopropyl acetate at 0 °C over about 3 seconds to prepare a supersaturated solution of bepotastine besilate. The degree of supersaturation of the supersaturated solution at the end of dropwise addition was 143. This value was higher than the actual measured value (36.4) of the critical degree of supersaturation shown in No. 57 in Table 3 mentioned above. After stirring was continued for 15 hours, the crystal was collected by filtration, followed by drying under reduced pressure at room temperature for 24 hours to obtain a spherulite of bepotastine besilate (0.99 g, 99%: calculated by measuring the supernatant concentration of the mother liquor by HPLC). The $d_{50}$ of the crystal thus obtained was 105 μm, the sharpness index was 2.15, and the sphericity was 0.98 ± 0.01 (see FIGs. 85 to 87).

Example 31

Production of Spherulite of Olopatadine (Crystallization by Reverse Addition)

To 1.0 g of olopatadine, 2.5 mL of methanol was added, followed by reflux to dissolve. This solution was added dropwise to 50 mL of ethyl acetate at 15 °C over about 1 second to prepare a supersaturated solution. The degree of supersaturation of the supersaturated solution at the end of dropwise addition was 15. This value was higher than the actual measured value (9.7) of the critical degree of supersaturation shown in No. 58 in Table 3 mentioned above.

One hour after the end of dropwise addition, a crystal had been already precipitated, and after 3.5 hours, the crystal was collected by filtration. Drying under reduced pressure was performed at room temperature to obtain a spherulite of olopatadine. The mean particle size of the spherulite thus obtained was 167 μm, and the sphericity of the spherulite was 0.96 ± 0.02 (see FIGs. 88 and 89).

Example 32

Building of Predictive Model of Critical Degree of Supersaturation (II)

Two-dimensional descriptors were calculated using alvaDesc 1.0 for 21 types of compounds for which a critical degree of supersaturation was obtained. When descriptors having the same value for all compounds, descriptors which could not be calculated for one or more compounds, and one descriptor of a set of descriptors having a correlation coefficient of 1.0 were deleted, 1,905 descriptors remained. Similarly, two-dimensional descriptors were calculated using alvaDesc 1.0 for 13 types of solvents used for obtaining a critical degree of supersaturation. When descriptors having the same value for all solvents, descriptors which could not be calculated for one or more solvents, and one descriptor of a set of descriptors having a correlation coefficient of 1.0 were deleted, 373 descriptors remained. Arrangement of a descriptor on a compound, a descriptor on a good solvent, a descriptor on a poor solvent, a solvent ratio, and a crystallization temperature was regarded as one piece of data, and a data set consisting of 58 pieces of data was created. When descriptors having the same value for 80% or more were deleted within the data set, 2,100 variables remained in total.

Descriptors on a compound (1,905 types)

MW, AMW, Sv, Se, Sp, Si, Mv, Me, Mp, Mi, GD, nAT, nSK, nTA, nBT, nBO, nBM, SCBO, RBN, RBF, nDB, nTB, nAB, nH, nC, nN, nO, nS, nF, nCL, nHM, nHet, nX, H%, C%, N%, O%, X%, nCsp3, nCsp2, nCsp, max_conj_path, nCIC, nCIR, TRS, Rperim, Rbrid, MCD, RFD, RCI, NRS, NNRS, nR05, nR06, nR07, nR08, nR09, nR10, nR11, nBnz, ARR, D/Dtr05, D/Dtr06, D/Dtr07, D/Dtr08, D/Dtr09, D/Dtr10, D/Dtr11, ZM1, ZM1V, ZM1Kup, ZM1Mad, ZM1Per, ZM1MulPer, ZM2, ZM2V, ZM2Kup, ZM2Mad, ZM2Per, ZM2MulPer, ON0, ON0V, ON1, ON1V, Qindex, BBI, DBI, SNar, HNar, GNar, Xt, Dz, Ram, BLI, Pol, LPRS, MSD, SPI, PJI2, ECC, AECC, DECC, MDDD, UNIP, CENT, VAR, ICR, MaxTD, MeanTD, MaxDD, MeanDD, SMTI, SMTIV, GMTI, GMTIV, Xu, CSI, Wap, S1K, S2K, S3K, PHI, PW2, PW3, PW4, PW5, MAXDN, MAXDP, DELS, TIE, Psi_i_s, Psi_i_0, Psi_i_1, Psi_i_t, Psi_i_0d, Psi_i_1d, Psi_i_1s, Psi_e_A, Psi_e_0, Psi_e_1, Psi_e_0d, BAC, LOC, MWC01, MWC02, MWC03, MWC04, MWC05, MWC06, MWC07, MWC08, MWC09, MWC10, SRW02, SRW04, SRW05, SRW06, SRW07, SRW08, SRW09, SRW10, MPC02, MPC03, MPC04, MPC05, MPC06, MPC07, MPC08, MPC09, MPC10, piPC01, piPC02, piPC03, piPC04, piPC05, piPC06, piPC07, piPC08, piPC09, piPC10, TWC, TPC, piID, PCR, PCD, CID, BID, X0, X1, X2, X3, X4, X5, X0A, X1A, X2A, X3A, X4A, X5A, X0v, X1v, X2v, X3v, X4v, X5v, X0Av, X1Av, X2Av, X3Av, X4Av, X5Av, X0sol, X1sol, X2sol, X3sol, X4sol, X5sol, XMOD, RDCHI, RDSQ, X1Kup, X1Mad, X1Per, X1MulPer, ISIZ, AAC, IDE, IDM, IDDE, IDDM, IDET, IDMT, IVDE, IVDM, Ges, rGes, S0K, HVcpx, HDcpx, Uindex, Vindex, Xindex, Yindex, IC0, IC1, IC2, IC3, IC4, IC5, TICO, TIC1, TIC2, TIC3, TIC4, TIC5, SICO, SIC1, SIC2, SIC3, SIC4, SIC5, CIC0, CIC1, CIC2, CIC3, CIC4, CIC5, BICO, BIC1, BIC2, BIC3, BIC4, BIC5, J_A, SpPos_A, SpPosLog_A, SpMax_A, SpMaxA_A, SpDiam_A, SpMAD_A, Ho_A, EE_A, VE1_A, VE2_A, VE3 A, VElsign A, VE2signA, VR1_A, VR2_A, VR3_A, Wi_D, AVS_D, H_D, Chi_D, ChiA_D, J_D, HyWi_D, SpPos_D, SpPosA_D, SpPosLog_D, SpMaxA_D, SpDiam_D, Ho_D, SM2_D, SM3_D, SM4_D, SM5_D, SM6_D, QW_L, TI1_L, TI2_L, STN_L, SpPosA_L, SpPosLog_L, SpMax_L, SpMaxA_L, SpDiam_L, SpAD_L, SpMAD_L, Ho_L, EE_L, SM2_L, SM3_L, SM4_L, SM5_L, SM6_L, VE1_L, VE2_L, VE3_L, VE1sign_L, VE2sign_L, VE3sign_L, VR1_L, VR2_L, VR3_L, AVS_X, H_X, Chi_X, ChiA_X, J_X, HyWi_X, SpPos_X, SpPosA_X, SpPosLog_X, SpMaxA_X, SpDiam_X, SpMAD_X, Ho_X, EE_X, SM2_X, SM3_X, SM4_X, SM5_X, SM6_X, VE1_X, VE2_X, VE3_X, VE1sign_X, VE2sign_X, VR1_X, VR2_X, VR3_X, Wi_H2, WiA_H2, AVS_H2, Chi_H2, ChiA_H2, J_H2, HyWi_H2, SpPos_H2, SpPosA_H2, SpPosLog_H2, SpMax_H2, SpMaxA_H2, SpDiam_H2, Ho_H2, EE_H2, SM2_H2, SM3_H2, SM4_H2, SM5_H2, SM6_H2, VE1_H2, VE2_H2, VE3_H2, VE1sign_H2, VE2sign_H2, VR1_H2, VR2_H2, VR3_H2, Wi_Dt, AVS_Dt, H_Dt, Chi_Dt, ChiA_Dt, J_Dt, HyWi_Dt, SpPos_Dt, SpPosA_Dt, SpPosLog_Dt, SpMax_Dt, SpMaxA_Dt, SpDiam_Dt, Ho_Dt,

SM2_Dt, SM3_Dt, SM4_Dt, SM5_Dt, SM6_Dt, Wi_D/Dt, WiA_D/Dt, AVS_D/Dt, H_D/Dt, Chi_D/Dt, ChiA_D/Dt, J_D/Dt, HyWi_D/Dt, SpPos_D/Dt, SpPosA_D/Dt, SpPosLog_D/Dt, SpMax_D/Dt, SpMaxA_D/Dt, SpDiam_D/Dt, Ho_D/Dt, EE_D/Dt, SM2_D/Dt, SM3_D/Dt, SM4_D/Dt, SM5_D/Dt, SM6_D/Dt, Wi_Dz(Z), WiA_Dz(Z), AVS_Dz(Z), H_Dz(Z), Chi_Dz(Z), ChiA_Dz(Z), J_Dz(Z), HyWi_Dz(Z), SpAbs_Dz(Z), SpPos_Dz(Z), SpPosA_Dz(Z), SpPosLog_Dz(Z), SpMax _Dz(Z), SpMaxA_Dz(Z), SpDiam_Dz(Z), SpAD_Dz(Z), SpMAD_Dz(Z), Ho_Dz(Z), SM1_Dz(Z), SM2_Dz(Z), SM3_Dz(Z), SM4_Dz(Z), SM5_Dz(Z), SM6_Dz(Z), VE1_Dz(Z), VE2_Dz(Z), VE3_Dz(Z), VElsign_Dz(Z), VE2sign_Dz(Z), VR1_Dz(Z), VR2_Dz(Z), VR3_Dz(Z), WiDz(m), WiA_Dz(m), AVS_Dz(m), H_Dz(m), Chi_Dz(m), ChiA_Dz(m), J_Dz(m), HyWi_Dz(m), SpAbs_Dz(m), SpPos_Dz(m), SpPosA_Dz(m), SpPosLog_Dz(m), SpMax_Dz(m), SpMaxA_Dz(m), SpDiam_Dz(m), SpAD_Dz(m), SpMAD_Dz(m), Ho_Dz(m), SM1_Dz(m), SM2_Dz(m), SM3_Dz(m), SM4_Dz(m), SM5_Dz(m), SM6_Dz(m), VE1_Dz(m), VE2_Dz(m), VE3_Dz(m), VElsign_Dz(m), VE2sign_Dz(m), VR1_Dz(m), VR2_Dz(m), VR3_Dz(m), Wi_Dz(v), WiA_Dz(v), AVS_Dz(v), H_Dz(v), Chi_Dz(v), ChiA_Dz(v), J_Dz(v), HyWi_Dz(v), SpAbs_Dz(v), SpPos_Dz(v), SpPosA_Dz(v), SpPosLog_Dz(v), SpMaxA_Dz(v), SpDiam_Dz(v), SpAD_Dz(v), SpMAD_Dz(v), Ho_Dz(v), EE_Dz(v), SM1_Dz(v), SM2_Dz(v), SM3_Dz(v), SM4_Dz(v), SM5_Dz(v), SM6_Dz(v), VE1_Dz(v), VE2_Dz(v), VE3_Dz(v), VElsign_Dz(v), VE2sign_Dz(v), VE3sign_Dz(v), VR1_Dz(v), VR2_Dz(v), VR3_Dz(v), Wi_Dz(e), WiA_Dz(e), AVS_Dz(e), H_Dz(e), Chi_Dz(e), ChiA_Dz(e), J_Dz(e), HyWi_Dz(e), SpAbs_Dz(e), SpPos_Dz(e), SpPosA_Dz(e), SpPosLog_Dz(e), SpMax_Dz(e), SpMaxA_Dz(e), SpDiam_Dz(e), SpAD_Dz(e), SpMAD_Dz(e), Ho_Dz(e), EE_Dz(e), SM1_Dz(e), SM2_Dz(e), SM3_Dz(e), SM4_Dz(e), SM5_Dz(e), SM6_Dz(e), VE1_Dz(e), VE2_Dz(e), VE3_Dz(e), VElsign_Dz(e), VE2sign_Dz(e), VR1_Dz(e), VR2_Dz(e), VR3_Dz(e), Wi_Dz(p), WiA_Dz(p), AVS_Dz(p), H_Dz(p), Chi_Dz(p), ChiA_Dz(p), J_Dz(p), HyWi_Dz(p), SpAbs_Dz(p), SpPos_Dz(p), SpPos _Dz(p), SpPosLog_Dz(p), SpMax_Dz(p), SpMaxA_Dz(p), SpDiam_Dz(p), SpAD_Dz(p), SpMAD_Dz(p), Ho_Dz(p), EE Dz(p), SM1_Dz(p), SM2_Dz(p), SM3_Dz(p), SM4_Dz(p), SM5_Dz(p), SM6_Dz(p), VE1_Dz(p), VE2_Dz(p), VE3_Dz(p), VElsign_Dz(p), VE2sign_Dz(p), VE3sign_Dz(p), VR1_Dz(p), VR2_Dz(p), VR3_Dz(p), Wi_Dz(i), WiA_Dz(i), AVS_Dz(i), H_Dz(i), Chi_Dz(i), ChiA_Dz(i), J_Dz(i), HyWi_Dz(i), SpAbs_Dz(i), SpPos_Dz(i), SpPosA_Dz(i), SpPosLog_Dz(i), SpMaxA_Dz(i), SpDiam_Dz(i), SpAD_Dz(i), SpMAD_Dz(i), Ho_Dz(i), EE_Dz(i), SM1_Dz(i), SM2_Dz(i), SM3_Dz(i), SM4_Dz(i), SM5_Dz(i), SM6_Dz(i), VE1_Dz(i), VE2_Dz(i), VE3_Dz(i), VElsign_Dz(i), VE2sign_Dz(i), VR1_Dz(i), VR2_Dz(i), VR3_Dz(i), Wi_B(m), WiA_B(m), AVS_B(m), Chi_B(m), ChiA_B(m), J_B(m), HyWi_B(m), SpAbs_B(m), SpPos_B(m), SpPosA_B(m), SpPosLog_B(m), SpMax_B(m), SpMaxA_B(m), SpDiam_B(m), SpAD_B(m), SpMAD_B(m), Ho_B(m), EE_B(m), SM1_B(m), SM2_B(m), SM3_B(m), SM4_B(m), SM5_B(m), SM6_B(m), VE1_B(m), VE2_B(m), VE3_B(m), VElsign_B(m), VE2sign_B(m), VE3sign_B(m), VR1_B(m), VR2_B(m), VR3_B(m), Wi_B(v), WiA_B(v), AVS_B(v), Chi_B(v), ChiA_B(v), J_B(v), HyWi_B(v), SpAbs_B(v), SpPos_B(v), SpPosA_B(v), SpPosLog_B(v), SpMax_B(v), SpMaxA_B(v), SpDiam_B(v), SpAD_B(v), SpMAD_B(v), Ho_B(v), EE_B(v), SM1_B(v), SM2_B(v), SM3_B(v), SM4_B(v), SM5_B(v), SM6_B(v), VE1_B(v), VE2_B(v), VE3_B(v), VE1sign_B(v), VE2sign_B(v), VE3sign_B(v), VR1_B(v), VR2_B(v), VR3_B(v), Wi_B(e), WiA_B(e), AVS_B(e), Chi_B(e), ChiA_B(e), J_B(e), HyWi_B(e), SpAbs_B(e), SpPos_B(e), SpPosA_B(e), SpPosLog_B(e), SpMax_B(e), SpMaxA_B(e), SpDiam_B(e), SpAD_B(e), SpMAD_B(e), Ho_B(e), EE_B(e), SM1_B(e), SM2_B(e), SM3_B(e), SM4_B(e), SM5_B(e), SM6_B(e), VE1_B(e), VE2_B(e), VE3_B(e), VElsign_B(e), VE2sign_B(e), VE3sign_B(e), VR1_B(e), VR2_B(e), VR3_B(e), Wi_B(p), WiA_B(p), AVS_B(p), Chi_B(p), ChiA_B(p), J_B(p), HyWi_B(p), SpAbs_B(p), SpPos_B(p), SpPosA_B(p), SpPosLog_B(p), SpMax_B(p), SpMaxA_B(p), SpDiam_B(p), SpAD_B(p), SpMAD_B(p), Ho_B(p), EE_B(p), SM1_B(p), SM2_B(p), SM3_B(p), SM4_B(p), SM5_B(p), SM6_B(p), VE1_B(p), VE2_B(p), VE3_B(p), VElsign_B(p), VE2sign_B(p), VE3sign_B(p), VR1_B(p), VR2_B(p), VR3_B(p), Wi_B(i), WiA_B(i), AVS_B(i), Chi_B(i), ChiA_B(i), J_B(i), HyWi_B(i), SpAbs_B(i), SpPos_B(i), SpPosA_B(i), SpPosLog_B(i), SpMax_B(i), SpMaxA_B(i), SpDiam_B(i), SpAD_B(i), SpMAD B(i), Ho B(i), EE B(i), SM1_B(i), SM2 B(i), SM3 B(i), SM4 B(i), SM5_B(i), SM6_B(i), VE1_B(i), VE2_B(i), VE3_B(i), VE1sign_B(i), VE2sign_B(i), VE3sign_B(i), VR1_B(i), VR2_B(i), VR3_B(i), Wi_B(s), WiA_B(s), AVS_B(s), Chi_B(s), ChiA_B(s), J_B(s), HyWi_B(s), SpAbs_B(s), SpPos_B(s), SpPosA_B(s), SpPosLog_B(s), SpMax_B(s), SpMaxA_B(s), SpDiam_B(s), SpAD_B(s), SpMAD_B(s), Ho_B(s), EE_B(s), SM1_B(s), SM2_B(s), SM3_B(s), SM4_B(s), SM5_B(s), SM6_B(s), VE1_B(s), VE2_B(s), VE3_B(s), VE1sign_B(s), VE2sign_B(s), VE3sign_B(s), VR1_B(s), VR2_B(s), VR3_B(s), ATS1m, ATS2m, ATS3m, ATS4m, ATS5m, ATS6m, ATS7m, ATS8m, ATS1v, ATS2v, ATS3v, ATS4v, ATS5v, ATS6v, ATS7v, ATS8v, ATS1e, ATS2e, ATS3e, ATS4e, ATS5e, ATS6e, ATS7e, ATS8e, ATS1p, ATS2p, ATS3p, ATS4p, ATS5p, ATS6p, ATS7p, ATS8p, ATS1i, ATS2i, ATS3i, ATS4i, ATS5i, ATS6i, ATS7i, ATS8i, ATS1s, ATS2s, ATS3s, ATS4s, ATS5s, ATS6s, ATS7s, ATS8s, ATSC1m, ATSC2m, ATSC3m, ATSC4m, ATSC5m, ATSC6m, ATSC7m, ATSC8m, ATSC1v, ATSC2v, ATSC3v, ATSC4v, ATSC5v, ATSC6v, ATSC7v, ATSC8v, ATSC1e, ATSC2e, ATSC3e, ATSC4e, ATSC5e, ATSC6e, ATSC7e, ATSC8e, ATSC1p, ATSC2p, ATSC3p, ATSC4p, ATSC5p, ATSC6p, ATSC7p, ATSC8p, ATSC1i, ATSC2i, ATSC3i, ATSC4i, ATSC5i, ATSC6i, ATSC7i, ATSC8i, ATSC1s, ATSC2s, ATSC3s, ATSC4s, ATSC5s, ATSC6s, ATSC7s, ATSC8s, MATS1m, MATS2m, MATS3m, MATS4m, MATS5m, MATS6m, MATS7m, MATS8m, MATS1v, MATS2v, MATS3v, MATS4v, MATS5v, MATS6v, MATS7v, MATS8v, MATS1e, MATS2e, MATS3e, MATS4e, MATS5e, MATS6e, MATS7e, MATS8e, MATS1p, MATS2p, MATS3p, MATS4p, MATS5p, MATS6p, MATS7p, MATS8p, MATS1i, MATS2i, MATS3i, MATS4i, MATS5i, MATS6i, MATS7i, MATS8i, MATS1s, MATS2s, MATS3s, MATS4s, MATS5s, MATS6s, MATS7s, MATS8s, GATS1m, GATS2m, GATS3m, GATS4m, GATS5m, GATS6m,

GATS7m, GATS8m, GATS1v, GATS2v, GATS3v, GATS4v, GATS5v, GATS6v, GATS7v, GATS8v, GATS1e, GATS2e, GATS3e, GATS4e, GATS5e, GATS6e, GATS7e, GATS8e, GATS1p, GATS2p, GATS3p, GATS4p, GATS5p, GATS6p, GATS7p, GATS8p, GATS1i, GATS2i, GATS3i, GATS4i, GATS5i, GATS6i, GATS7i, GATS8i, GATS1s, GATS2s, GATS3s, GATS4s, GATS5s, GATS6s, GATS7s, GATS8s, GGI1, GGI2, GGI3, GGI4, GGI5, GGI6, GGI7, GGI8, GGI9, GGI10, JGI1, JGI2, JGI3, JGI4, JGI5, JGI6, JGI7, JGI8, JGI9, JGI10, JGT, SpMax1_Bh(m), SpMax2_Bh(m), SpMax3_Bh(m), SpMax4_Bh(m), SpMax5_Bh(m), SpMax6_Bh(m), SpMax7_Bh(m), SpMax8_Bh(m), SpMax1_Bh(v), SpMax2_Bh(v), SpMax3_Bh(v), SpMax4_Bh(v), SpMax5_Bh(v), SpMax6_Bh(v), SpMax7_Bh(v), SpMax8_Bh(v), SpMax1_Bh(e), SpMax2_Bh(e), SpMax3_Bh(e), SpMax4_Bh(e), SpMax5_Bh(e), SpMax6_Bh(e), SpMax7_Bh(e), SpMax8_Bh(e), SpMax1_Bh(p), SpMax2_Bh(p), SpMax3_Bh(p), SpMax4_Bh(p), SpMax5_Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i), SpMax8_Bh(i), SpMax1_Bh(s), SpMax2_Bh(s), SpMax3_Bh(s), SpMax4_Bh(s), SpMax5_Bh(s), SpMax6_Bh(s), SpMax7_Bh(s), SpMax8_Bh(s), SpMin1_Bh(m), SpMin2_Bh(m), SpMin3_Bh(m), SpMin4_Bh(m), SpMin5_Bh(m), SpMin6_Bh(m), SpMin7_Bh(m), SpMin8_Bh(m), SpMin1_Bh(v), SpMin2_Bh(v), SpMin3_Bh(v), SpMin4_Bh(v), SpMin5_Bh(v), SpMin6_Bh(v), SpMin7_Bh(v), SpMin8_Bh(v), SpMin1Bh(e), SpMin2_Bh(e), SpMin3_Bh(e), SpMin4_Bh(e), SpMin5_Bh(e), SpMin6_Bh(e), SpMin7_Bh(e), SpMin8_Bh(e), SpMin1Bh(p), SpMin2_Bh(p), SpMin3 _Bh(p), SpMin4 _Bh(p), SpMin5 _Bh(p), SpMin6 _Bh(p), SpMin7 _Bh(p), SpMin8_Bh(p), SpMin1Bh(i), SpMin2_Bh(i), SpMin3_Bh(i), SpMin4_Bh(i), SpMin5_Bh(i), SpMin6_Bh(i), SpMin7_Bh(i), SpMin8_Bh(i), SpMin1Bh(s), SpMin2_Bh(s), SpMin3_Bh(s), SpMin4_Bh(s), SpMin5_Bh(s), SpMin6_Bh(s), SpMin7_Bh(s), SpMin8_Bh(s), P_VSA_LogP_1, P_VSA_LogP_2, P_VSA_LogP_3, P_VSA_LogP_4, P_VSA_LogP_5, P_VSA_LogP_6, P_VSA_LogP_7, P_VSA_LogP_8, P_VSA_MR_1, P_VSA_MR_2, P_VSA_MR_3, P_VSA_MR_4, P_VSA_MR_5, P_VSA_MR_6, P_VSA_MR_7, P_VSA_MR_8, P_VSA_m_1, P_VSA_m_2, P_VSA_m_3, P_VSA_m_4, P_VSA_v_2, P_VSA_v_3, P_VSA_e_2, P_VSA_e_3, P_VSA_e_4, P_VSA_e_5, P_VSA_p_1, PVSA_p2, P_VSA_i_1, P_VSA_i_2, P_VSA_i_3, P_VSA_i_4, P_VSA_s_2, P_VSA_s_3, P_VSA_s_4, P_VSA_s_5, P_VSA_s_6, P_VSA_ppp_L, P_VSA_ppp_P, P_VSA_ppp_N, P_VSA_ppp_D, P_VSA_ppp_A, P_VSA_ppp_ar, P_VSA_ppp_con, P_VSA_ppp_hal, P_VSA_ppp_cyc, P_VSA_ppp_ter, Eta_alpha, Eta_alpha_A, Eta_epsi, Eta_epsi_A, Eta_betaS, Eta_betaS A, Eta_betaP, Eta_betaP_A, Eta_beta, Eta_beta_A, Eta_C, Eta_C_A, Eta_L, Eta_L_A, Eta_F, Eta_F_A, Eta_FL, Eta_FL_A, Eta_B, Eta_B_A, Eta_sh_p, Eta_sh_y, Eta_sh_x, Eta DAlphaA, Eta_D_AlphaB, Eta_epsi_2, Eta_epsi_3, Eta_epsi_4, Eta_epsi_5, Eta_D_epsiA, Eta_D_epsiB, Eta_D_epsiC, Eta_DepsiD, Eta_psi1, Eta_D_psiA, Eta_D_beta, Eta_D_beta_A, SpMax EA, SpMaxA_EA, SpDiam_EA, SpAD_EA, SpMAD_EA, SpMax_EA(ed), SpMaxA_EA(ed), SpDiam_EA(ed), SpAD_EA(ed), SpMAD_EA(ed), SpMax_EA(bo), SpMaxA_EA(bo), SpDiam_EA(bo), SpAD_EA(bo), SpMAD_EA(bo), SpMax_EA(dm), SpMaxA_EA(dm), SpDiam_EA(dm), SpAD_EA(dm), SpMAD_EA(dm), SpMax_EA(ri), SpMaxA_EA(ri), SpDiam_EA(ri), SpAD_EA(ri), SpMAD EA(ri), SpMax_AEA(ed), SpMaxA_AEA(ed), SpDiam_AEA(ed), SpAD_AEA(ed), SpMAD_AEA(ed), SpMax_AEA(bo), SpMaxA_AEA(bo), SpDiam_AEA(bo), SpAD_AEA(bo), SpMAD_AEA(bo), SpMax_AEA(dm), SpMaxA_AEA(dm), SpDiam_AEA(dm), SpAD_AEA(dm), SpMAD_AEA(dm), SpMax_AEA(ri), SpMaxA_AEA(ri), SpDiam_AEA(ri), SpAD_AEA(ri), SpMAD_AEA(ri), Chi0_EA, Chi1_EA, Chi0_EA(ed), Chi1_EA(ed), Chi0_EA(bo), Chi1_EA(bo), Chi0_EA(dm), Chi1_EA(dm), Chi0_EA(ri), Chi1_EA(ri), SM02_EA, SM03_EA, SM04_EA, SM05_EA, SM06_EA, SM07_EA, SM08_EA, SM09_EA, SM10_EA, SM11_EA, SM12_EA, SM13_EA, SM14_EA, SM15_EA, SM02_EA(ed), SM03_EA(ed), SM04_EA(ed), SM05_EA(ed), SM06_EA(ed), SM07_EA(ed), SM08_EA(ed), SM09_EA(ed), SM10_EA(ed), SM11_EA(ed), SM12_EA(ed), SM13_EA(ed), SM14_EA(ed), SM15_EA(ed), SM02_EA(bo), SM03_EA(bo), SM04_EA(bo), SM05_EA(bo), SM06_EA(bo), SM07_EA(bo), SM08_EA(bo), SM09_EA(bo), SM10_EA(bo), SM11_EA(bo), SM12_EA(bo), SM13_EA(bo), SM14_EA(bo), SM15_EA(bo), SM02_EA(dm), SM03_EA(dm), SM04_EA(dm), SM05_EA(dm), SM06_EA(dm), SM07_EA(dm), SM08_EA(dm), SM09_EA(dm), SM10_EA(dm), SM11_EA(dm), SM12_EA(dm), SM13_EA(dm), SM14_EA(dm), SM15_EA(dm), SM02_EA(ri), SM03_EA(ri), SM04_EA(ri), SM05_EA(ri), SM06_EA(ri), SM07_EA(ri), SM08_EA(ri), SM09_EA(ri), SM10_EA(ri), SM11_EA(ri), SM12_EA(ri), SM13_EA(ri), SM14_EA(ri), SM15_EA(ri), SM02_AEA(ed), SM03_AEA(ed), SM04_AEA(ed), SM05_AEA(ed), SM06_AEA(ed), SM07_AEA(ed), SM08_AEA(ed), SM09_AEA(ed), SM10_AEA(ed), SM11_AEA(ed), SM12_AEA(ed), SM13_AEA(ed), SM14_AEA(ed), SM15_AEA(ed), SM02_AEA(bo), SM03_AEA(bo), SM04_AEA(bo), SM05_AEA(bo), SM06_AEA(bo), SM07_AEA(bo), SM08_AEA(bo), SM10_AEA(bo), SM11_AEA(bo), SM12_AEA(bo), SM13_AEA(bo), SM14_AEA(bo), SM15_AEA(bo), SM02_AEA(dm), SM03_AEA(dm), SM04_AEA(dm), SM05_AEA(dm), SM06_AEA(dm), SM07_AEA(dm), SM08_AEA(dm), SM09_AEA(dm), SM11_AEA(dm), SM12_AEA(dm), SM13_AEA(dm), SM14_AEA(dm), SM15_AEA(dm), SM02_AEA(ri), SM03_AEA(ri), SM04_AEA(ri), SM05_AEA(ri), SM06_AEA(ri), SM07_AEA(ri), SM08_AEA(ri), SM09_AEA(ri), SM10_AEA(ri), SM12_AEA(ri), SM13_AEA(ri), SM14_AEA(ri), SM15_AEA(ri), Eig06_EA, Eig11_EA, Eig14_EA, Eig05_EA(ed), Eig10_EA(ed), Eig13_EA(ed), Eig14_EA(ed), Eig02 _EA(bo), Eig05 _EA(bo), Eig06_EA(bo), Eig07_EA(bo), Eig08_EA(bo), Eig09_EA(bo), Eig10_EA(bo), Eig11_EA(bo), Eig12_EA(bo), Eig13_EA(bo), Eig14_EA(bo), Eig15_EA(bo), Eig01_EA(dm), Eig02_EA(dm), Eig03_EA(dm), Eig04_EA(dm), Eig05_EA(dm), Eig06_EA(dm), Eig07_EA(dm), Eig08_EA(dm), Eig09_EA(dm), Eig10_EA(dm), Eig11_EA(dm), Eig12_EA(dm), Eig13_EA(dm),

Eig14_EA(dm), Eig02_EA(ri), Eig03_EA(ri), Eig04_EA(ri), Eig05_EA(ri), Eig06_EA(ri), Eig07_EA(ri), Eig08_EA(ri), Eig09_EA(ri), Eig10_EA(ri), Eig11_EA(ri), Eig12_EA(ri), Eig13_EA(ri), Eig14_EA(ri), Eig15_EA(ri), Eig01_AEA(ed), Eig02_AEA(ed), Eig03_AEA(ed), Eig04_AEA(ed), Eig05_AEA(ed), Eig06_AEA(ed), Eig07_AEA(ed), Eig08_AEA(ed), Eig09_AEA(ed), Eig10_AEA(ed), Eig11_AEA(ed), Eig12_AEA(ed), Eig13_AEA(ed), Eig14_AEA(ed), Eig15_AEA(ed), Eig02_AEA(bo), Eig03_AEA(bo), Eig04_AEA(bo), Eig05_AEA(bo), Eig06_AEA(bo), Eig07_AEA(bo), Eig08_AEA(bo), Eig09_AEA(bo), Eig10_AEA(bo), Eig11_AEA(bo), Eig12_AEA(bo), Eig13_AEA(bo), Eig14_AEA(bo), Eig15_AEA(bo), Eig01_AEA(dm), Eig02_AEA(dm), Eig03_AEA(dm), Eig04_AEA(dm), Eig05_AEA(dm), Eig06_AEA(dm), Eig07_AEA(dm), Eig08_AEA(dm), Eig09_AEA(dm), Eig10_AEA(dm), Eig11_AEA(dm), Eig12_AEA(dm), Eig13_AEA(dm), Eig14_AEA(dm), Eig15_AEA(dm), Eig02_AEA(ri), Eig03_AEA(ri), Eig04_AEA(ri), Eig05_AEA(ri), Eig06_AEA(ri), Eig07_AEA(ri), Eig08_AEA(ri), Eig09_AEA(ri), Eig10_AEA(ri), Eig11_AEA(ri), Eig12_AEA(ri), Eig13_AEA(ri), Eig14_AEA(ri), Eig15_AEA(ri), nCp, nCs, nCt, nCq, nCrs, nCrt, nCrq, nCar, nCbH, nCb-, nCconj, nR=Ct, nRCOOH, nRCOOR, nRCONHR, nArCONHR, nRCONR2, nArCONR2, nCONN, nN=C-N<, nRNH2, nRNHR, nRNR2, nArNR2, nN(CO)2, nROH, nOHs, nOHt, nROR, nArOR, nSO, nArX, nPyrrolidines, nImidazoles, nThiophenes, nPyridines, nHDon, nHAcc, C-001, C-002, C-003, C-005, C-006, C-007, C-008, C-009, C-011, C-024, C-025, C-026, C-027, C-028, C-029, C-033, C-034, C-035, C-040, C-041, C-042, C-044, H-046, H-047, H-048, H-049, H-050, H-051, H-052, H-053, H-054, O-056, O-058, O-059, O-060, N-067, N-068, N-072, N-073, N-074, N-075, S-107, S-109, SsCH3, SssCH2, SaaCH, SsssCH, StsC, SdssC, SaasC, SaaaC, SssssC, SsNH2, SssNH, SsssN, SdsN, SaaN, StN, SaasN, SaaNH, SsOH, SdO, SssO, SaaS, SdssS, SsF, SsCl, NsCH3, NssCH2, NaaCH, NsssCH, NdssC, NaasC, NaaaC, NssssC, NssNH, NsssN, NdsN, NaaN, NtN, NaasN, NaaNH, NdO, NssO, NdssS, CATS2D_00_DD, CATS2D_03_DD, CATS2D_05_DD, CATS2D_06_DD, CATS2D_08_DD, CATS2D 09_DD, CATS2D_02_DA, CATS2D_03_DA, CATS2D_04_DA, CATS2D_05_DA, CATS2D 06_DA, CATS2D_07_DA, CATS2D_08_DA, CATS2D_09_DA, CATS2D_03_DP, CATS2D_06_DP, CATS2D_02_DN, CATS2D_04_DN, CATS2D_05_DN, CATS2D_02_DL, CATS2D_03_DL, CATS2D_04_DL, CATS2D_05_DL, CATS2D_06_DL, CATS2D_07_DL, CATS2D_08_DL, CATS2D_09_DL, CATS2D_00_AA, CATS2D_02_AA, CATS2D_03_AA, CATS2D_04_AA, CATS2D_05_AA, CATS2D_06_AA, CATS2D_07_AA, CATS2D_08_AA, CATS2D_09_AA, CATS2D_02_AP, CATS2D_03_AP, CATS2D_04_AP, CATS2D_05_AP, CATS2D_06_AP, CATS2D_08_AP, CATS2D_09_AP, CATS2D_04_AN, CATS2D_05_AN, CATS2D_07_AN, CATS2D_08_AN, CATS2D_02_AL, CATS2D_03_AL, CATS2D_04_AL, CATS2D_05_AL, CATS2D_06_AL, CATS2D_07_AL, CATS2D_08_AL, CATS2D_09_AL, CATS2D_02_PN, CATS2D_04_PN, CATS2D_02_PL, CATS2D_03_PL, CATS2D_04_PL, CATS2D_05_PL, CATS2D_07_PL, CATS2D_08_PL, CATS2D_09_PL, CATS2D_00_NN, CATS2D_01_NL, CATS2D_02_NL, CATS2D_03_NL, CATS2D_04_NL, CATS2D_05_NL, CATS2D_06_NL, CATS2D_07_NL, CATS2D_08_NL, CATS2D_00_LL, CATS2D_01_LL, CATS2D_02_LL, CATS2D_03_LL, CATS2D_04_LL, CATS2D_05_LL, CATS2D_06_LL, CATS2D_07_LL, CATS2D_08_LL, CATS2D_09_LL, SHED_DD, SHED_DA, SHED_DP, SHED DN, SHED DL, SHED AA, SHED AP, SHED AN, SHED AL, SHED PN, SHED_PL, SHED_NN, SHED NL, SHED_LL, T(N..N), T(N..O), T(N..S), T(N..F), T(N..C1), T(O..O), T(O..S), T(O.. C1), B01[C-O], B01[C-F], B01[O-S], B02[C-F], B02[N-N], B02[N-O], B02[N-S], B02[O-O], B03[N-N], B03[N-O], B03[N-S], B03[O-O], B04[C-S], B04[C-F], B04[N-N], B04[N-O], B04[N-S], B04[O-O], B04[O-S], B05[C-C], B05[C-O], B05[C-S], B05[C-F], B05[N-N], B05[N-O], B05[N-S], B05[O-O], B05[O-S], B05[O-C1], B06[C-C], B06[C-N], B06[C-O], B06[C-F], B06[N-N], B06[N-O], B06[O-O], B07[C-C], B07[C-N], B07[C-O], B07[C-S], B07[C-F], B07[N-N], B07[N-O], B07[N-S], B07[O-O], B07[O-S], B08[C-C], B08[C-N], B08[C-O], B08[C-S], B08[N-N], B08[N-O], B08[O-O], B09[C-C], B09[C-N], B09[C-O], B09[C-S], B09[C-F], B09[C-C1], B09[N-N], B09[N-O], B09[O-O], B10[C-C], B10[C-N], B10[C-O], B10[N-N], B10[N-O], B10[O-O], F01[C-C], F01[C-N], F01[C-O], F01[C-S], F01[O-S], F02[C-C], F02[C-N], F02[C-O], F02[C-S], F02[C-F], F02[N-N], F02[N-O], F02[N-S], F02[O-O], F03[C-C], F03[C-N], F03[C-O], F03[C-S], F03[C-C1], F03[N-N], F03[N-O], F03[O-O], F04[C-C], F04[C-N], F04[C-O], F04[C-S], F04[C-C1], F04[N-N], F04[N-O], F04[N-S], F04[O-O], F04[O-S], F05[C-C], F05[C-N], F05[C-O], F05[C-S], F05[C-F], F05[C-C1], F05[N-N], F05[NO], F05[N-S], F05[O-O], F05[O-C1], F06[C-C], F06[C-N], F06[C-O], F06[C-S], F06[C-F], F06[C-C1], F06[N-N], F06[N-O], F06[O-O], F07[C-C], F07[C-N], F07[C-O], F07[C-S], F07[C-F], F07[C-C1], F07[N-N], F07[N-O], F07[O-O], F07[O-S], F08[C-C], F08[C-N], F08[C-O], F08[C-S], F08[C-C1], F08[N-N], F08[N-O], F08[O-O], F09[C-C], F09[C-N], F09[C-O], F09[C-S], F09[C-C1], F09[N-N], F09[N-O], F09[O-O], F10[C-C], F10[C-N], F10[C-O], F10[N-N], F10[N-O], F10[O-O], Uc, Ui, Hy, TPSA(NO), TPSA(Tot), MLOGP, MLOGP2, SAtot, SAacc, VvdwMG, VvdwZAZ, PDI, BLTD48, BLTA96, Ro5, DLS_01, DLS_02, DLS_03, DLS_04, DLS_05, DLS_06, DLS_07, DLS_cons, LLS_01, LLS_02.

Descriptors on a solvent (373 types)

MW, AMW, Sv, Se, Sp, Si, Mv, Me, Mp, Mi, GD, RBF, H%, C%, O%, MCD, ZM1Kup, ZM1Mad, ZM1Per, ZM1MulPer, ZM2Kup, ZM2Mad, ZM2Per, ZM2MulPer, ON0, ON0V, ON1, ON1V, DBI, SNar, HNar, GNar, Xt, Dz, LPRS, MSD, SPI, AECC, DECC, MDDD, ICR, MeanTD, MeanDD, S1K, S2K, S3K, PHI, PW2, PW3, PW4, PW5, MAXDN, MAXDP, DELS, LOC, MWC01, MWC02, MWC03, MWC04, MWC05, MWC06, MWC07, MWC08, MWC09, MWC10, SRW02, SRW04, SRW06, SRW08, SRW10, MPC01, MPC02, MPC03, MPC04, MPC05, piPC01, piPC02, piPC03,

piPC04, piPC05, TWC, TPC, piID, PCD, CID, BID, ISIZ, IAC, AAC, IDE, IDM, IDDE, IDDM, IDET, IDMT, IVDE, IVDM, HVcpx, HDcpx, Uindex, Vindex, Xindex, Yindex, IC0, IC1, IC2, IC3, IC4, IC5, TIC0, TIC1, TIC2, TIC3, TIC4, TIC5, SIC0, SIC1, SIC2, SIC3, SIC4, SIC5, CIC0, CIC1, CIC2, CIC3, CIC4, CIC5, BIC0, BIC1, BIC2, BIC3, BIC4, BIC5, ATS1m, ATS2m, ATS3m, ATS4m, ATS5m, ATS6m, ATS1v, ATS2v, ATS3v, ATS4v, ATS5v, ATS6v, ATS1e, ATS2e, ATS3e, ATS4e, ATS5e, ATS6e, ATS1p, ATS2p, ATS3p, ATS4p, ATS5p, ATS6p, ATS1i, ATS2i, ATS3i, ATS4i, ATS5i, ATS6i, ATSC1m, ATSC2m, ATSC3m, ATSC4m, ATSC5m, ATSC6m, ATSC1v, ATSC2v, ATSC3v, ATSC4v, ATSC5v, ATSC6v, ATSC1e, ATSC2e, ATSC3e, ATSC4e, ATSC5e, ATSC6e, ATSC1p, ATSC2p, ATSC3p, ATSC4p, ATSC5p, ATSC6p, ATSC1i, ATSC2i, ATSC3i, ATSC4i, ATSC5i, ATSC6i, MATS1m, MATS2m, MATS3m, MATS4m, MATS5m, MATS6m, MATS1v, MATS2v, MATS3v, MATS4v, MATS5v, MATS6v, MATS1e, MATS2e, MATS3e, MATS4e, MATS5e, MATS6e, MATS1p, MATS2p, MATS3p, MATS4p, MATS5p, MATS6p, MATS1i, MATS2i, MATS3i, MATS4i, MATS5i, MATS6i, GATS1m, GATS2m, GATS3m, GATS4m, GATS5m, GATS1v, GATS2v, GATS3v, GATS4v, GATS5v, GATS1e, GATS2e, GATS3e, GATS4e, GATS5e, GATS1p, GATS2p, GATS3p, GATS4p, GATS5p, GATS1i, GATS2i, GATS3i, GATS4i, GATS5i, GGI1, GGI2, GGI3, JGI1, JGI2, JGI3, JGT, SpMax1 Bh(m), SpMax2_Bh(m), SpMax3_Bh(m), SpMax4_Bh(m), SpMax5_Bh(m), SpMax6_Bh(m), SpMax7_Bh(m), SpMax8_Bh(m), SpMax1 Bh(v), SpMax2_Bh(v), SpMax3_Bh(v), SpMax4_Bh(v), SpMax5_Bh(v), SpMax6_Bh(v), SpMax7_Bh(v), SpMax1_Bh(e), SpMax2_Bh(e), SpMax3_Bh(e), SpMax4_Bh(e), SpMax5_Bh(e), SpMax6_Bh(e), SpMax7_Bh(e), SpMax8_Bh(e), SpMax1_Bh(p), SpMax2_Bh(p), SpMax3_Bh(p), SpMax4_Bh(p), SpMax5_Bh(p), SpMax6_Bh(p), SpMax7_Bh(p), SpMax8_Bh(p), SpMax1_Bh(i), SpMax2_Bh(i), SpMax3_Bh(i), SpMax4_Bh(i), SpMax5_Bh(i), SpMax6_Bh(i), SpMax7_Bh(i), SpMax8_Bh(i), SpMin1_Bh(m), SpMin2_Bh(m), SpMin3_Bh(m), SpMin4_Bh(m), SpMin5_Bh(m), SpMin1_Bh(v), SpMin2_Bh(v), SpMin3_Bh(v), SpMin4_Bh(v), SpMin5_Bh(v), SpMin1_Bh(e), SpMin2_Bh(e), SpMin3_Bh(e), SpMin4_Bh(e), SpMin1_Bh(p), SpMin2 _Bh(p), SpMin3 _Bh(p), SpMin4_Bh(p), SpMin5_Bh(p), SpMin1_Bh(i), SpMin2_Bh(i), SpMin3_Bh(i), SpMin4_Bh(i), PVSA_LogP_1, P_VSA_LogP_2, P_VSA_LogP_3, P_VSA_LogP_4, P_VSA_LogP_5, P_VSA_LogP_7, P_VSA_MR_1, P_VSA_MR_2, P_VSA_MR_3, P_VSA_MR_5, P_VSA_MR_6, P_VSA_m_1, P_VSA_m_2, P_VSA_m_3, P_VSA_v_2, P_VSA_v_3, P_YSA_e_2, P_VSA_e_5, P_VSA_i_2, P_VSA_i_3, P_VSA_s_2, P_VSA_s_3, P_VSA_s_4, P_VSA_s_6, P_VSA_ppp_L, P_VSA_ppp_D, P VSA_ppp_cyc, P_VSA_ppp_ter, SsCH3, SssCH2, SsssCH, SdssC, SsOH, SdO, SssO, SHED_AL, SHED_LL, Uc, Ui, Hy, AMR, TPSA(NO), TPSA(Tot), MLOGP2, ALOGP, ALOGP2, SAtot, SAdon, VvdwMG, VvdwZAZ, PDI, BLTF96, DLS_02, DLS_04, DLS_05, DLS_cons.

(Step 1) Identification of Important Descriptors by LASSO

In order to identify descriptors which are important for prediction of a critical degree of supersaturation among 2,100 descriptors, LASSO was applied. Of 58 types of data sets, 80% (46 types) were used as learning data and 20% (12 types) were used as validation data, and 3-fold cross validation was performed. The evaluation index of cross validation for determining a hyperparameter $\lambda$ was a coefficient of determination Q2. In order to consider the randomness of data splitting, LASSO was repeated 1,000 times, and it was judged that a model in which the coefficient of determination R2 for validation data is 0.50 or more has high prediction accuracy and high validity. The number of building of a model with R2 of 0.50 or more was 223, the mean R2 for the top 100 times was 0.71, and the mean number of descriptors building a model was 10.

LASSO is one of linear regression methods. In the least-squares method, a regression coefficient is determined so that the sum of squares of errors is minimized, while in LASSO, a regression coefficient is set so that the sum of squares of errors and the sum of absolute values of the regression coefficient are minimized. In other words, when the explanatory variable is defined as X, the objective variable is defined as y, the regression coefficient is defined as b, and the number of the explanatory variables is defined as m, a set of b such that the following function G is minimized is determined:

[Equation 14]

$$G = \|y - Xb\|^2 + \lambda \sum_{i=1}^{m} |b_i|$$

where $\lambda$ is a weight parameter, which determines which of the sum of squares of errors or the sum of absolute values of the regression coefficient is to be emphasized. Since the results greatly vary depending on the value of $\lambda$, generally $\lambda$ is determined by cross validation, etc. In LASSO, the regression coefficient is likely to become 0 in the process in which the sum of absolute values of the regression coefficient is made smaller, resulting in deletion of unnecessary

descriptors.

Descriptors which are selected in a model in which the coefficient of determination R2 is high and which have a larger regression coefficient in the model are considered to be important descriptors. Thus, for each descriptor selected in a model with R2 of 0.50 or more, the product of R2 and the regression coefficient was calculated, and its sum was defined as a score of importance. The results of arrangement of 20 descriptors in descending order of the score of importance are shown in Table 9. Regarding the breakdown of 20 descriptors, the types of descriptors on a compound include 2D autocorrelations (6 types), PVSA-like descriptors (4 types), edge adjacency indices (2 types), Burden eigenvalues (2 types), drug-like indices (1 type), and topological indices (1 type), the descriptors on a good solvent include Burden eigenvalues (2 types), the descriptors on a poor solvent include 2D autocorrelations (1 type), and the descriptors on experimental conditions include temperature. For the definition of the descriptors, see Non-Patent Literature: Roberto Todeschini, Viviana Consonni (2009) "Molecular Descriptors for Chemoinformatics" Wiley-VCH Verlag GmbH & Co. KGaA.

[Table 9]

Table 9

| Importance of descriptor | Information source | Name of descriptor | Type of descriptor | Sign of regression coefficient | Score of importance |
|---|---|---|---|---|---|
| 1 | Compound | MATS5i | 2D autocorrelations | + | 0.117 |
| 2 | Good solvent | SpMax5_ Bh (m) | Burden eigenvalues | + | 0.109 |
| 3 | Compound | SM03_ EA (dm) | Edge adjacency indices | + | 0.088 |
| 4 | Compound | P_VSA_MR 6 | P_VSA-like descriptors | - | 0.073 |
| 5 | Compound | MAXDP | Topological indices | - | 0.069 |
| 6 | Compound | MATS6m | 2D autocorrelations | - | 0.055 |
| 7 | Compound | DLS_04 | Drug-like indices | + | 0.048 |
| 8 | Compound | P_VSA_ s_3 | P VSA-like descriptors | - | 0.045 |
| 9 | Compound | GATS8s | 2D autocorrelations | - | 0.039 |
| 10 | Compound | ATSC1e | 2D autocorrelations | + | 0.034 |
| 11 | Compound | P_VSA_MR_ 8 | P_VSA-like descriptors | - | 0.030 |
| 12 | Compound | GATS5i | 2D autocorrelations | - | 0.028 |
| 13 | Experimental conditions | Temp | Temperature | + | 0.026 |
| 14 | Compound | SM13_ AEA(ri) | Edge adjacency indices | - | 0.025 |
| 15 | Compound | MATS2s | 2D autocorrelations | + | 0.023 |
| 16 | Compound | P_VSA_ LogP_ 2 | P_VSA-like descriptors | + | 0.017 |

(continued)

| Importance of descriptor | Information source | Name of descriptor | Type of descriptor | Sign of regression coefficient | Score of importance |
|---|---|---|---|---|---|
| 17 | Poor solvent | MATS3v | 2D autocorrelations | - | 0.016 |
| 18 | Compound | SpMax1_ Bh (m) | Burden eigenvalues | - | 0.014 |
| 19 | Good solvent | SpMax5_ Bh (v) | Burden eigenvalues | + | 0.013 |
| 20 | Compound | SpMax1_ Bh (p) | Burden eigenvalues | - | 0.011 |

Table 10 shows the mean R2 for the top 100 times of the coefficient of determination R2 for validation data when LASSO was performed 1,000 times using one and two of seven descriptor groups consisting of six descriptor groups including descriptors selected as important descriptors on a compound, namely, Moran and Geary 2D autocorrelations (96 types), P_VSA-like descriptors (42 types), edge adjacency indices (278 types), Burden eigenvalues (96 types), drug-like indices (10 types), topological indices (54 types), and the others descriptor group (839 types), descriptors on a good solvent, descriptors on a poor solvent, a solvent ratio, and a temperature. In Table 10, a descriptor in which the mean R2 for the top 100 times is a value of 0.50 or more, and preferably 0.65 or more, or a combination of the descriptors has relatively high importance. When Moran and Geary 2D autocorrelations were used or when both of drug-like indices and edge adjacency indices were used, a model with high prediction accuracy was built. Using descriptors with a value of 0.50 or more in Table 10, for example, 2D autocorrelations or edge adjacency indices, or a combination of descriptors with a value of 0.50 or more, for example, 2D autocorrelations and P_VSA-like descriptors, 2D autocorrelations and drug-like indices, 2D autocorrelations and edge adjacency indices, 2D autocorrelations and Burden eigenvalues, 2D autocorrelations and topological indices, 2D autocorrelations and others, P_VSA-like descriptors and edge adjacency indices, drug-like indices and edge adjacency indices, drug-like indices and Burden eigenvalues, drug-like indices and topological indices, edge adjacency indices and Burden eigenvalues, or edge adjacency indices and topological indices, a model can be produced. In terms of building a model with higher prediction accuracy, preferably using descriptors with a value of 0.65 or more in Table 10, for example, 2D autocorrelations, or a combination of descriptors with a value of 0.65 or more, for example, 2D autocorrelations and P_VSA-like descriptors, 2D autocorrelations and drug-like indices, 2D autocorrelations and edge adjacency indices, 2D autocorrelations and Burden eigenvalues, 2D autocorrelations and topological indices, 2D autocorrelations and others, or drug-like indices and edge adjacency indices, a model can be produced.
[Table 10]

Table 10

| | 2D autocorrelations | P_VSA-like descriptors | Drug-like indices | Edge adjacency indices | Burden eigenvalues | Topological indices | Others |
|---|---|---|---|---|---|---|---|
| 2D autocorrelations | 0.68 | 0.73 | 0.67 | 0.68 | 0.70 | 0.73 | 0.67 |
| P_VSA-like descriptors | 0.73 | 0.34 | 0.42 | 0.50 | 0.42 | 0.44 | 0.40 |
| Drug-like indices | 0.67 | 0.42 | 0.25 | 0.77 | 0.62 | 0.51 | 0.48 |
| Edge adjacency indices | 0.68 | 0.50 | 0.77 | 0.53 | 0.52 | 0.51 | 0.47 |
| Burden eigenvalues | 0.70 | 0.42 | 0.62 | 0.52 | 0.43 | 0.47 | 0.43 |
| Topological indices | 0.73 | 0.44 | 0.51 | 0.51 | 0.47 | 0.23 | 0.39 |

(continued)

| | 2D autocorrelat ions | P_VSA-like descriptors | Drug-like indices | Edge adjacency indices | Burden eigenvalues | Topological indices | Others |
|---|---|---|---|---|---|---|---|
| Others | 0.67 | 0.40 | 0.48 | 0.47 | 0.43 | 0.39 | 0.40 |

(Step 2) Semi-supervised Learning by PCA-PLS

From the database chembl_23 of ChEMBL (https://www.ebi.ac.uk/chembl/) and the database of PubChem (https://pubchem.ncbi.nlm.nih.gov/), 50,000 types of respective compounds were randomly extracted. Of these, using 88,902 types of compounds combining 88,881 types in which 16 important descriptors on a compound identified in Step 1 could be calculated with the above-mentioned 21 types of compounds, principal component analysis was performed to reduce dimension. Regarding the number of components here, the number of components of 11, in which the cumulative contribution rate exceeds 90% for the first time, was used.

Using a total of 15 variables combining 11 components of the compound obtained by principal component analysis with 2 important descriptors on a good solvent, 1 important descriptor on a poor solvent, and a crystallization temperature as explanatory variables, partial least squares regression (PLSR) was performed with the logarithm of a critical degree of supersaturation as an objective variable.

Of 58 types of experimental data, randomly, 70% (40 types) were used as learning data, and 30% (18 types) were used as validation data. When the number of components in PLS was determined using the R2 value in 4-fold cross validation as an evaluation function, a model with R2 for learning data of 0.856 and R2 for validation data of 0.856 was obtained in the case of the number of components of 3. FIG. 90 is a diagram in which predictive values for experimental values of a critical degree of supersaturation were plotted.

Using the model, a predictive value of a critical degree of supersaturation and a prediction interval for the substrates in Table 3 were calculated. The values obtained by converting the results outputted in a logarithmic scale to values in a linear scale are shown in Table 11. As the type of data in the table, "validation" represents data used as validation data, and "learning" represents data used as learning data.

[Table 11-1]

Table 11

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 1 | Esomeprazole magnesium hvdrate | MeOH | H2O | 1 | 35 | 1.4 | Validation | 1.7 | 1.1 | 2.7 |
| 2 | Lansoprazole | MeOH | H2O | 3 | 0 | 6.9 | Learning | 8.9 | 5.6 | 14.3 |
| 3 | Clopidogrel sulfate | H2O | 2-BuOH | 222 | 20 | 9.7 | Learning | 9.3 | 5.8 | 14.8 |
| 4 | Ketotifen fumarate | MeOH | IPA | 5 | 0 | 2.5 | Validation | 3.1 | 2.0 | 5.0 |
| 5 | Ketotifen fumarate | MeOH | IPA | 5 | 20 | 4 | Validation | 3.8 | 2.4 | 6.0 |
| 6 | Ketotifen fumarate | MeOH | 2-BuOH | 5 | 0 | 2.6 | Learning | 3.4 | 2.1 | 5.4 |
| 7 | Ketotifen fumarate | MeOH | 2-BuOH | 5 | 20 | 4.4 | Learning | 4.1 | 2.5 | 6.5 |
| 8 | Clarithromycin | THF | H2O | 25 | 0 | 21 | Validation | 20.6 | 12.9 | 32.8 |
| 9 | Clarithromycin | THF | H2O | 20 | 0 | 26 | Learning | 20.6 | 12.9 | 32.8 |
| 10 | Clarithromycin | THF | H2O | 25 | 10 | 17 | Learning | 21.8 | 13.6 | 34.7 |
| 11 | Azithromycin | IPA | H2O | 10 | 0 | 3.2 | Validation | 4.8 | 3.0 | 7.7 |
| 12 | DL-glutamic acid | H2O | Acetone | 5 | 0 | 45.8 | Validation | 34.9 | 21.9 | 55.6 |
| 13 | DL-glutamic acid | H2O | THF | 5 | 0 | 24.3 | Learning | 21.8 | 13.6 | 34.7 |
| 30 | Theophylline magnesium salt | Acetone | H2O | 3 | 0 | 6.3 | Validation | 6.4 | 4.0 | 10.2 |
| 39 | Vildagliptin | EtOH | TBME | 10 | 0 | 9.2 | Learning | 10.3 | 6.5 | 16.5 |
| 41 | Vildagliptin | MEK | Toluene | 10 | 0 | 8.5 | Learning | 9.2 | 5.8 | 14.7 |
| 56 | Valacyclovir hydrochloride | H2O | 2-BuOH | 35 | 10 | 48.6 | Learning | 53.8 | 33.7 | 85.9 |
| 38 | Tramadol hydrochloride | EtOH | TBME | 30 | 10 | 22.4 | Learning | 17.2 | 10.8 | 27.5 |
| 22 | Escitalopram oxalate | H2O | 2-BuOH | 15 | 0 | 3.7 | Learning | 3.4 | 2.1 | 5.4 |

(continued)

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 26 | Dabigatran etexilate methanesulfonate | EtOH | EtOAc | 10 | 20 | 4.3 | Learning | 4.8 | 3.0 | 7.6 |
| 35 | Pilsicainide hydrochloride anhydride | IPA | Toluene | 10 | 5 | 27 | Learning | 26.5 | 16.6 | 42.3 |
| 18 | Azithromycin | IPA | H2O | 10 | 20 | 11.1 | Validation | 5.0 | 3.1 | 7.9 |
| 17 | Azithromycin | MeOH | H2O | 20 | 0 | 7.5 | Learning | 3.8 | 2.4 | 6.0 |
| 16 | Azithromycin | EtOH | H2O | 20 | 0 | 7.1 | Validation | 4.8 | 3.0 | 7.7 |
| 15 | Azithromycin | Acetone | H2O | 20 | 0 | 6.2 | Learning | 4.8 | 3.0 | 7.7 |
| 45 | Linagliptin | EtOH | TBME | 15 | 0 | 7.9 | Learning | 6.4 | 4.0 | 10.1 |
| 21 | Escitalopram oxalate | H2O | 2-BuOH | 30 | 0 | 2.8 | Validation | 3.4 | 2.1 | 5.4 |
| 23 | Escitalopram oxalate | H2O | IPA | 15 | 0 | 4 | Learning | 3.2 | 2.0 | 5.0 |
| 46 | Linagliptin | EtOH | TBME | 15 | 20 | 6.7 | Learning | 7.0 | 4.4 | 11.2 |
| 14 | Azithromycin | IPA | H2O | 20 | 0 | 2.6 | Learning | 4.8 | 3.0 | 7.7 |

[Table 11-2]

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 25 | Dabigatran etexilate methanesulfonate | EtOH | EtOAc | 10 | 5 | 2.9 | Validation | 4.4 | 2.8 | 7.1 |
| 27 | Dabigatran etexilate methanesulfonate | EtOH | EtOAc | 10 | 35 | 5.2 | Learning | 5.3 | 3.3 | 8.5 |
| 24 | Dabigatran etexilate methanesulfonate | MeOH | TBME | 10 | 20 | 3.3 | Learning | 3.6 | 2.3 | 5.8 |
| 20 | Lansoprazole | MeOH | H2O | 3 | 15 | 12.6 | Learning | 10.4 | 6.5 | 16.6 |
| 47 | Glutathione | H2O | EtOH | 5 | 20 | 18.7 | Learning | 25.9 | 16.3 | 41.4 |
| 50 | Mirabegron | MeOH | TBME | 10 | 0 | 4 | Learning | 3.3 | 2.1 | 5.2 |
| 52 | Mirabegron | EtOH | TBME | 10 | 0 | 5.6 | Learning | 4.4 | 2.8 | 7.0 |
| 33 | Teneligliptin hydrobromide hvdrate | MeOH | 1-BuOH | 10 | 20 | 7.9 | Validation | 6.7 | 4.2 | 10.8 |
| 32 | Teneligliptin hydrobromide hydrate | MeOH | 1-BuOH | 10 | 0 | 5.2 | Learning | 4.9 | 3.1 | 7.8 |
| 34 | Teneligliptin hydrobromide hydrate | MeOH | 1-BuOH | 10 | 40 | 12.6 | Learning | 9.8 | 6.1 | 15.6 |
| 48 | Mirabegron | H2O | MeOH | 1.25 | 0 | 1.6 | Learning | 2.9 | 1.8 | 4.6 |
| 49 | Mirabegron | H2O | MeOH | 1.25 | 10 | 3.2 | Validation | 3.0 | 1.9 | 4.8 |
| 51 | Mirabegron | MeOH | TBME | 10 | 10 | 4.3 | Learning | 3.4 | 2.2 | 5.5 |
| 53 | Mirabegron | EtOH | TBME | 10 | 10 | 5.4 | Learning | 4.6 | 2.9 | 7.4 |
| 28 | Theophylline magnesium salt | MeOH | H2O | 3 | 0 | 3.5 | Learning | 5.1 | 3.2 | 8.1 |

(continued)

| | Substrate | Solvent 1 | Solvent 2 | Solvent 2/ solvent 1 | Temperature during crystallization | Critical degree of supersaturation S | Type of data | Critical degree of supersaturation S | Lower 95% prediction | Upper 95% prediction |
|---|---|---|---|---|---|---|---|---|---|---|
| | | | | Ratio | (°C) | Experimental value | | Predictive value | | |
| 29 | Theophylline magnesium salt | EtOH | H2O | 3 | 0 | 3.9 | Learning | 6.4 | 4.0 | 10.2 |
| 31 | Theophylline magnesium salt | IPA | H2O | 3 | 0 | 6.6 | Learning | 6.4 | 4.0 | 10.2 |
| 54 | Tolvaptan | MeOH | H2O | 0.4 | 0 | 4.8 | Validation | 6.6 | 4.1 | 10.5 |
| 55 | Tolvaptan | MeOH | H2O | 0.4 | 10 | 7.1 | Validation | 6.8 | 4.3 | 10.9 |
| 37 | Tramadol hydrochloride | IPA | TBME | 30 | 10 | 11.1 | Validation | 17.2 | 10.8 | 27.5 |
| 36 | Tramadol hydrochloride | MeOH | i-PrOAc | 20 | 10 | 4.2 | Learning | 7.1 | 4.5 | 11.4 |
| 19 | Esomeprazole magnesium hvdrate | EtOH | H2O | 1 | 35 | 1.6 | Learning | 1.4 | 0.9 | 2.3 |
| 40 | Vildagliptin | EtOH | TBME | 10 | 10 | 7 | Learning | 10.7 | 6.7 | 17.1 |
| 42 | Vildagliptin | IPA | TBME | 10 | 0 | 11 | Learning | 10.3 | 6.5 | 16.5 |
| 43 | Vildagliptin | IPA | TBME | 10 | 10 | 11.6 | Learning | 10.7 | 6.7 | 17.1 |
| 44 | Vildagliptin | IPA | TBME | 10 | 20 | 11.9 | Validation | 11.3 | 7.1 | 18.0 |
| 57 | Bepotastine besilate | EtOH | AcOiPr | 10 | 0 | 36.4 | Learning | 15.5 | 9.7 | 24.7 |
| 58 | Olopatadine | MeOH | EtOAc | 20 | 15 | 9.7 | Validation | 7.3 | 4.6 | 11.6 |

Example 33

Building of Predictive Model of Critical Degree of Supersaturation (III)

First, using Jmol (http://jmol.sourceforge.net/), a structure model of a compound is produced based on the SDF file, and 512 captured images (snapshot, size: 512 × 512, 24 bpp) taken by rotating each structure model at 45-degree intervals around each of the X-axis, the Y-axis, and the Z-axis are produced. The SDF file of learning data is inputted, and a captured image of each compound is produced. The captured image of each compound is stored in a predetermined folder in which a critical degree of supersaturation was recorded together with information on a solvent and a solution temperature during crystallization. The model is one of unmodified AlexNet (University of Toronto) in which the output layer is replaced by Support Vector Regression (SVR), and using Keras, the predictive model is subjected to transfer learning.

Furthermore, using test data including the captured image of a compound, information on a solvent, and an actual measured value of a critical degree of supersaturation, the prediction performance was confirmed by the external validation method. Specifically, the captured image in the test data is inputted into the learning model, and the outputted predictive value of a critical degree of supersaturation is compared with the actual measured value included in the test data to investigate the correlation.

INDUSTRIAL APPLICABILITY

According to the method of the present invention, it is possible to reproducibly obtain a specifically-shaped crystal (in particular, a spherulite) of a compound. The spherulite of a compound has wide applicability in the fields of pharmaceutical manufacturing, pesticide manufacturing, food manufacturing, printing technology and organic electronic devices.

REFERENCE SIGNS LIST

100: Information processor
102: Input device
103: Display device
110: Storage device
120: CPU

**Claims**

1. A method for producing a spherulite of a compound, which comprises:

(1) a step of preparing a supersaturated solution of the compound having a degree of supersaturation equal to or higher than a critical degree of supersaturation required to obtain the spherulite of the compound; and
(2) a step of precipitating the spherulite of the compound from the supersaturated solution.

2. The method according to claim 1, wherein the step (1) is performed under a condition where nucleation does not occur until a degree of supersaturation equal to or higher than a critical degree of supersaturation is reached.

3. The method according to claim 1, which further comprises a step of removing crystal nuclei produced by the time a degree of supersaturation equal to or higher than a critical degree of supersaturation is reached in the step (1).

4. The method according to any one of claims 1 to 3, which further comprises a step of inputting data including:

information on a compound obtained as a spherulite, and
at least one of information on a solvent used for crystallization and a solution temperature during crystallization into a predictive model of a critical degree of supersaturation required to obtain the spherulite of the compound, and outputting a predictive value of the critical degree of supersaturation from the predictive model, wherein the critical degree of supersaturation in the step (1) is the predictive value.

5. The method according to claim 4, wherein, when the predictive value is outputted as a numerical value range, the critical degree of supersaturation in the step (1) is a lower limit of the numerical value range.

6. The method according to any one of claims 1 to 3, wherein the critical degree of supersaturation is an actual measured value.

7. The method according to any one of claims 1 to 6, wherein a sphericity of the spherulite is 0.60 or more.

8. The method according to any one of claims 1 to 7, wherein the compound obtained as a spherulite is a compound selected from the following (1) and (2):

(1) a compound represented by the following formula I, or a tautomer thereof, or an optical isomer thereof, or a salt thereof, or a solvate thereof:

[Chemical Formula 1]

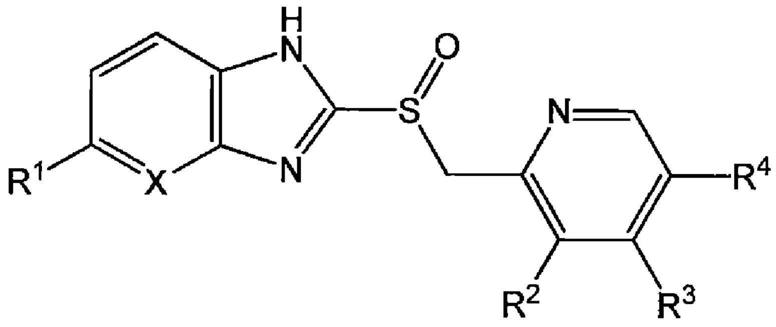

I

wherein

X is CH or N,
$R^1$ is a hydrogen atom or an optionally substituted $C_{1-6}$ alkoxy group, and
$R^2$, $R^3$, and $R^4$ are the same or different and each represent a hydrogen atom, an optionally substituted $C_{1-6}$ alkyl group, an optionally substituted $C_{1-6}$ alkoxy group, or an optionally substituted amino group;

(2) azithromycin, duloxetine, clarithromycin, lanthanum carbonate, glutamic acid, clopidogrel, ketotifen, escitalopram, dabigatran etexilate, theophylline, teneligliptin, pilsicainide, tramadol, vildagliptin, linagliptin, glutathione, mirabegron, tolvaptan, valacyclovir, bepotastine, olopatadine, or an optical isomer thereof, or a salt thereof, or a solvate thereof.

9. The method according to any one of claims 1 to 8, wherein the compound obtained as a spherulite is esomeprazole or lansoprazole, or a salt thereof, or a solvate thereof.

10. The method according to any one of claims 1 to 9, wherein the compound obtained as a spherulite is esomeprazole magnesium trihydrate.

11. A spherulite of a compound which is produced by the method according to any one of claims 1 to 10.

12. A spherulite of a compound selected from the following (1) and (2):

(1) a compound represented by the following formula I, or a tautomer thereof, or an optical isomer thereof, or a salt thereof, or a solvate thereof:

[Chemical Formula 2]

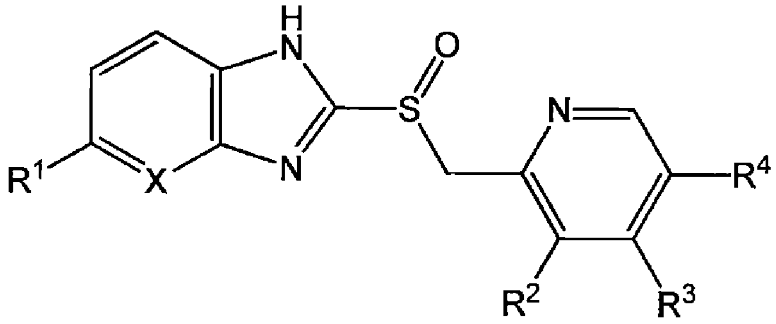

I

wherein

X is CH or N,
$R^1$ is a hydrogen atom or an optionally substituted $C_{1-6}$ alkoxy group, and
$R^2$, $R^3$, and $R^4$ are the same or different and each represent a hydrogen atom, an optionally substituted $C_{1-6}$ alkyl group, an optionally substituted $C_{1-6}$ alkoxy group, or an optionally substituted amino group;

(2) azithromycin, duloxetine, clarithromycin, lanthanum carbonate, glutamic acid, clopidogrel, ketotifen, escitalopram, dabigatran etexilate, theophylline, teneligliptin, pilsicainide, tramadol, vildagliptin, linagliptin, glutathione, mirabegron, tolvaptan, valacyclovir, bepotastine, olopatadine, or an optical isomer thereof, or a salt thereof, or a solvate thereof.

**13.** The spherulite according to claim 12, wherein the compound selected from (1) and (2) is esomeprazole or lansoprazole, or a salt thereof, or a solvate thereof.

**14.** The spherulite according to claim 12 or 13, wherein the compound selected from (1) and (2) is esomeprazole magnesium trihydrate.

**15.** The spherulite according to any one of claims 12 to 14, wherein the sphericity is 0.60 or more.

**16.** A device for predicting a critical degree of supersaturation required to obtain a spherulite of a target compound, the device comprising:

a storage unit for recording a predictive model of a critical degree of supersaturation which is previously learned so as to input data including information on a compound obtained as a spherulite and at least one of information on a solvent used for crystallization and a solution temperature during crystallization, and to output a predictive value of a critical degree of supersaturation required to obtain the spherulite of the compound; and
a calculation unit for inputting data including information on a target compound obtained as a spherulite and at least one of information on a solvent used for crystallization of the target compound and a solution temperature during crystallization into the predictive model, and calculating a predictive value of a critical degree of supersaturation required to obtain the spherulite of the target compound.

**17.** A method for predicting a critical degree of supersaturation required to obtain a spherulite of a compound, which comprises a step of inputting data including information on a compound obtained as a spherulite and at least one of information on a solvent used for crystallization and a solution temperature during crystallization into a predictive model of a critical degree of supersaturation required to obtain the spherulite of the compound, and outputting a predictive value of a critical degree of supersaturation from the predictive model.

**18.** A computer program for predicting a critical degree of supersaturation required to obtain a spherulite of a compound, which makes a computer run a process including a step of inputting data including information on a compound obtained as a spherulite and at least one of information on a solvent used for crystallization and a solution temperature

during crystallization into a predictive model of a critical degree of supersaturation required to obtain the spherulite of the compound, and outputting a predictive value of a critical degree of supersaturation from the predictive model.

FIG. 1

(a) S = 9.0 (b) S = 4.0 (c) S = 3.4

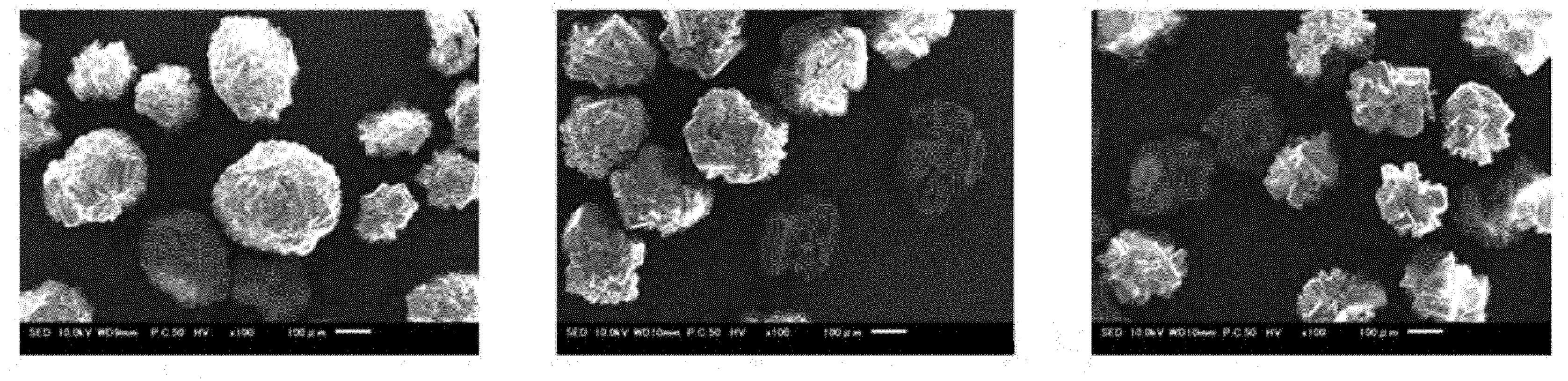

Sphericity $0.78 \pm 0.05$ | Sphericity $0.60 \pm 0.14$ | Sphericity $0.47 \pm 0.23$

FIG. 2

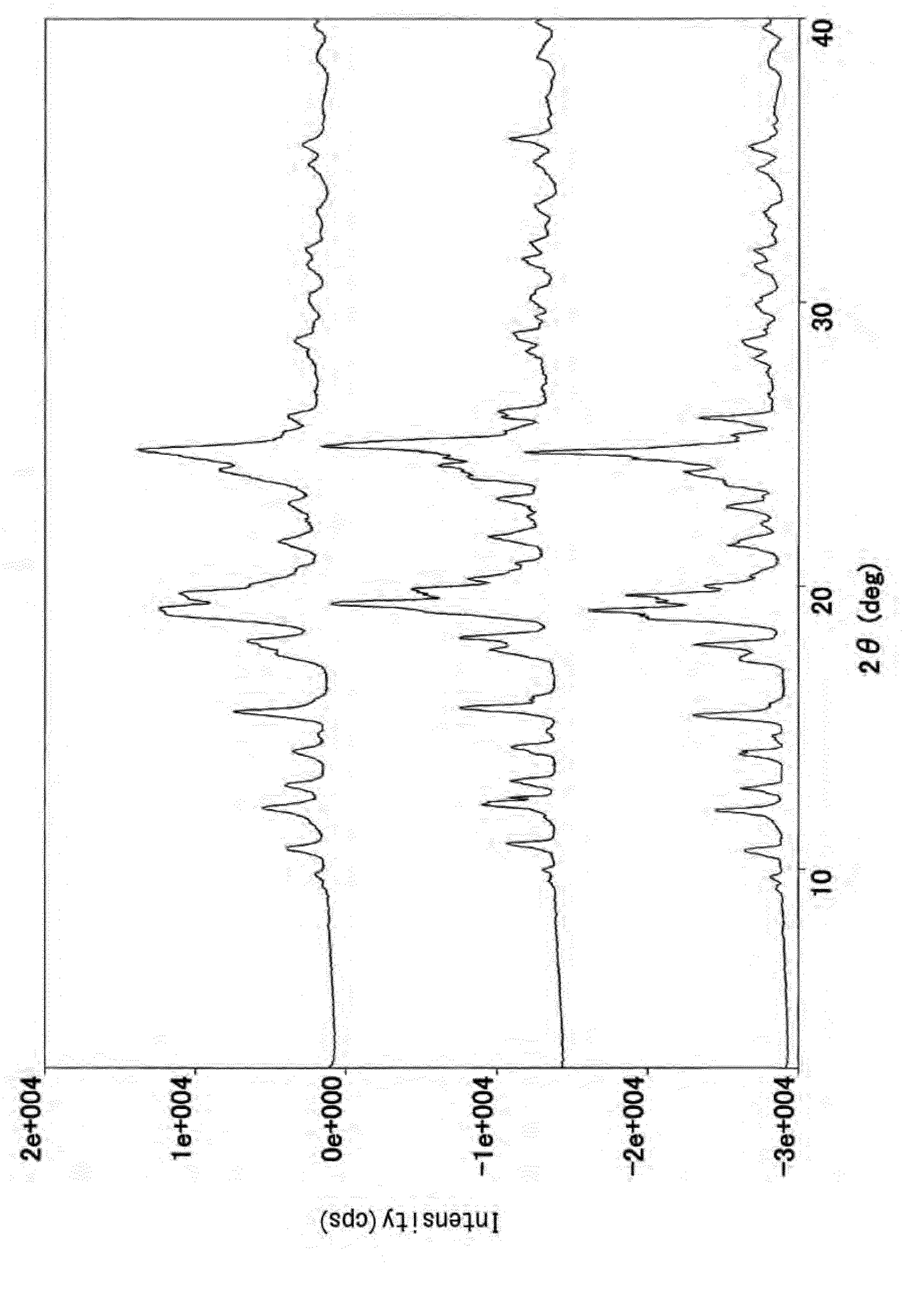
2e+004
1e+004
0e+000
−1e+004
−2e+004
−3e+004
Intensity (cps)
10
20
30
40
2θ (deg)

FIG. 3

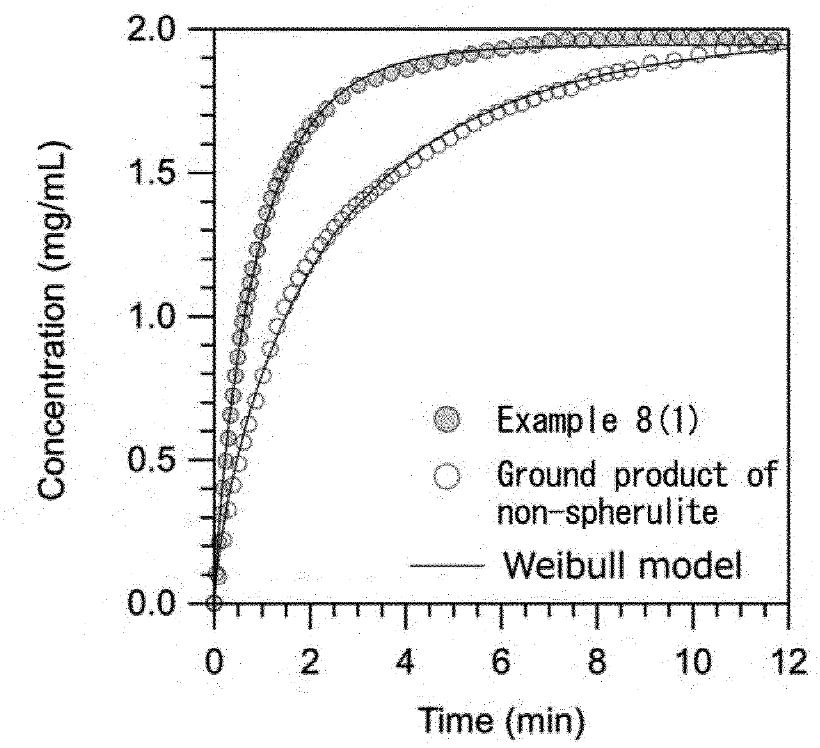
2.0
1.5
1.0
0.5
0.0
Concentration (mg/mL)
0
2
4
6
8
10
12
Time (min)
Example 8(1)
Ground product of non-spherulite
Weibull model

FIG. 4

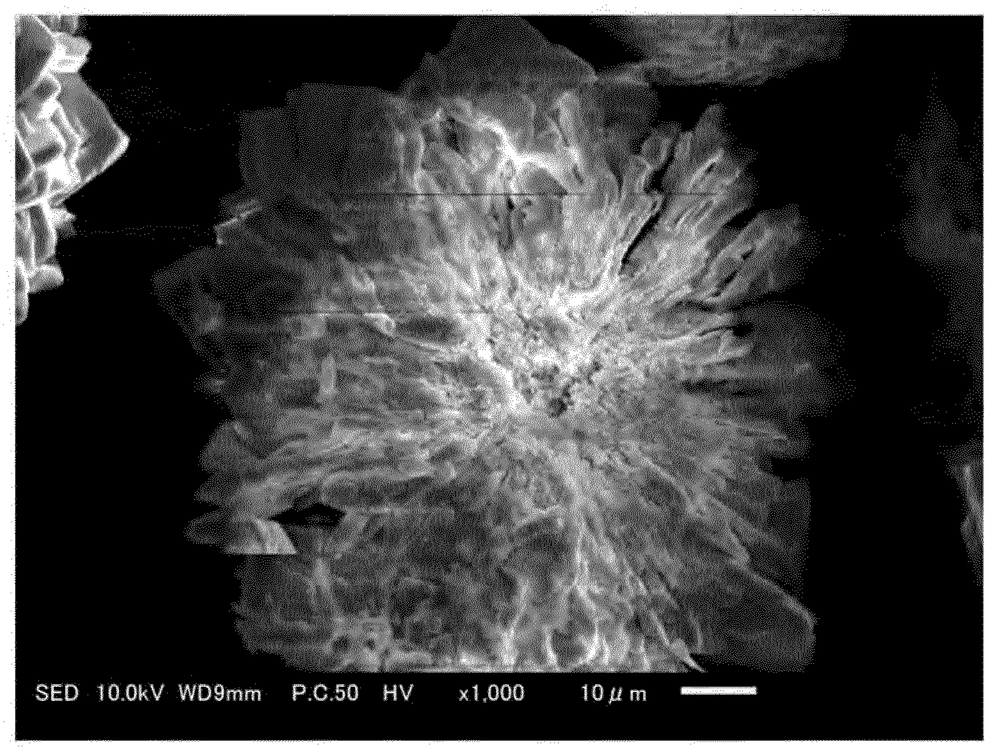
SED 10.0kV WD9mm P.C.50 HV x1,000 10 μm

FIG. 5

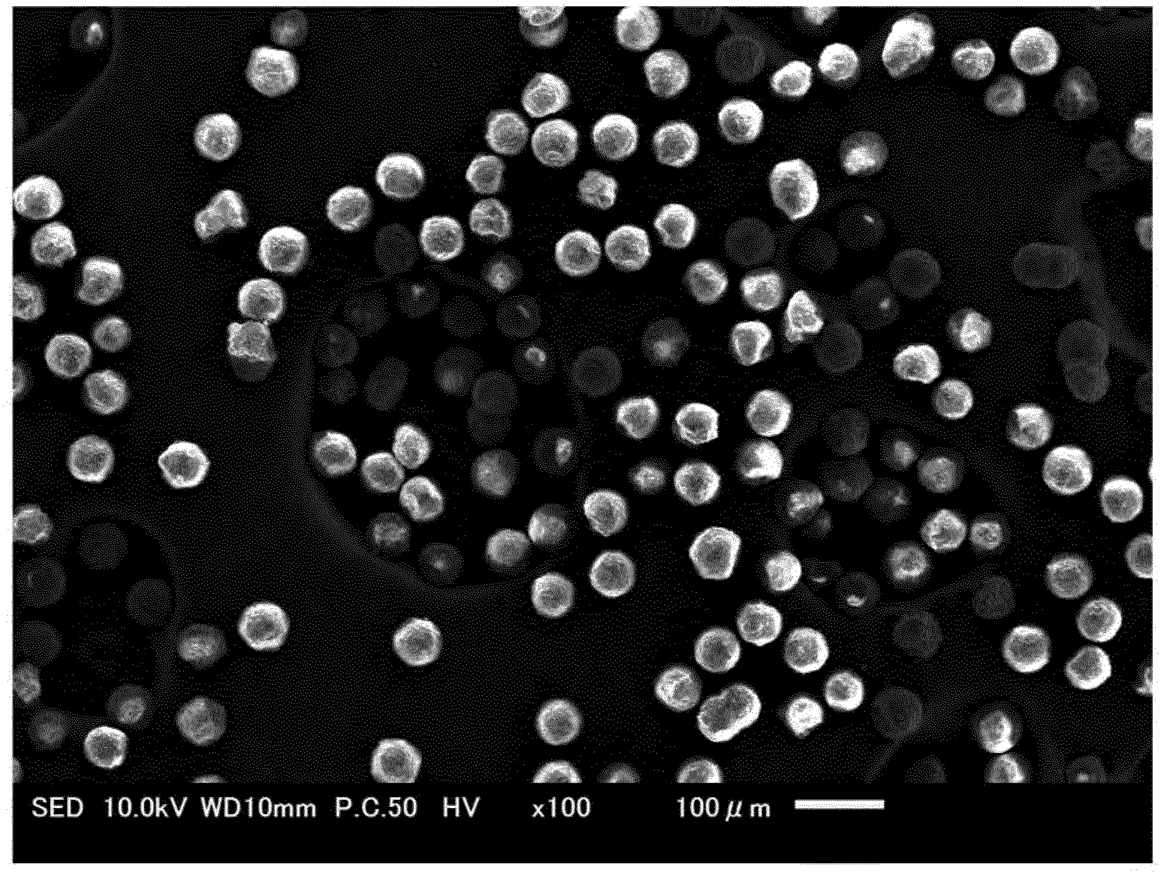
SED 10.0kV WD10mm P.C.50 HV x100 100 μm

FIG. 6

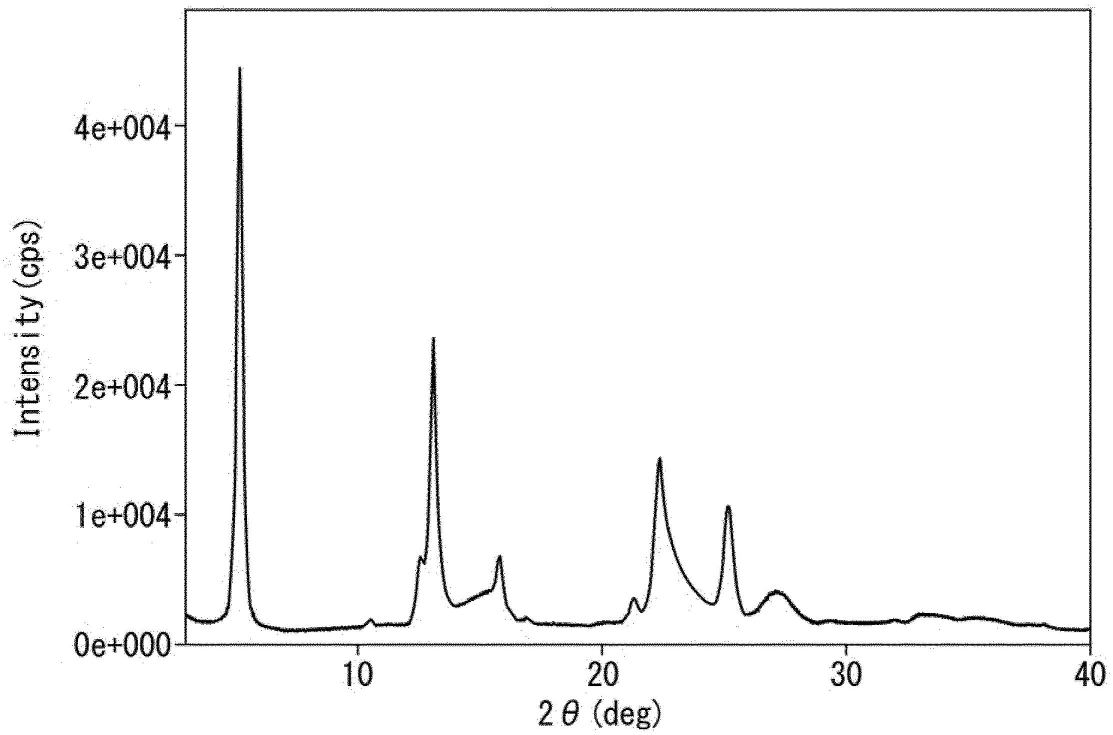
4e+004
3e+004
2e+004
1e+004
0e+000
Intensity (cps)
10
20
30
40
2 θ (deg)

FIG. 7

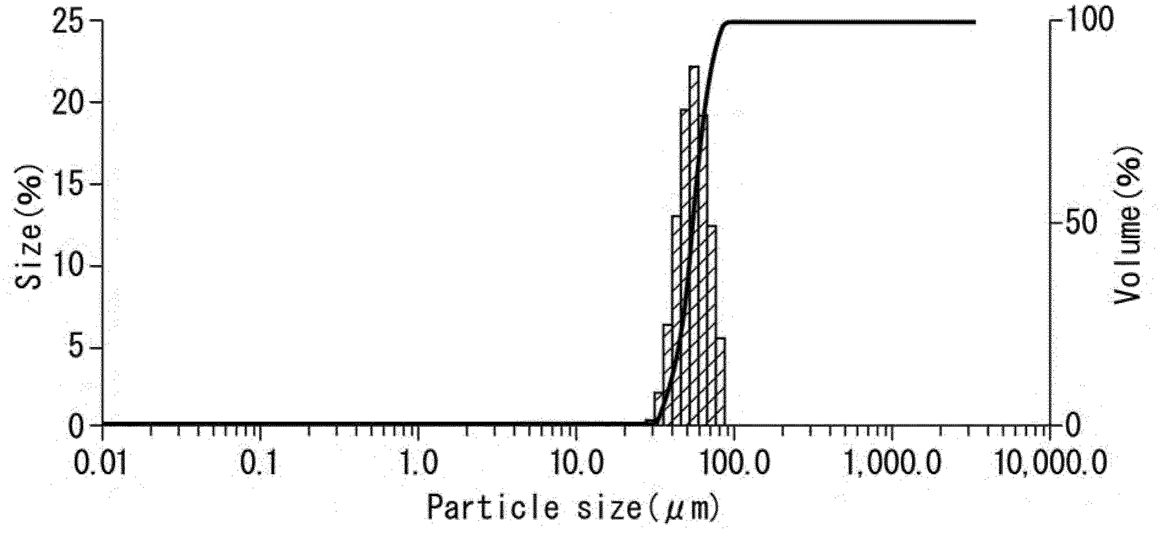


| $d_{10}$ | $d_{50}$ | $d_{90}$ |
|---|---|---|
| 40.7μm | 54.6μm | 72.5μm |

FIG. 8

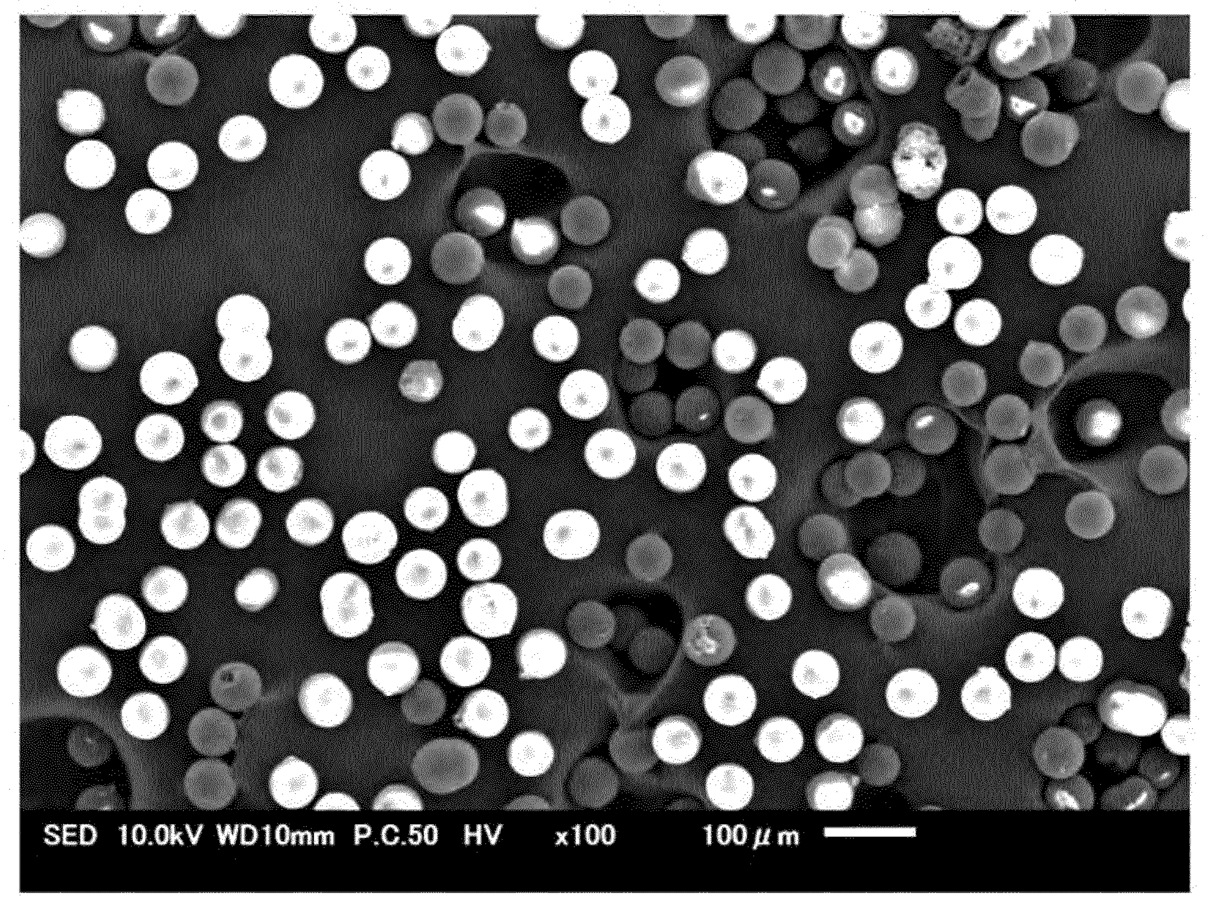

FIG. 9

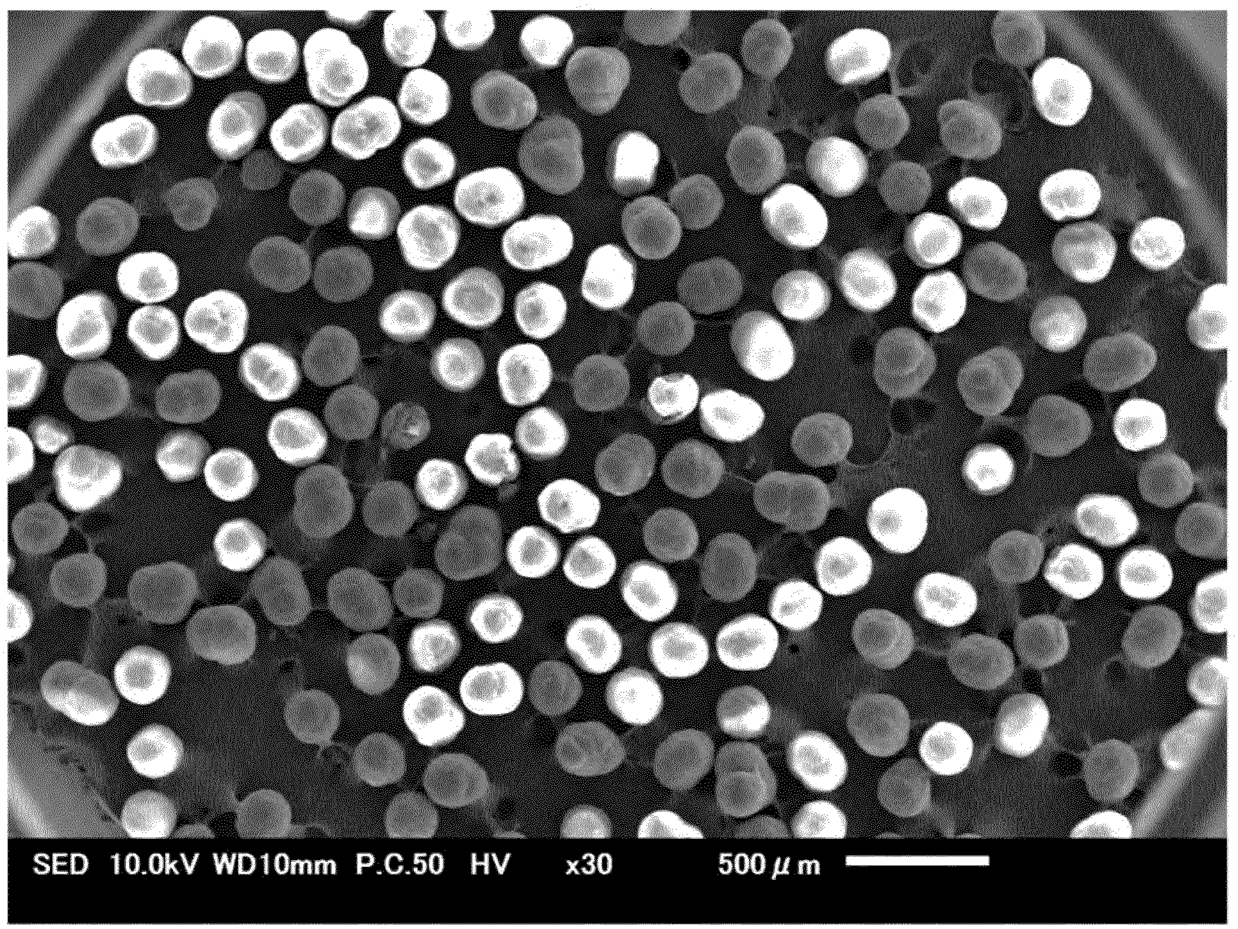

FIG. 10

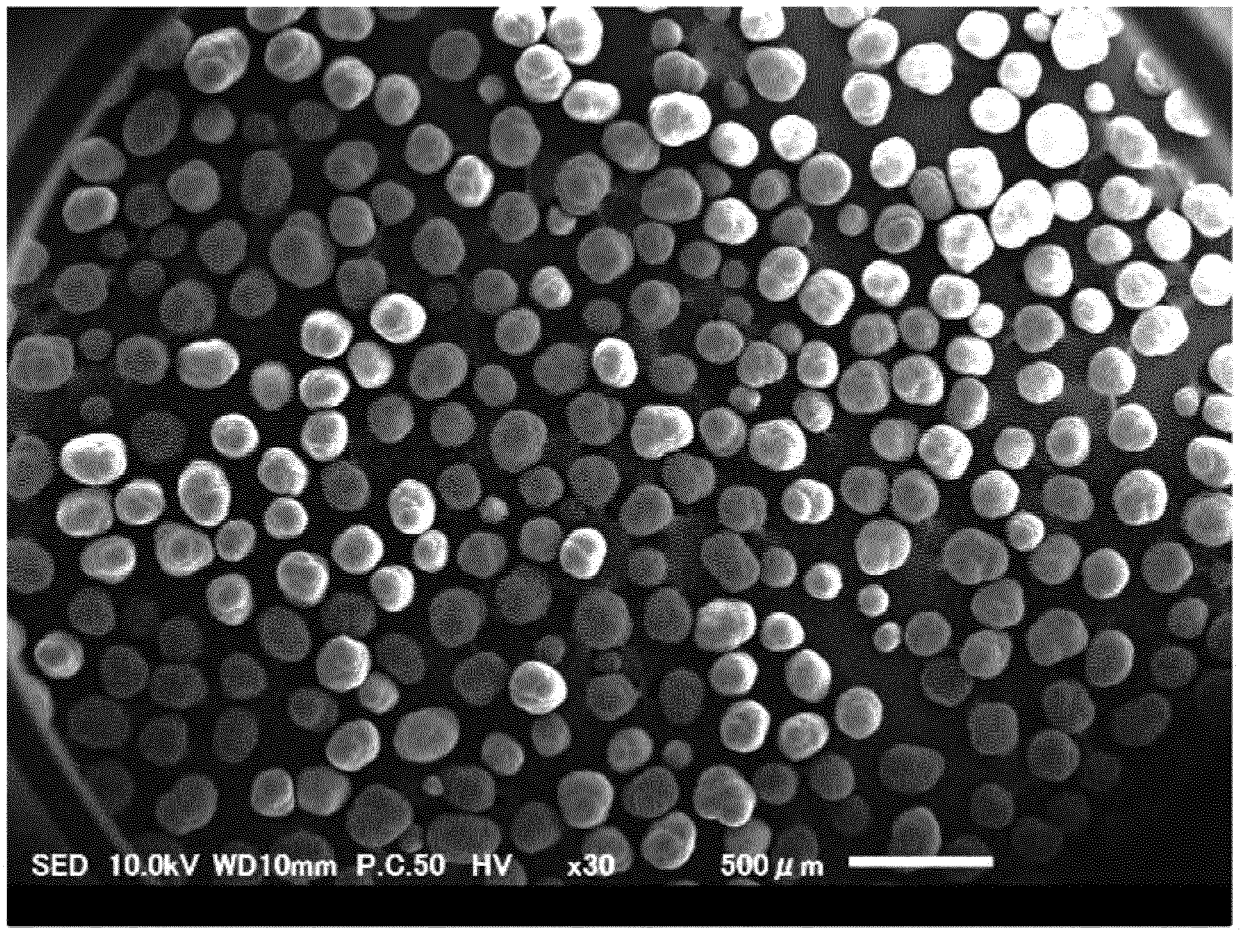

FIG. 11

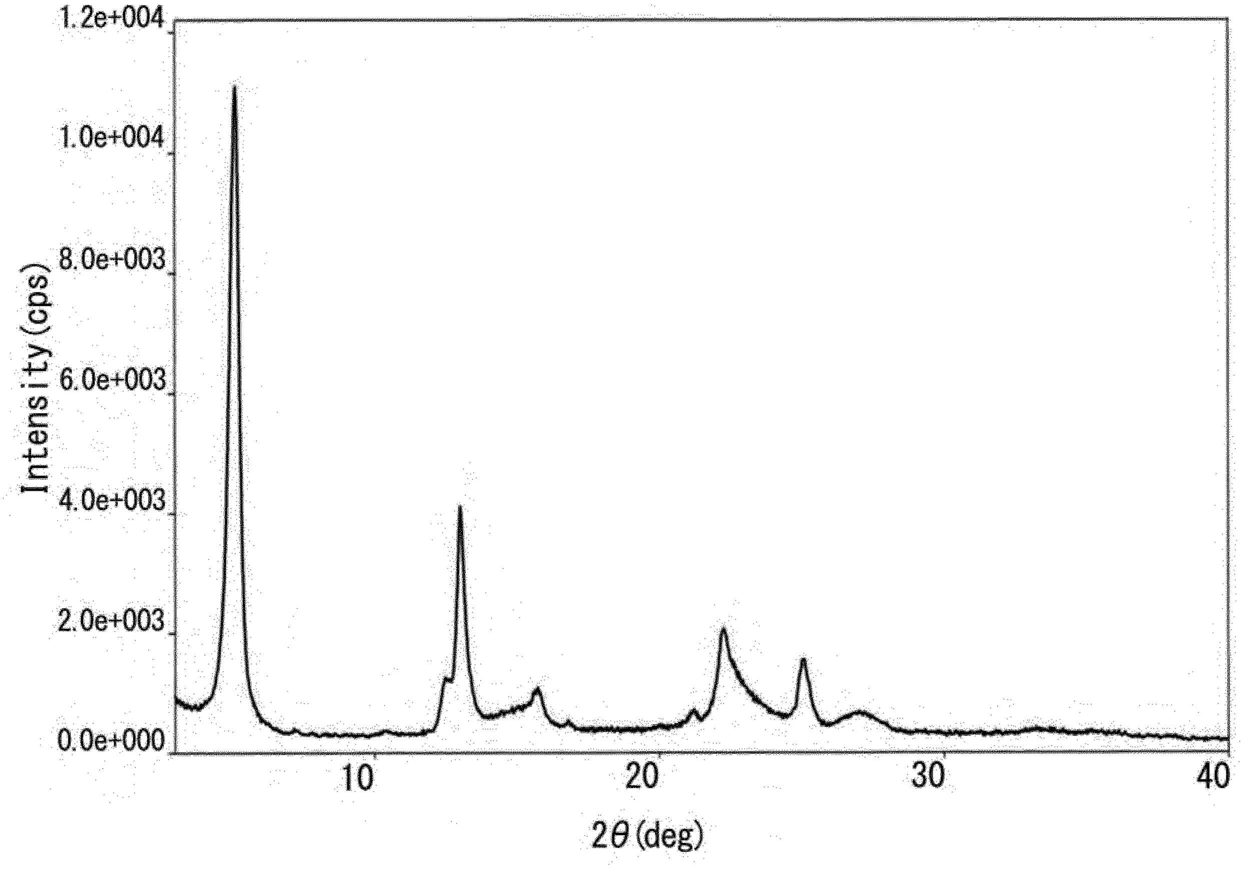
1.2e+004
1.0e+004
8.0e+003
6.0e+003
4.0e+003
2.0e+003
0.0e+000
Intensity (cps)
10
20
30
40
2θ (deg)

FIG. 12

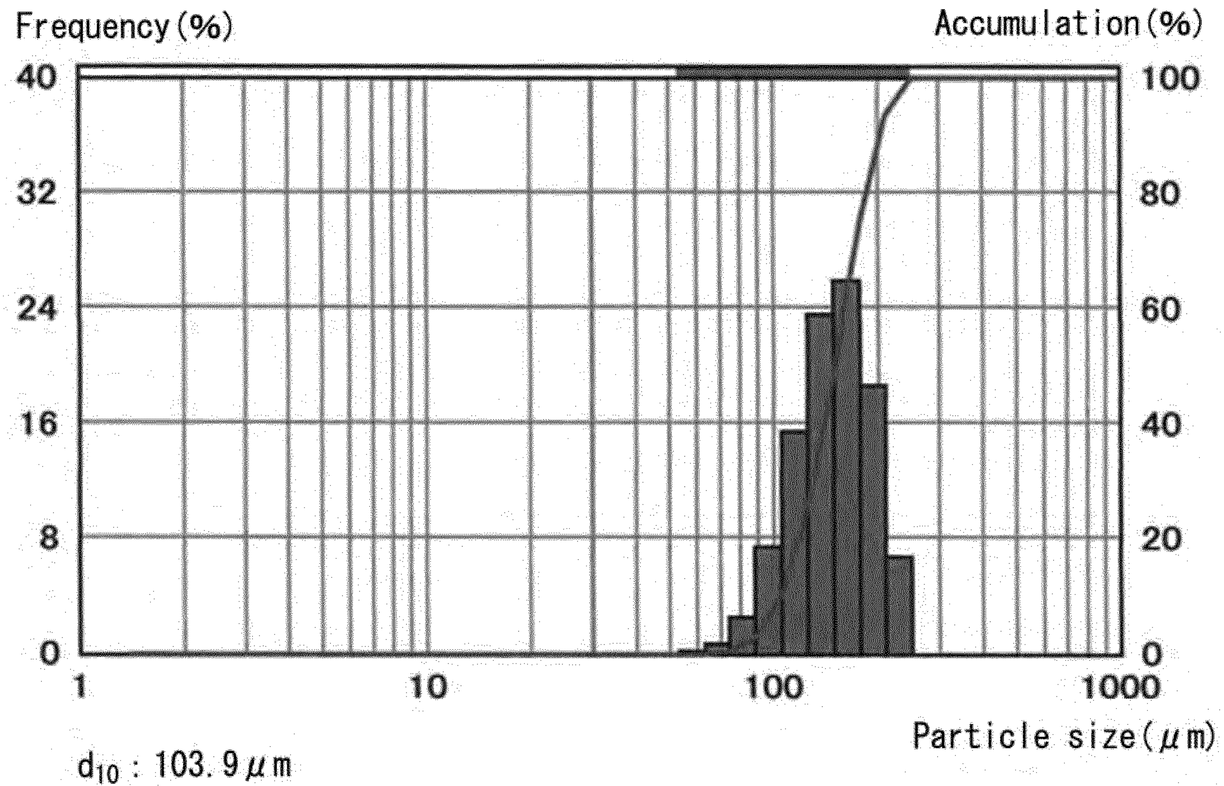


$d_{10}$ : 103.9 μm
$d_{50}$ : 149.9 μm
$d_{90}$ : 203.5
Mode diameter: 192.9 μm
Sharpness index: 1.40

FIG. 13

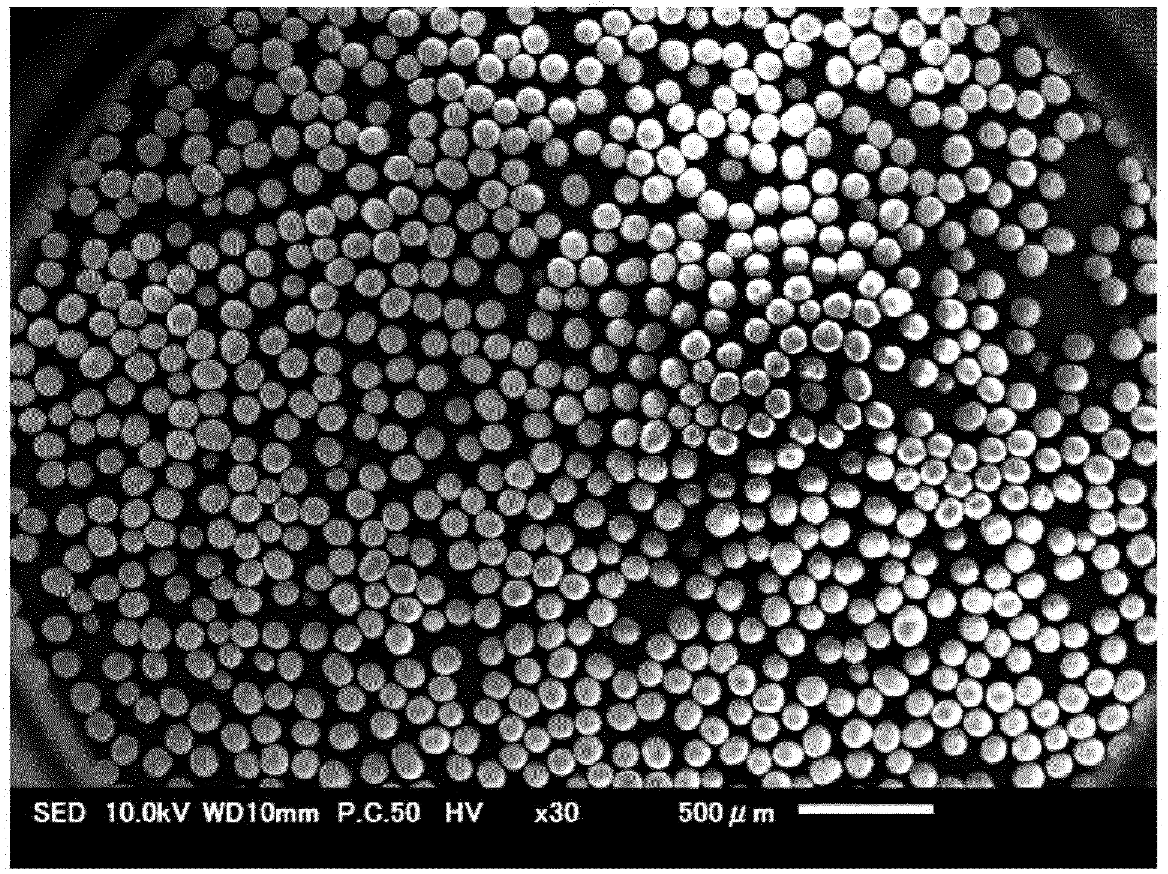
SED 10.0kV WD10mm P.C.50 HV x30 500 μm

FIG. 14

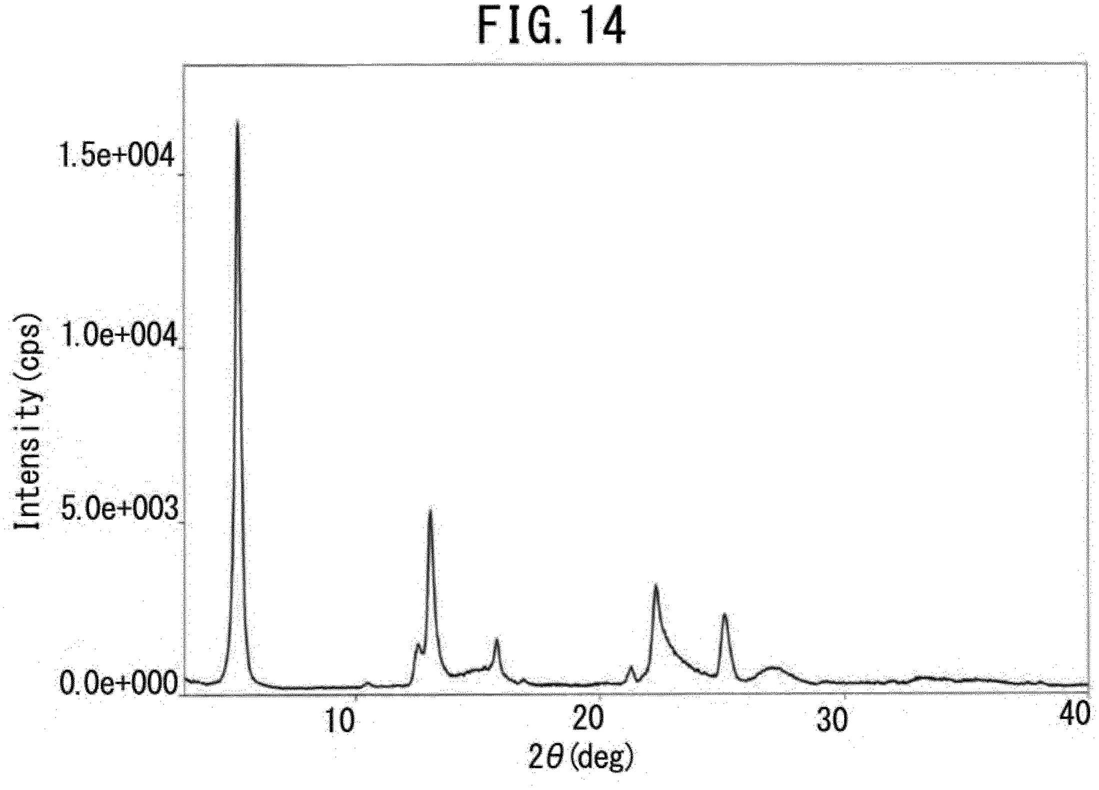
1.5e+004
1.0e+004
5.0e+003
0.0e+000
Intensity (cps)
10
20
30
40
2θ (deg)

FIG. 15

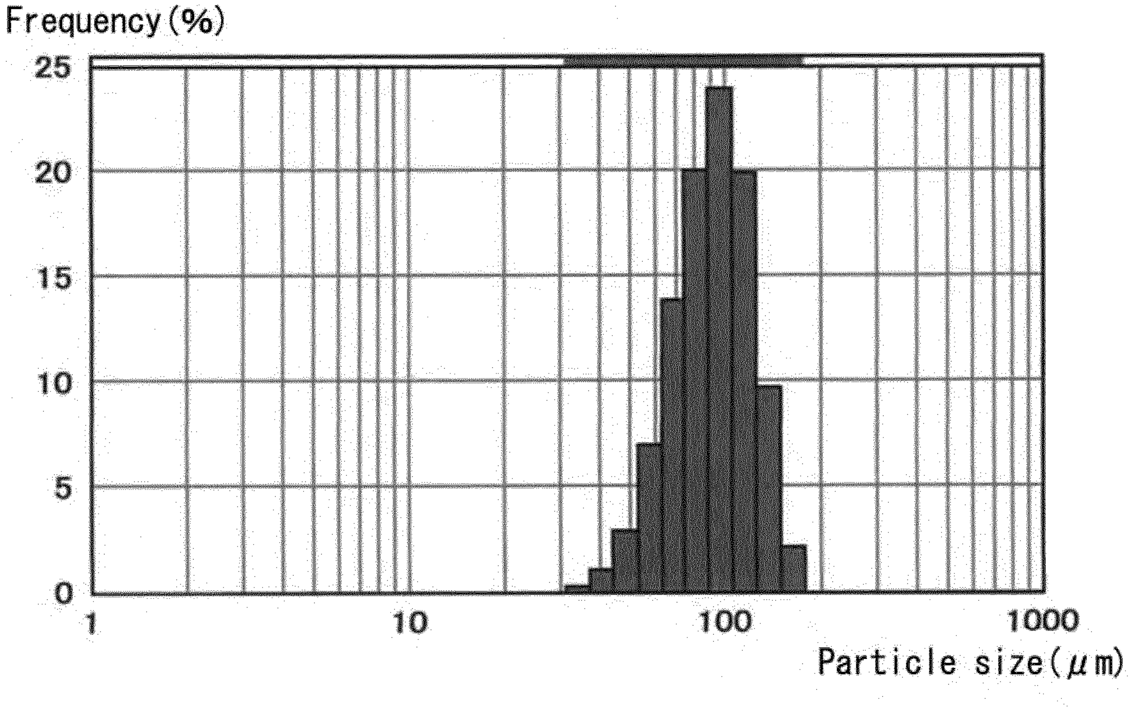

FIG. 16

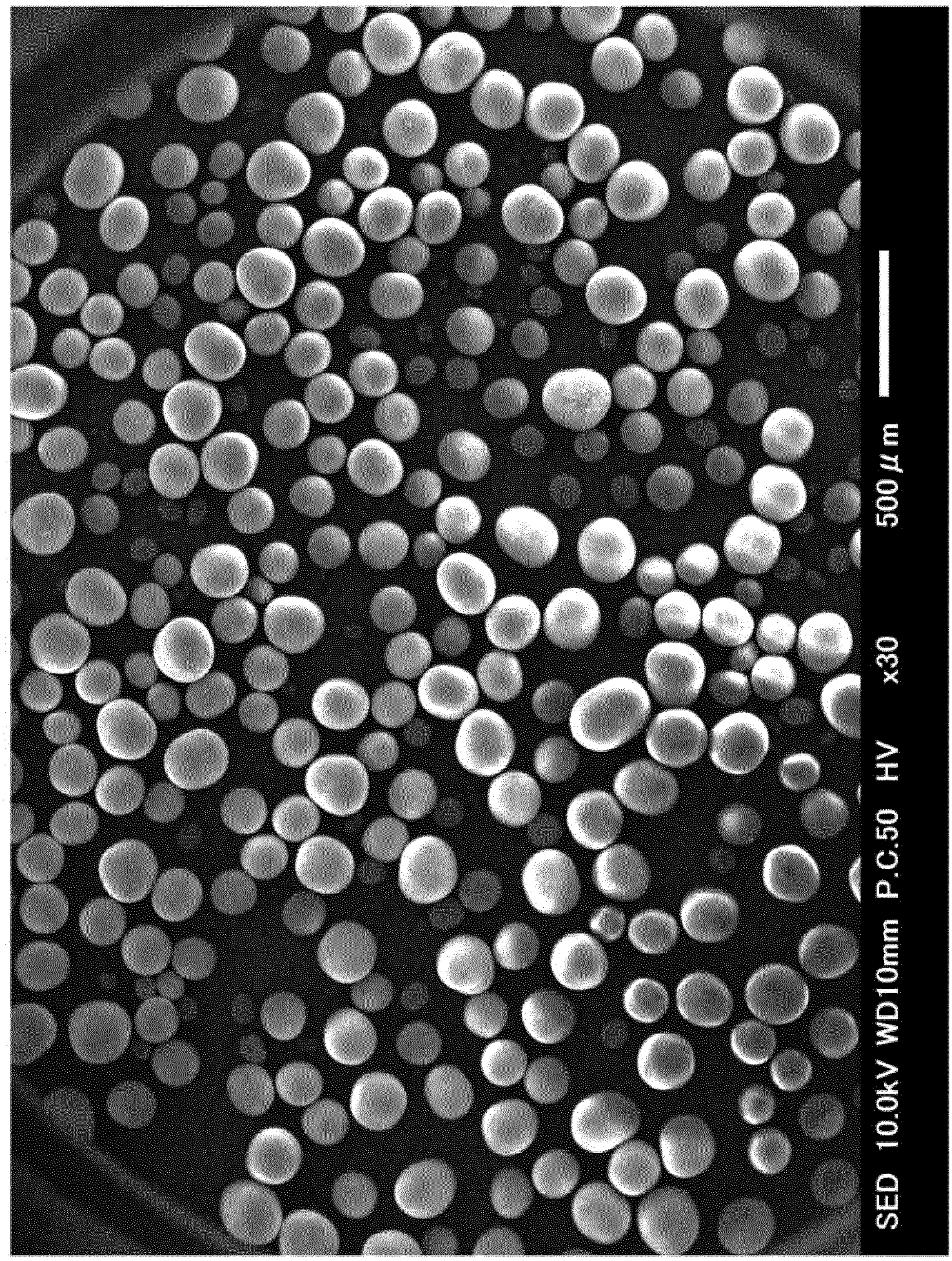

FIG. 17

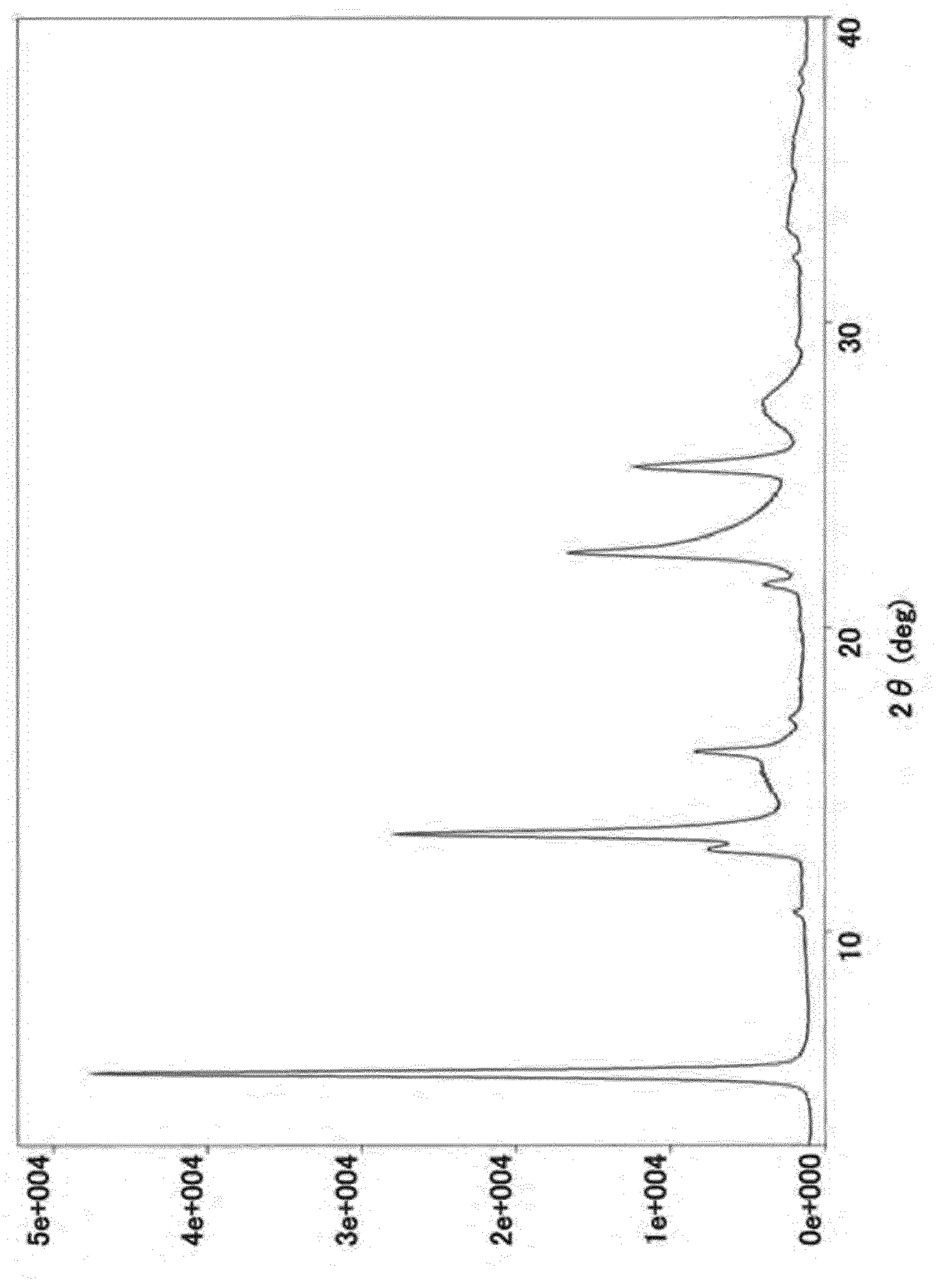

FIG. 18

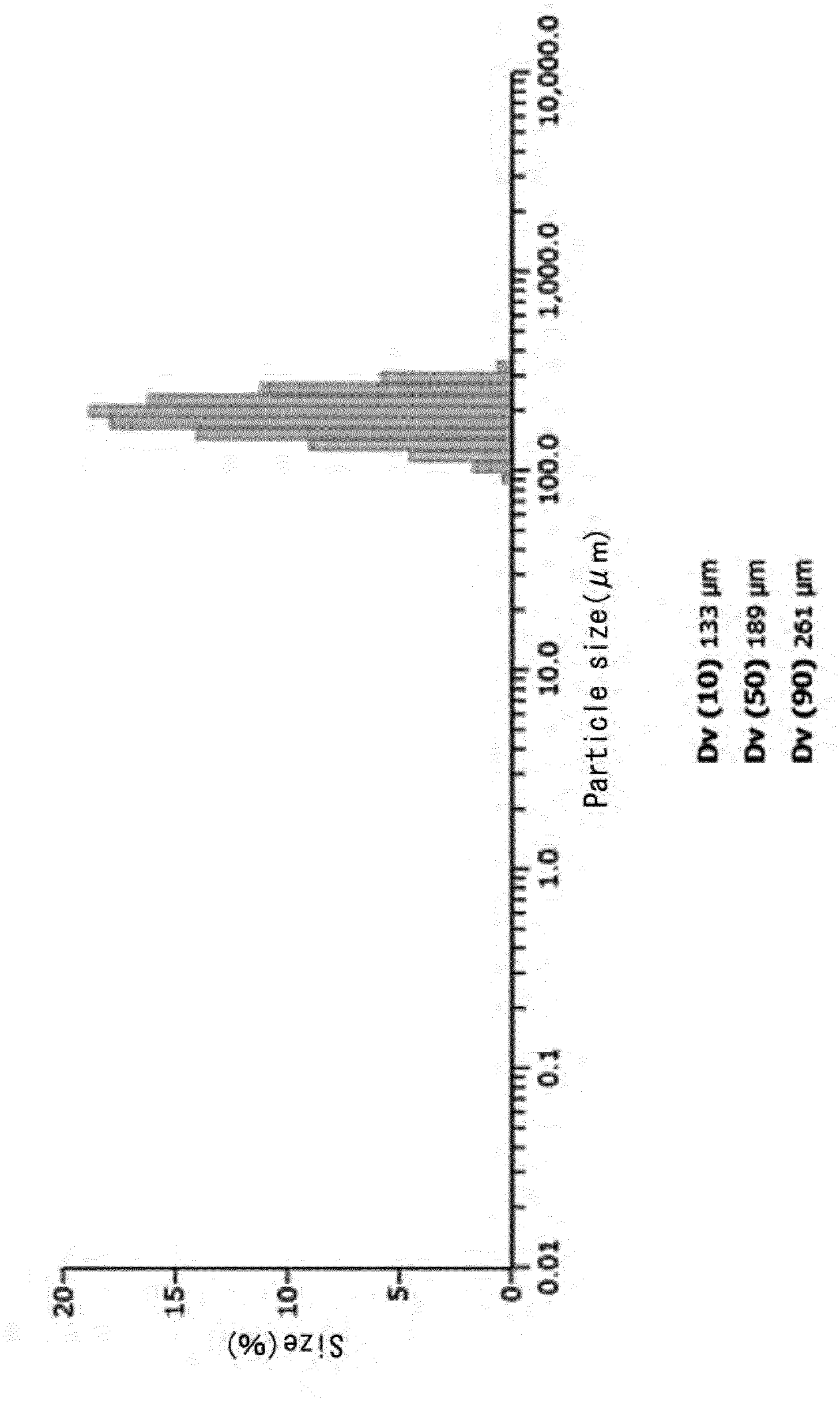

Size (%)
20
15
10
5
0
0.01
0.1
1.0
10.0
100.0
1,000.0
10,000.0
Particle size (μm)
Dv (10) 133 μm
Dv (50) 189 μm
Dv (90) 261 μm

FIG. 19

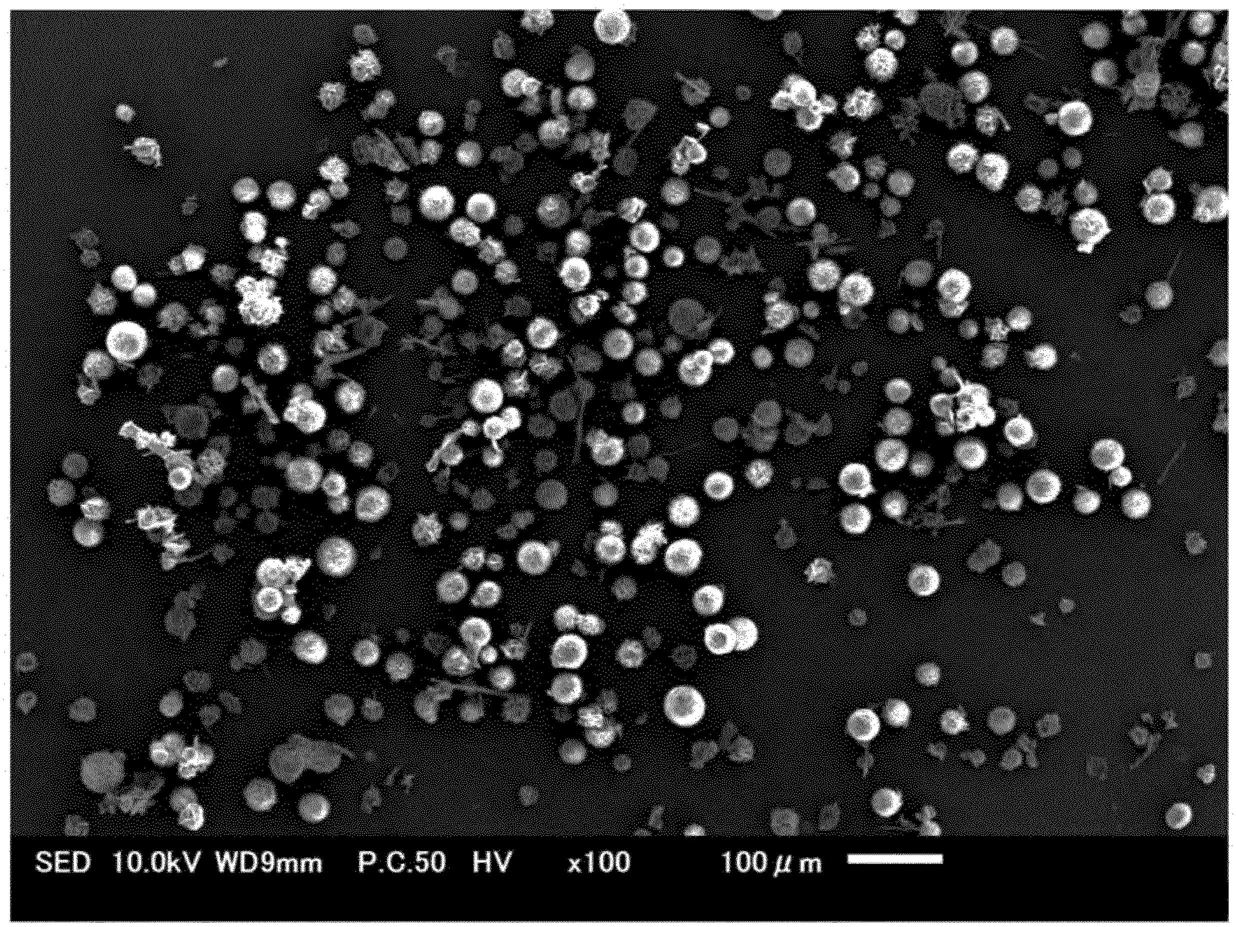

FIG. 20

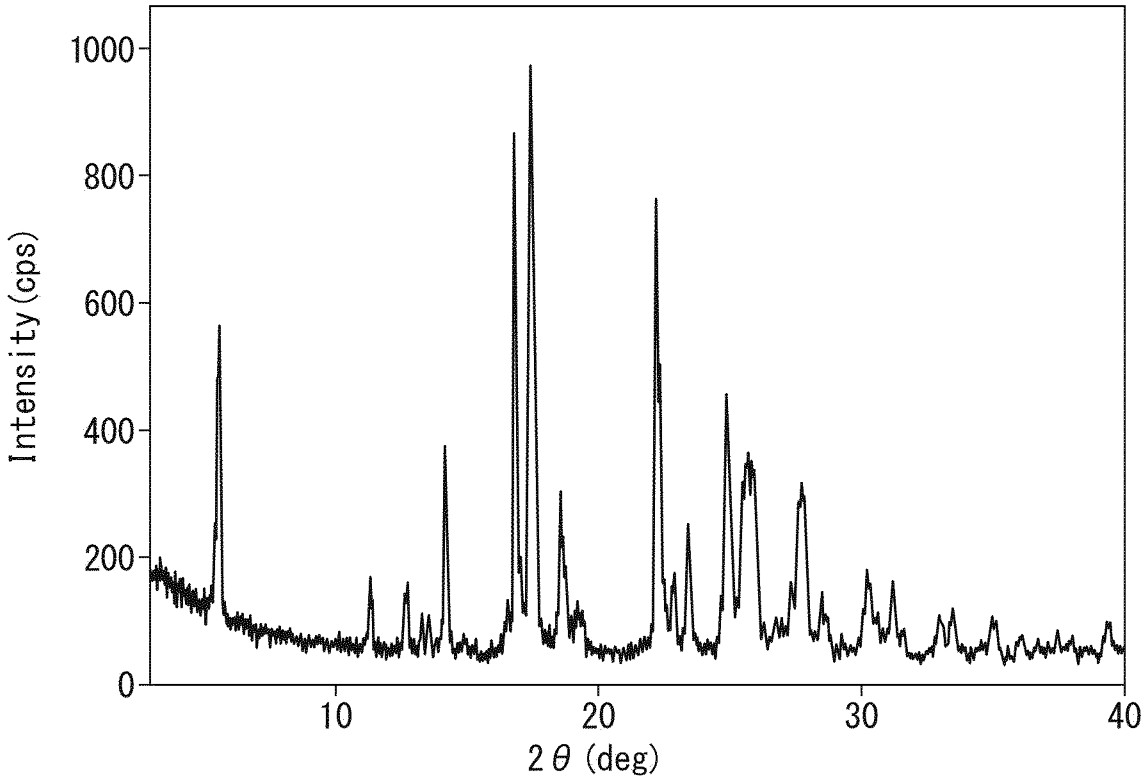

FIG. 21

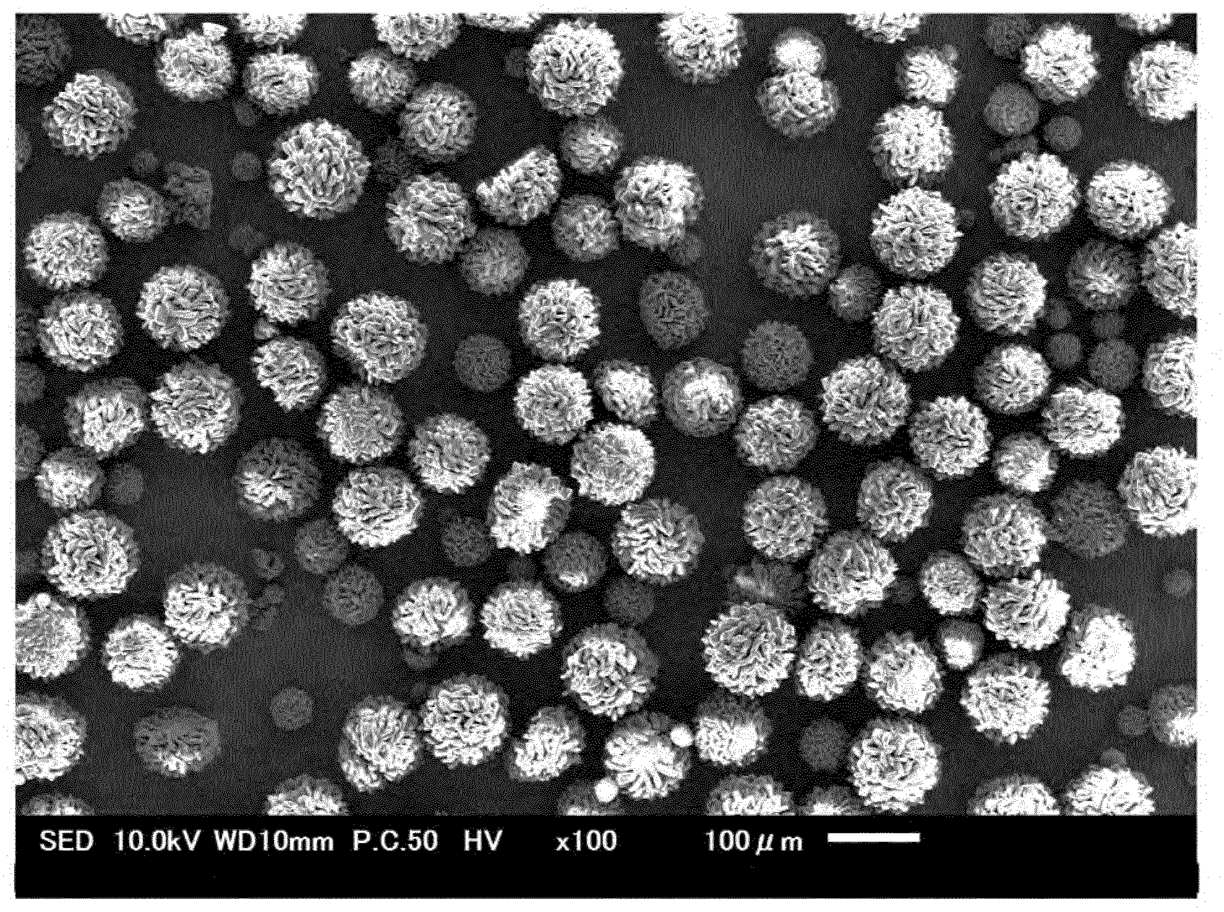

FIG. 22

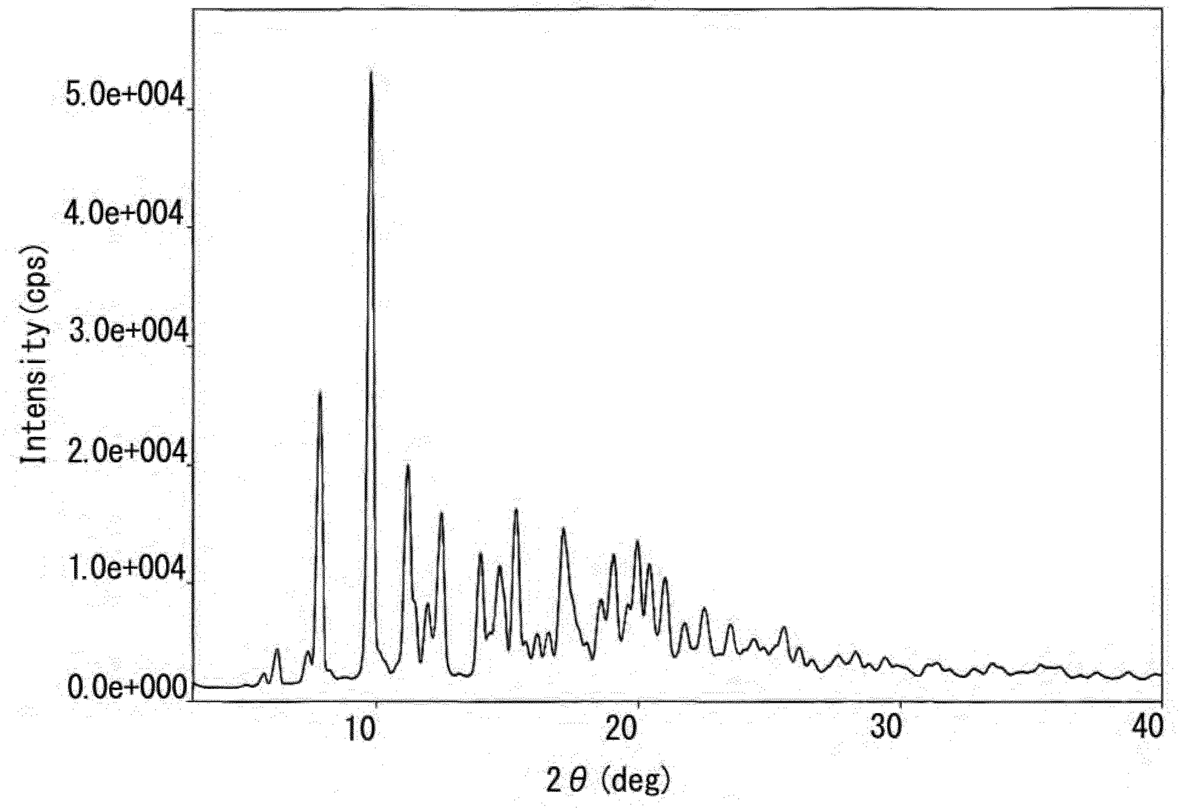
Intensity(cps)
5.0e+004
4.0e+004
3.0e+004
2.0e+004
1.0e+004
0.0e+000
10
20
30
40
2θ (deg)

FIG. 23

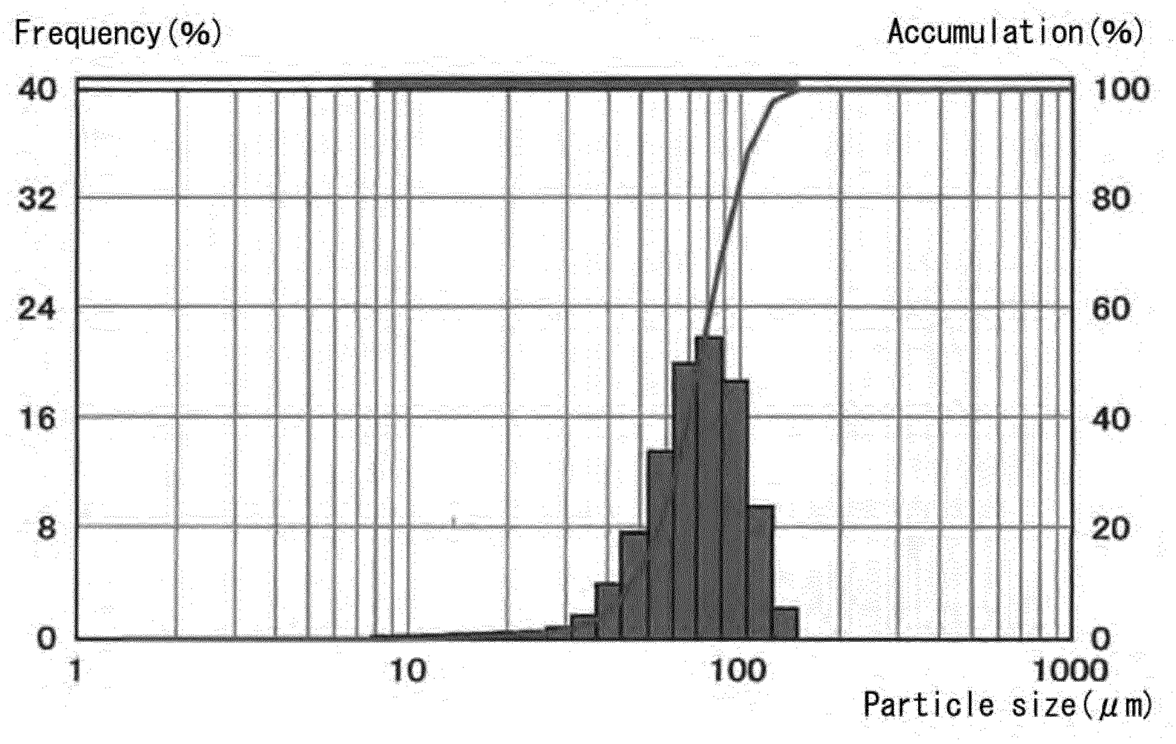
Frequency(%)
Accumulation(%)
40
32
24
16
8
0
100
80
60
40
20
0
1
10
100
1000
Particle size(μm)

FIG. 24

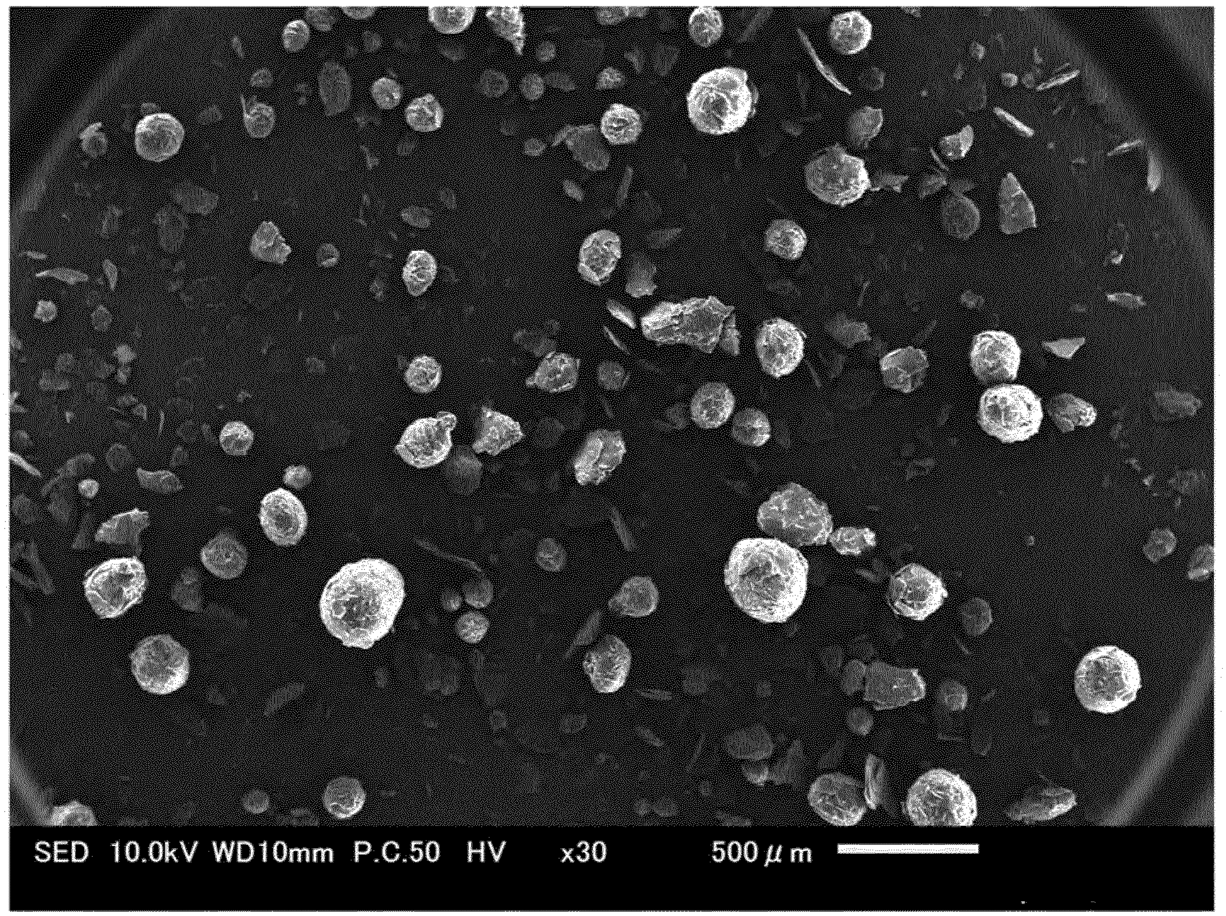

FIG. 25

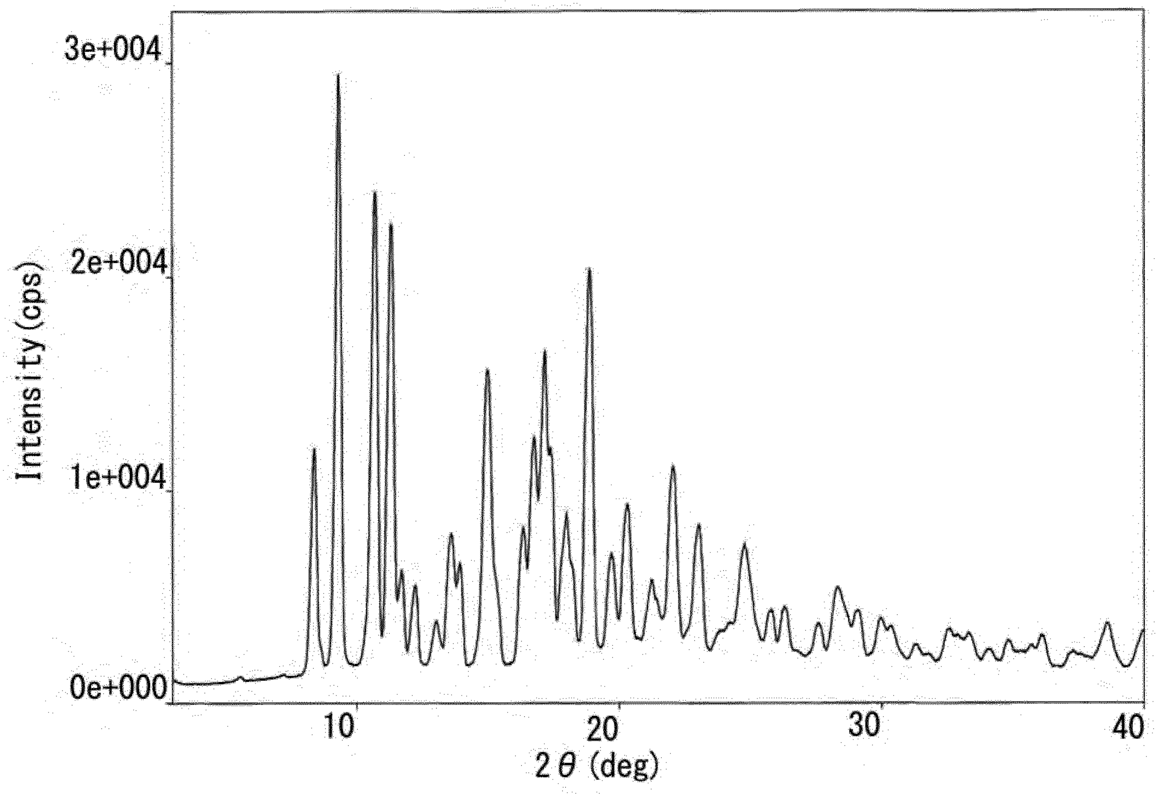
3e+004
2e+004
1e+004
0e+000
Intensity (cps)
10
20
30
40
2θ (deg)

FIG. 26

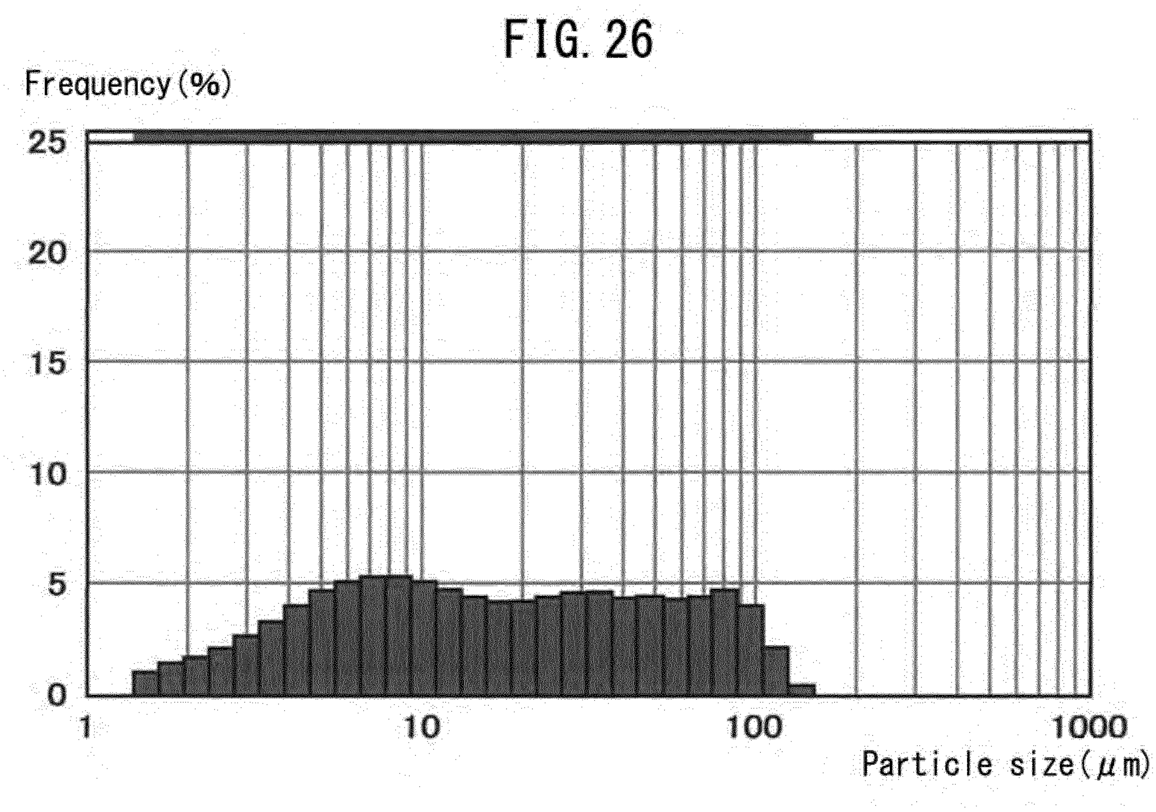
Frequency (%)
25
20
15
10
5
0
1
10
100
1000
Particle size (μm)

FIG. 27

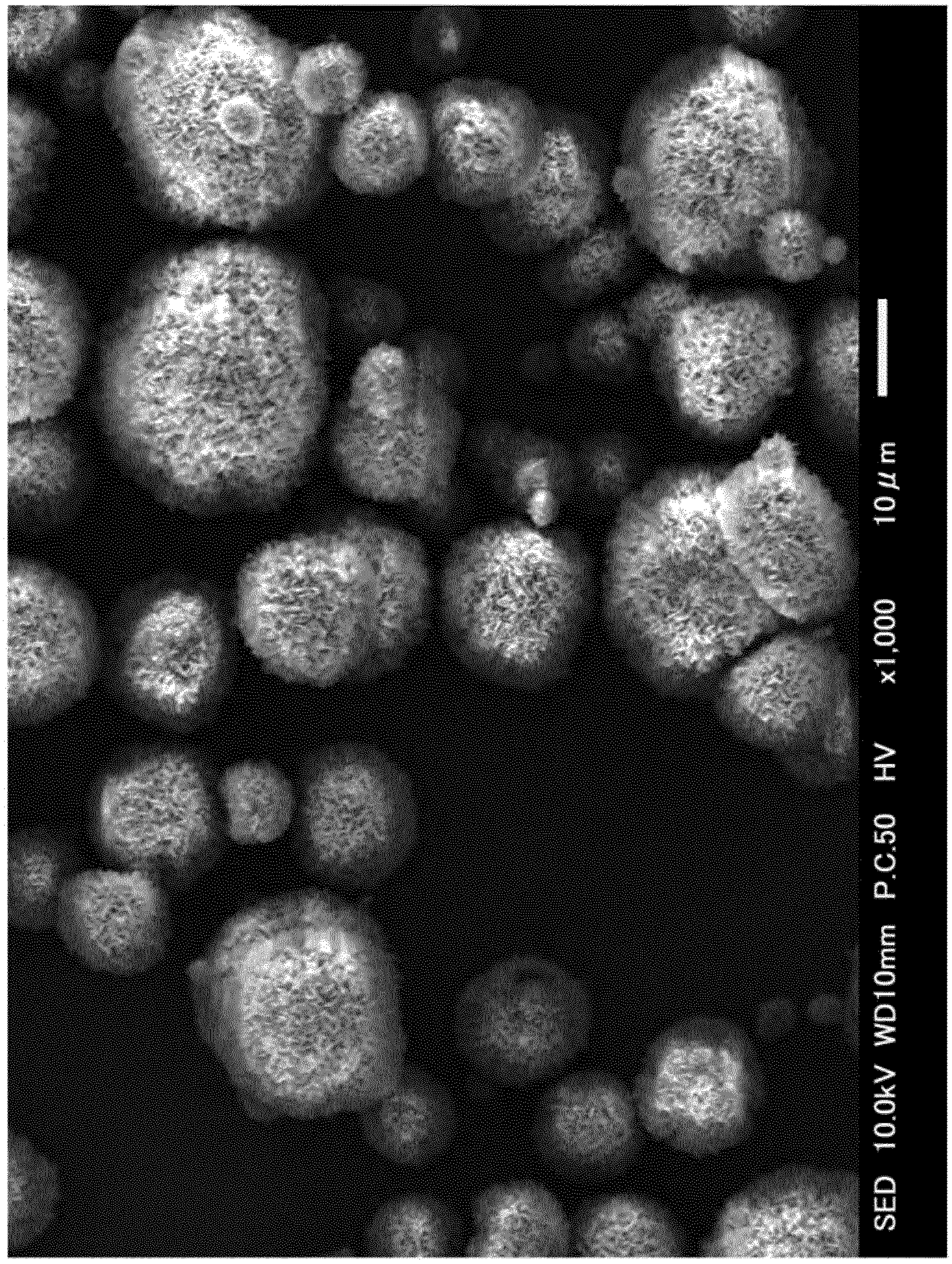

FIG. 28

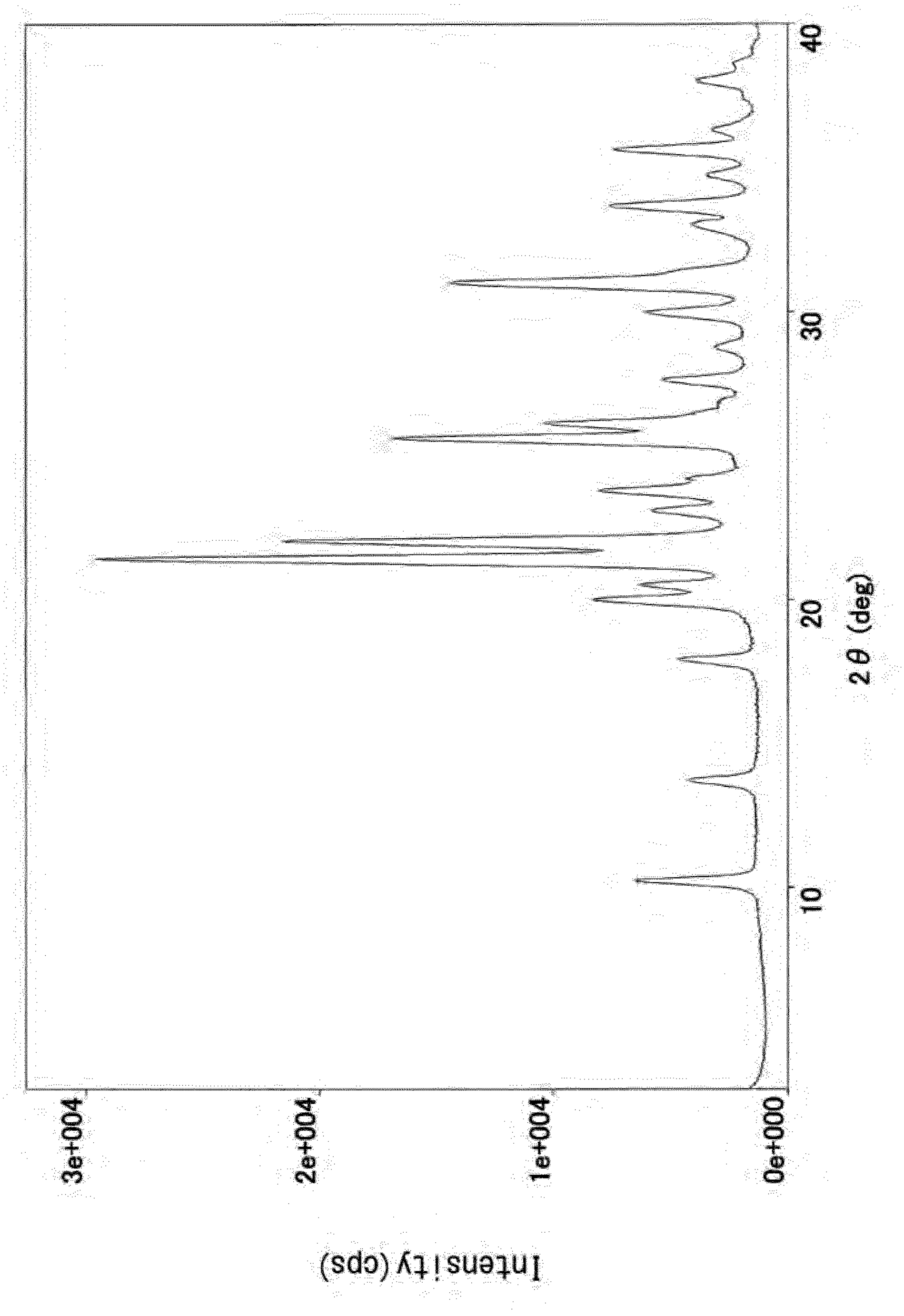

FIG. 29

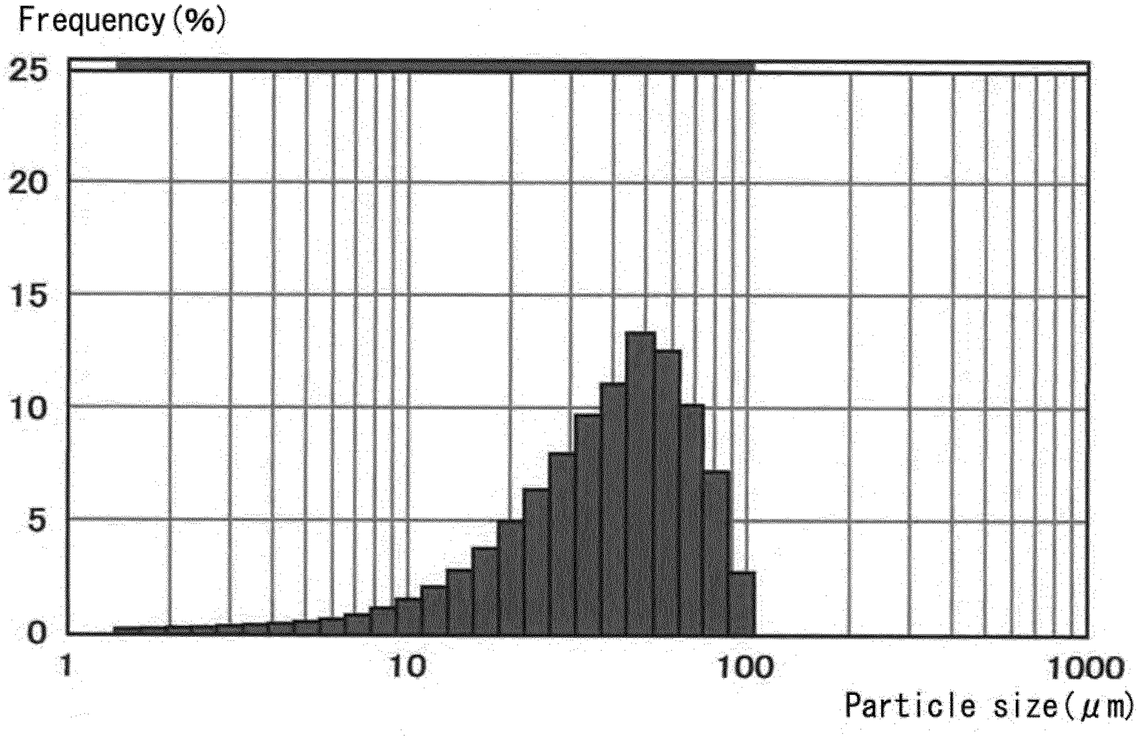
Frequency (%)
25
20
15
10
5
0
1
10
100
1000
Particle size (μm)

FIG. 30

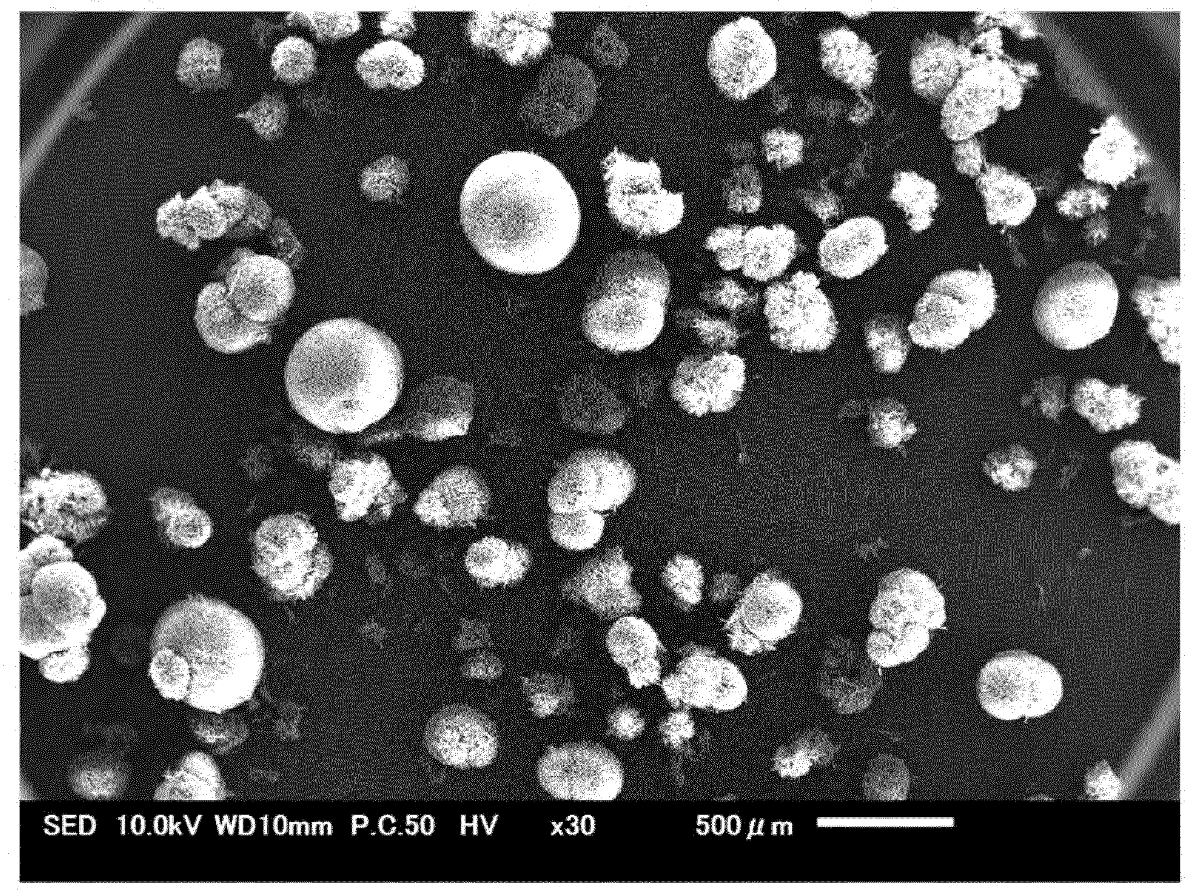
SED 10.0kV WD10mm P.C.50 HV x30 500 μm

FIG. 31

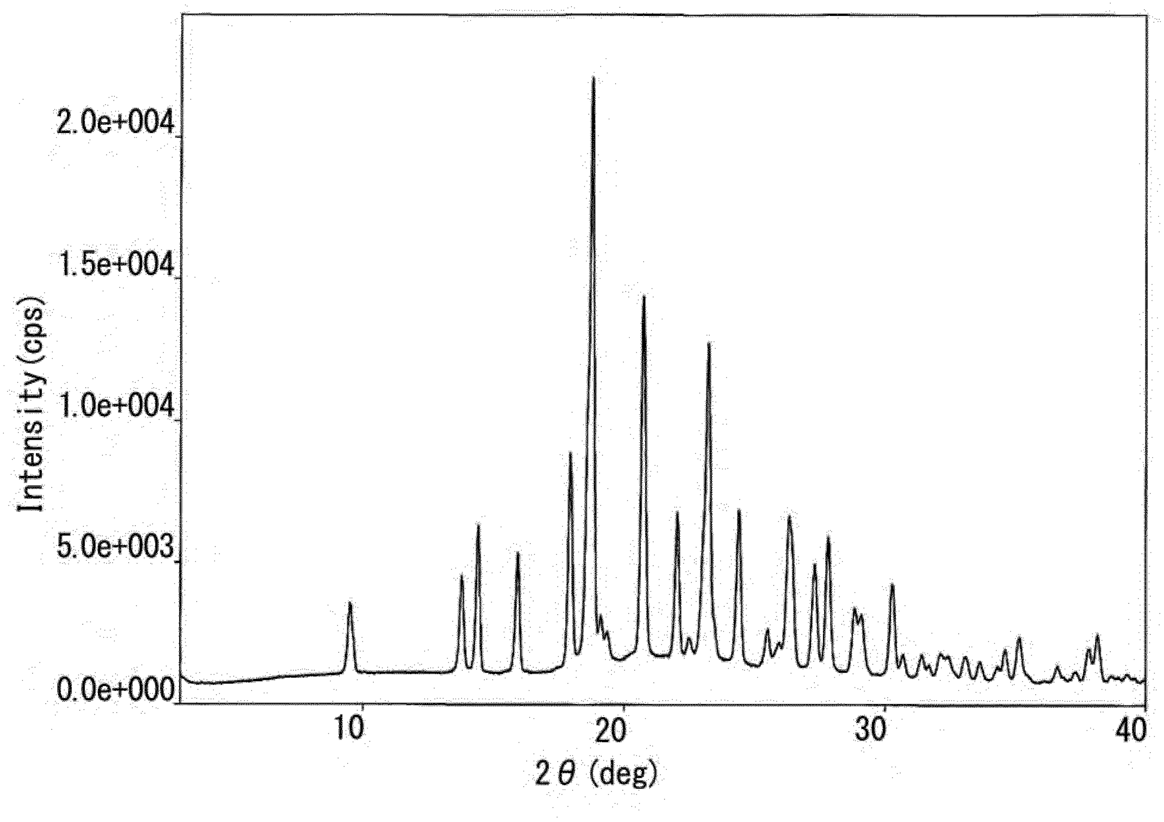
2.0e+004
1.5e+004
1.0e+004
5.0e+003
0.0e+000
Intensity(cps)
10
20
30
40
2θ (deg)

FIG. 32

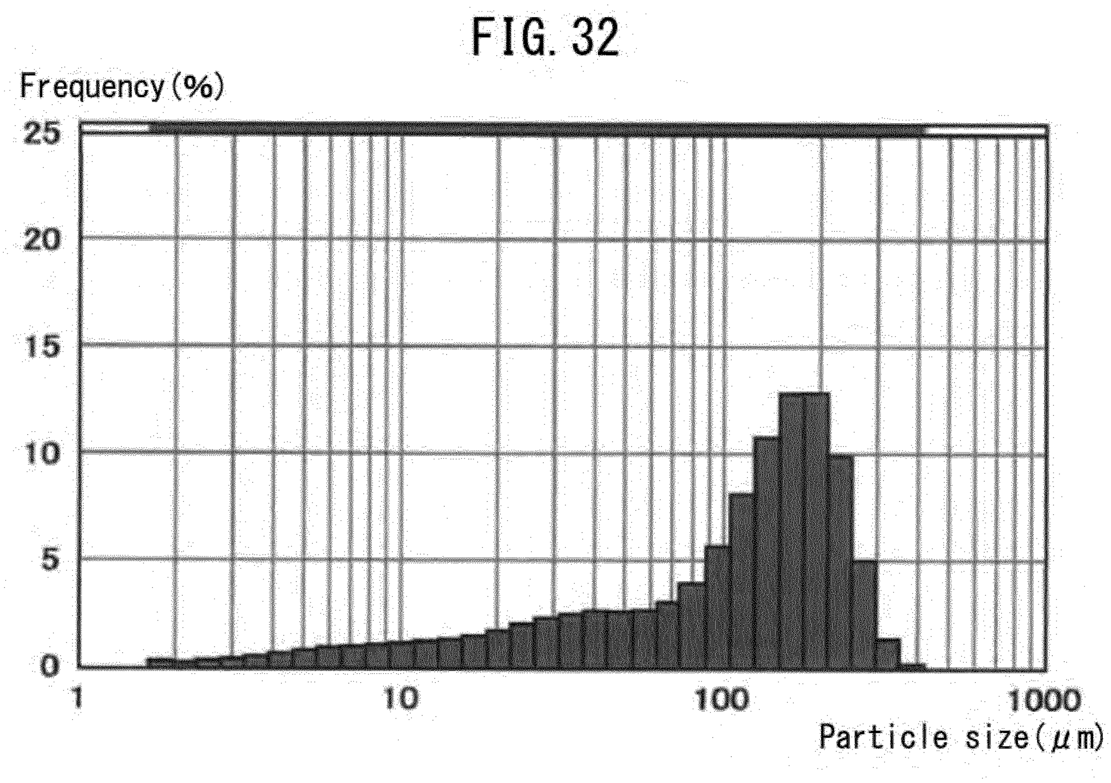
Frequency(%)
25
20
15
10
5
0
1
10
100
1000
Particle size(μm)

FIG. 33

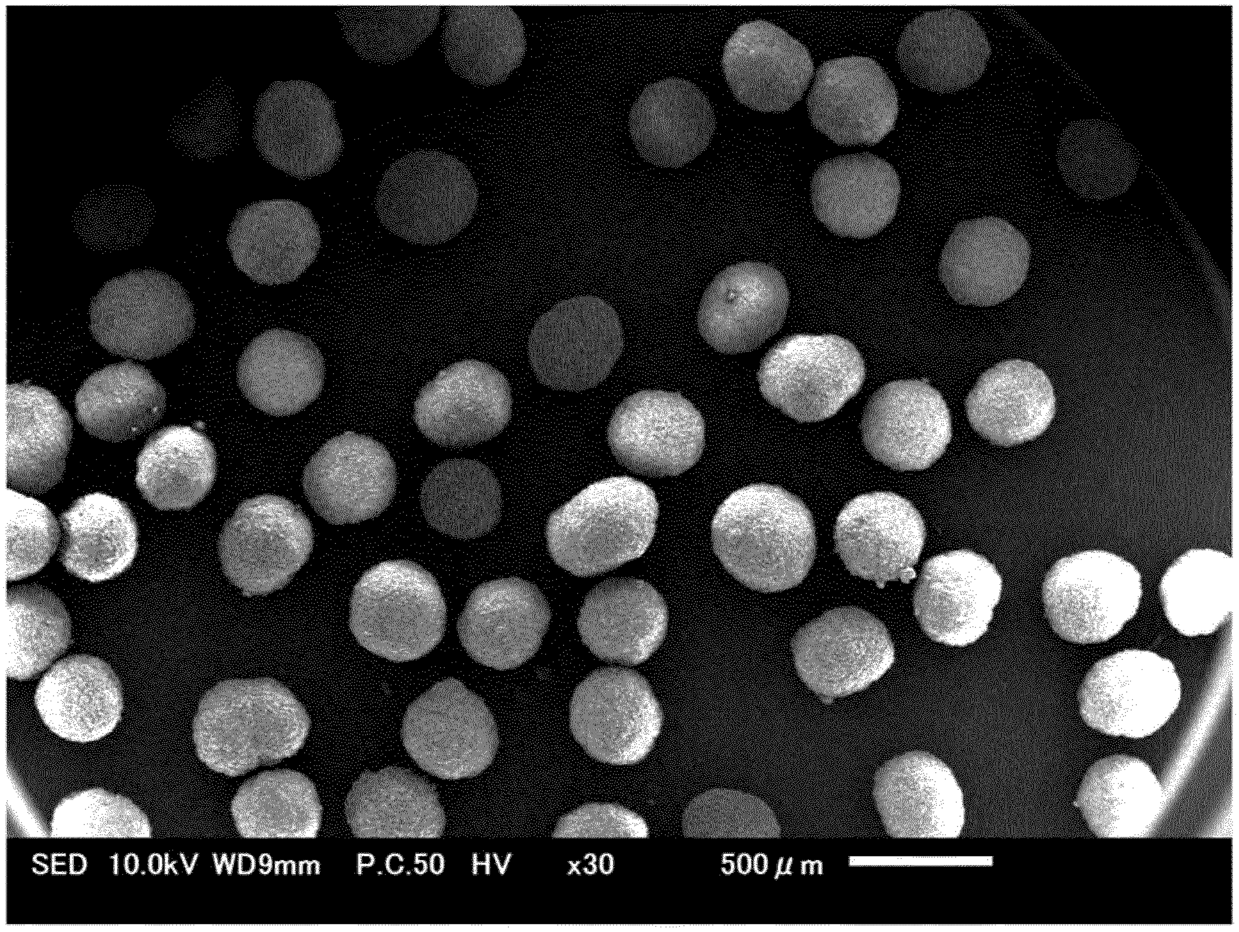

FIG. 34

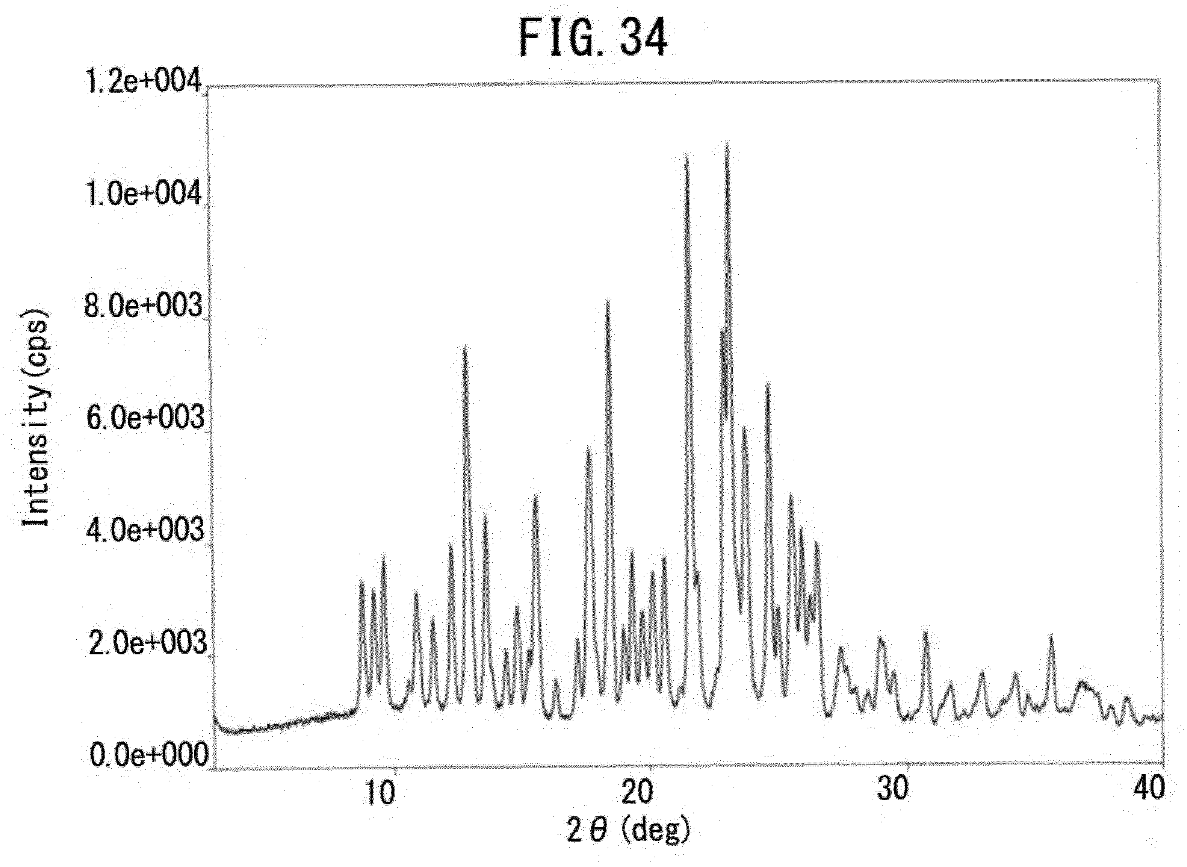
Intensity (cps)
1.2e+004
1.0e+004
8.0e+003
6.0e+003
4.0e+003
2.0e+003
0.0e+000
10
20
30
40
2θ (deg)

FIG. 35

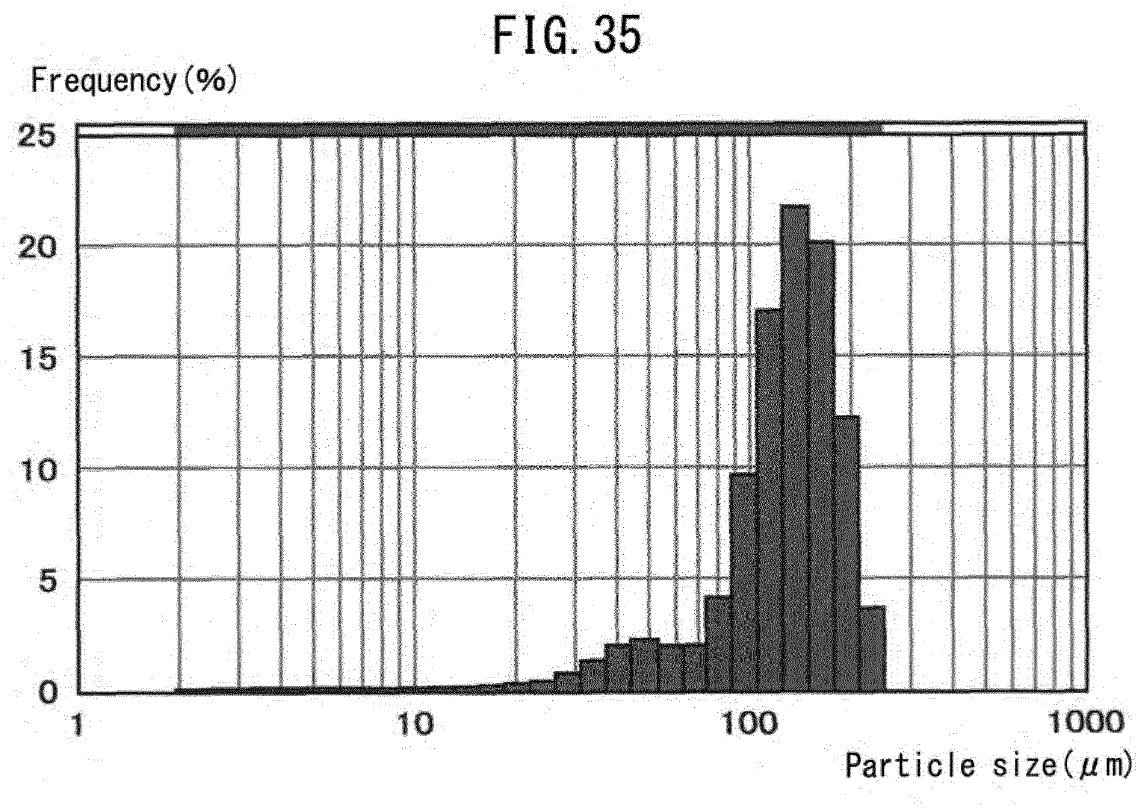
Frequency (%)
25
20
15
10
5
0
1
10
100
1000
Particle size (μm)

FIG. 36

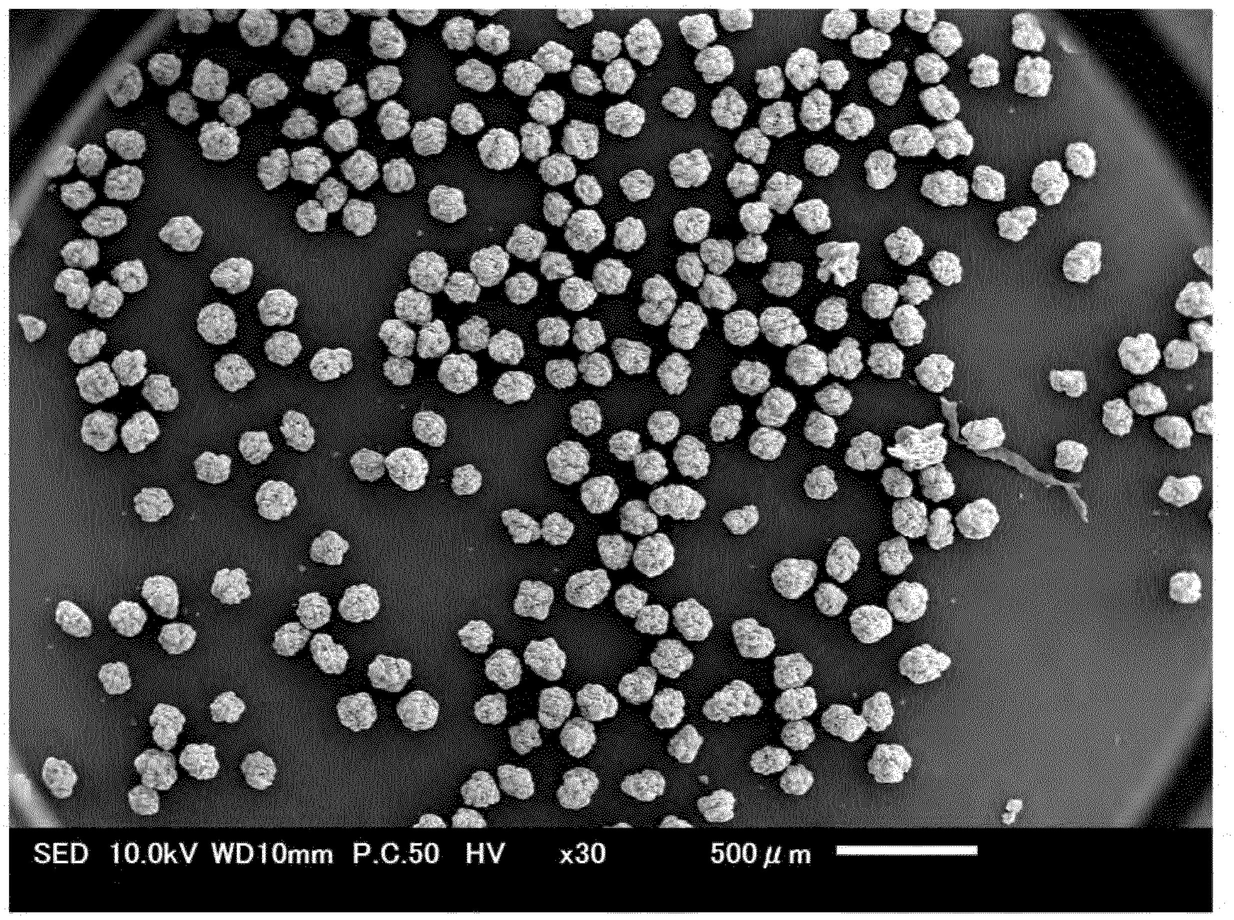

FIG. 37

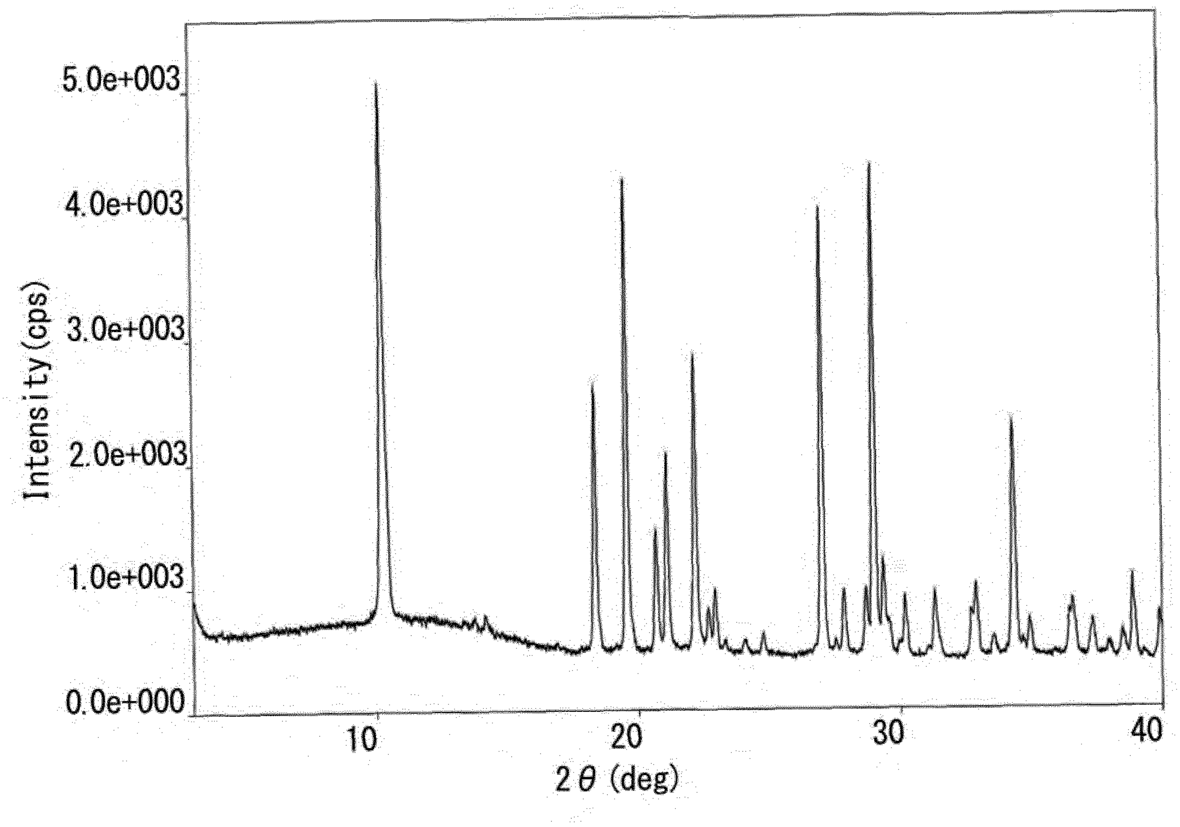
5.0e+003
4.0e+003
3.0e+003
2.0e+003
1.0e+003
0.0e+000
Intensity (cps)
10
20
30
40
2θ (deg)

FIG. 38

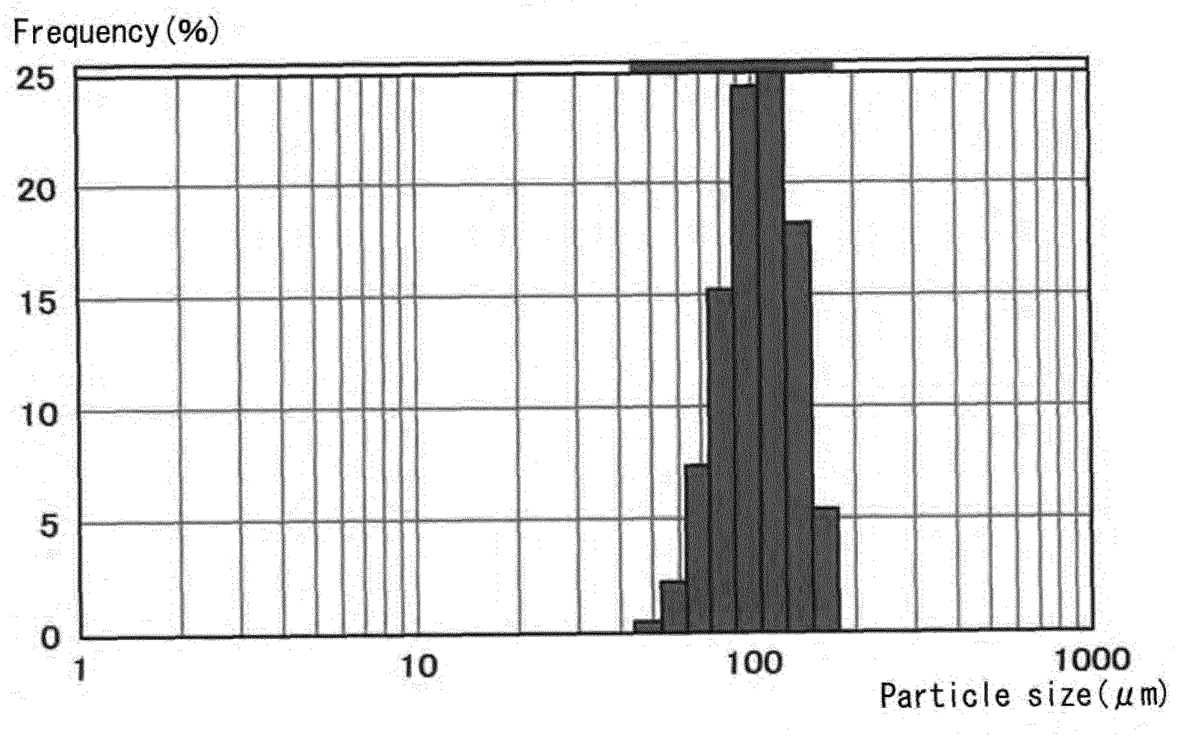
Frequency (%)
25
20
15
10
5
0
1
10
100
1000
Particle size (μm)

FIG. 39

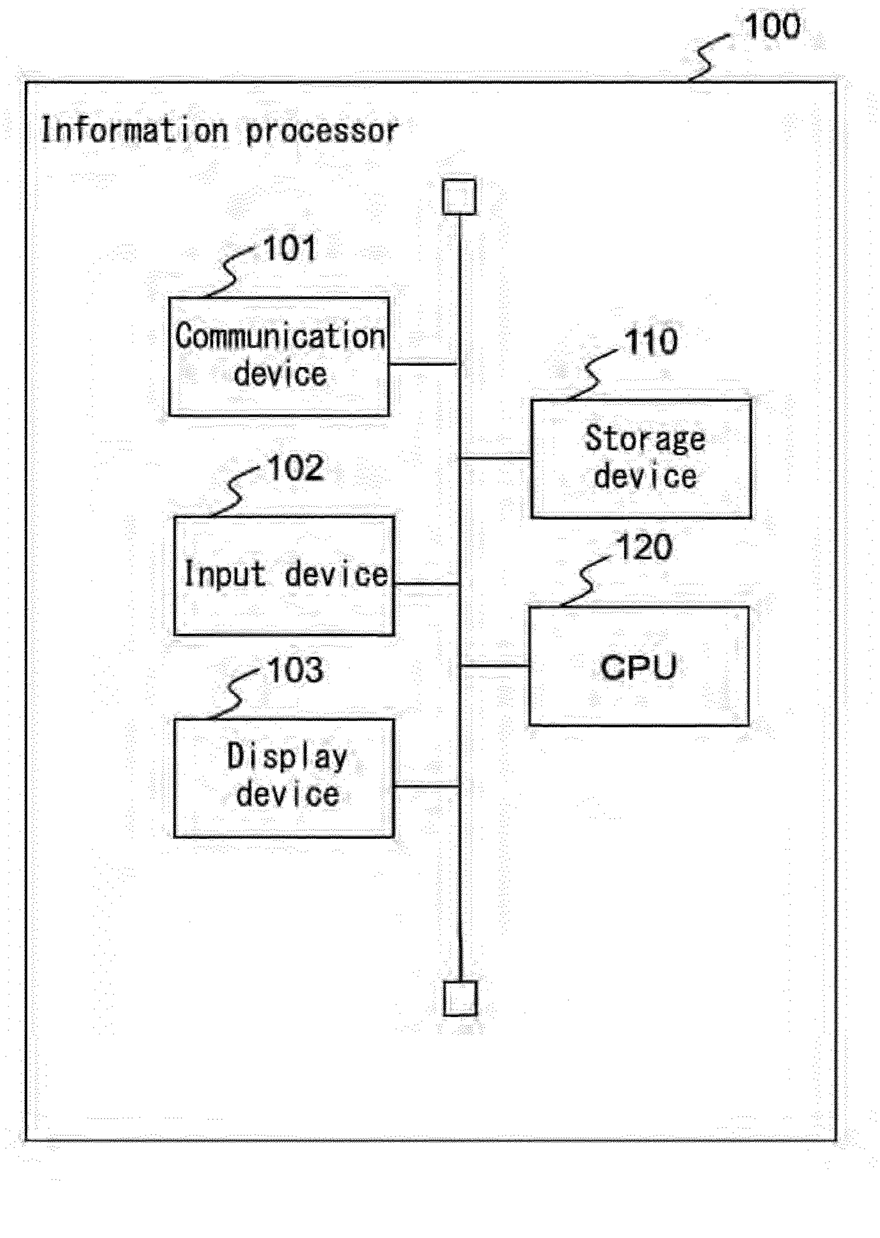
100
Information processor
101
Communication device
102
Input device
103
Display device
110
Storage device
120
CPU

FIG. 40

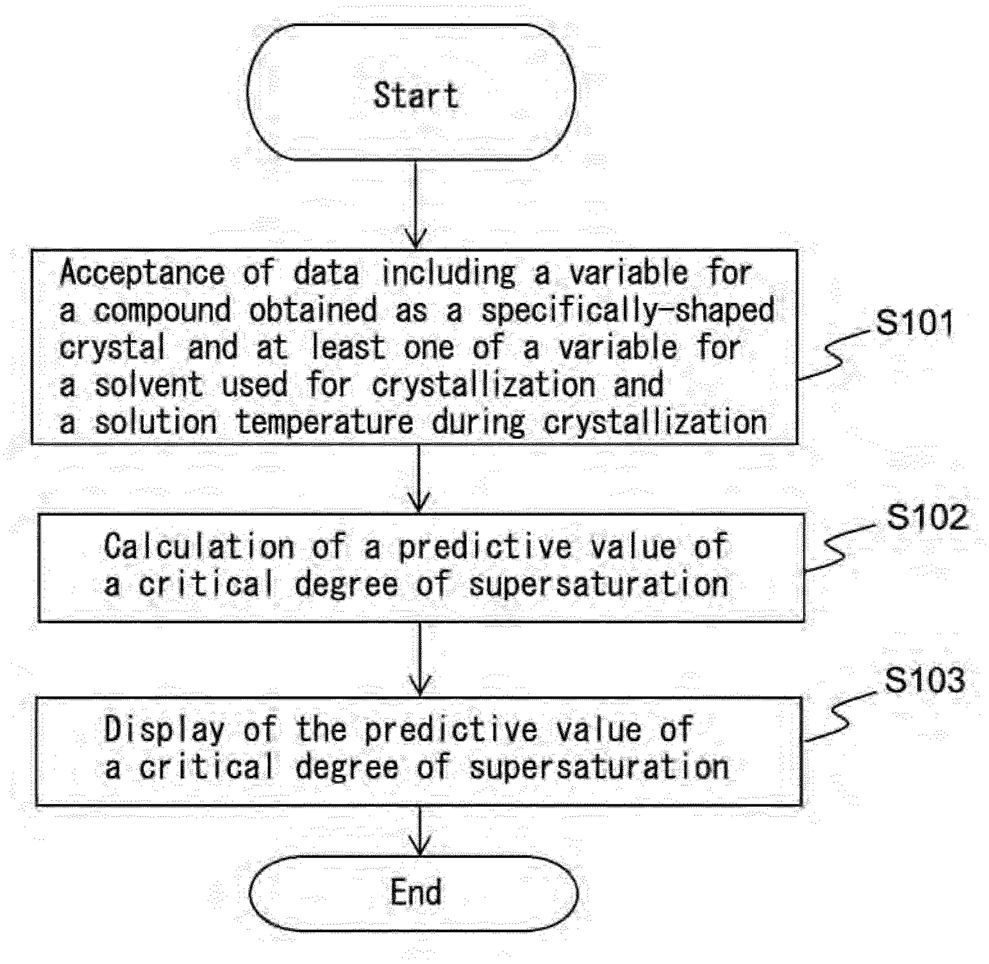
Start
Acceptance of data including a variable for a compound obtained as a specifically-shaped crystal and at least one of a variable for a solvent used for crystallization and a solution temperature during crystallization
S101
Calculation of a predictive value of a critical degree of supersaturation
S102
Display of the predictive value of a critical degree of supersaturation
S103
End

FIG. 41

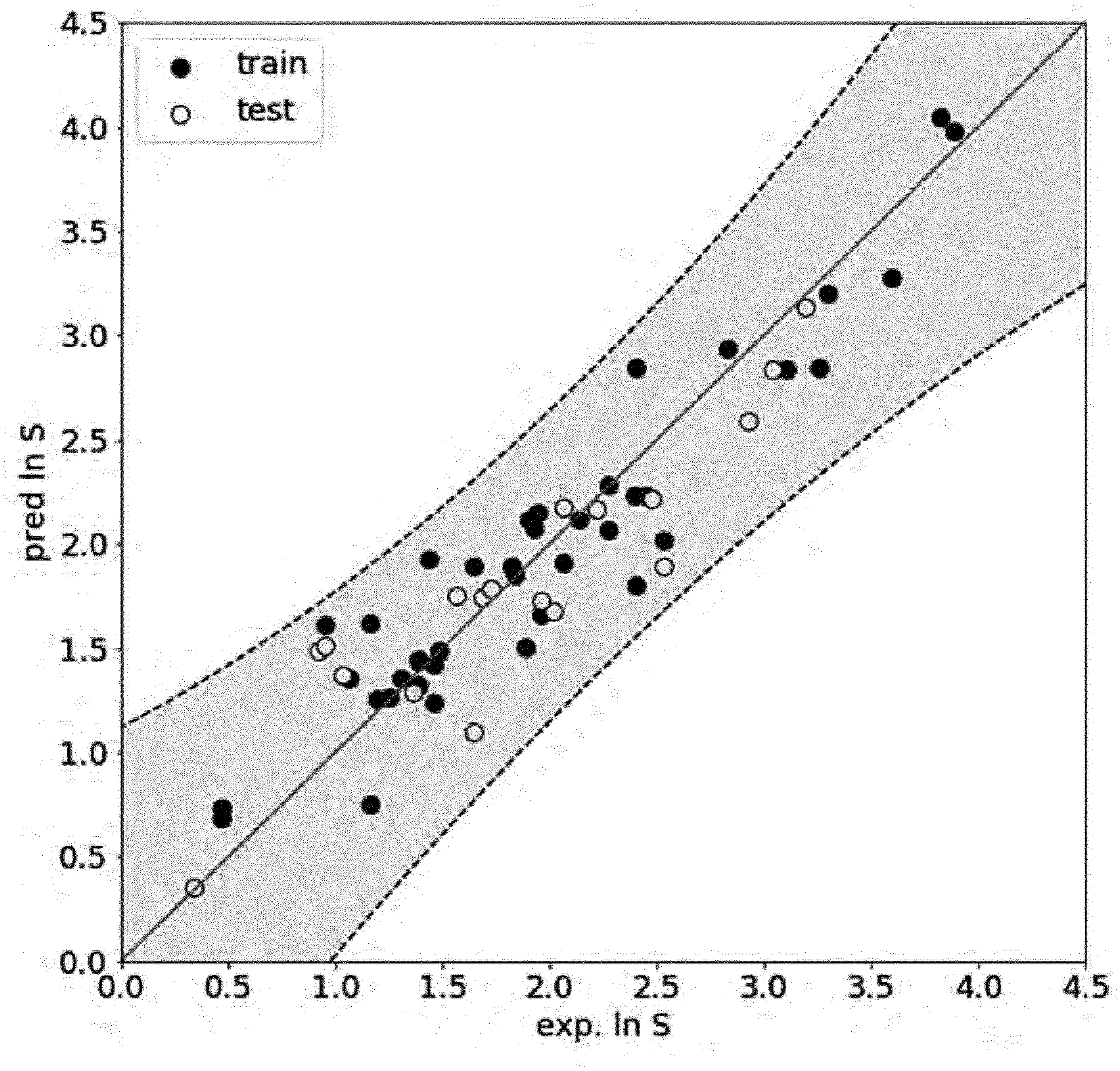
train
test
4.5
4.0
3.5
3.0
2.5
2.0
1.5
1.0
0.5
0.0
pred ln S
0.0 0.5 1.0 1.5 2.0 2.5 3.0 3.5 4.0 4.5
exp. ln S

FIG. 42

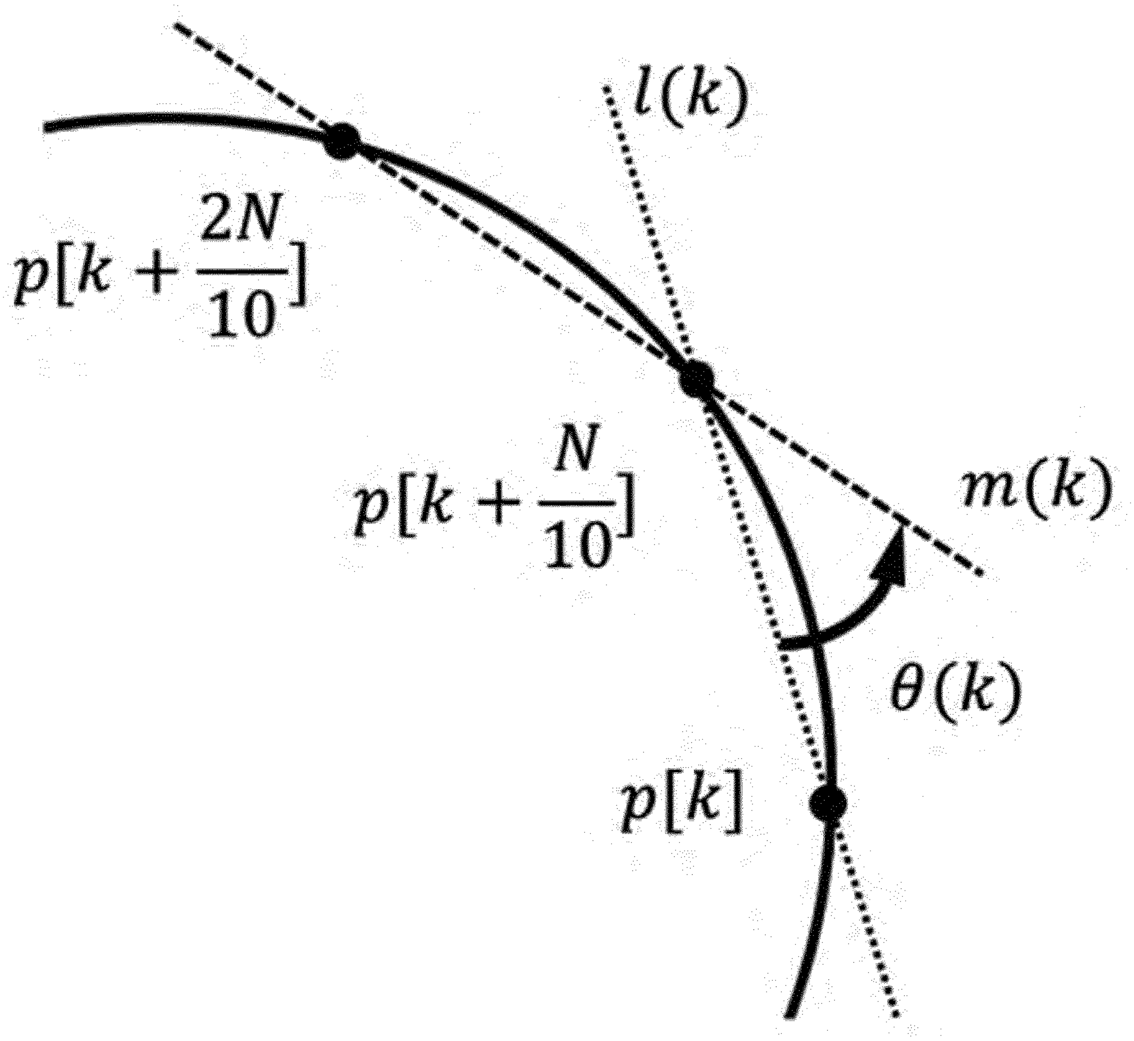

FIG. 43

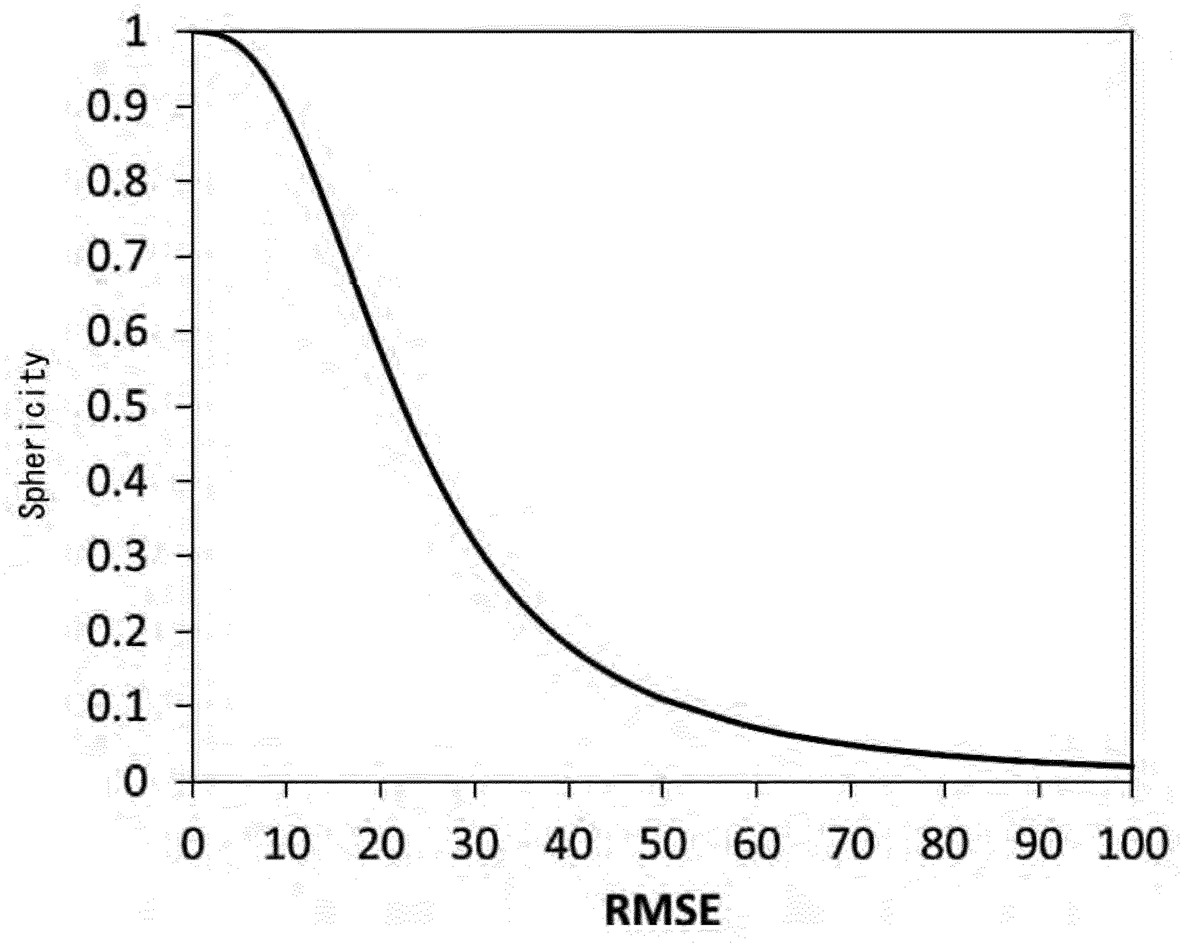
1
0.9
0.8
0.7
0.6
0.5
0.4
0.3
0.2
0.1
0
Sphericity
0
10
20
30
40
50
60
70
80
90
100
RMSE

FIG. 44

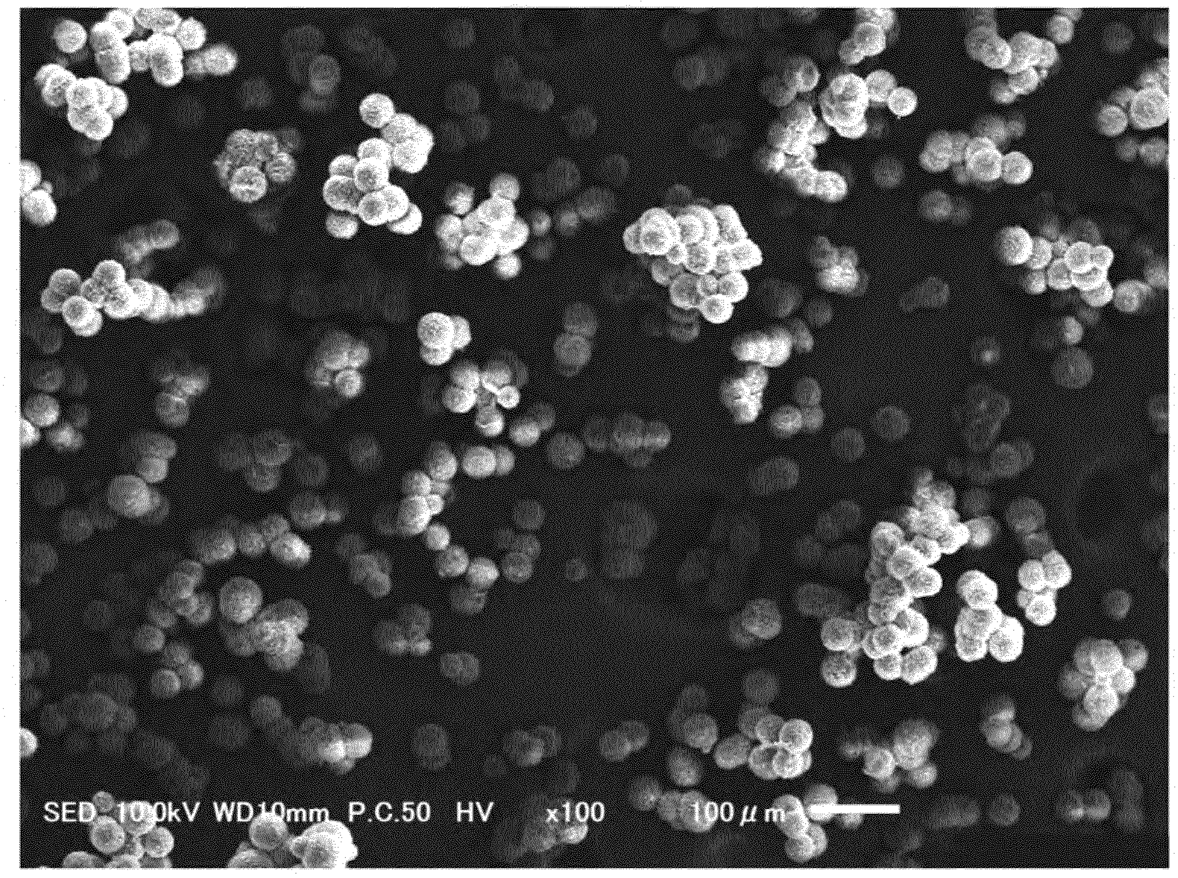
SED 10.0kV WD10mm P.C.50 HV x100 100 μm

FIG. 45

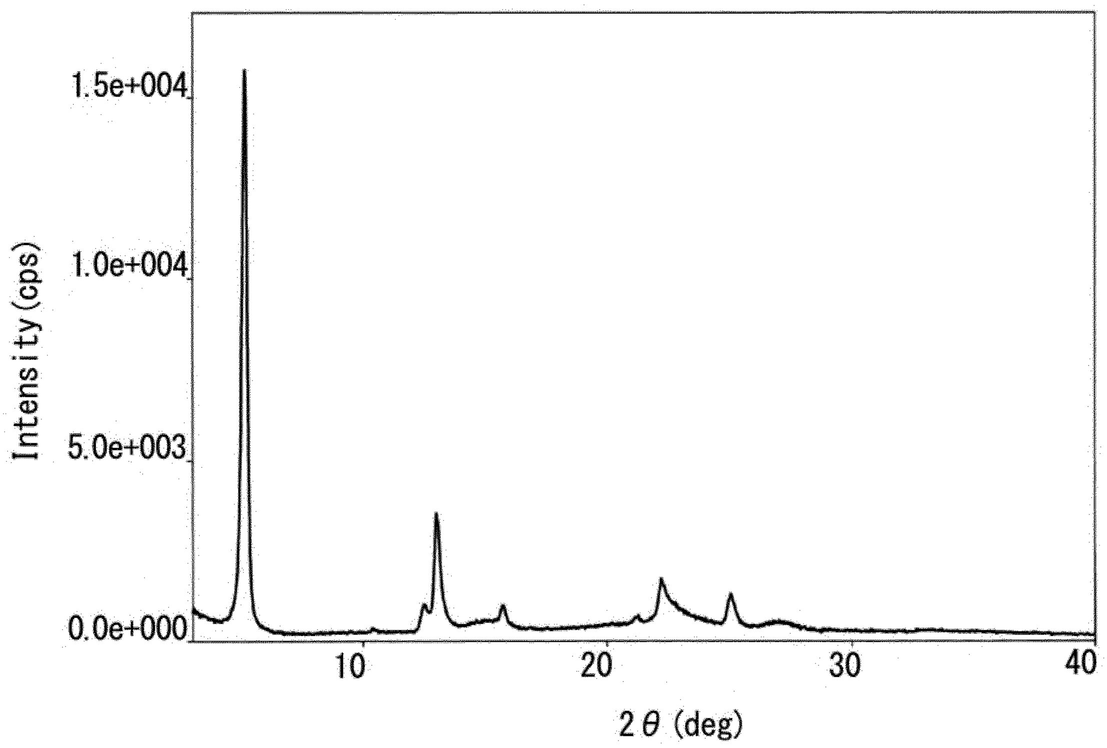
1.5e+004
1.0e+004
5.0e+003
0.0e+000
Intensity (cps)
10
20
30
40
2θ (deg)

FIG. 46

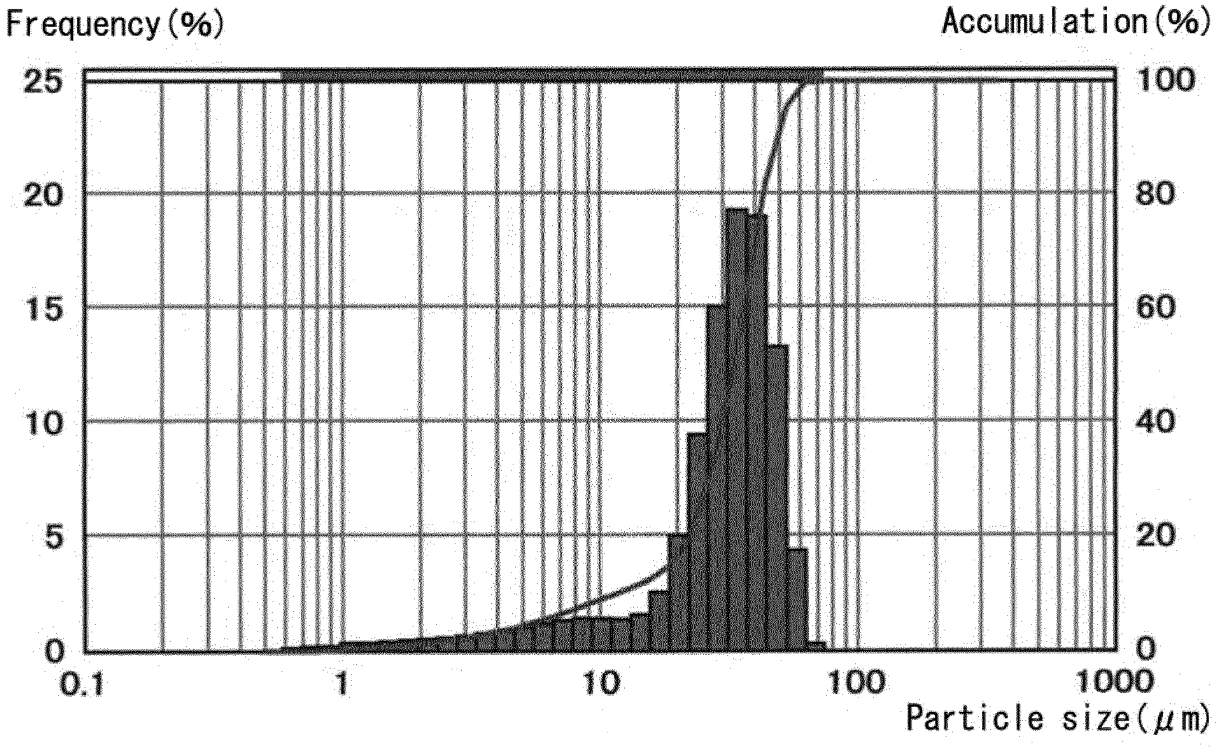
Frequency (%)
Accumulation (%)
25
20
15
10
5
0
100
80
60
40
20
0
0.1
1
10
100
1000
Particle size (μm)

FIG. 47

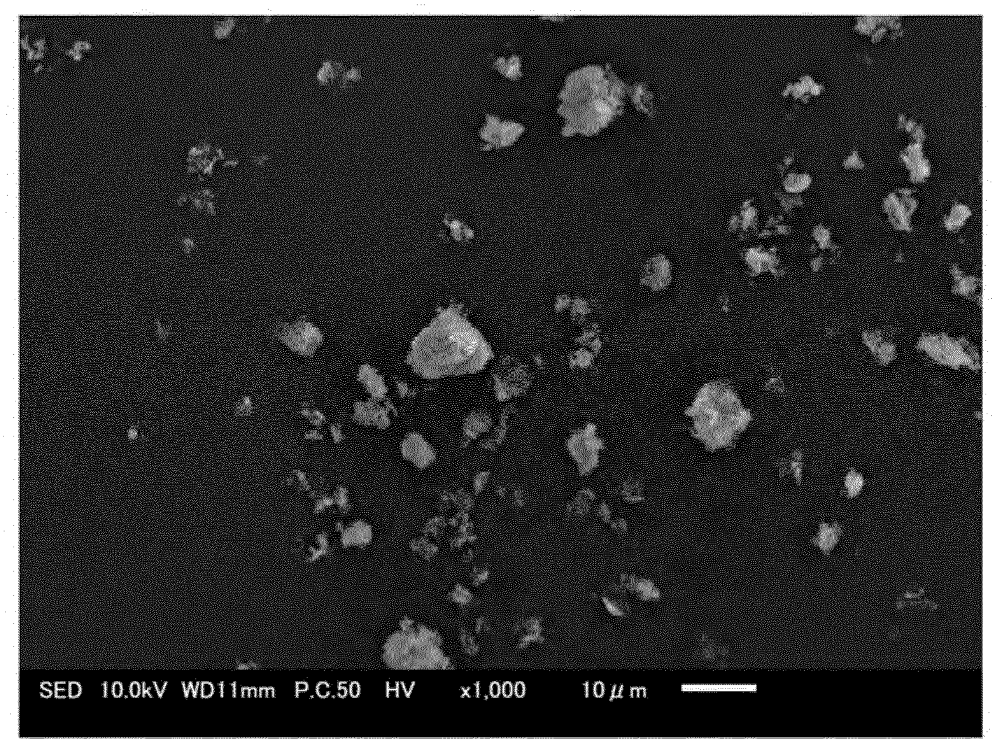
SED 10.0kV WD11mm P.C.50 HV x1,000 10μm

FIG. 48

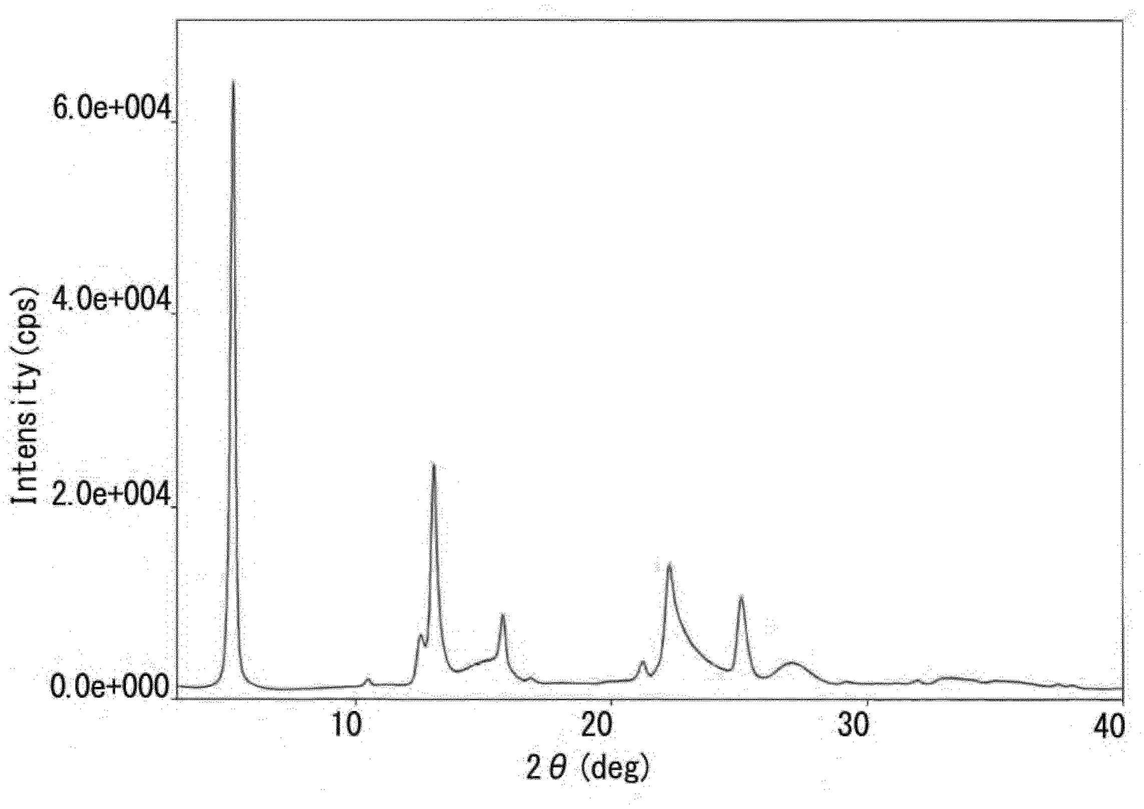
6.0e+004
4.0e+004
2.0e+004
0.0e+000
Intensity(cps)
10
20
30
40
2θ (deg)

FIG. 49

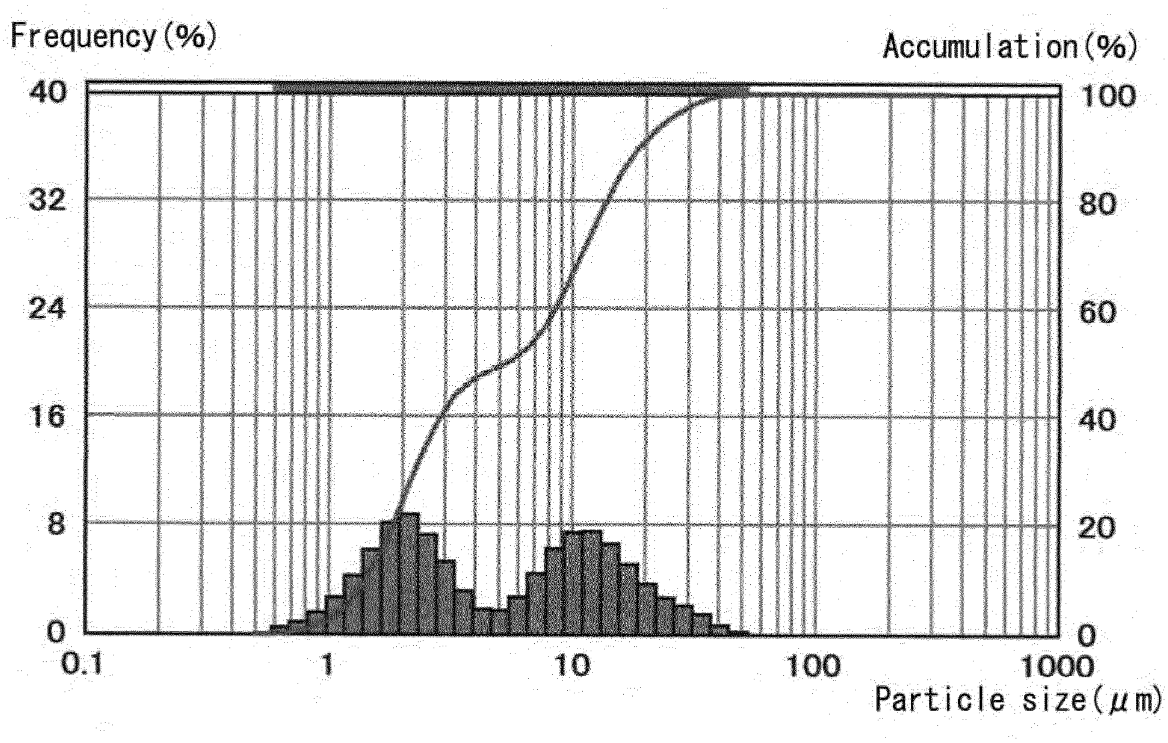
Frequency(%)
Accumulation(%)
40
32
24
16
8
0
100
80
60
40
20
0
0.1
1
10
100
1000
Particle size(μm)

FIG. 50

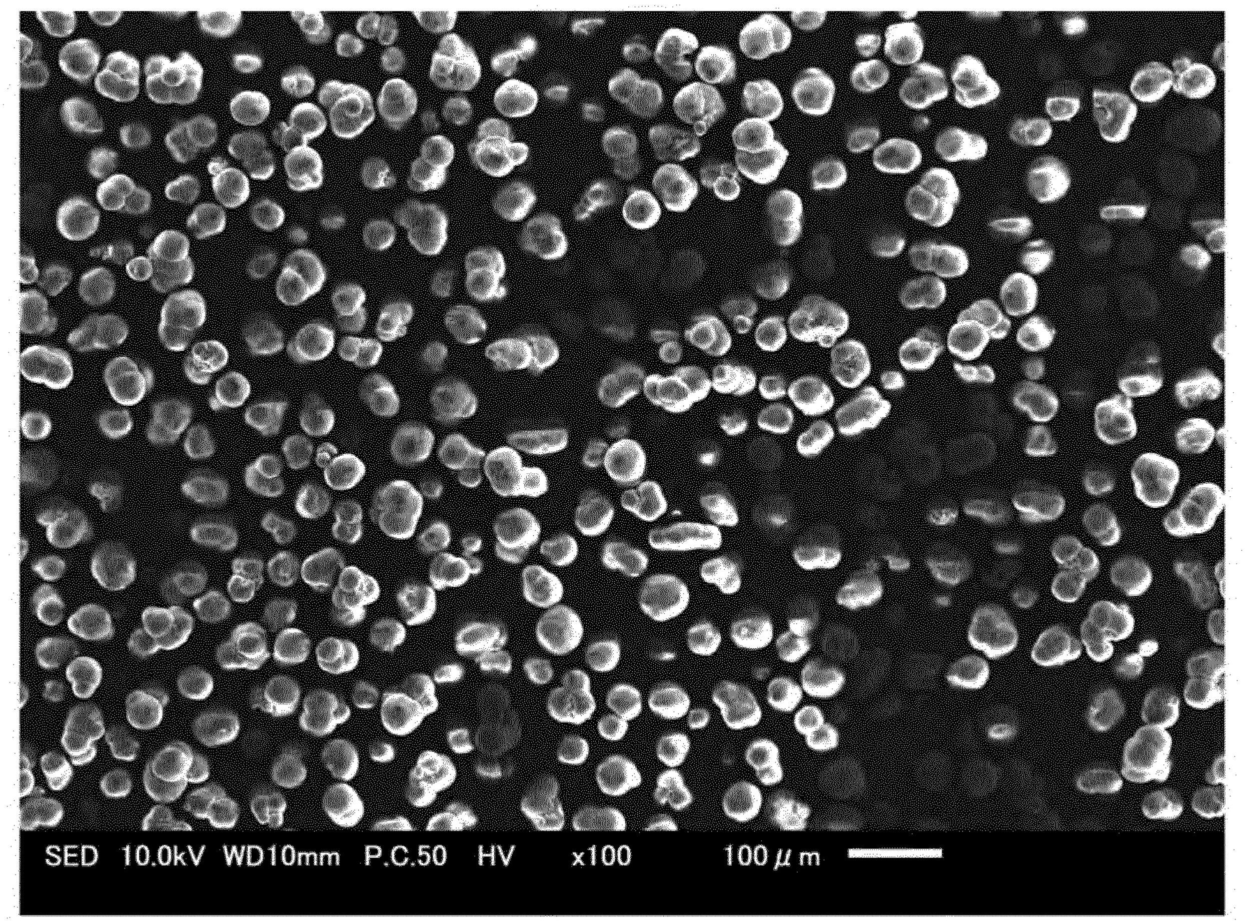

FIG. 51

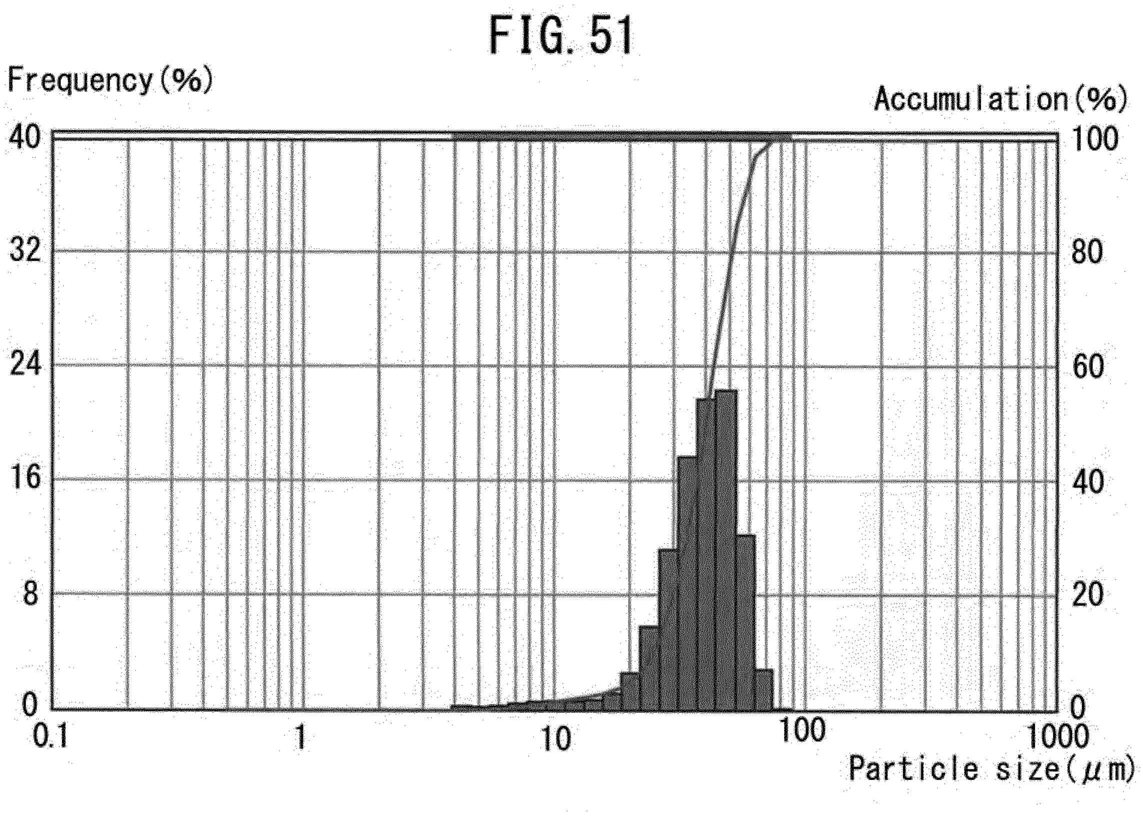
Frequency (%)
Accumulation (%)
40
32
24
16
8
0
100
80
60
40
20
0
0.1
1
10
100
1000
Particle size (μm)

FIG. 52

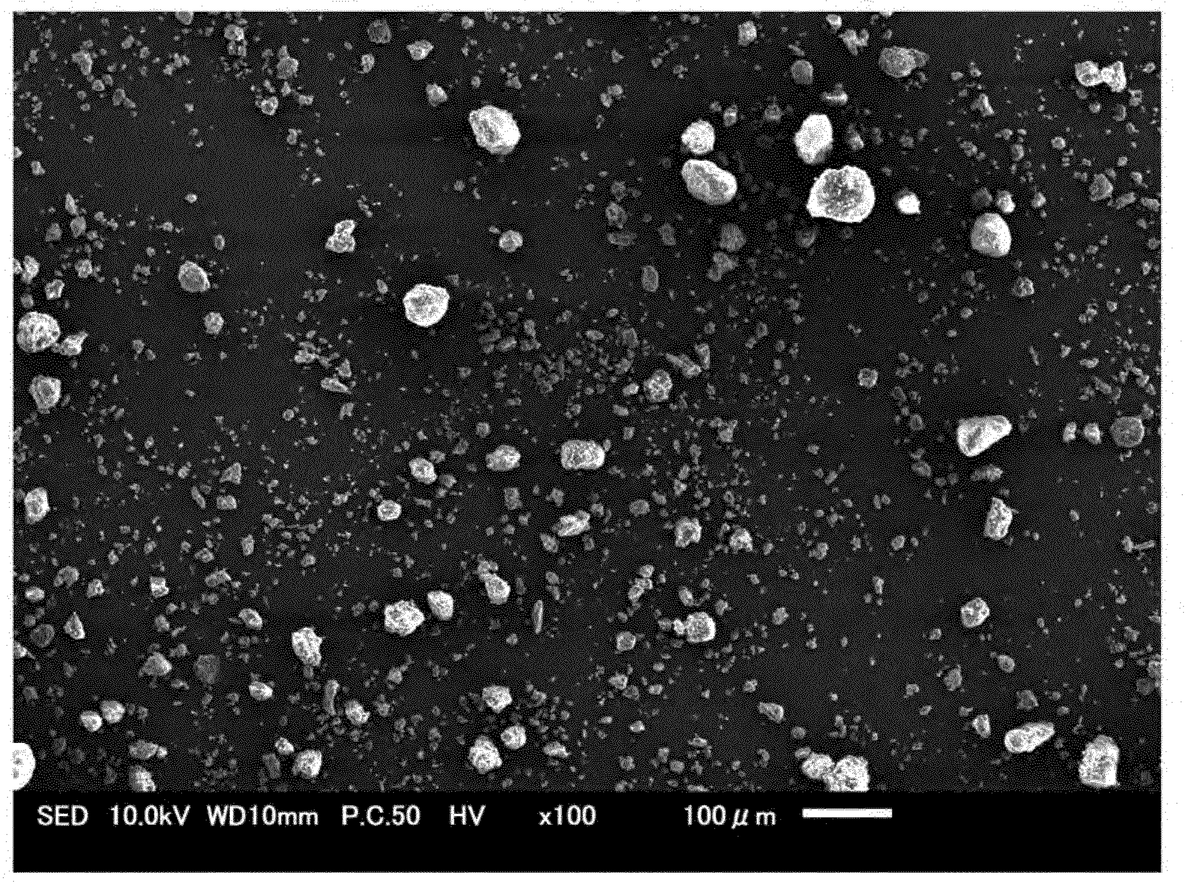
SED 10.0kV WD10mm P.C.50 HV x100 100 μm

FIG. 53

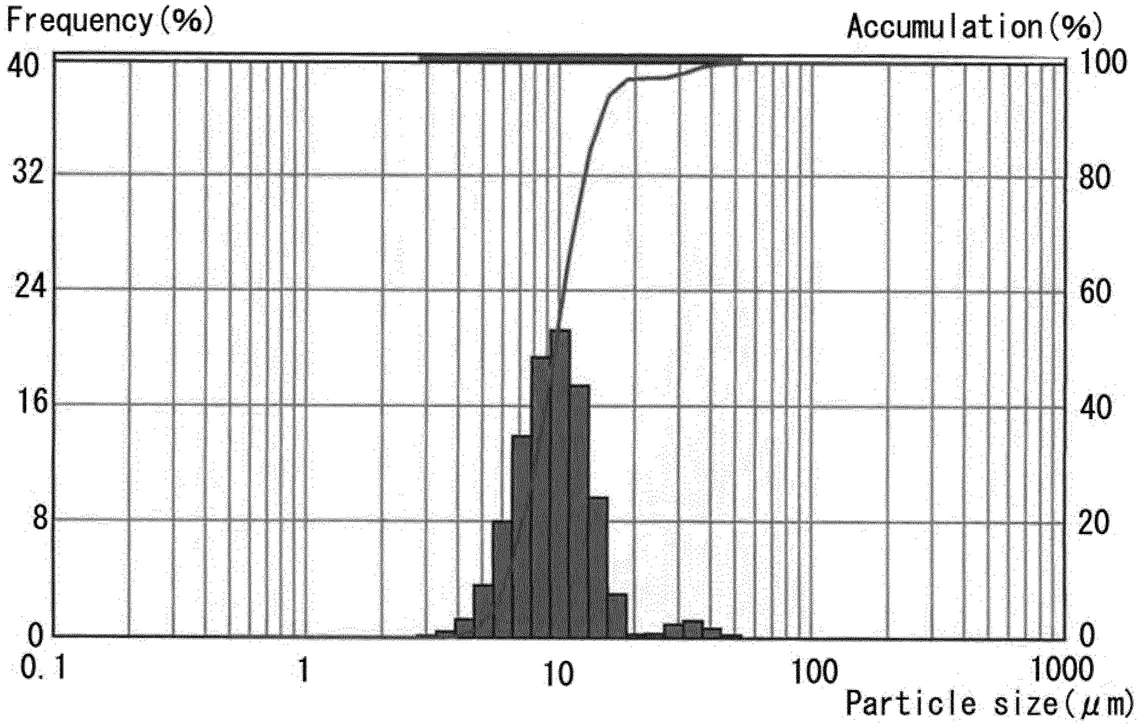
Frequency (%)
Accumulation (%)
40
32
24
16
8
0
100
80
60
40
20
0
0.1
1
10
100
1000
Particle size (μm)

FIG. 54

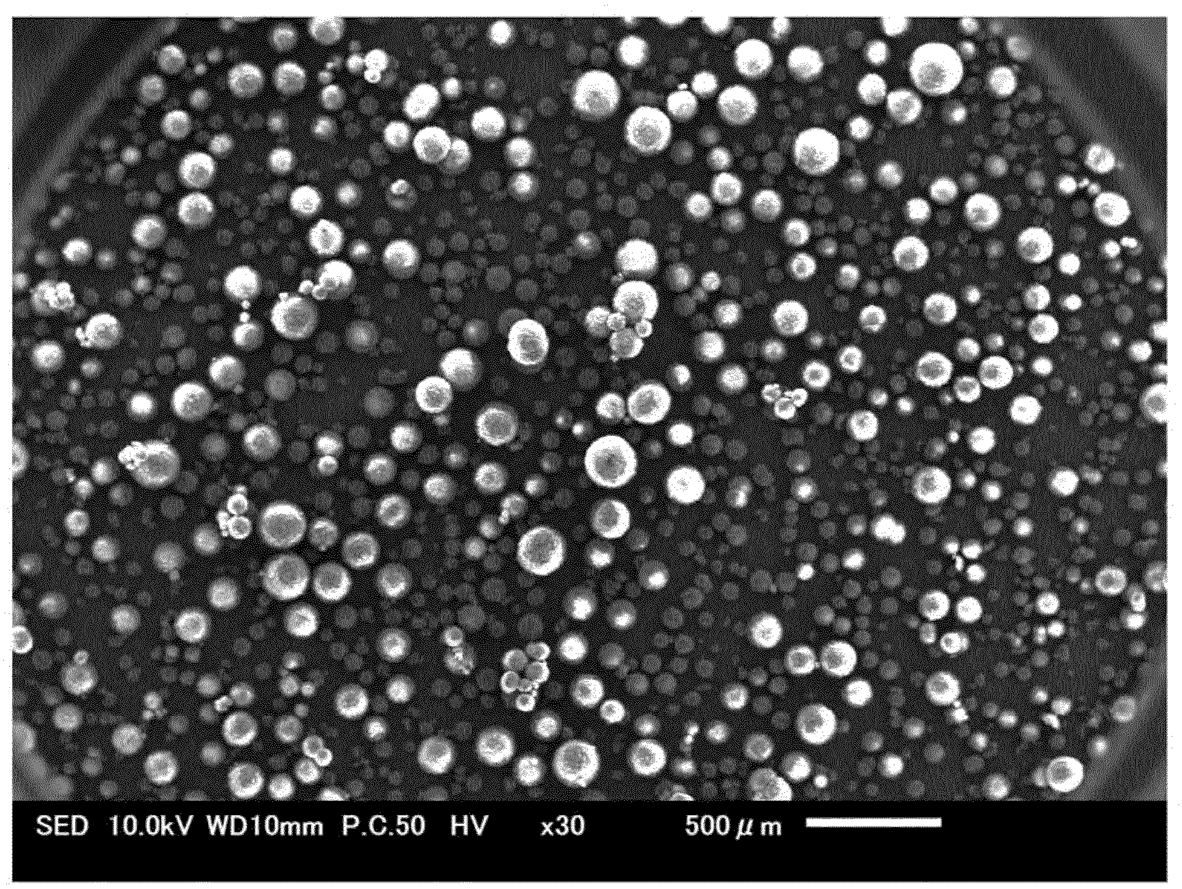
SED 10.0kV WD10mm P.C.50 HV x30 500μm

FIG. 55

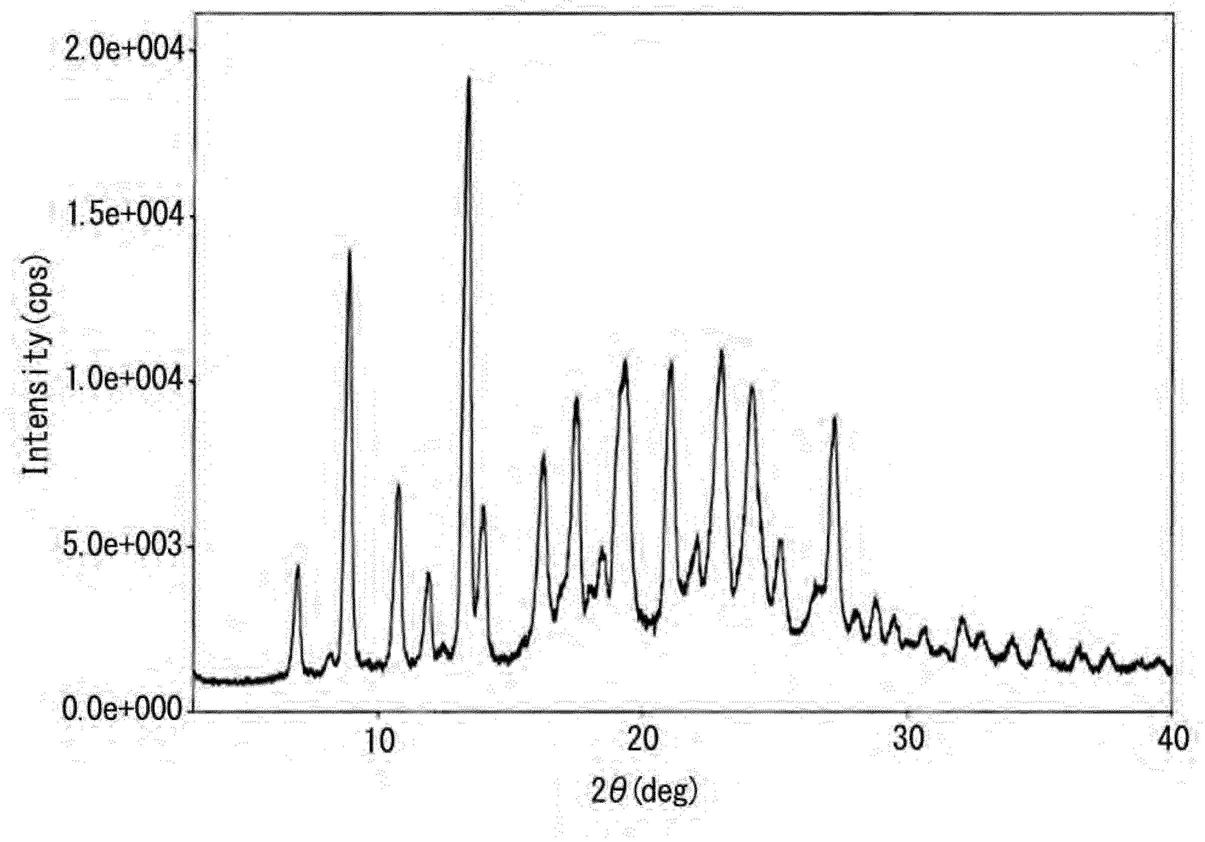
2.0e+004
1.5e+004
1.0e+004
5.0e+003
0.0e+000
Intensity (cps)
10
20
30
40
2θ (deg)

FIG. 56

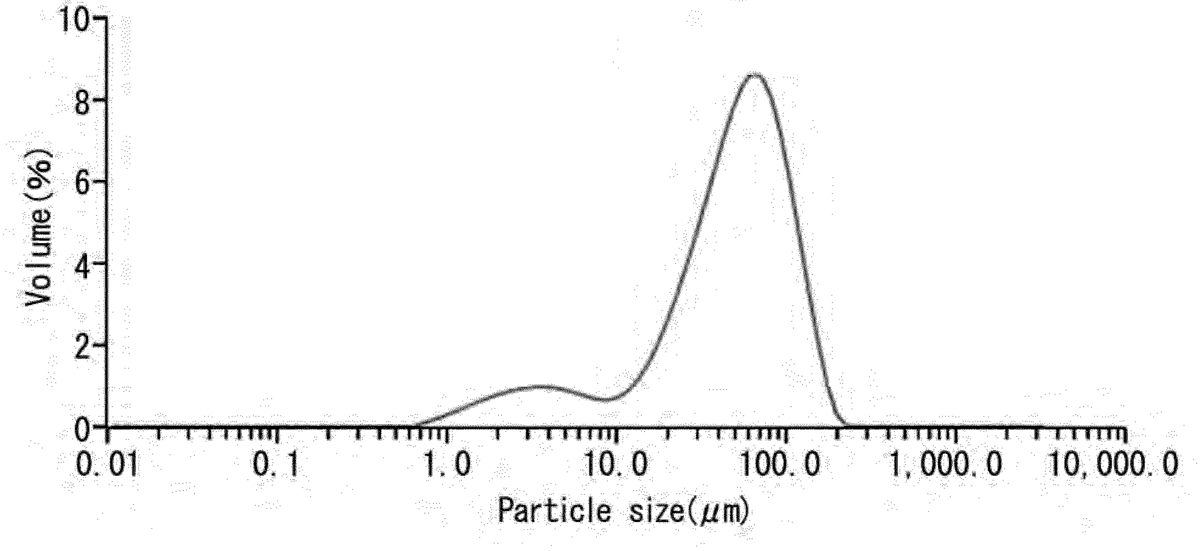
10
8
6
4
2
0
Volume (%)
0.01
0.1
1.0
10.0
100.0
1,000.0
10,000.0
Particle size(μm)

FIG. 57

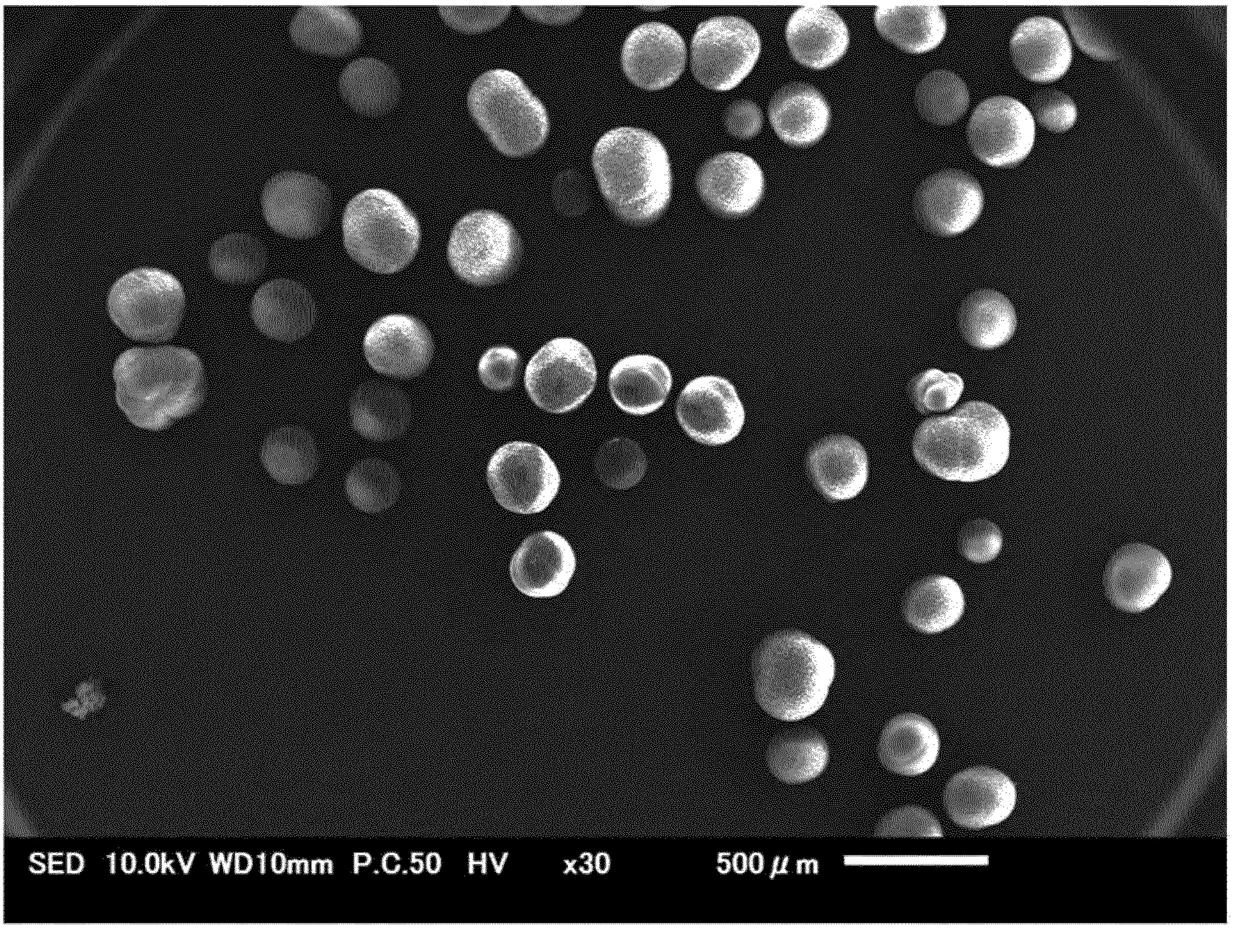

FIG. 58

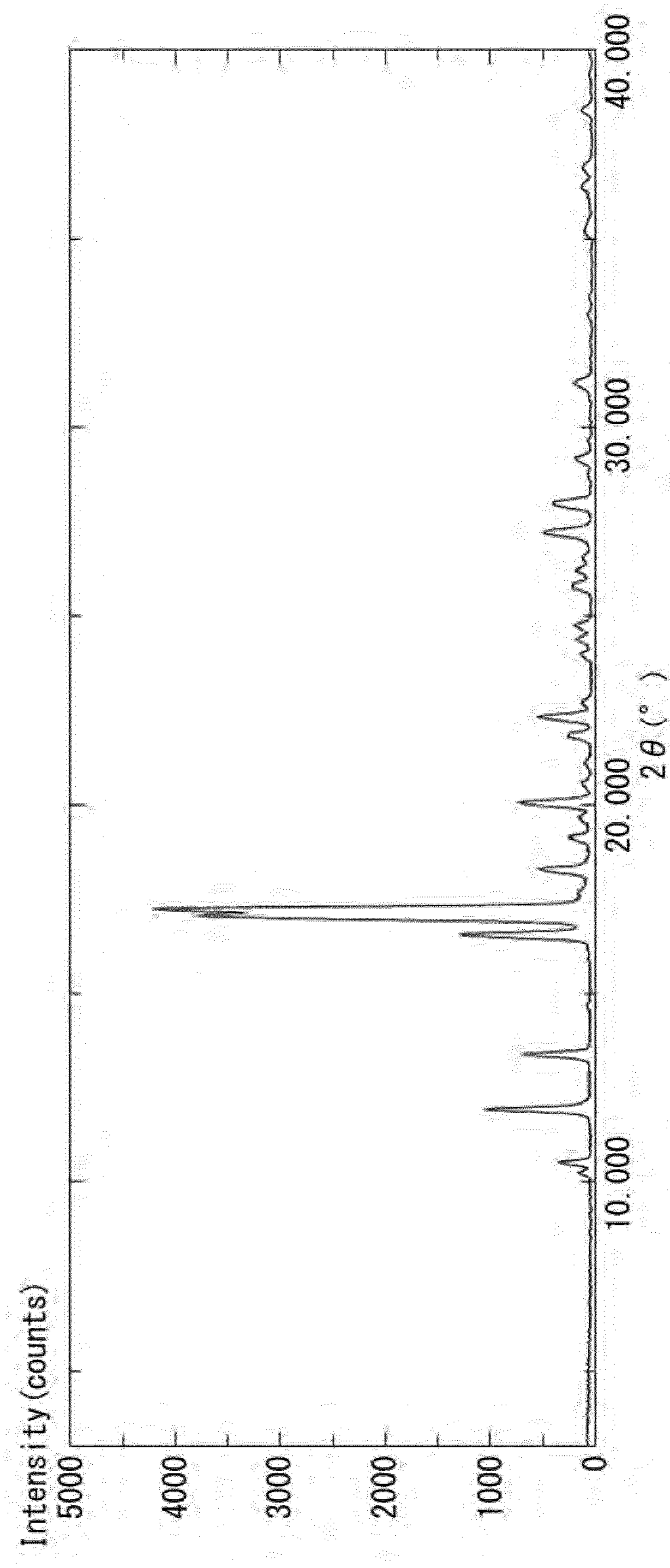
Intensity (counts)
5000
4000
3000
2000
1000
0
10.000
20.000
30.000
40.000
2θ(°)

FIG. 59

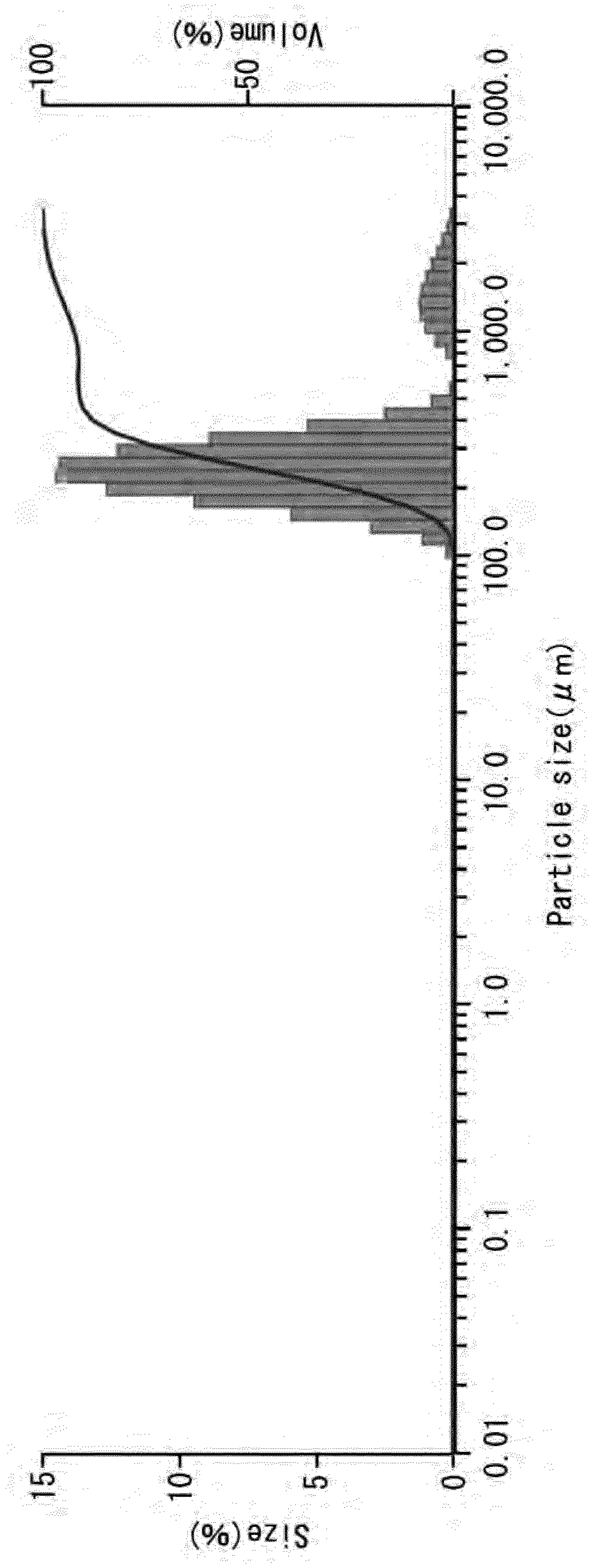
Volume (%)
100
50
Size (%)
15
10
5
0
0.01
0.1
1.0
10.0
100.0
1,000.0
10,000.0
Particle size (μm)

FIG. 60

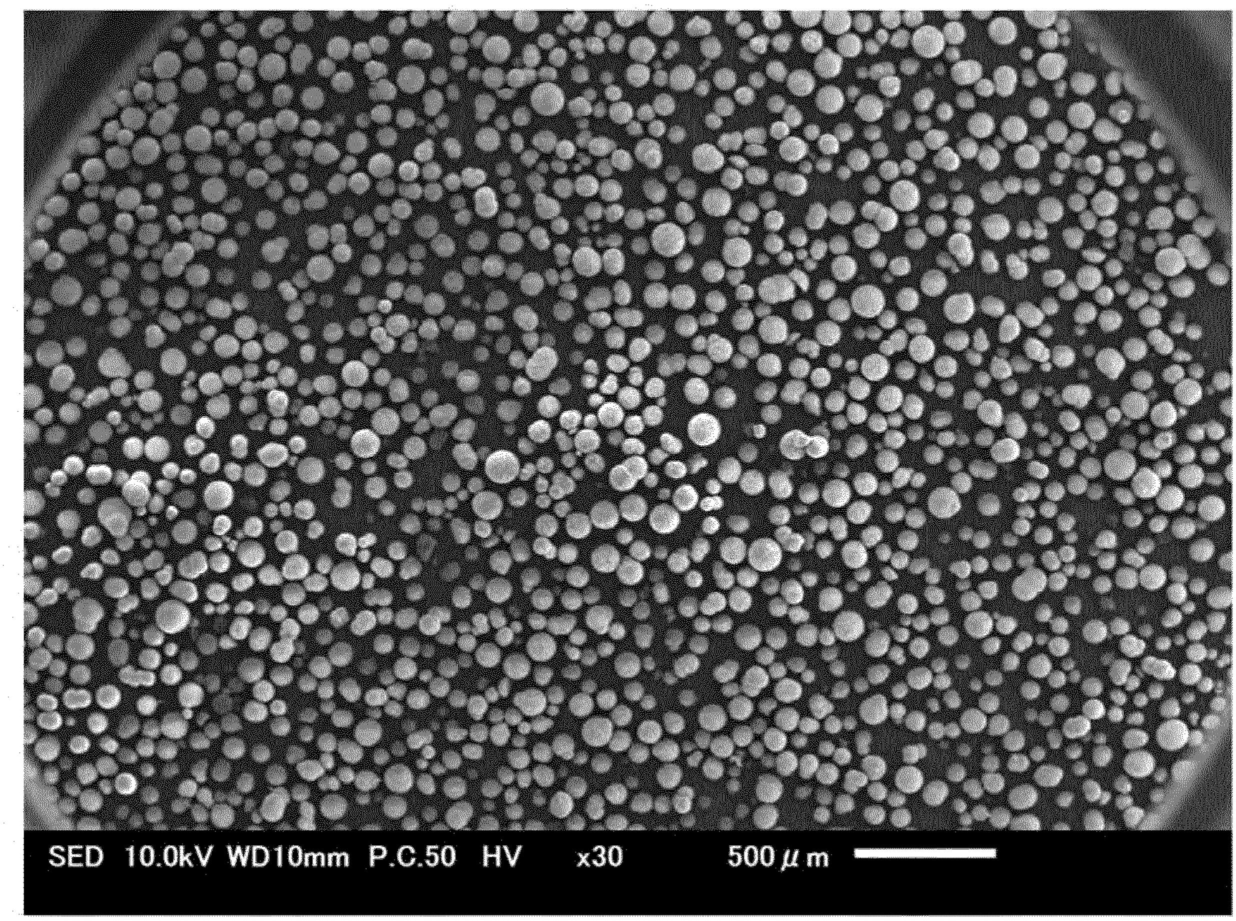

FIG. 61

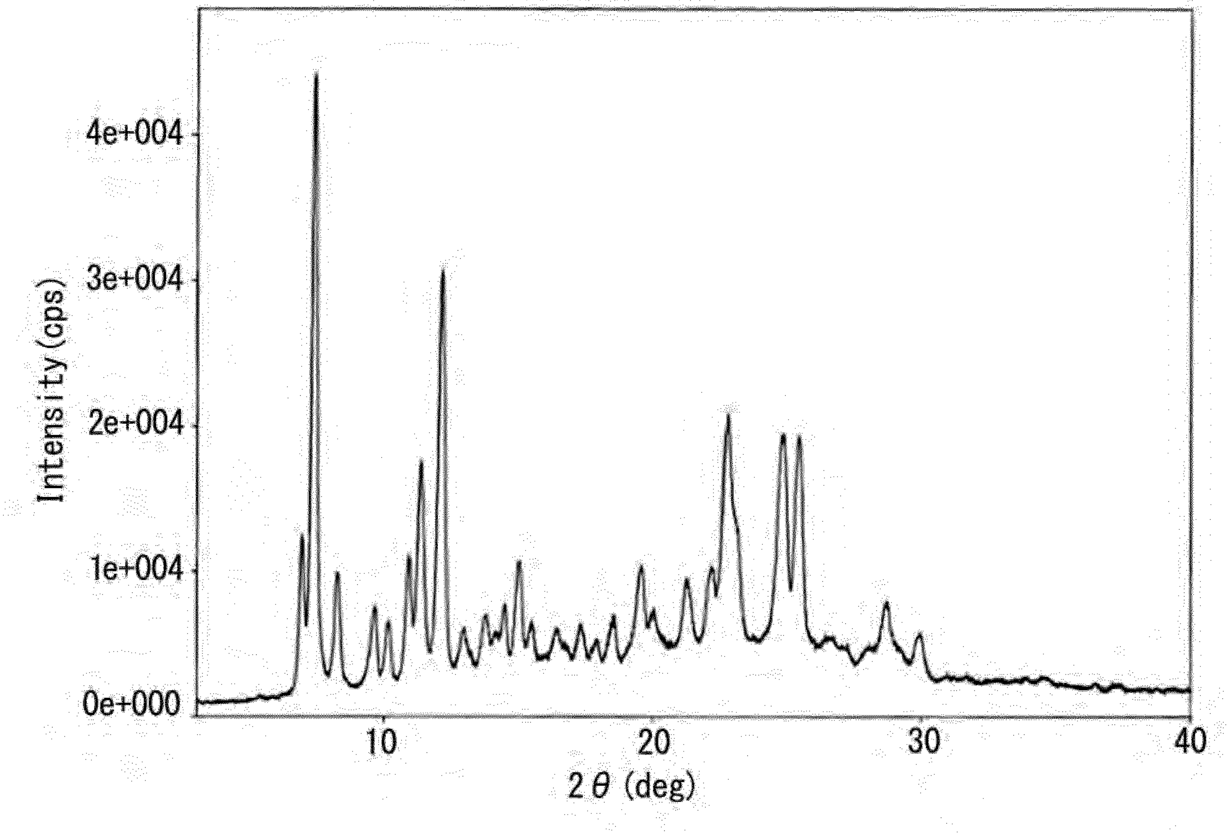
4e+004
3e+004
2e+004
1e+004
0e+000
Intensity(cps)
10
20
30
40
2θ (deg)

FIG. 62

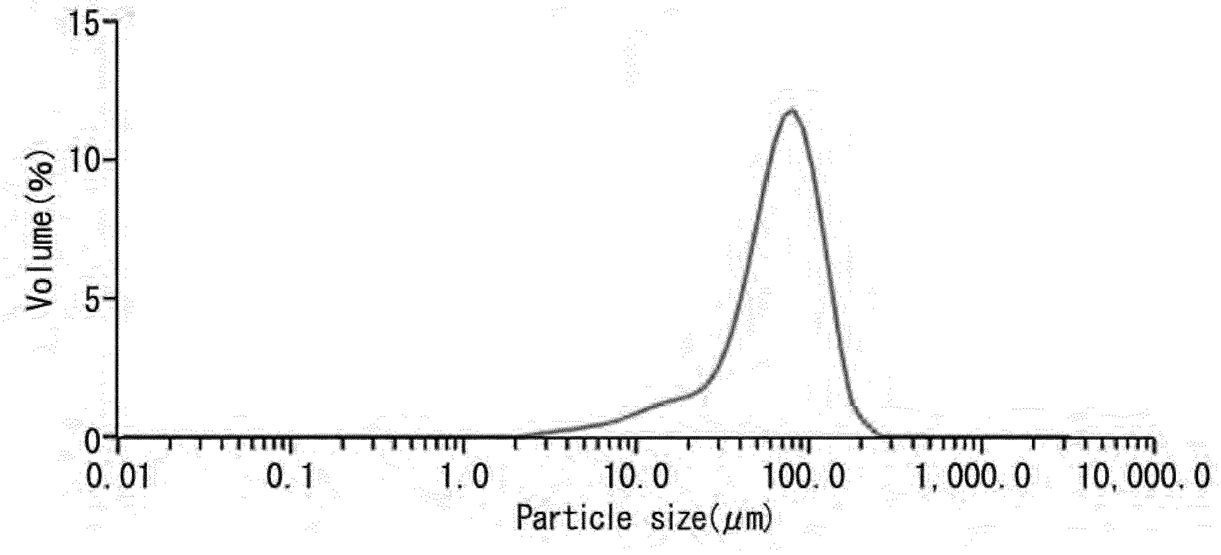
15
10
5
0
Volume(%)
0.01
0.1
1.0
10.0
100.0
1,000.0
10,000.0
Particle size(μm)

FIG. 63

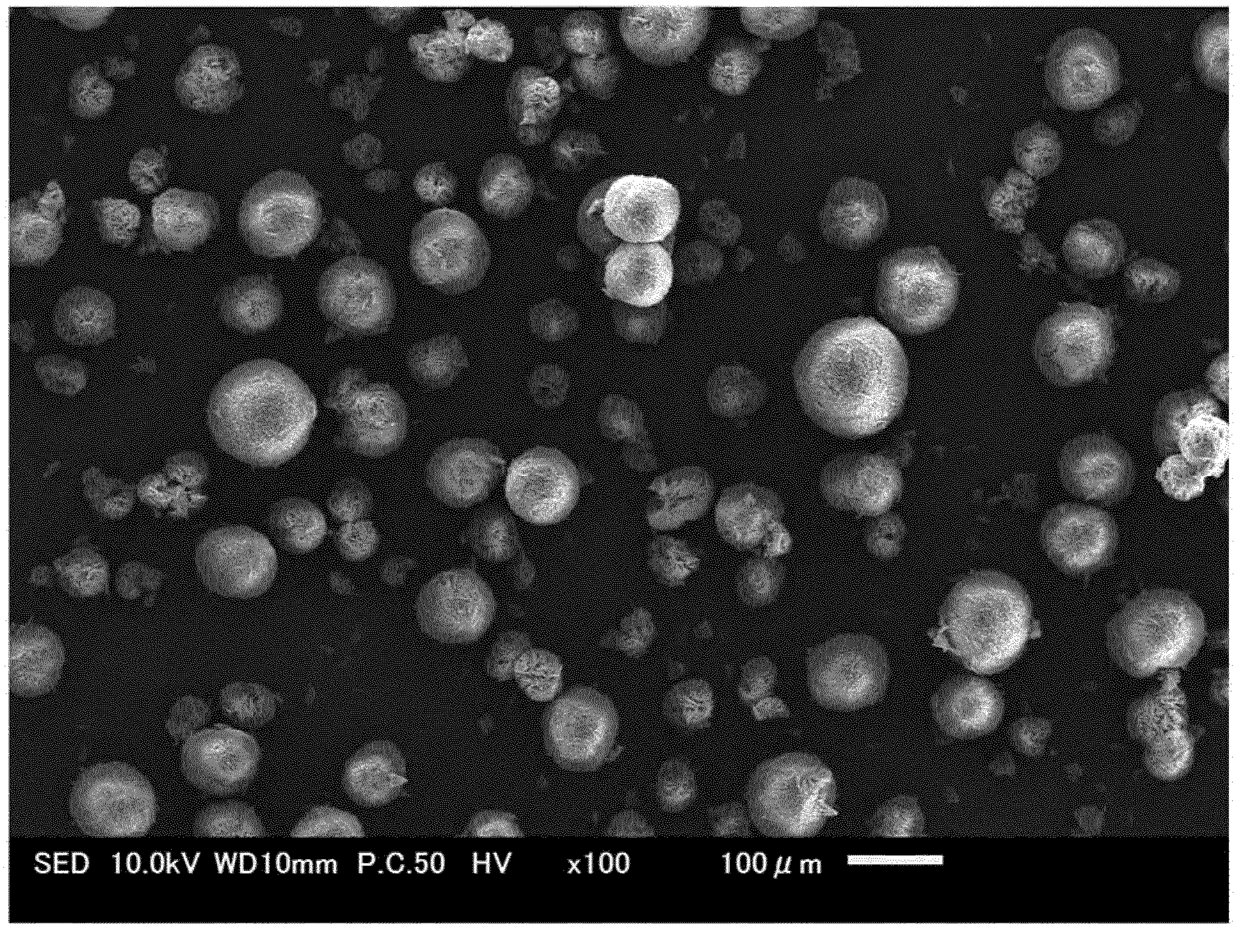

FIG. 64

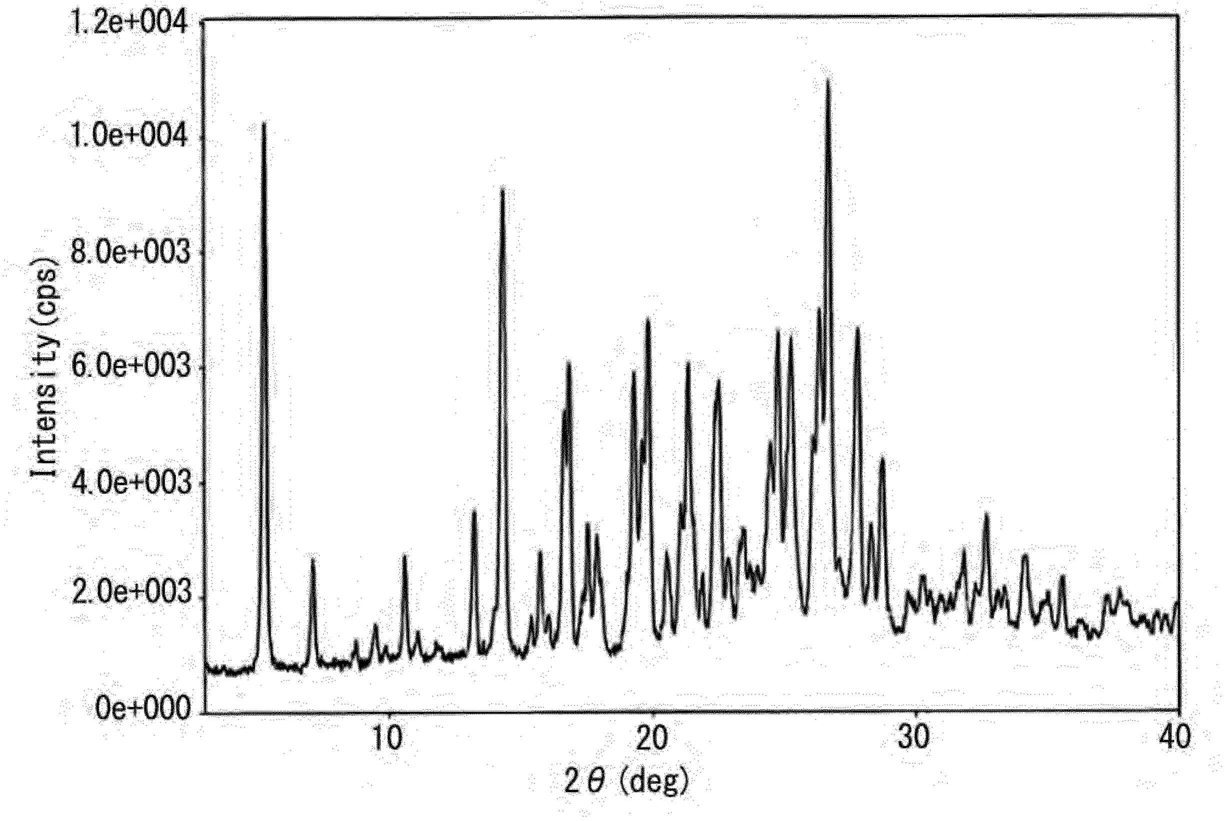
1.2e+004
1.0e+004
8.0e+003
6.0e+003
4.0e+003
2.0e+003
0e+000
Intensity (cps)
10
20
30
40
2θ (deg)

FIG. 65

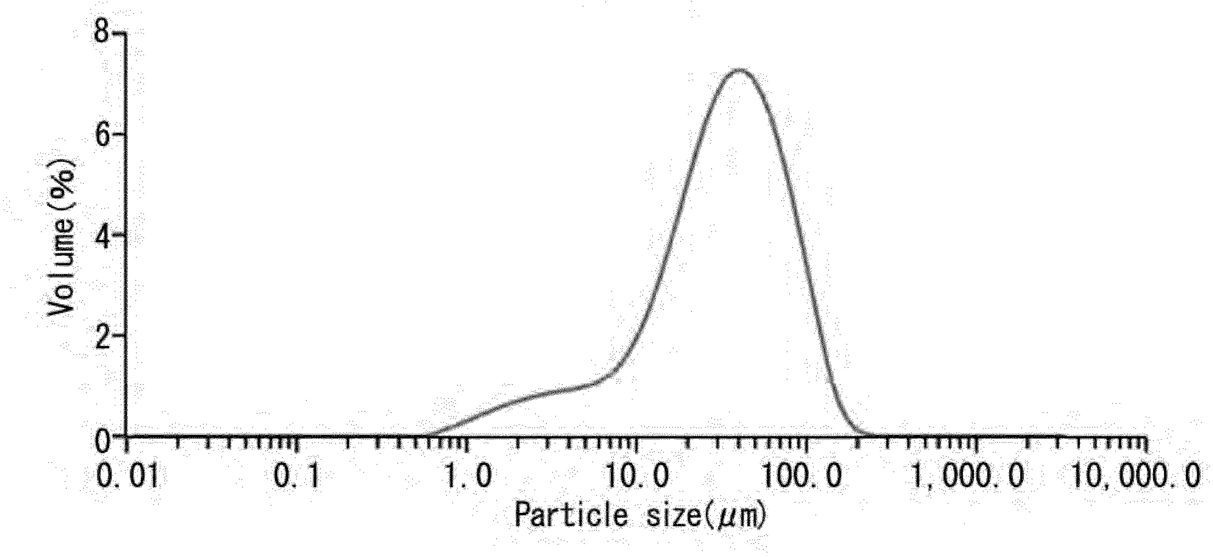
8
6
4
2
0
Volume (%)
0.01
0.1
1.0
10.0
100.0
1,000.0
10,000.0
Particle size(μm)

FIG. 66

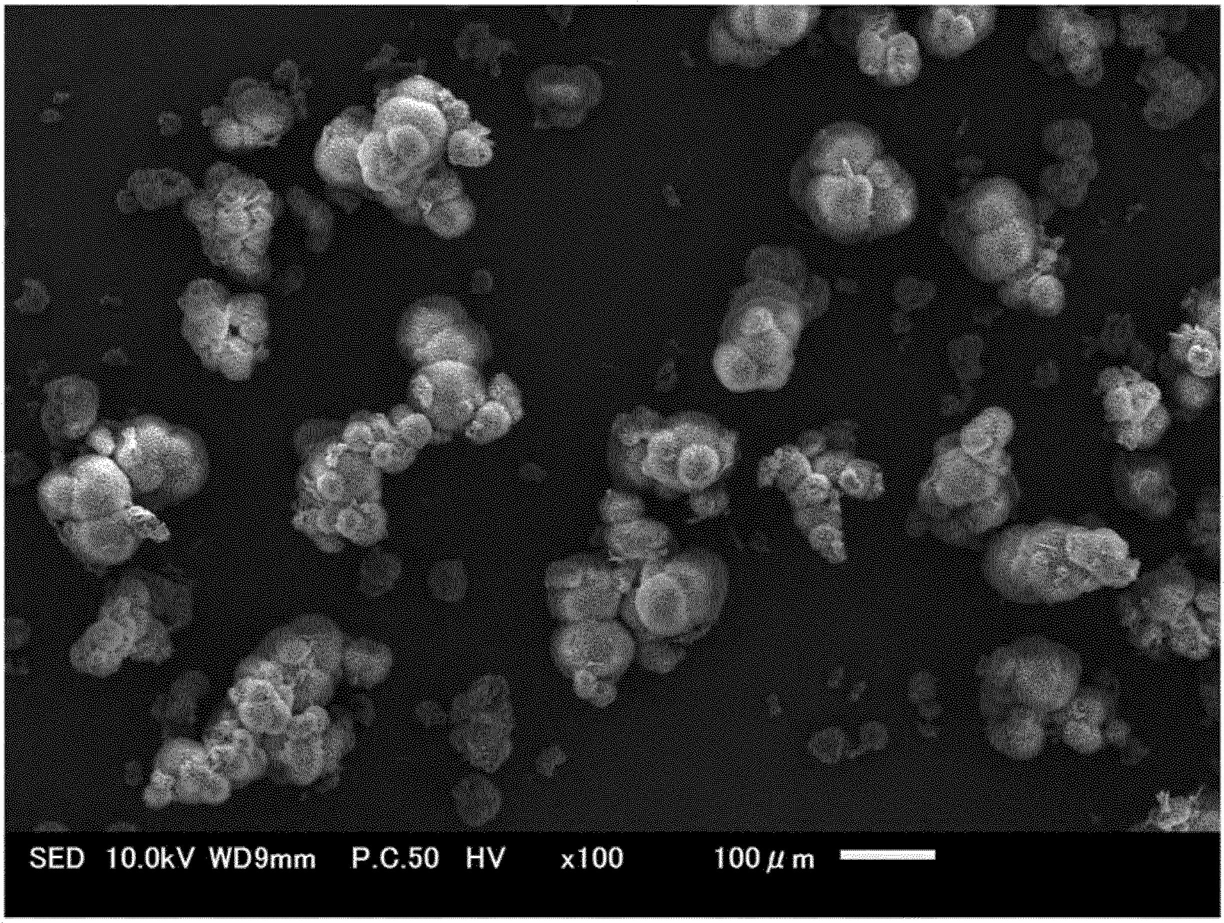

FIG. 67

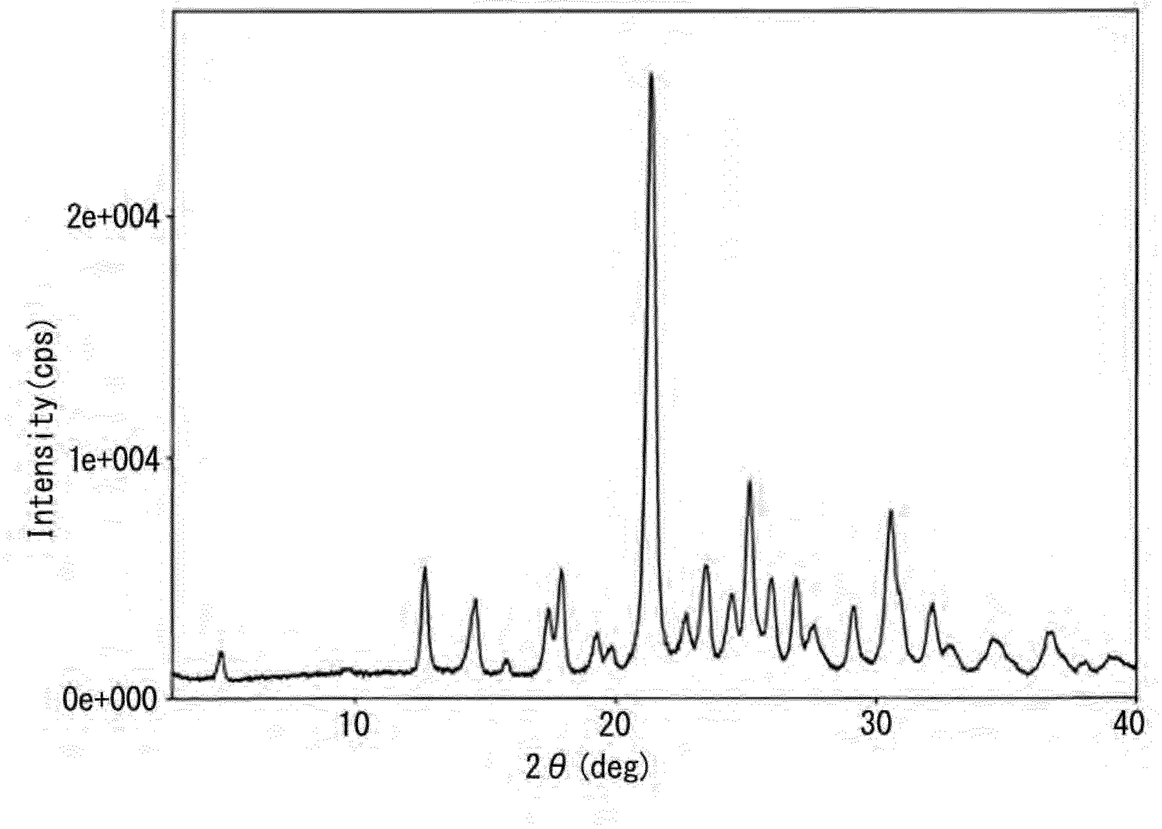
2e+004
1e+004
0e+000
Intensity (cps)
10
20
30
40
2θ (deg)

FIG. 68

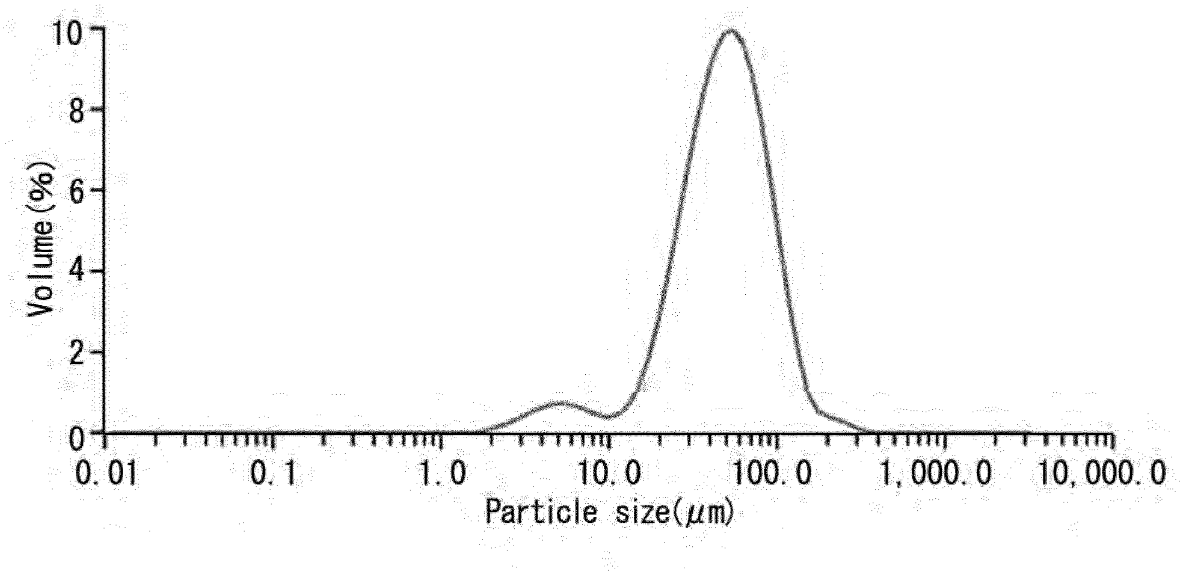
10
8
6
4
2
0
Volume (%)
0.01
0.1
1.0
10.0
100.0
1,000.0
10,000.0
Particle size(μm)

FIG. 69

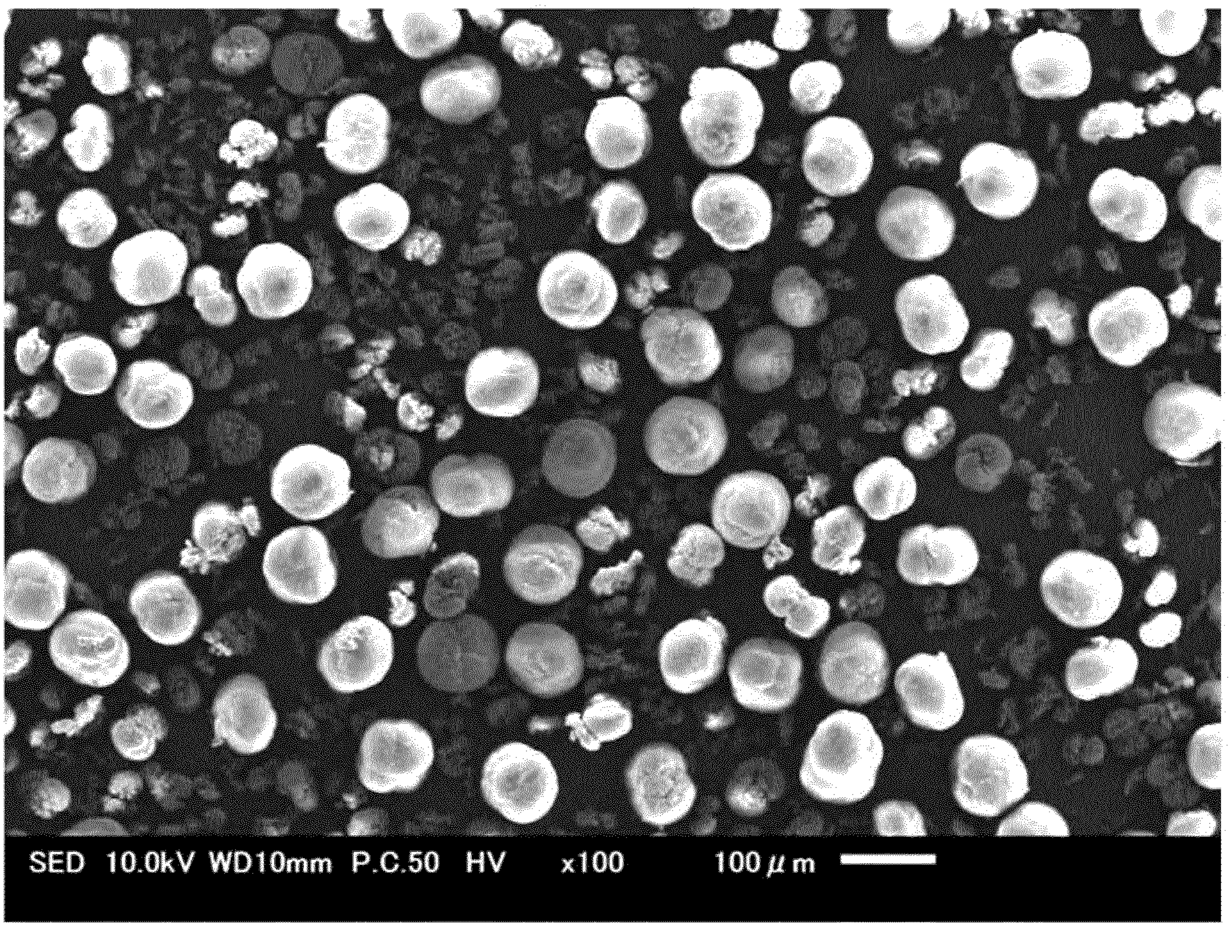

FIG. 70

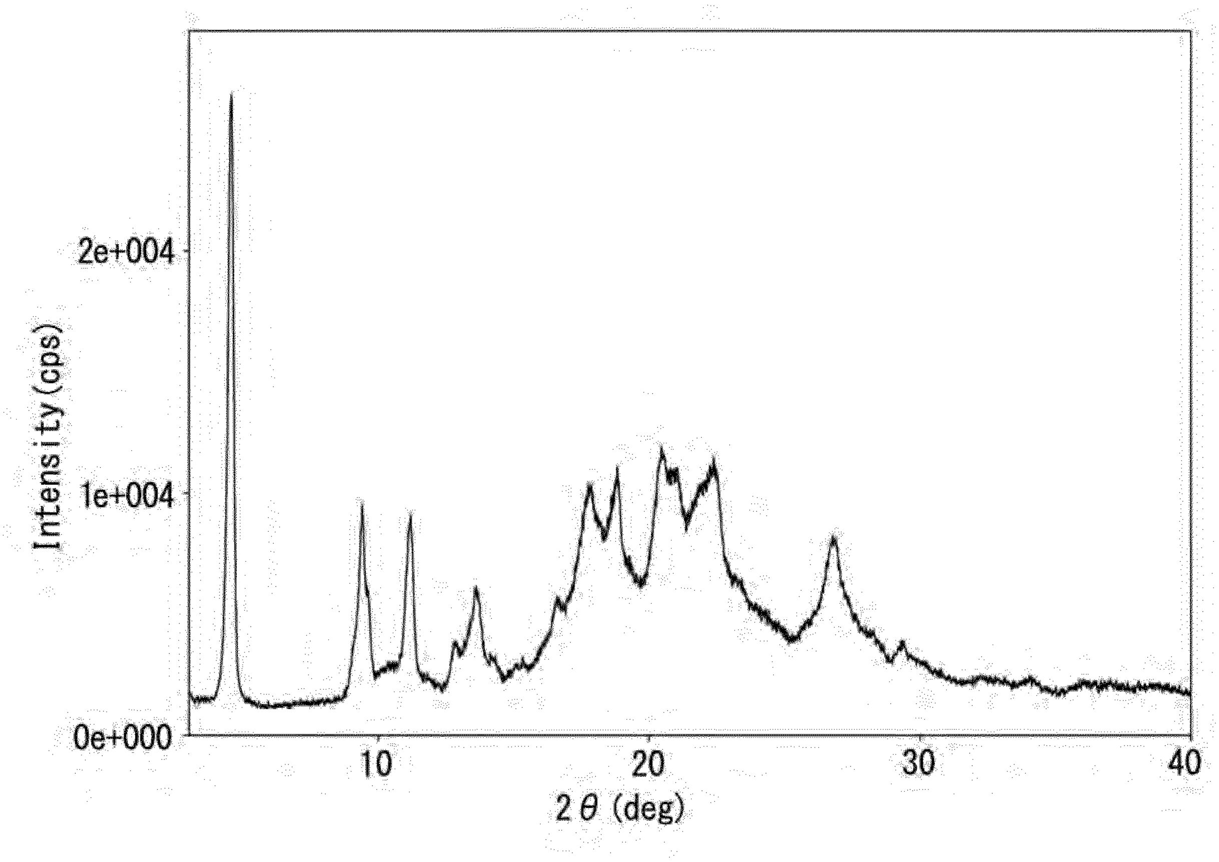

FIG. 71

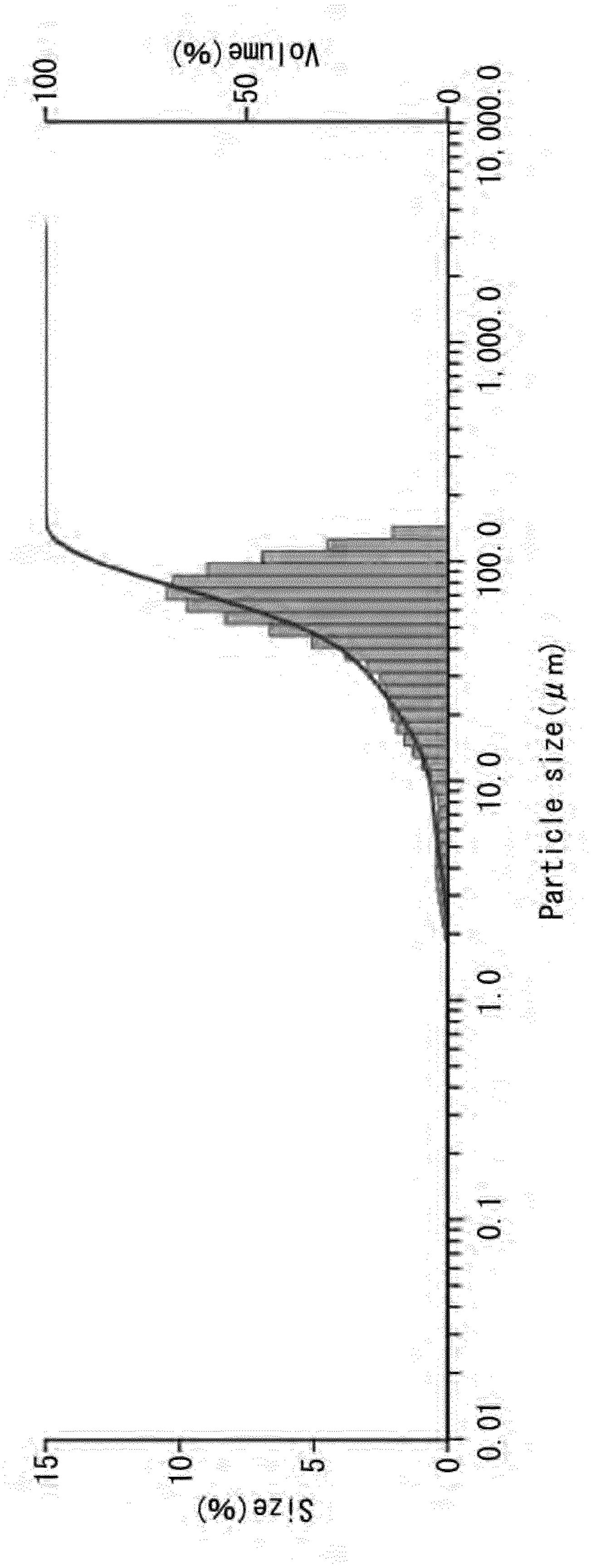
Volume (%)
100
50
0
Size (%)
15
10
5
0
0.01
0.1
1.0
10.0
100.0
1,000.0
10,000.0
Particle size (μm)

FIG. 72

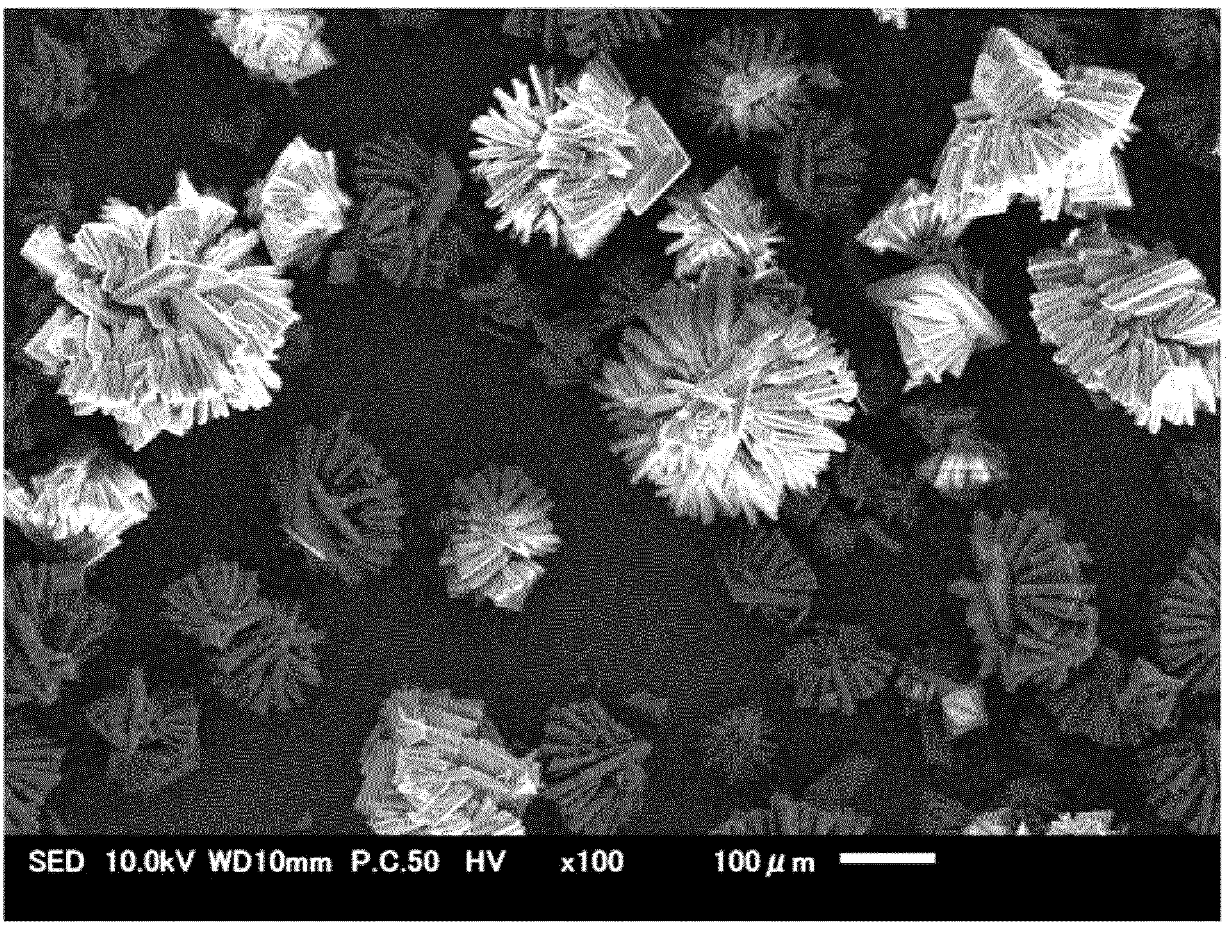

FIG. 73

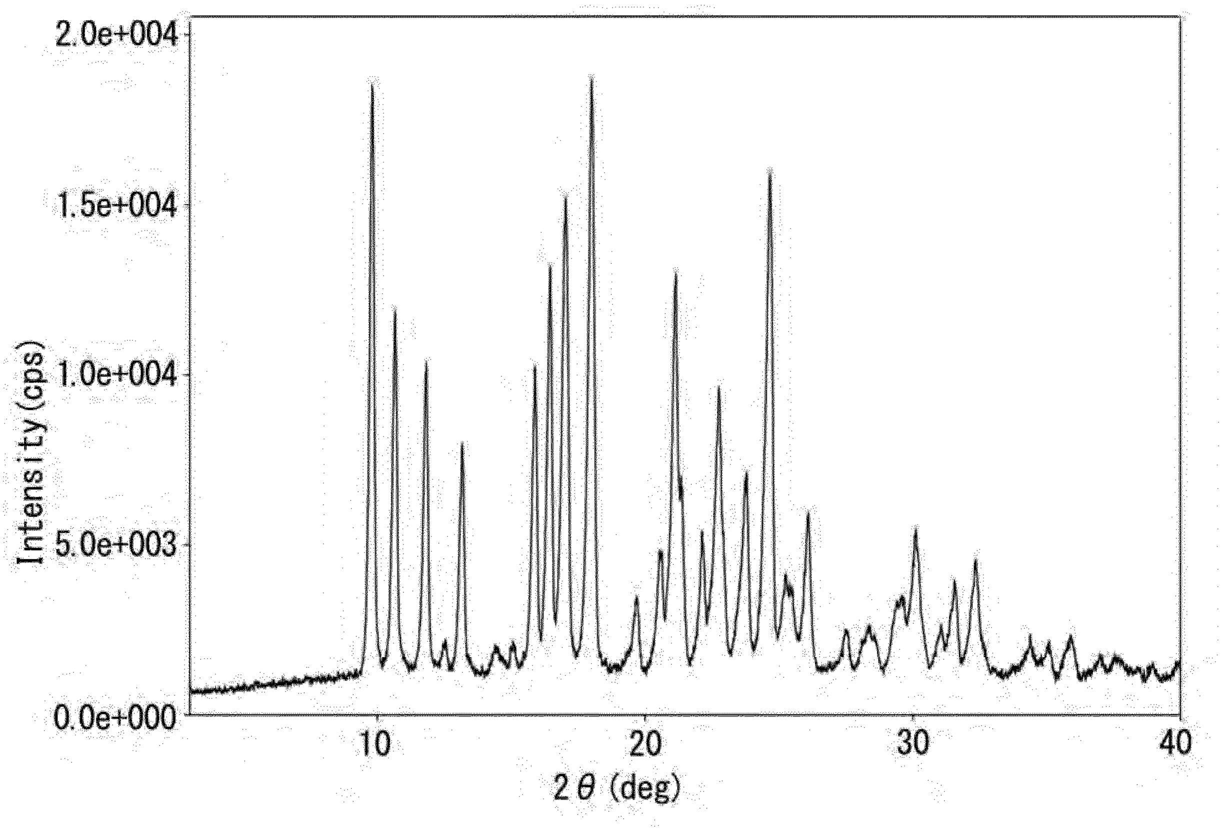
2.0e+004
1.5e+004
1.0e+004
5.0e+003
0.0e+000
Intensity (cps)
10
20
30
40
2θ (deg)

FIG. 74

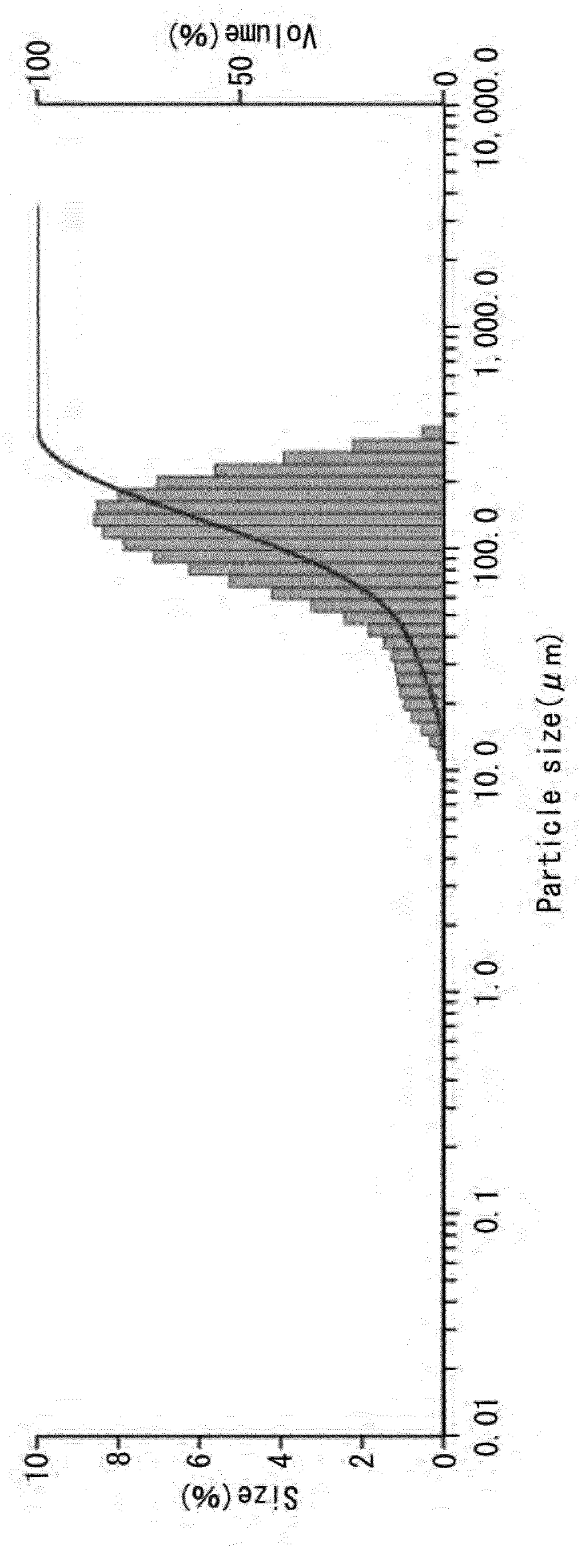
Size (%)
10
8
6
4
2
0
Volume (%)
100
50
0
0.01
0.1
1.0
10.0
100.0
1,000.0
10,000.0
Particle size (μm)

FIG. 75

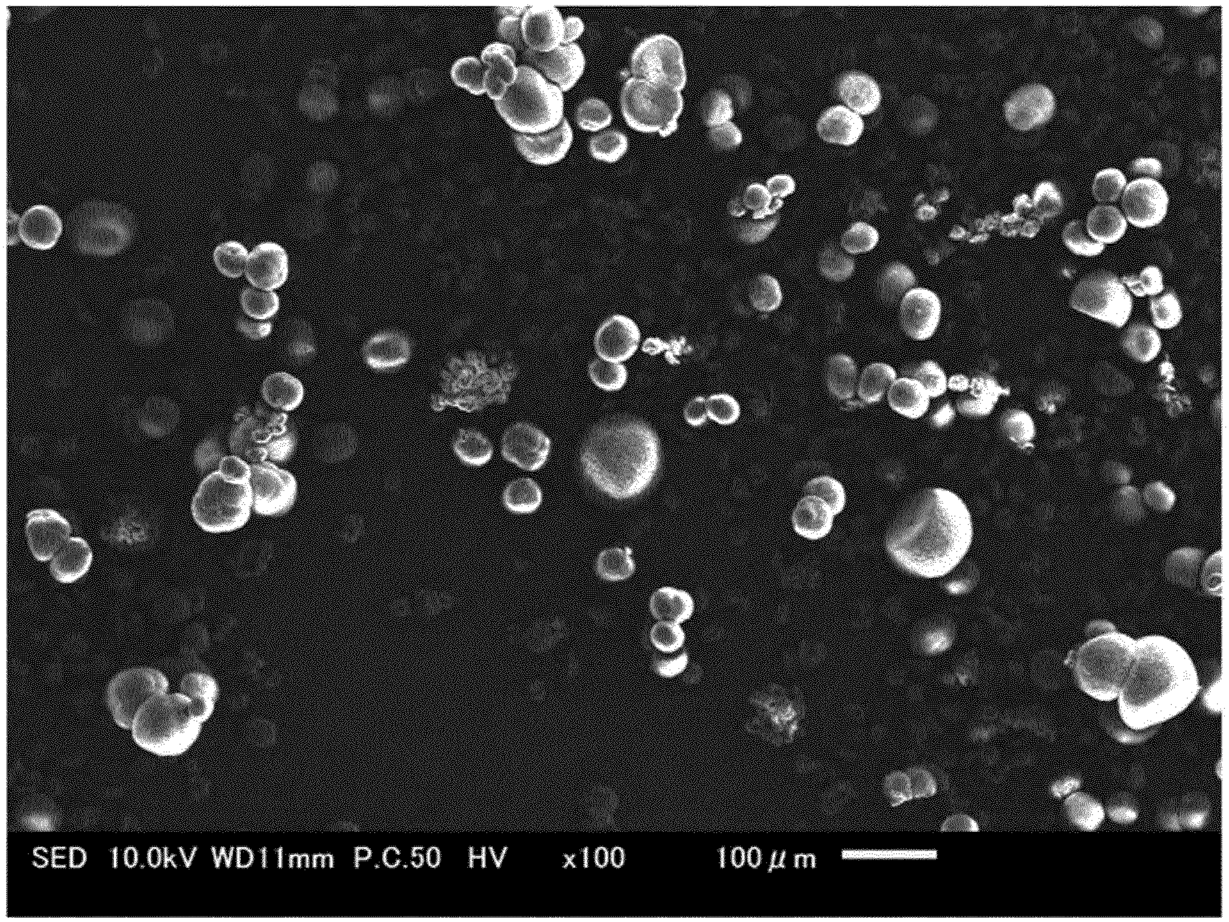

FIG. 76

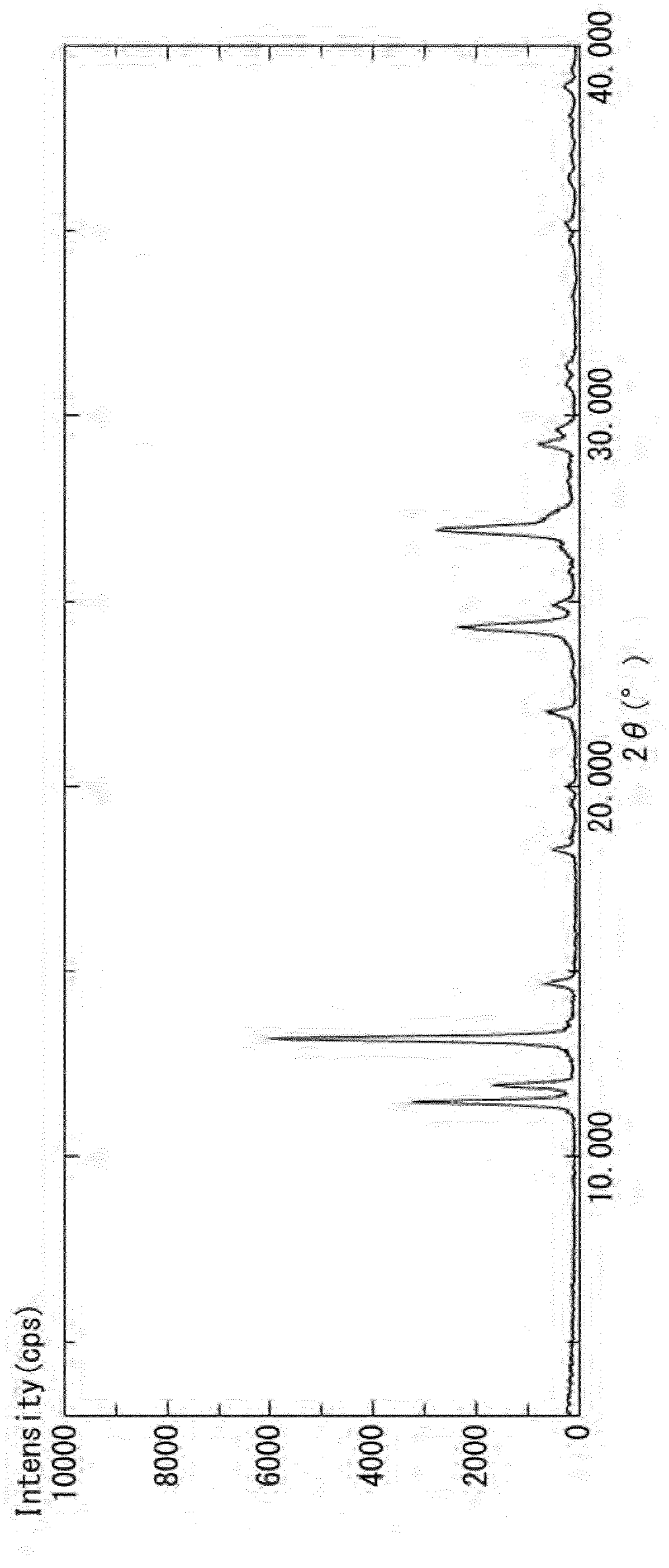
Intensity (cps)
10000
8000
6000
4000
2000
0
10.000
20.000
30.000
40.000
2θ (°)

FIG. 77

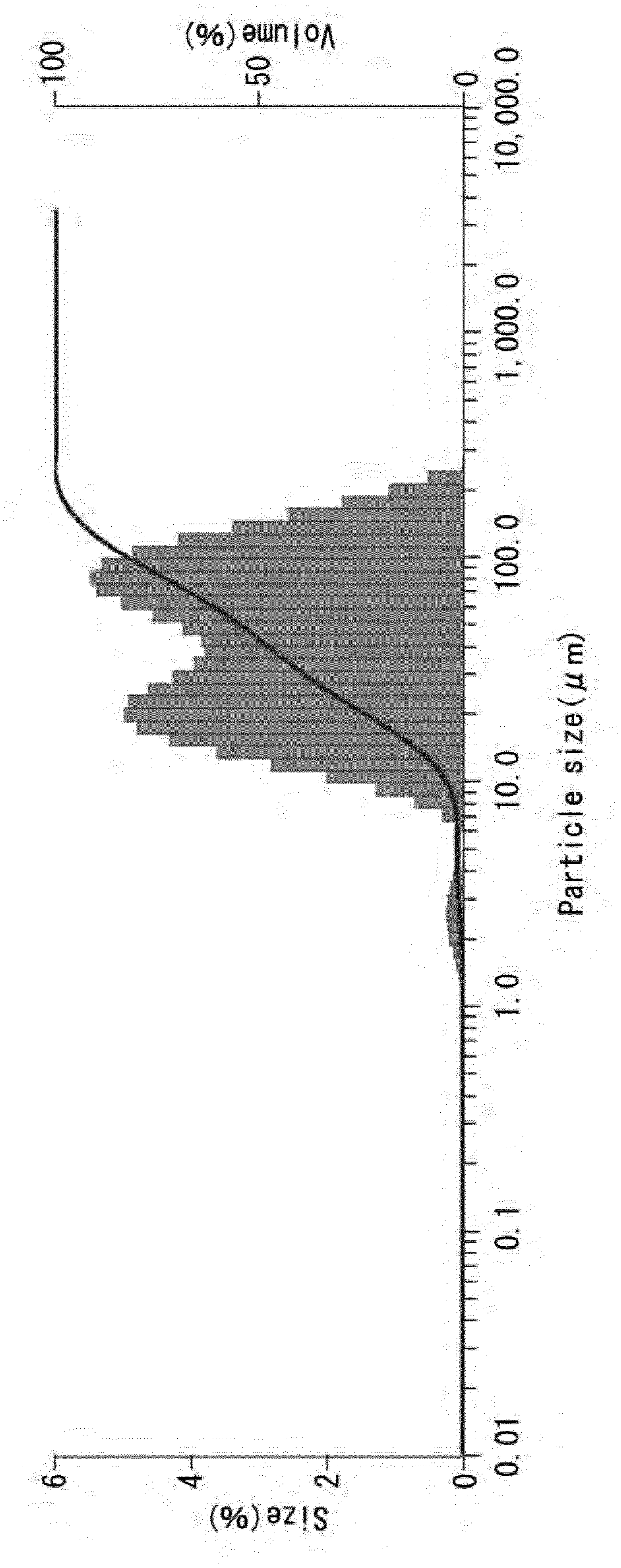
Volume (%)
100
50
0
Size (%)
6
4
2
0
0.01
0.1
1.0
10.0
100.0
1,000.0
10,000.0
Particle size (μm)

FIG. 78

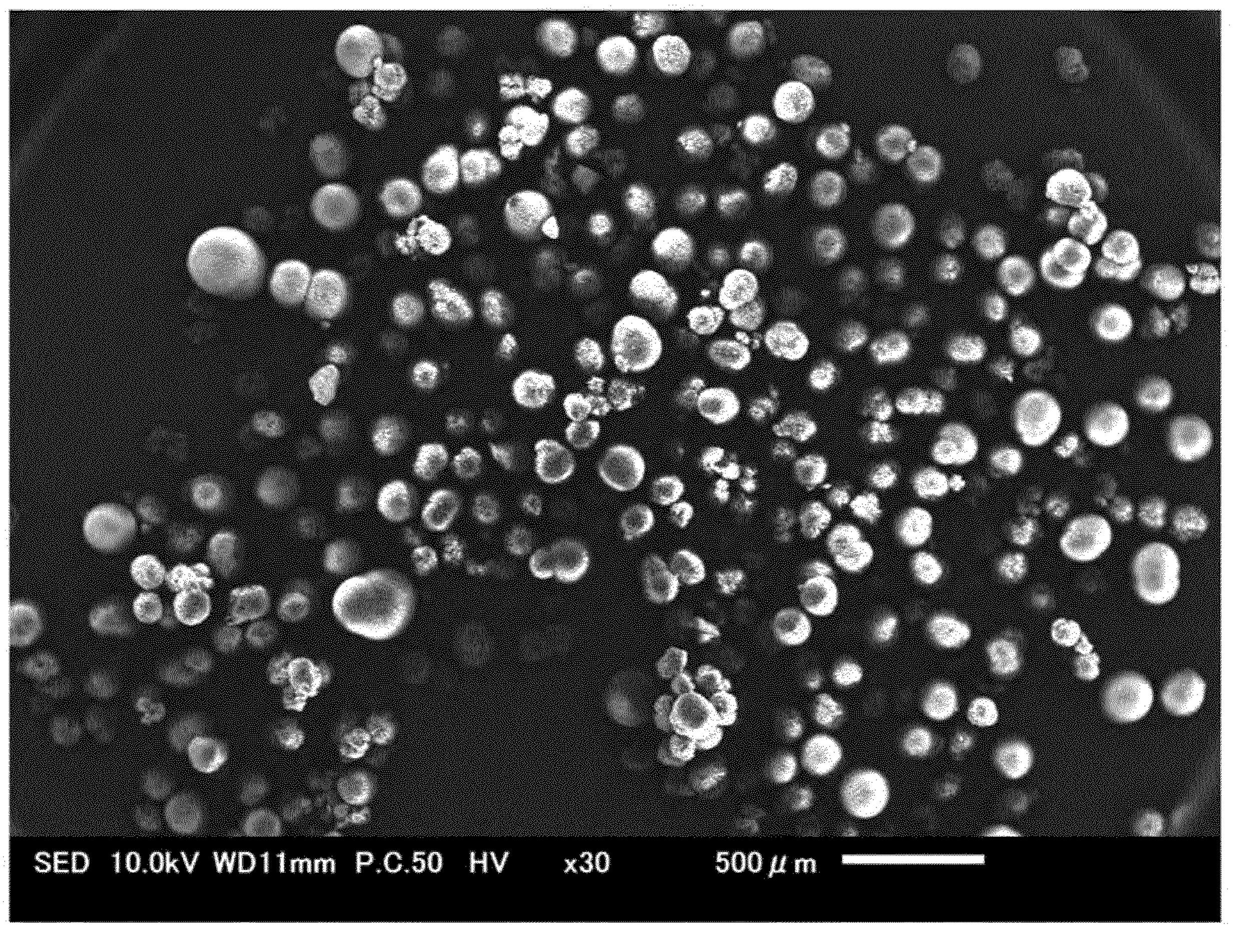

FIG. 79

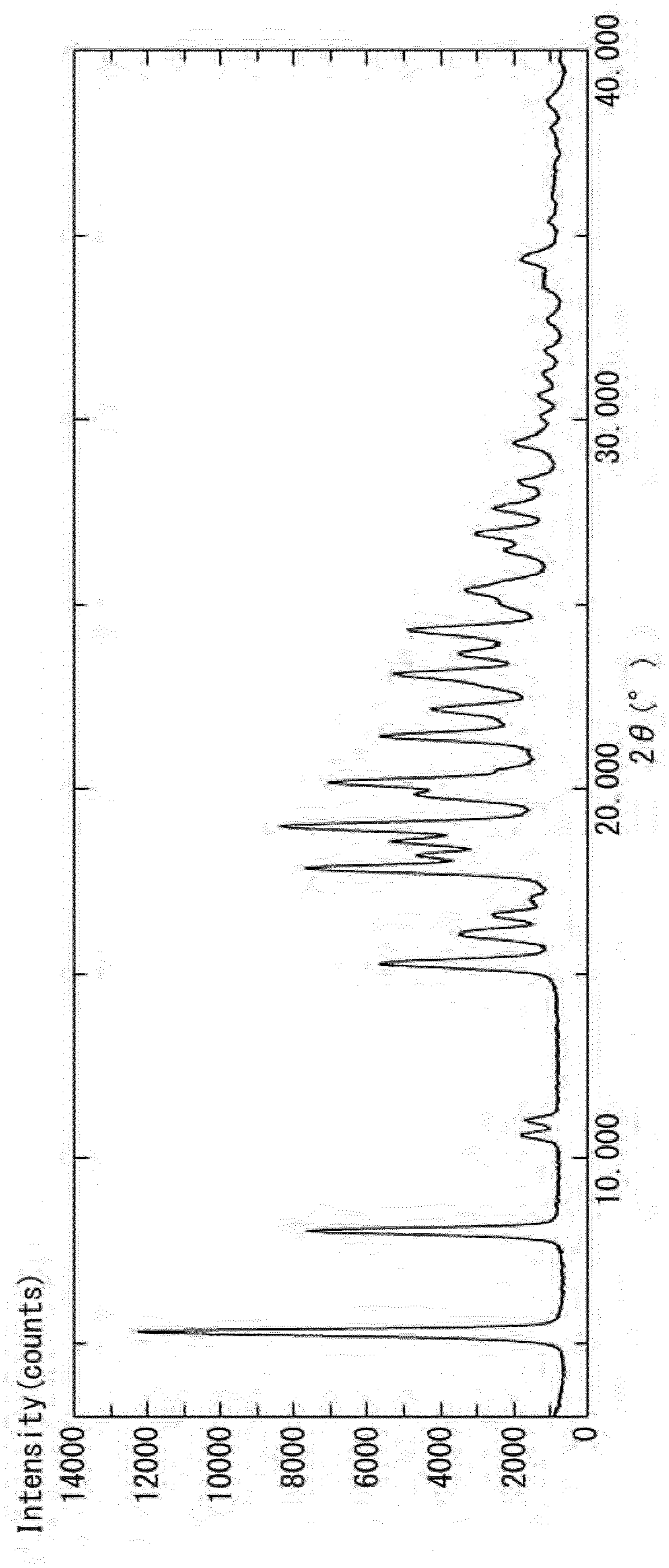
Intensity (counts)
14000
12000
10000
8000
6000
4000
2000
0
10.000
20.000
30.000
40.000
2θ (°)

FIG. 80

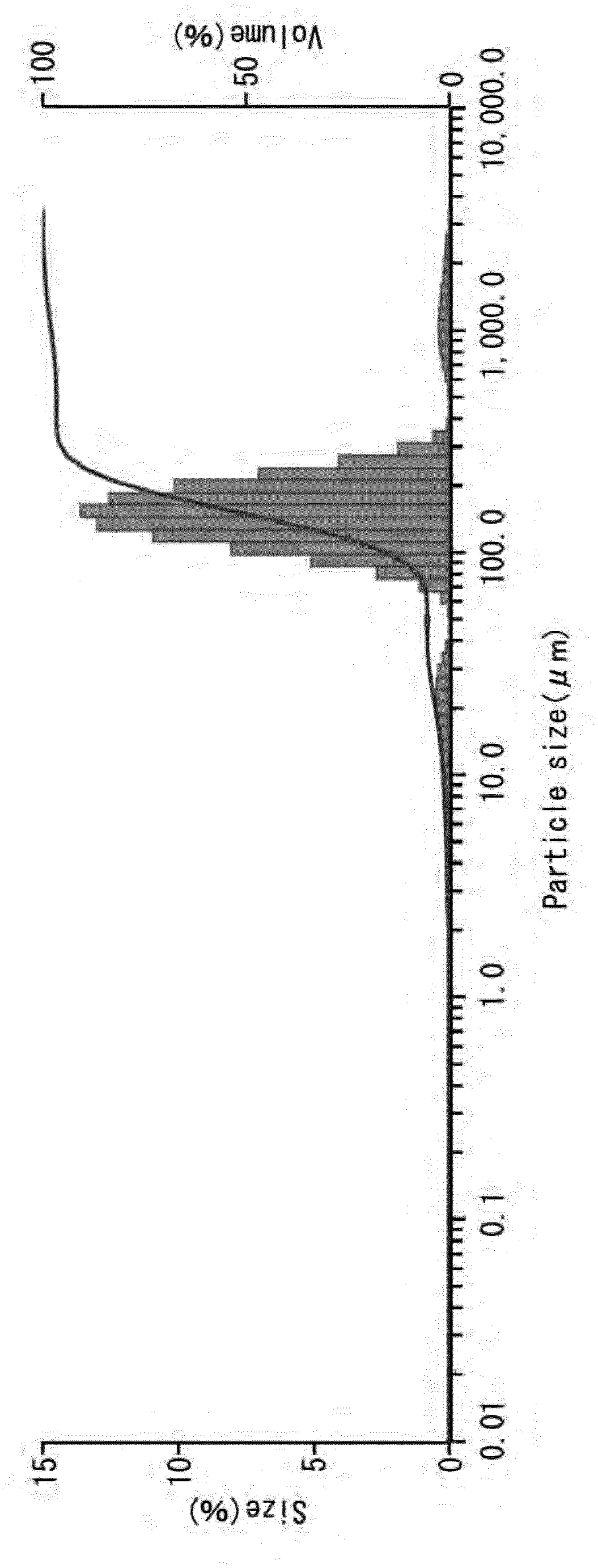
Volume (%)
100
50
0
Size (%)
15
10
5
0
0.01
0.1
1.0
10.0
100.0
1,000.0
10,000.0
Particle size (μm)

FIG. 81

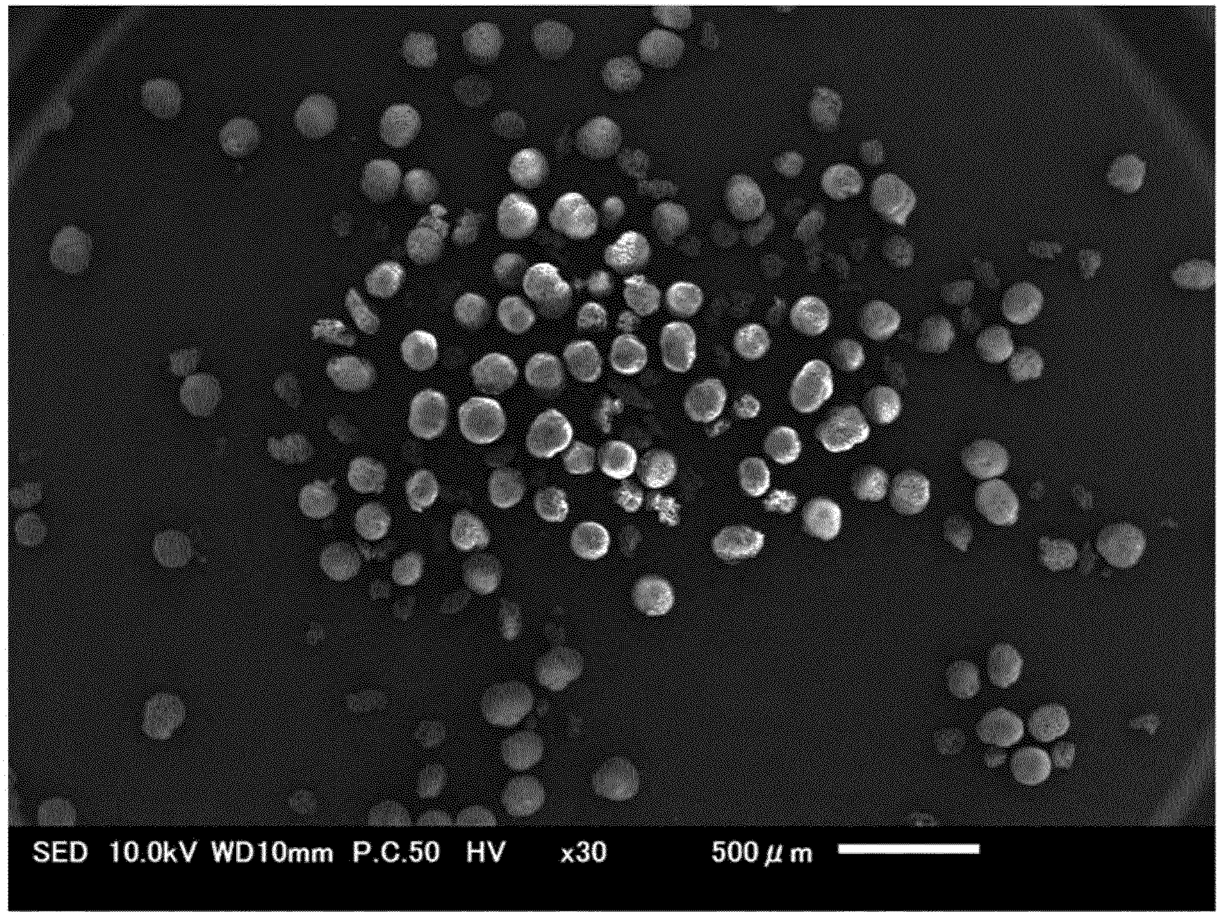

FIG. 82

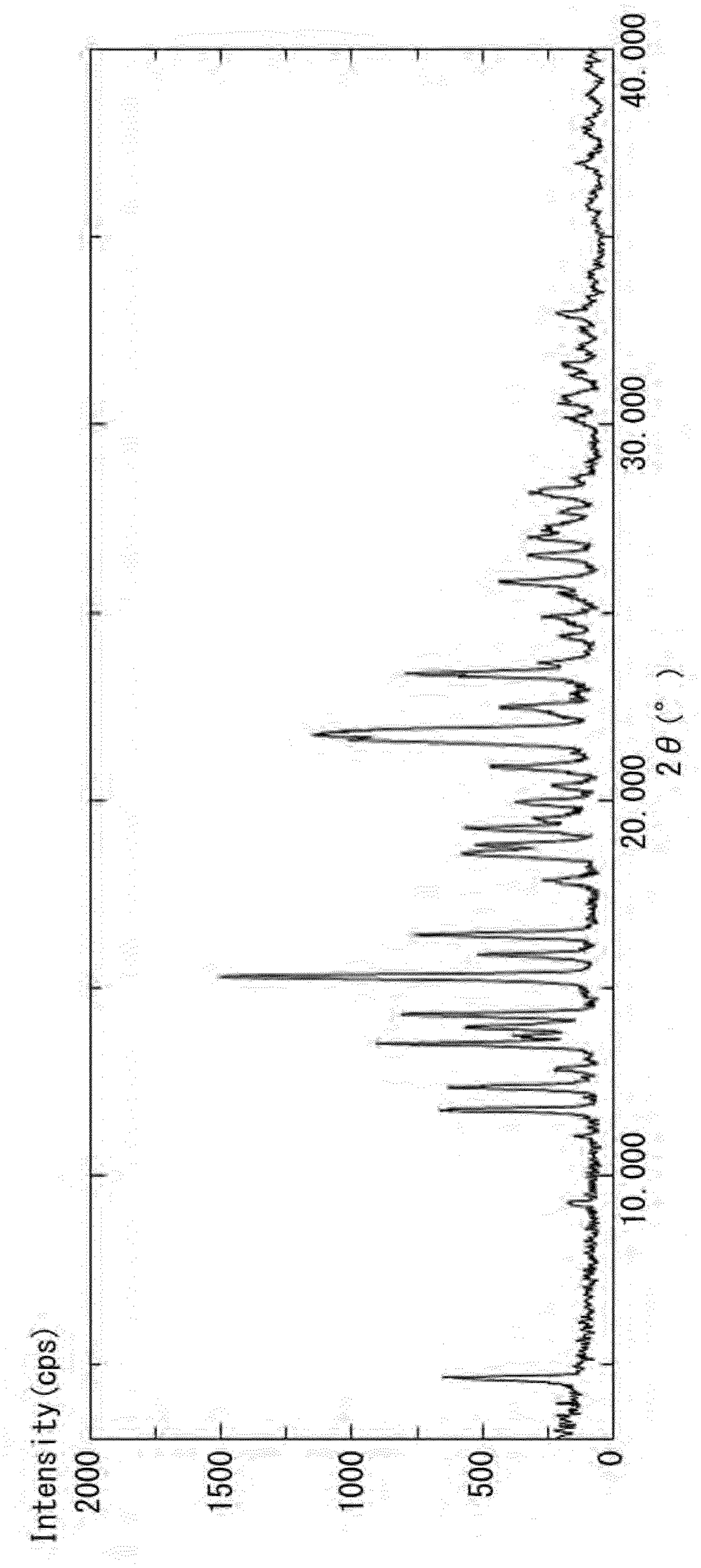
Intensity (cps)
2000
1500
1000
500
0
10.000
20.000
30.000
40.000
2θ (°)

FIG. 83

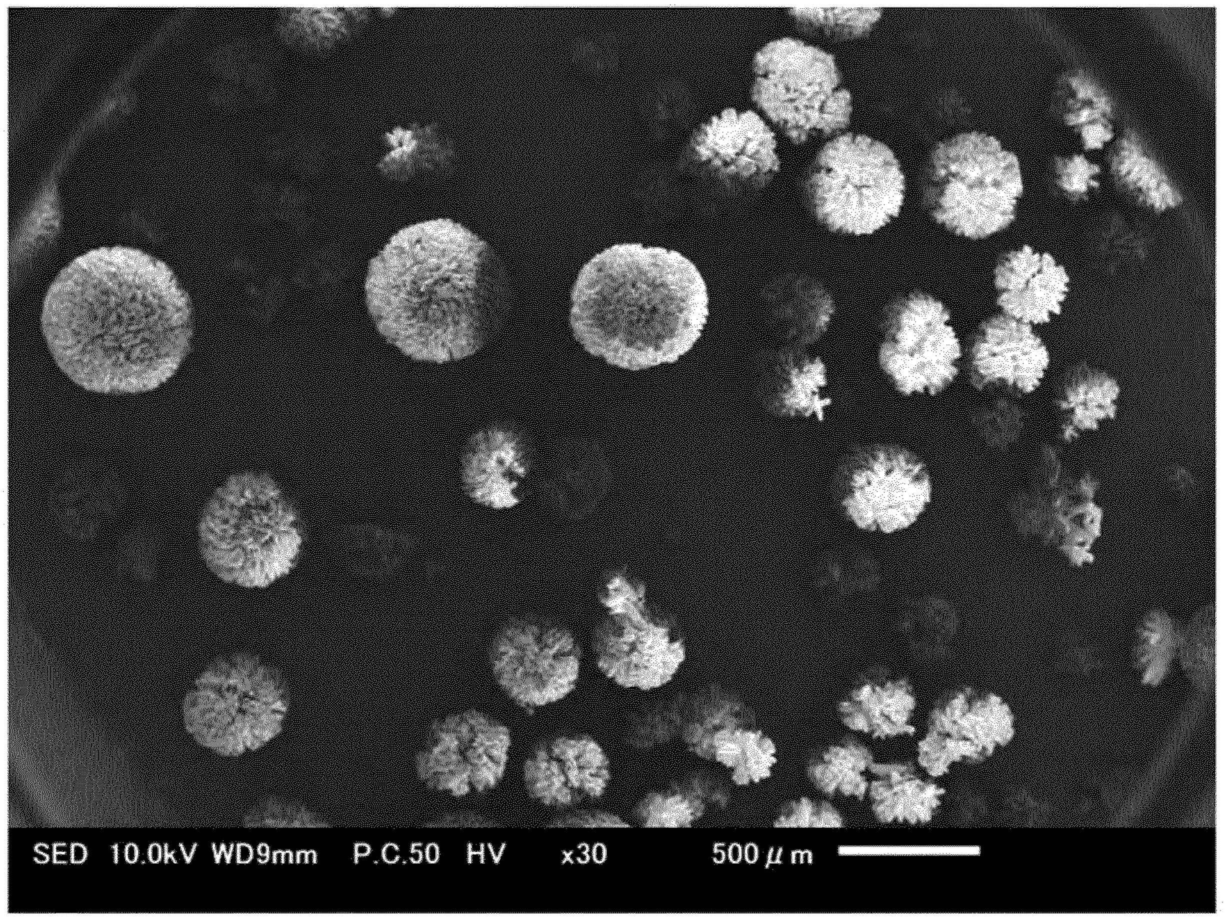

FIG. 84

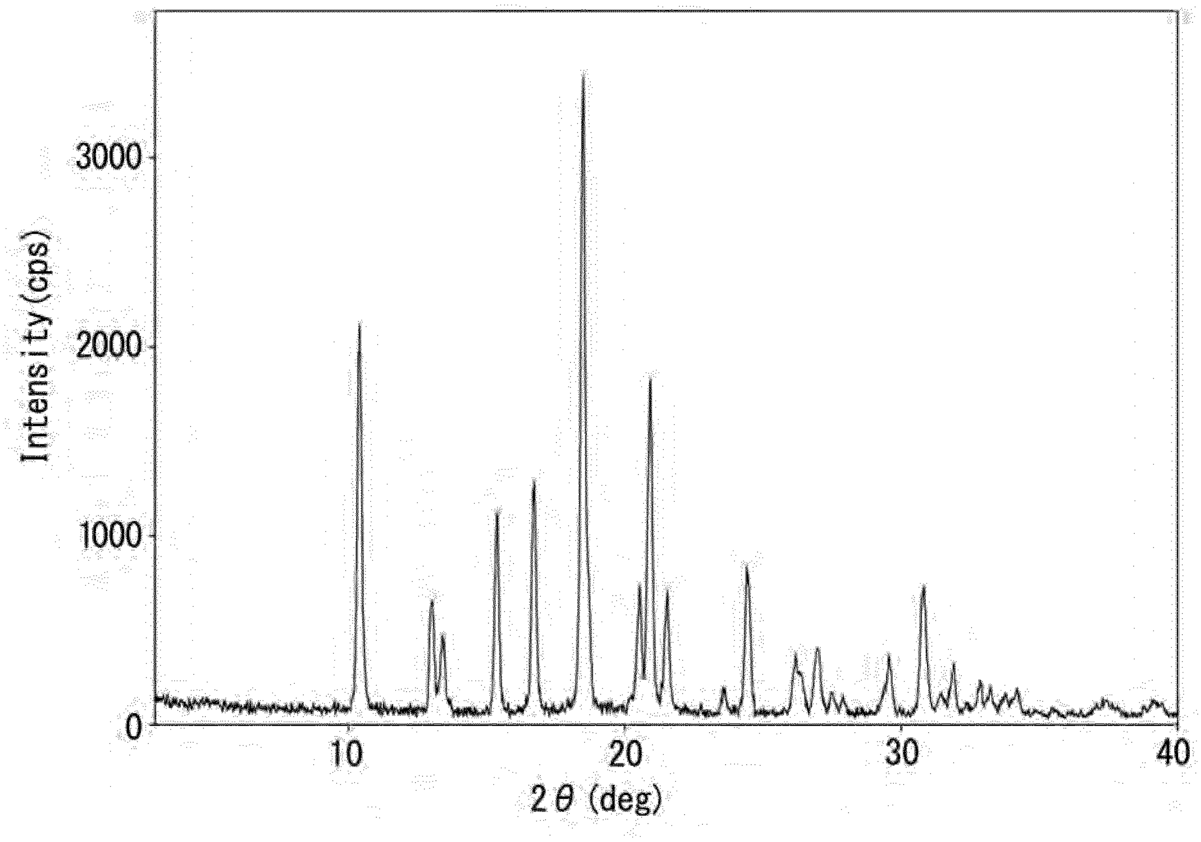

FIG. 85

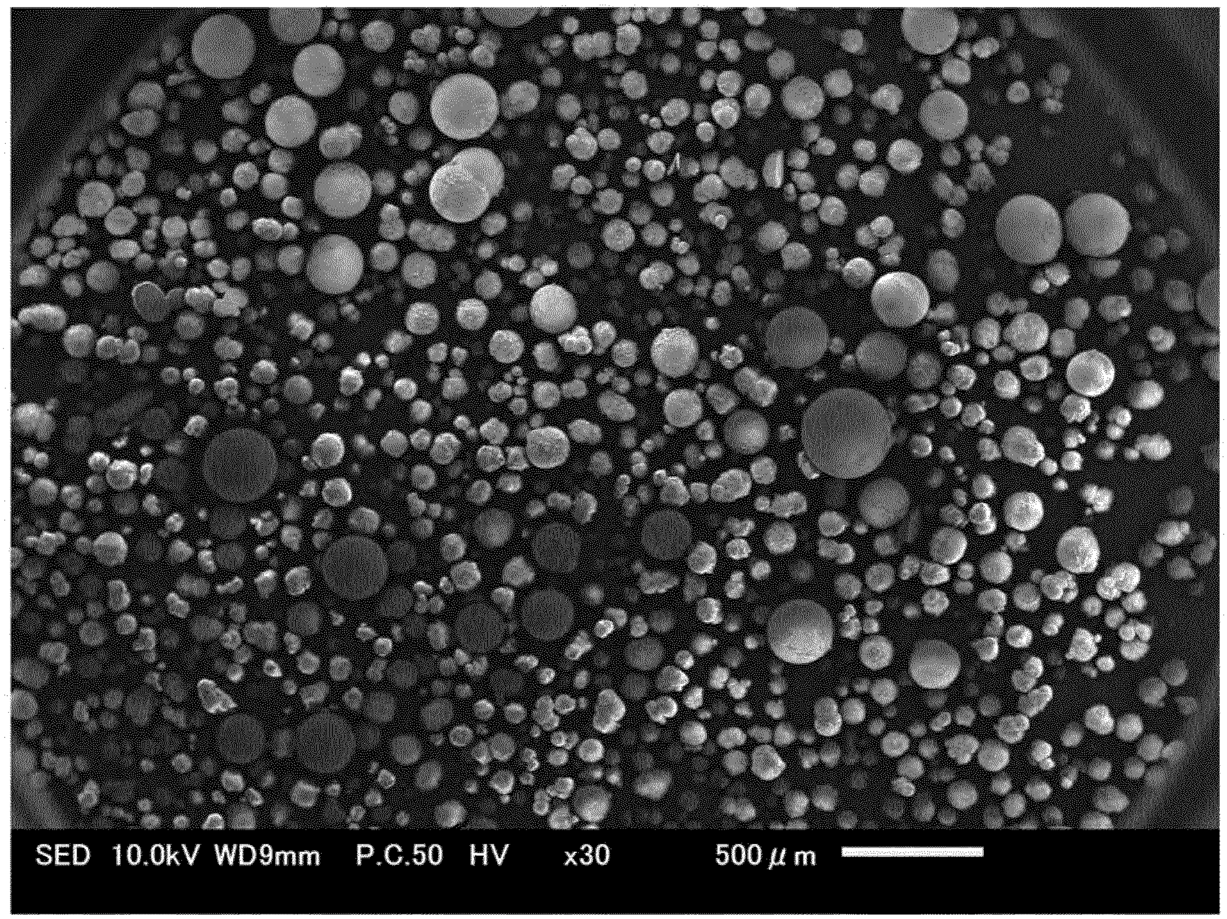

FIG. 86

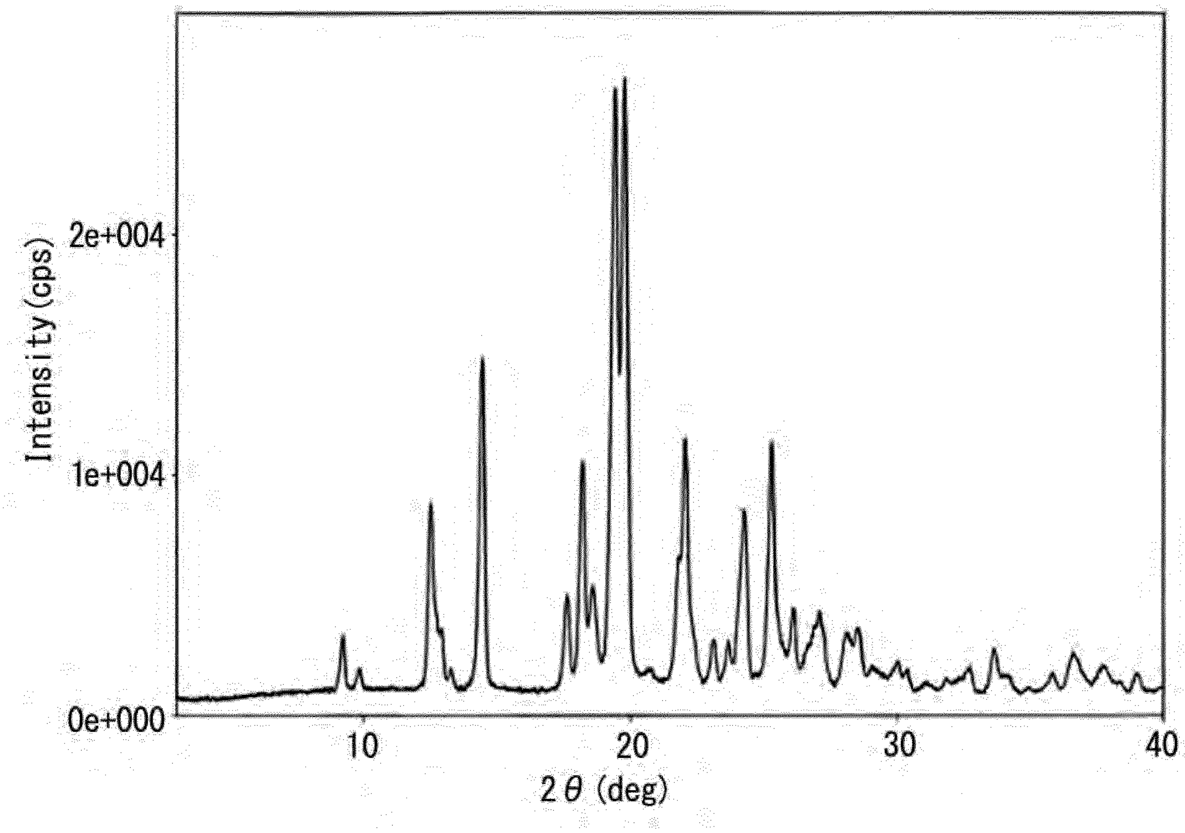
2e+004
1e+004
0e+000
Intensity (cps)
10
20
30
40
2θ (deg)

FIG. 87

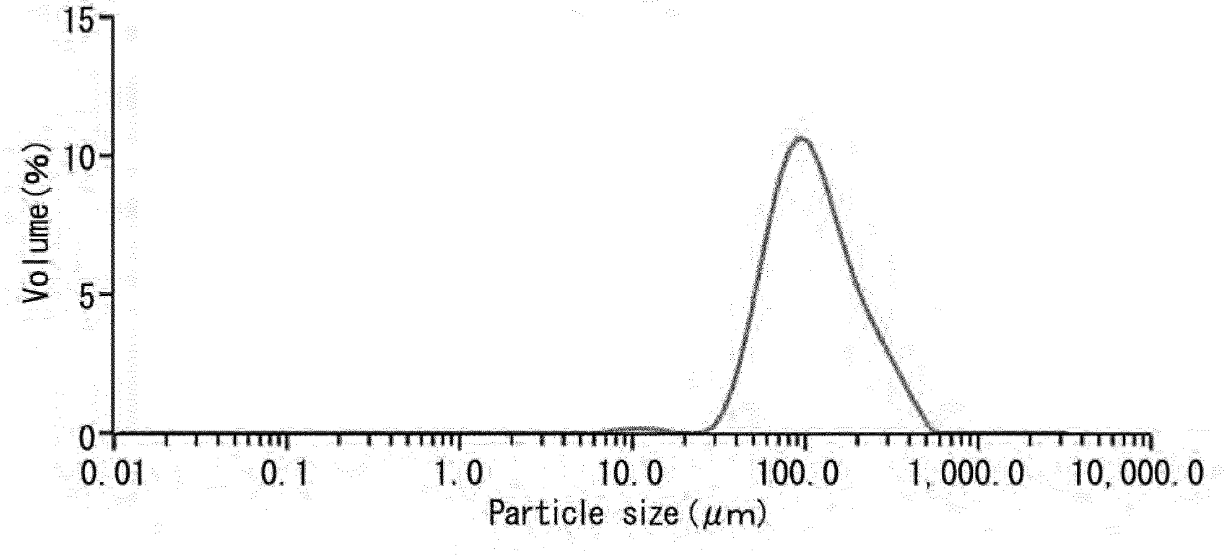
15
10
5
0
Volume (%)
0.01
0.1
1.0
10.0
100.0
1,000.0
10,000.0
Particle size (μm)

FIG. 88

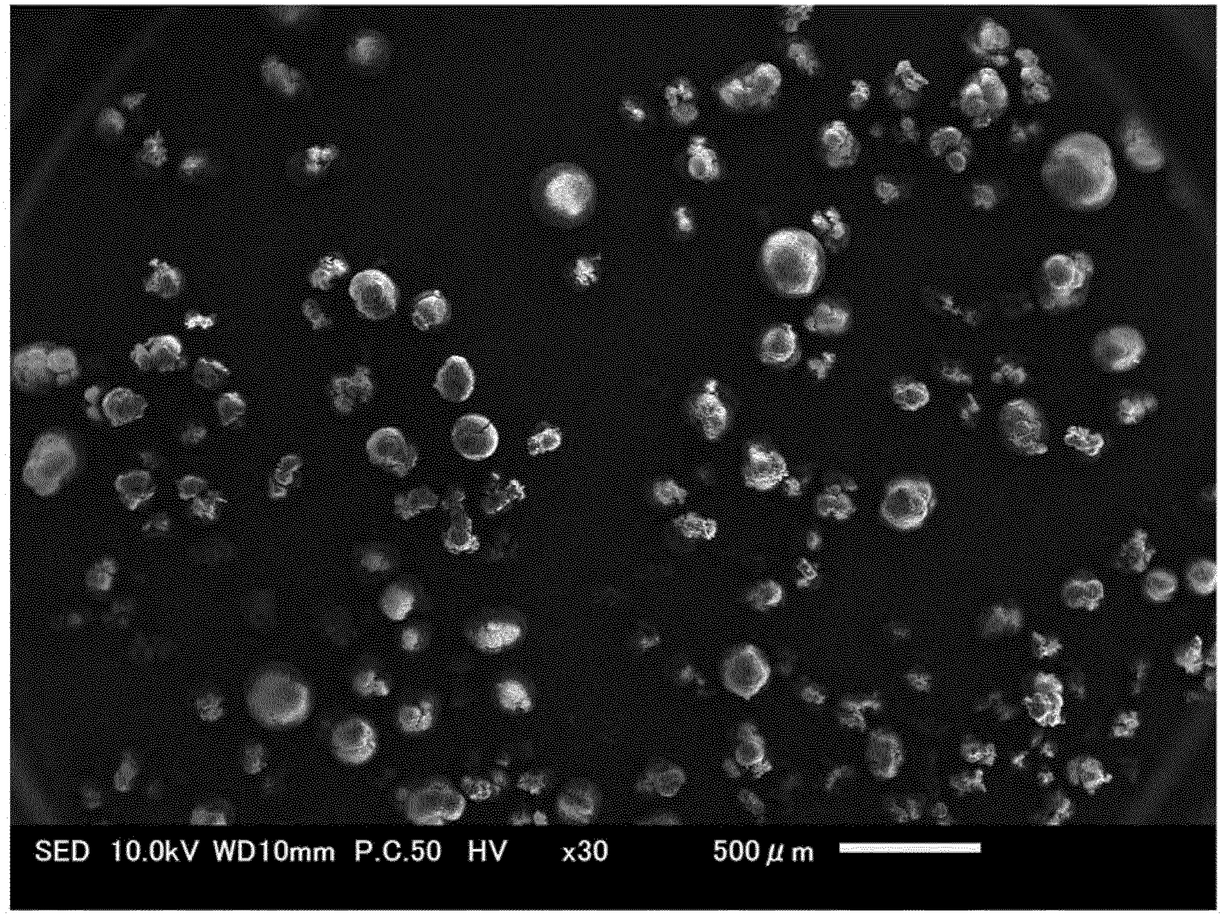

FIG. 89

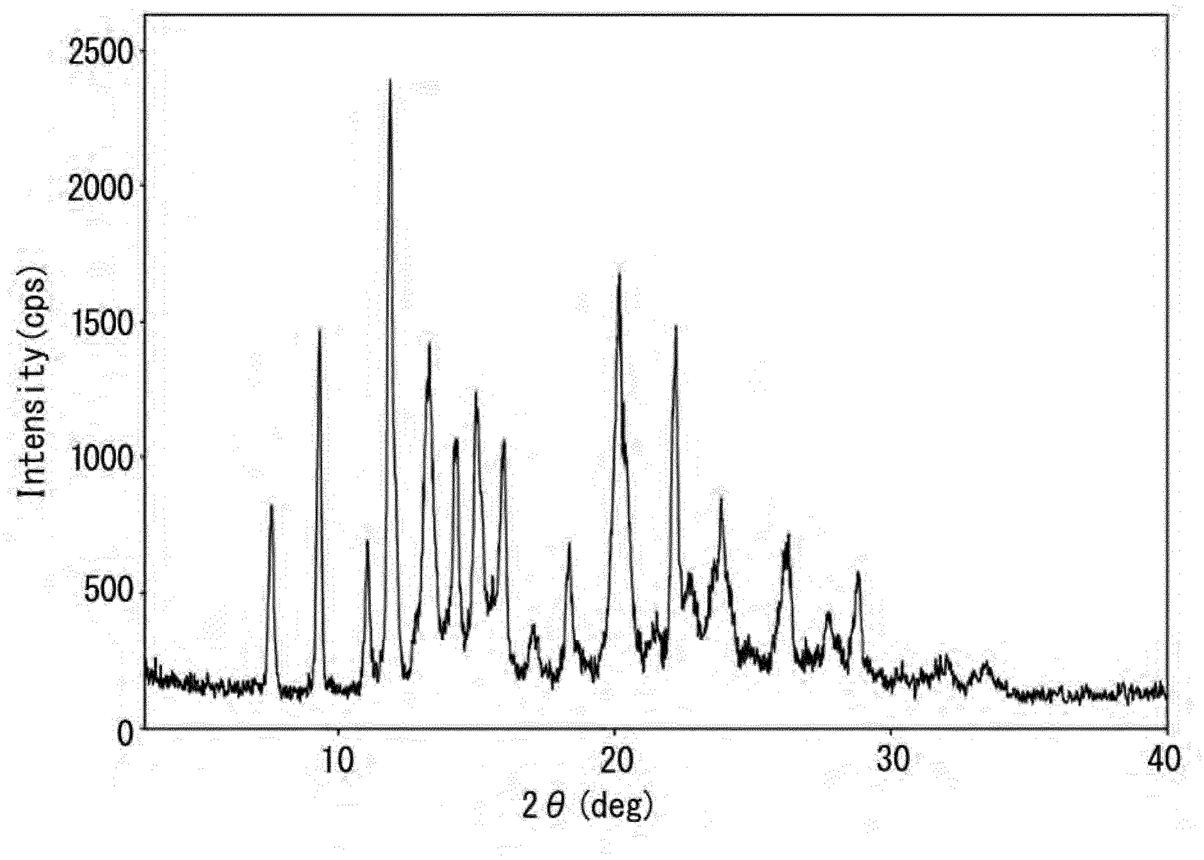

FIG. 90

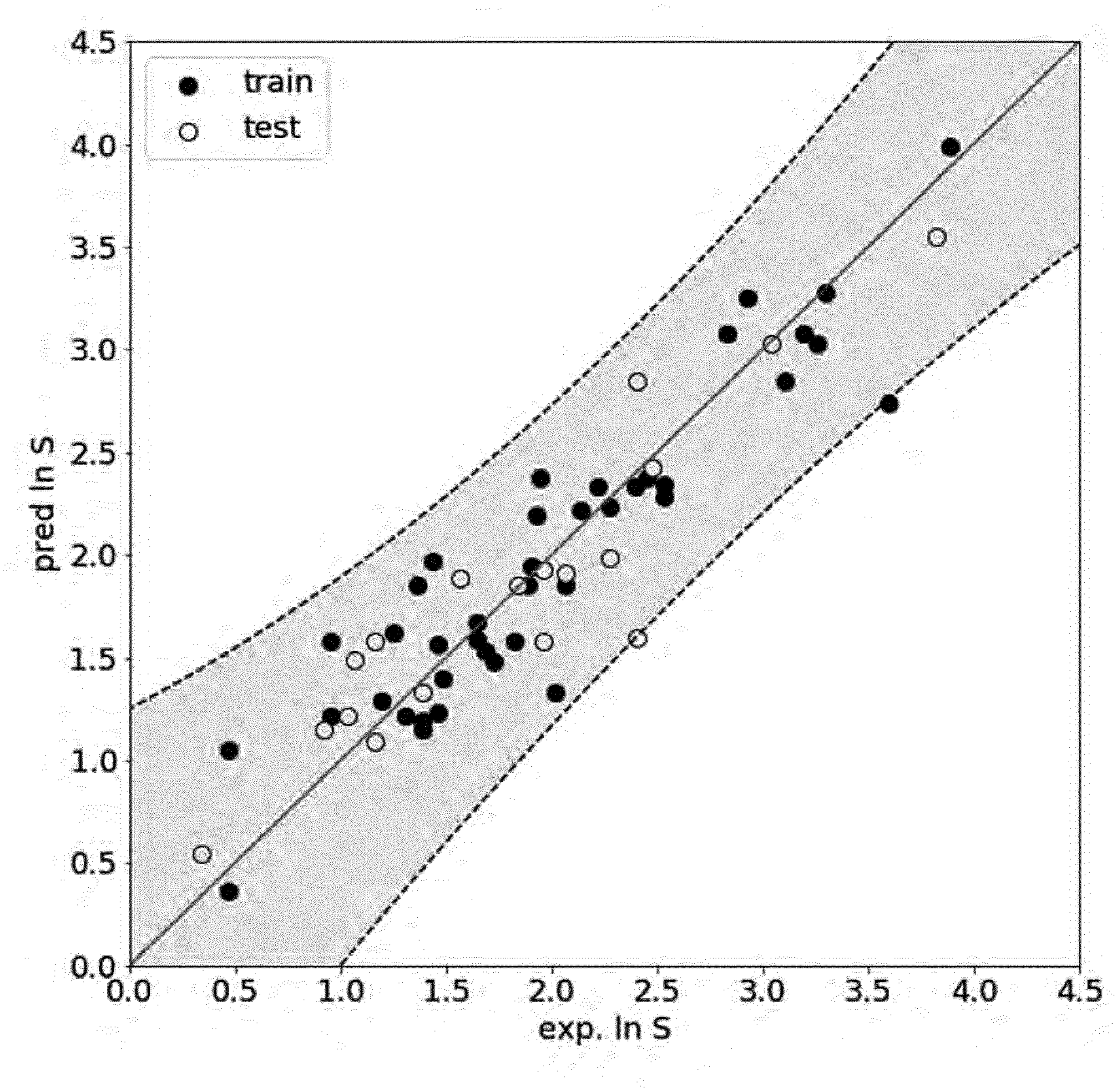

FIG. 91

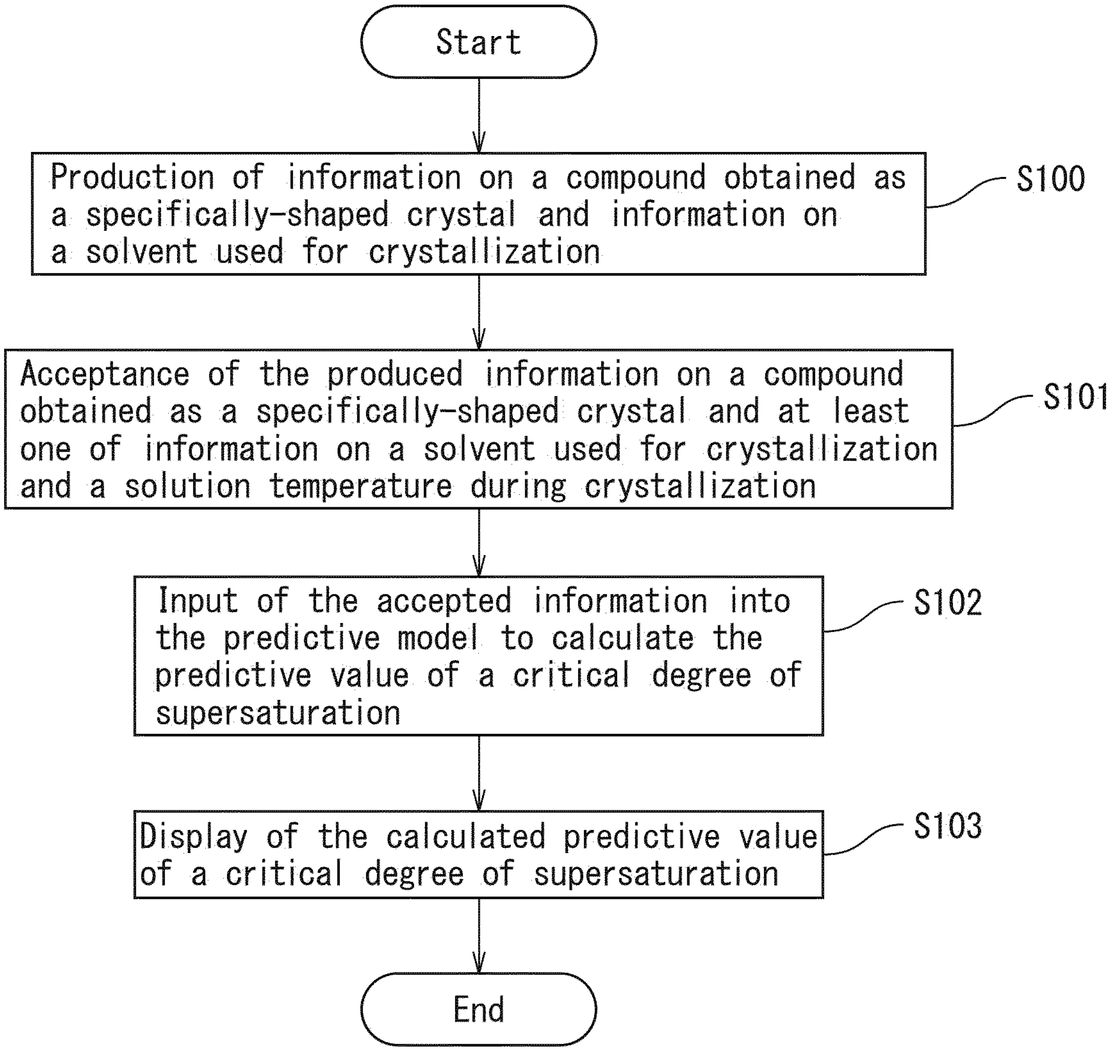
Start
Production of information on a compound obtained as a specifically-shaped crystal and information on a solvent used for crystallization
S100
Acceptance of the produced information on a compound obtained as a specifically-shaped crystal and at least one of information on a solvent used for crystallization and a solution temperature during crystallization
S101
Input of the accepted information into the predictive model to calculate the predictive value of a critical degree of supersaturation
S102
Display of the calculated predictive value of a critical degree of supersaturation
S103
End

**INTERNATIONAL SEARCH REPORT**

International application No. PCT/JP2019/032722

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl. C30B29/54, C07D333/20, C07D401/12, C07D409/04, C07D495/04, C07H1/06, C07H17/00, C07H17/08, C30B7/08, C30B29/60, A61K31/198, A61K31/381, A61K31/4365, A61K31/4439, A61K31/4535, A61K31/7048, A61K31/7052, A61K33/24

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus (JDreamIII), JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | CN 104817571 A (TAINJIN UNIVERSITY) 05 August 2015, paragraphs [0010]-[0077] (Family: none) | 1,2,6-8,11, 12,15 |
| X | CHEN, Mingyang et al., "Spherical Crystallization and the Mechanism of Clopidogrel Hydrogen Sulfate", Chemical Engineering Technology, 20 April 2018, vol. 41, no. 6, pp. 1259-1265 | 11,12,15 |
| X | US 2003/0059837 A1 (LEVISON et al.) 27 March 2003, paragraphs [0014]-[0078] & JP 2003-519698 A | 4,5,16,17,18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 November 2019 (01.11.2019) | 19 November 2019 (19.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/032722

C (Continuation). DOCUMENTS CONSIDERED TO BE RELEVANT

| Category* | Citation of document, with indication, where appropriate, of the relevant passages | Relevant to claim No. |
|---|---|---|
| A | US 2011/0009637 A1 (BRAGA et al.) 13 January 2011, paragraphs [0015]-[0048] (Family: none) | 9,10,13,14 |
| A | 有田稔彦，難水溶性薬剤の無添加ナノ粒子化による飛躍的溶解速度向上と過飽和溶解，日本化学会講演予稿集，vol. 93rd, no. 3, 日本化学会，08 March 2013, pp. 707, non-official translation (ARITA, toshihiko, "Significant improvement in dissolution rate of poorly water-soluble drugs due to additive-free nanoparticles, and supersaturated dissolution", Lecture preprints of the Chemical Society of Japan, The Chemical Society of Japan) | 1-18 |

Form PCT/ISA/210 (continuation of second sheet) (January 2015)

**INTERNATIONAL SEARCH REPORT**

International application No.
PCT/JP2019/032722

CLASSIFICATION OF SUBJECT MATTER
C30B29/54(2006.01)i, C07D333/20(2006.01)i, C07D401/12(2006.01)i, C07D409/04(2006.01)i, C07D495/04(2006.01)i, C07H1/06(2006.01)i, C07H17/00(2006.01)i, C07H17/08(2006.01)i, C30B7/08(2006.01)i, C30B29/60(2006.01)i, A61K31/198(2006.01)n, A61K31/381(2006.01)n, A61K31/4365(2006.01)n, A61K31/4439(2006.01)n, A61K31/4535(2006.01)n, A61K31/7048(2006.01)n, A61K31/7052(2006.01)n, A61K33/24(2019.01)n

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S58143832 A
- JP H1279869 A


### Non-patent literature cited in the description


- **ICHIRO SUNAGAWA.** Crystals: Growth, Morphology and Perfection. KYORITSU SHUPPAN CO., LTD, 2003, 47-48
- **F. ESPITALIER ; B. BISCANS ; J.-R. AUTHELIN ; C. LAGUERIE.** *Institution of Chemical Engineers,* 1997, vol. 75 (2), 257-267
- **L. GRANASY et al.** *Nature Materials,* 2004, vol. 3, 645-650
- Molecular Descriptors for Chemoinformatics. **ROBERTO TODESCHINI.** Viviana Consonni. Wiley-VCH Verlag GmbH & Co. KGaA, 2009